(19) Europäisches Patentamt
European Patent Office
Office européen des brevets

(11) EP 4 105 207 A1

(12) # EUROPEAN PATENT APPLICATION
published in accordance with Art. 153(4) EPC

(43) Date of publication:
**21.12.2022 Bulletin 2022/51**

(21) Application number: **21754125.9**

(22) Date of filing: **08.02.2021**

(51) International Patent Classification (IPC):
***C07D 401/12*** (2006.01) ***C07D 471/10*** (2006.01)
***C07D 487/00*** (2006.01) ***C07D 241/02*** (2006.01)
***A61K 31/66*** (2006.01) ***A61K 31/675*** (2006.01)
***A61P 35/00*** (2006.01)

(52) Cooperative Patent Classification (CPC):
**A61K 31/66; A61K 31/675; A61P 35/00; C07D 241/02; C07D 401/12; C07D 471/10; C07D 487/00**

(86) International application number:
**PCT/CN2021/075994**

(87) International publication number:
**WO 2021/160087 (19.08.2021 Gazette 2021/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.02.2020 CN 202010094824**
**02.02.2021 CN 202110142695**

(71) Applicant: **Betta Pharmaceuticals Co., Ltd**
**Yuhang**
**Hangzhou**
**Zhejiang 311100 (CN)**

(72) Inventors:
- **LIU, Xiangyong**
  **Beijing 100176 (CN)**
- **QIU, Changyong**
  **Beijing 100176 (CN)**
- **LIU, Mengqiang**
  **Beijing 100176 (CN)**
- **SONG, Xiaodong**
  **Beijing 100176 (CN)**
- **SHEN, Qichao**
  **Beijing 100176 (CN)**
- **DU, Guolong**
  **Beijing 100176 (CN)**
- **SHENG, Haitong**
  **Beijing 100176 (CN)**
- **DING, Lieming**
  **Hangzhou, Zhejiang 311100 (CN)**
- **WANG, Jiabing**
  **Beijing 100176 (CN)**

(74) Representative: **Graf von Stosch Patentanwaltsgesellschaft mbH**
**Prinzregentenstraße 22**
**80538 München (DE)**

(54) **QUINOLYL PHOSPHINE OXIDE COMPOUND, AND COMPOSITION AND APPLICATION THEREOF**

(57) The present invention relates to a compound of formula (I), a method for using the compound as an EGFR inhibitor, and a pharmaceutical composition comprising the compound. The compound can be used for treatment, prevention or alleviation of diseases or disorders such as cancer or infection.

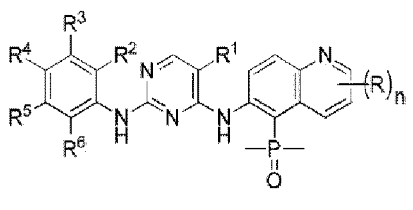

(I)

EP 4 105 207 A1

## Description

### FIELD OF THE INVENTION

The present invention relates to a pharmaceutically active compound, a deuterated compound (a hydrogen substituted by a deuterium) and a pharmaceutically acceptable salt thereof, which can be used in the treatment or prevention of diseases or medical conditions mediated by epidermal growth factor receptors in certain mutant forms (for example, L858R activating mutant, Exon19 deletion activating mutant, T790M resistance mutant and C797S resistance mutant). The present invention also relates to a pharmaceutical composition containing said compound, and a method of using said compound, deuterated compound and a salt thereof for treating diseases mediated by various forms of EGFR mutants.

### BACKGROUND OF THE INVENTION

Epidermal growth factor receptor (EGFR) is a transmembrane glycoprotein belonging to the ErbB family of tyrosine kinase receptors. The activation of EGFR leads to autophosphorylation of receptor tyrosine kinases, which initiates a cascade reaction of downstream signaling pathways involved in regulating cell proliferation, differentiation and survival. EGFR is abnormally activated by various mechanisms, such as receptor overexpression, mutation, ligand-dependent receptor dimerization, and ligand-independent receptor activation, and is related to the development of a variety of human cancers.

Inhibition of EGFR is one of the key goals of cancer treatment. Although previous generations of EGFR-TKIs developed rapidly, the problem of drug resistance has also emerged with the development of drugs. After the first and second generations of EGFR-TKI being used, most of the drug resistance was caused by the T790M mutation generated in the ATP binding domain. Recently developed third-generation irreversible inhibitors against T790M, such as osimertinib, have very good inhibitory activities, but drug resistance emerged inevitably. The EGFR-C797S mutation is the most common secondary mutation that causes resistance to third-generation EGFR-TKI. C797S mutation refers to a missense mutation in which the cysteine at position 797 of EGFR exon 20 is replaced by serine, which is located in the tyrosine kinase region of EGFR. C797S mutation prevents osimertinib from continuing forming covalent bonds in the ATP-binding domain, thereby losing the effect of inhibiting EGFR activation, resulting in drug resistance.

Early patent applications WO2018108064, WO2018115218, WO2018181777 disclosed a series of fourth-generation EGFR inhibitors, but there is still a need for EGFR C797S inhibitors with a better selectivity. In the present invention, the applicant has discovered a small molecule that can be used as fourth-generation EGFR inhibitor, and its activity can be used to treat cancer and/or infectious diseases. It is contemplated that these small molecules can have better activity, higher safety, and the like, which are essential to effective drugs for promoting human health.

### SUMMARY OF THE INVENTION

The present invention relates to a quinolinyl phosphine oxide compound capable of inhibiting EGFR, and these compounds can be used in the treatment of cancer and infectious diseases.

The first object of the present invention is to provide a compound of formula I, or a stereoisomer, a tautomer, a deuterated compound, a pharmaceutically acceptable salt, a prodrug, a chelate, a non-covalent complex or a solvate thereof:

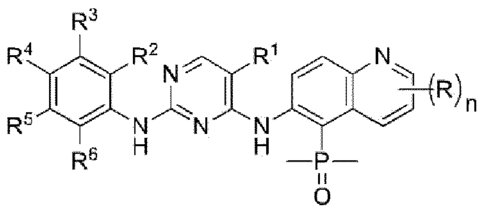

Formula I

wherein,

R is each independently selected from the group consisting of H, halogen, CN, $NO_2$, OH, -NR'R'', $-C_{1-6}$ alkyl, $-C_{1-6}$ alkoxy, $-(CH_2)_p(O(CH_2)_q)_rCH_3$, $-C_{3-6}$ cycloalkyl, 3-6 membered heterocyclyl, aryl, heteroaryl, $-CR^7=CR^8$, and

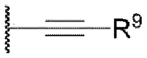 ; wherein the -$C_{1-6}$ alkyl, -$C_{1-6}$ alkoxy, 3-6 membered heterocyclyl, aryl and heteroaryl are optionally substituted by halogen, OH, $NH_2$, aryl, heteroaryl, -$C_{1-6}$ alkyl, or -$C_{3-6}$ cycloalkyl;

R', R", $R^7$, $R^8$ and $R^9$ are each independently selected from the group consisting of H, CN, -$C_{1-6}$ alkyl, -$C_{3-6}$ cycloalkyl and 3-6 membered heterocyclyl; wherein the -$C_{1-6}$ alkyl, -$C_{3-6}$ cycloalkyl and 3-6 membered heterocyclyl are optionally substituted by halogen, OH, $NH_2$, aryl, heteroaryl, -$C_{1-6}$ alkyl, -$C_{3-6}$ cycloalkyl, or 3-6 membered heterocyclyl;

$R^1$ is selected from the group consisting of H, halogen, CN, -$NR^{10}R^{11}$, OH, -$C_{1-6}$ alkyl, - $C_{1-6}$ alkoxy, -$C_{3-6}$ cycloalkyl and 3-6 membered heterocyclyl; wherein the -$C_{1-6}$ alkyl, -$C_{1-6}$ alkoxy, -$C_{3-6}$ cycloalkyl and 3-6 membered heterocyclyl are optionally substituted by halogen, OH, $NH_2$, or -$C_{1-6}$ alkyl;

$R^{10}$ and $R^{11}$ are each independently selected from the group consisting of H, -$C_{1-6}$ alkyl and -$C_{3-6}$ cycloalkyl;

$R^2$, $R^3$ and $R^6$ are each independently selected from the group consisting of H, halogen, CN, -$NR^{12}R^{13}$, OH, -$C_{1-6}$ alkyl, -$C_{1-6}$ alkoxy, -$C_{3-6}$ cycloalkyl and 3-6 membered heterocyclyl; wherein the -$C_{1-6}$ alkyl, -$C_{1-6}$ alkoxy, -$C_{3-6}$ cycloalkyl and 3-6 membered heterocyclyl are optionally substituted by halogen, OH, $NH_2$, or -$C_{1-6}$ alkyl; and $R^2$, $R^3$ and $R^6$ are not H at the same time;

$R^{12}$ and $R^{13}$ are each independently selected from the group consisting of H, -$C_{1-6}$ alkyl and -$C_{3-6}$ cycloalkyl;

$R^4$ and $R^5$ are each independently selected from the group consisting of H, halogen, CN, $NO_2$, OH, -$NR^{14}R^{15}$, -$C_{1-6}$ alkyl, aryl, heteroaryl, -$C_{1-6}$ alkoxy, -$C_{3-6}$ cycloalkyl and 3-8 membered heterocyclyl; wherein, the -$C_{1-6}$ alkyl, aryl, heteroaryl, -$C_{1-6}$ alkoxy, -$C_{3-6}$ cycloalkyl and 3-8 membered heterocyclyl are optionally substituted by halogen, OH, $NH_2$, - $C_{1-6}$ alkyl, halogen-substituted $C_{1-6}$ alkyl, hydroxy-substituted $C_{1-6}$ alkyl, -$C_{1-6}$ alkoxy, - $NR^{14}R^{15}$, -$C_{3-6}$ cycloalkyl, substituted $C_{3-6}$ cycloalkyl, 3-6 membered heterocyclyl, substituted 3-6 membered heterocyclyl, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and -$NR^{14}R^{15}$; and $R^4$ and $R^5$ are not H at the same time;

$R^{14}$ and $R^{15}$ are each independently selected from the group consisting of H, -$C_{1-6}$ alkyl, - $C_{3-6}$ cycloalkyl, -$C_{3-6}$ heterocyclyl; wherein, the -$C_{1-6}$ alkyl, -$C_{3-6}$ cycloalkyl and -$C_{3-6}$ heterocyclyl are optionally substituted by -$C_{1-6}$ alkyl, -$C_{1-6}$ alkoxy, -$NR^{16}R^{17}$ and -$C_{3-6}$ heterocyclyl, or heterocyclyl substituted by one or more $C_{1-6}$ alkyl; $R^{16}$ and $R^{17}$ are each independently H or -$C_{1-6}$ alkyl;

n is an integer selected from 1 to 3;

p is an integer selected from 1 to 3;

q is an integer selected from 0 to 3;

r is an integer selected from 1 to 3.

In certain embodiments, n is 1.

In certain embodiments, n is 2.

In certain embodiments, n is 3.

In certain embodiments, R is independently selected from the group consisting of halogen, CN, $NO_2$, OH, -$C_{1-6}$ alkyl, -$C_{3-6}$ cycloalkyl, 3-6 membered heterocyclyl, heteroaryl, and 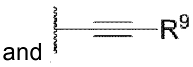 ; wherein $R^9$ is defined as described in any of the embodiments of the present invention.

In certain embodiments, R is each independently selected from the group consisting of halogen, CN, $NO_2$, OH, -$CF_3$, -$CH_3$, -$CH_2CH_3$, -$CH_2CH_2CH_3$, -$CH(CH_3)_2$, -$CH_2CH(CH_3)_2$,

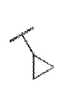, 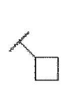, 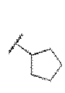, 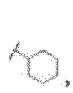, 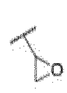, 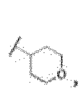, 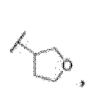, 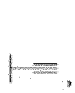, 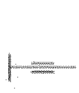, 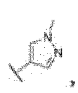, 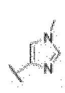, 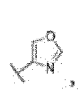, and 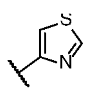.

In certain embodiments, $R^1$ is selected from the group consisting of halogen, CN, - $NR^{10}R^{11}$ and OH; wherein the $R^{10}$, $R^{11}$ are defined as described in any of the embodiments of the present invention.

In certain embodiments, $R^1$ is selected from halogen; wherein the halogen is fluorine, chlorine, bromine or iodine.

In certain embodiments, $R^1$ is fluoro or bromo.

In certain embodiments, $R^2$ and $R^3$ are each independently selected from the group consisting of H, halogen, CN, -$NR^{12}R^{13}$, OH, -$C_{1-6}$ alkyl, -$C_{1-6}$ alkoxy, -$C_{3-6}$ cycloalkyl and 3-6 membered heterocyclyl, respectively; wherein the -$C_{1-6}$ alkyl, -$C_{1-6}$ alkoxy, -$C_{3-6}$ cycloalkyl and 3-6 membered heterocyclyl are optionally substituted by halogen, OH, $NH_2$, -$C_{1-6}$ alkyl; and $R^2$ and $R^3$ are not H at the same time; $R^{12}$ and $R^{13}$ are defined as described in any of the embodiments of the present invention.

In certain embodiments, $R^2$ is selected from the group consisting of H, -$C_{1-6}$ alkyl and - $C_{1-6}$ alkoxy.

In certain embodiments, $R^2$ is selected from -$C_{1-6}$ alkoxy.

In certain embodiments, $R^2$ is selected from the group consisting of H, -$CH_3$ or -$OCH_3$.

In certain embodiments, $R^3$ is selected from the group consisting of H or -$C_{1-6}$ alkoxy.

In certain embodiments, $R^3$ is selected from H or $-OCH_3$.

In certain embodiments, $R^4$ and $R^5$ are each independently selected from the group consisting of H, halogen, CN, $NO_2$, OH, $-NR^{14}R^{15}$, $-C_{1-6}$ alkyl, aryl, heteroaryl, $-C_{1-6}$ alkoxy, - $C_{3-6}$ cycloalkyl and 3-6 membered heterocyclyl; wherein the $-C_{1-6}$ alkyl, aryl, heteroaryl, $-C_{1-6}$ alkoxy, $-C_{3-6}$ cycloalkyl and 3-6 membered heterocyclyl are optionally substituted by halogen, OH, $NH_2$, $-C_{1-6}$ alkoxy, $-C_{3-6}$ cycloalkyl, substituted $C_{3-6}$ cycloalkyl, 3-6 membered heterocyclyl, substituted 3-6 membered heterocyclyl, aryl, or heteroaryl; and $R^4$ and $R^5$ are not H at the same time.

In certain embodiments, $R^4$ is selected from the group consisting of H, halogen, CN, $NO_2$, OH, $-NR^{14}R^{15}$, aryl, heteroaryl, $-C_{3-6}$ cycloalkyl and 3-6 membered heterocyclyl; wherein the aryl, heteroaryl, $-C_{3-6}$ cycloalkyl and 3-6 membered heterocyclyl are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$; $R^{14}$ and $R^{15}$ are defined as described in any of the embodiments of the present invention.

In certain embodiments, $R^4$ is selected from the group consisting of $-NR^{14}R^{15}$, aryl, heteroaryl, $-C_{3-6}$ cycloalkyl and 3-6 membered heterocyclyl; wherein the aryl, heteroaryl, $-C_{3-6}$ cycloalkyl and 3-6 membered heterocyclyl are optionally substituted by halogen, OH, $NH_2$, - $C_{1-6}$ alkyl, halogen-substituted $C_{1-6}$ alkyl, hydroxy-substituted $C_{1-6}$ alkyl, $-C_{1-6}$ alkoxy, - $NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and - $NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$; $R^{14}$ and $R^{15}$ are defined as described in any of the embodiments of the present invention.

In certain embodiments, $R^{14}$ and $R^{15}$ are each independently selected from the group consisting of H, $-C_{1-6}$ alkyl and $-C_{3-6}$ cycloalkyl.

In certain embodiments, $R^4$ is selected from the group consisting of

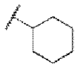, 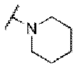, 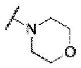, 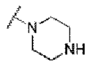, 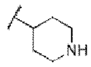,

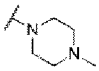, 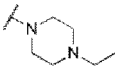, 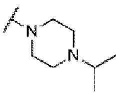, 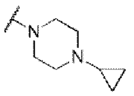, 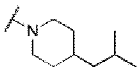, 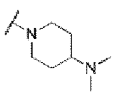, 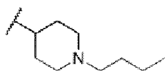,

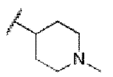, 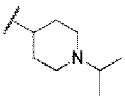, 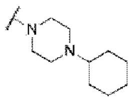, 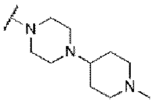, 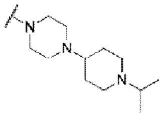, 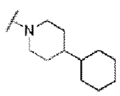,

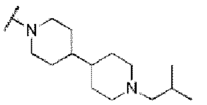, 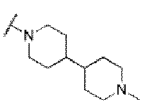, 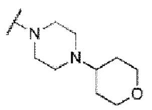, 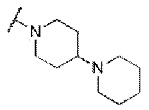, 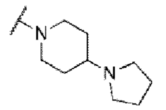,

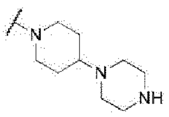, 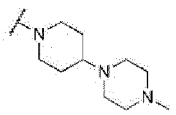, 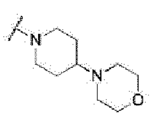, 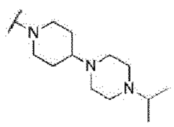, 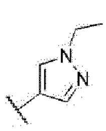, 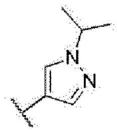,

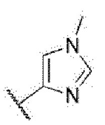, 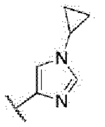, 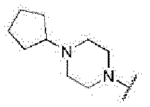, 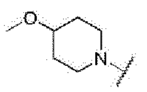, 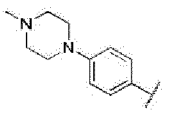, 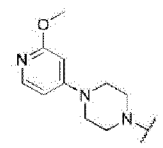,

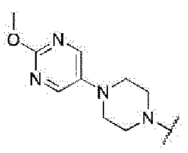, 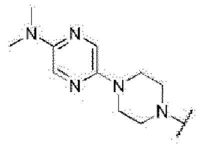, 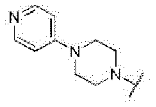, 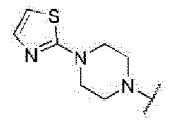, 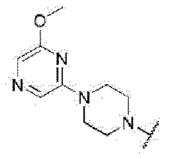,

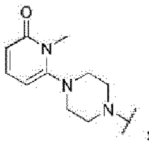, 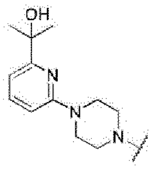, 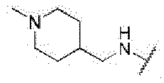, 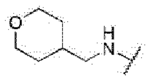, 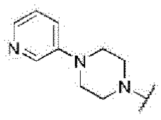, 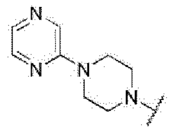,

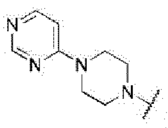, 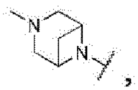, 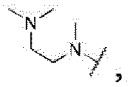, 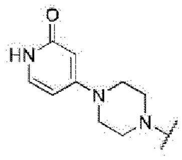.

In certain embodiments, $R^5$ is selected from the group consisting of H, halogen, CN, $NO_2$, OH, $-C_{1-6}$ alkyl, aryl, heteroaryl, and $-C_{1-6}$ alkoxy; wherein the $-C_{1-6}$ alkyl, aryl, heteroaryl, $-C_{1-6}$ alkoxy are each optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.

In certain embodiments, $R^5$ is selected from the group consisting of H, halogen, CN, $-C_{1-6}$ alkyl, heteroaryl, and $-C_{1-6}$ alkoxy; wherein the $-C_{1-6}$ alkyl, heteroaryl, $-C_{1-6}$ alkoxy are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.

In certain embodiments, $R^5$ is selected from the group consisting of H, CN, $CH_3$, $CH_2CH_3$, F, $OCH_3$,

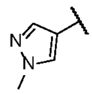, 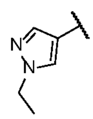, 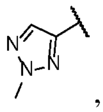, 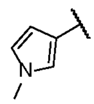 or 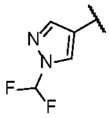.

In certain embodiments, $R^6$ is selected from the group consisting of H, CN, $-C_{1-6}$ alkyl, $-C_{3-6}$ cycloalkyl and 3-6 membered heterocyclyl; wherein the $-C_{1-6}$ alkyl, $-C_{3-6}$ cycloalkyl and 3-6 membered heterocyclyl are optionally substituted by halogen, OH, $NH_2$, $-C_{1-6}$ alkyl.

In certain embodiments, $R^6$ is selected from the group consisting of H, CN, $CH_3$, $CH_2CH_3$, $CH(CH_3)_2$,

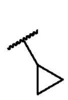, 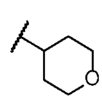.

In certain embodiments, the compound of formula I, or a stereoisomer, a tautomer, a deuterated compound, a pharmaceutically acceptable salt, a prodrug, a chelate, a non-covalent complex or a solvate thereof is represented by formula II:

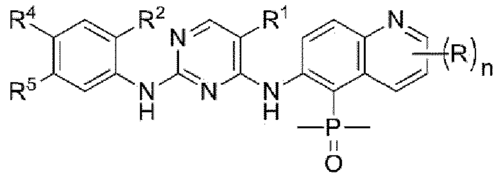

Formula II

wherein,

R is each independently selected from the group consisting of H, halogen, CN, $NO_2$, OH, -NR'R'', $-C_{1-6}$ alkyl, $-C_{1-6}$ alkoxy, $-(CH_2)_p(O(CH_2)_q)_rCH_3$, $-C_{3-6}$ cycloalkyl, 3-6 membered heterocyclyl, aryl, heteroaryl, $-CR^7=CR^8$ and

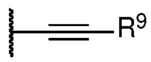

;

wherein the $-C_{1-6}$ alkyl, $-C_{1-6}$ alkoxy, 3-6 membered heterocyclyl, aryl and heteroaryl are optionally substituted by halogen, OH, $NH_2$, aryl, heteroaryl, $-C_{1-6}$ alkyl, $-C_{3-6}$ cycloalkyl;

R', R'', $R^7$, $R^8$ and $R^9$ are each independently selected from the group consisting of H, CN, $-C_{1-6}$ alkyl, $-C_{3-6}$ cycloalkyl and 3-6 membered heterocyclyl; wherein the $-C_{1-6}$ alkyl, $-C_{3-6}$ cycloalkyl and 3-6 membered heterocyclyl are optionally substituted by halogen, OH, $NH_2$, aryl, heteroaryl, $-C_{1-6}$ alkyl, $-C_{3-6}$ cycloalkyl, 3-6 membered heterocyclyl;

$R^1$ is selected from the group consisting of H, halogen, CN, $-NR^{10}R^{11}$, OH, $-C_{1-6}$ alkyl, - $C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, $-C_{1-6}$ alkyl;

$R^{10}$ and $R^{11}$ are each independently selected from the group consisting of H, $-C_{1-6}$ alkyl and $-C_{3-6}$ cycloalkyl;

$R^2$ is selected from the group consisting of H, halogen, CN, $-NR^{12}R^{13}$, OH, $-C_{1-6}$ alkyl, - $C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, $-C_{1-6}$ alkyl; and $R^2$, $R^3$ and $R^6$ are not H at the same time;

$R^{12}$ and $R^{13}$ are each independently selected from the group consisting of H, $-C_{1-6}$ alkyl and $-C_{3-6}$ cycloalkyl;

$R^4$ and $R^5$ are each independently selected from the group consisting of H, halogen, CN, $NO_2$, OH, $-NR^{14}R^{15}$, $-C_{1-6}$ alkyl, aryl, heteroaryl, $-C_{1-6}$ alkoxy, $-C_{3-6}$ cycloalkyl and 3-8 membered heterocyclyl; wherein the $-C_{1-6}$ alkyl, aryl, heteroaryl, $-C_{1-6}$ alkoxy, $-C_{3-6}$ cycloalkyl and 3-8 membered heterocyclyl are optionally substituted by halogen, OH, $NH_2$, - $C_{1-6}$ alkyl, halogen-substituted $C_{1-6}$ alkyl, hydroxy-substituted $C_{1-6}$ alkyl, $-C_{1-6}$ alkoxy, - $NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and - $NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$; and $R^4$ and $R^5$ are not H at the same time;

$R^{14}$ and $R^{15}$ are each independently selected from the group consisting of H, $-C_{1-6}$ alkyl, - $C_{3-6}$ cycloalkyl, $-C_{3-6}$ heterocyclyl; wherein the $-C_{1-6}$ alkyl, $-C_{3-6}$ cycloalkyl and $-C_{3-6}$ heterocyclyl are optionally substituted by $-C_{1-6}$ alkyl, $-C_{1-6}$ alkoxy, $-NR^{16}R^{17}$ and $-C_{3-6}$ heterocyclyl, or heterocyclyl substituted by one or more $C_{1-6}$ alkyl; $R^{16}$ and $R^{17}$ are each independently selected from H and $-C_{1-6}$ alkyl;

n is an integer selected from 1 to 3;

p is an integer selected from 1 to 3;

q is an integer selected from 0 to 3;

r is an integer selected from 1 to 3.

In certain embodiments, n is 1.

In certain embodiments, R is each independently selected from the group consisting of halogen, CN, $NO_2$, OH, $-C_{1-6}$ alkyl, $-C_{3-6}$ cycloalkyl, 3-6 membered heterocyclyl, heteroaryl, and

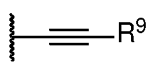

;

wherein $R^9$ is defined as described in any of the embodiments of the present invention.

In certain embodiments, R is each independently selected from the group consisting of halogen, CN, $NO_2$, OH,

$-CF_3$-$CH_3$, $-CH_2CH_3$, $-CH_2CH_2CH_3$, $-CH(CH_3)_2$, $-CH_2CH(CH_3)_2$,

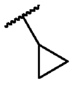, 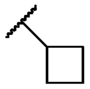, 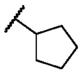, 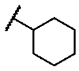, 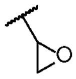, 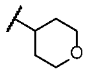, 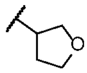, 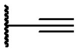, 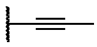, 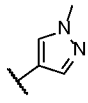, 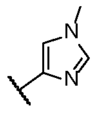, 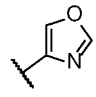,

and

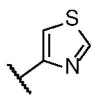.

In certain embodiments, $R^1$ is selected from the group consisting of halogen, CN, - $NR^{10}R^{11}$ and OH; wherein $R^{10}$ and $R^{11}$ are defined as described in any of the embodiments of the present invention.

In certain embodiments, $R^1$ is selected from halogen; wherein the halogen is fluorine, chlorine, bromine or iodine.

In certain embodiments, $R^1$ is fluoro or bromo.

In certain embodiments, $R^2$ is selected from the group consisting of H, halogen, CN, - $NR^{12}R^{13}$, OH, $-C_{1-6}$ alkyl, $-C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, $-C_{1-6}$ alkyl; $R^{12}$ and $R^{13}$ are defined as described in any of the embodiments of the present invention.

In certain embodiments, $R^2$ is selected from the group consisting of H, $-C_{1-6}$ alkyl and - $C_{1-6}$ alkoxy.

In certain embodiments, $R^2$ is selected from $-C_{1-6}$ alkoxy.

In certain embodiments, $R^2$ is selected from H, $-CH_3$ or $-OCH_3$.

In certain embodiments, $R^4$ is selected from the group consisting of H, halogen, CN, $NO_2$, OH, $-NR^{14}R^{15}$, aryl, heteroaryl, $-C_{3-6}$ cycloalkyl and 3-6 membered heterocyclyl; wherein the aryl, heteroaryl, $-C_{3-6}$ cycloalkyl and 3-6 membered heterocyclyl are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$; wherein $R^{14}$ and $R^{15}$ are defined as described in any of the embodiments of the present invention.

In certain embodiments, $R^4$ is selected from the group consisting of $-NR^{14}R^{15}$, aryl, heteroaryl, $-C_{3-6}$ cycloalkyl and 3-6 membered heterocyclyl; wherein the aryl, heteroaryl, $-C_{3-6}$ cycloalkyl and 3-6 membered heterocyclyl are optionally substituted by halogen, OH, $NH_2$, - $C_{1-6}$ alkyl, halogen-substituted $C_{1-6}$ alkyl, hydroxy-substituted $C_{1-6}$ alkyl, $-C_{1-6}$ alkoxy, - $NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and - $NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$; wherein $R^{14}$ and $R^{15}$ are defined as described in any of the embodiments of the present invention.

In certain embodiments, $R^4$ is selected from the group consisting of

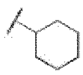, 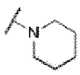, 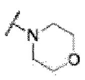, 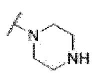, 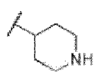,

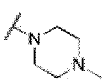, 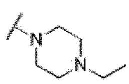, 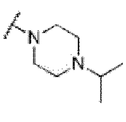, 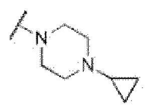, 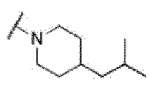, 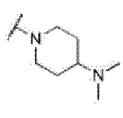, 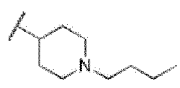,

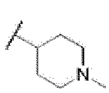, 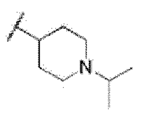, 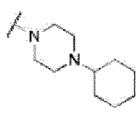, 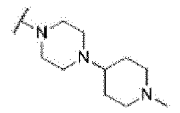, 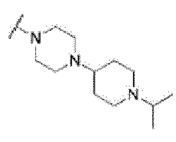, 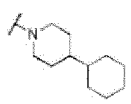,

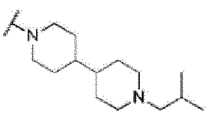, 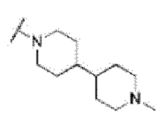, 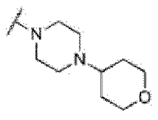, 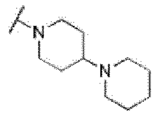, 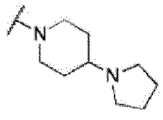,

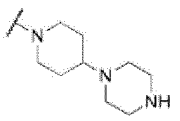, 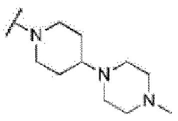, 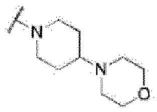, 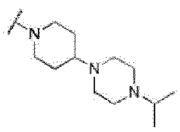, 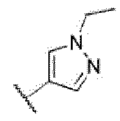, 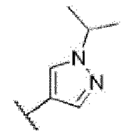,

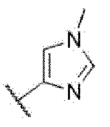, 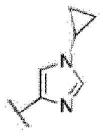, 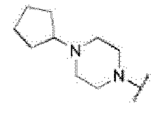, 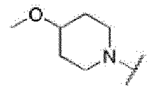, 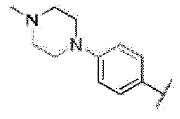, 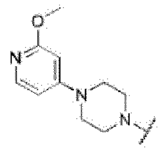,

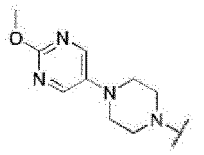, 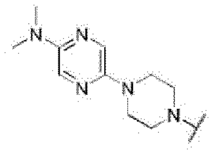, 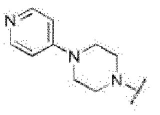, 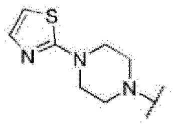, 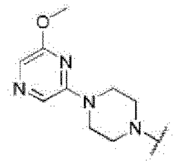,

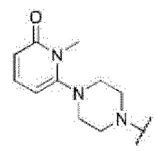, 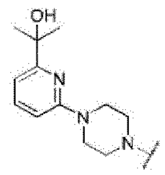, 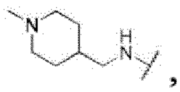, 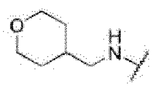, 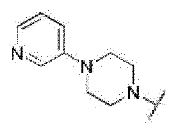, 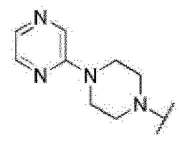,

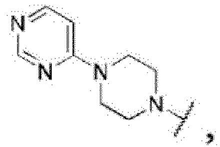, 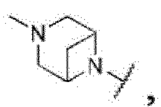, 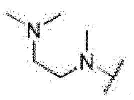, 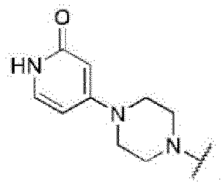.

In certain embodiments, $R^5$ is selected from the group consisting of H, halogen, CN, $NO_2$, OH, -$C_{1-6}$ alkyl, aryl, heteroaryl, -$C_{1-6}$ alkoxy; wherein the -$C_{1-6}$ alkyl, aryl, heteroaryl, - $C_{1-6}$ alkoxy are optionally substituted by halogen, OH, $NH_2$, -$C_{1-6}$ alkyl, halogen-substituted $C_{1-6}$ alkyl, hydroxy-substituted $C_{1-6}$ alkyl, -$C_{1-6}$ alkoxy, -$NR^{14}R^{15}$, -$C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and -$NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and -$NR^{14}R^{15}$.

In certain embodiments, $R^5$ is selected from the group consisting of H, halogen, CN, -$C_{1-6}$ alkyl, heteroaryl, -$C_{1-6}$ alkoxy; wherein the -$C_{1-6}$ alkyl, heteroaryl, -$C_{1-6}$ alkoxy are optionally substituted by halogen, OH, $NH_2$, -$C_{1-6}$ alkyl, halogen-substituted $C_{1-6}$ alkyl, hydroxy-substituted $C_{1-6}$ alkyl, -$C_{1-6}$ alkoxy, -$NR^{14}R^{15}$, -$C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and -$NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and -$NR^{14}R^{15}$.

In certain embodiments, $R^5$ is selected from the group consisting of H, CN, $CH_3$, $CH_2CH_3$, F, $OCH_3$,

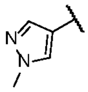, 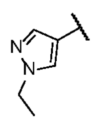, 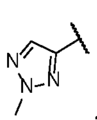, 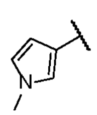 or 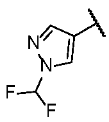.

In certain embodiments, the compound of formula I, or a stereoisomer, a tautomer, a deuterated compound, a pharmaceutically acceptable salt, a prodrug, a chelate, a non-covalent complex or a solvate thereof is represented by formula III:

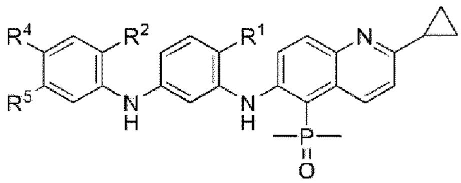

Formula III

wherein,

$R^1$ is selected from the group consisting of H, halogen, CN, $-NR^{10}R^{11}$, OH, $-C_{1-6}$ alkyl, - $C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, or $-C_{1-6}$ alkyl;

$R^{10}$ and $R^{11}$ are each independently selected from the group consisting of H, $-C_{1-6}$ alkyl and $-C_{3-6}$ cycloalkyl;

$R^2$ is selected from the group consisting of H, halogen, CN, $-NR^{12}R^{13}$, OH, $-C_{1-6}$ alkyl, - $C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, $-C_{1-6}$ alkyl;

$R^{12}$ and $R^{13}$ are each independently selected from the group consisting of H, $-C_{1-6}$ alkyl and $-C_{3-6}$ cycloalkyl;

$R^4$ and $R^5$ are each independently selected from the group consisting of H, halogen, CN, $NO_2$, OH, $-NR^{14}R^{15}$, $-C_{1-6}$ alkyl, aryl, heteroaryl, $-C_{1-6}$ alkoxy, $-C_{3-6}$ cycloalkyl and 3-8 membered heterocyclyl; wherein the $-C_{1-6}$ alkyl, aryl, heteroaryl, $-C_{1-6}$ alkoxy, $-C_{3-6}$ cycloalkyl and 3-8 membered heterocyclyl are optionally substituted by halogen, OH, $NH_2$, - $C_{1-6}$ alkyl, halogen-substituted $C_{1-6}$ alkyl, hydroxy-substituted $C_{1-6}$ alkyl, $-C_{1-6}$ alkoxy, - $NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and - $NR^{14}R^{15}5$, aryl, substituted aryl, heteroaryl, or heteroaryl substituted with one or more of $C_{1-6}$ alkyl, hydroxy-substituted $C_{1-6}$ alkyl, oxo, $C_{1-6}$ alkoxy, and $-NR^{14}R^{15}$; and $R^4$ and $R^5$ are not H at the same time;

$R^{14}$ and $R^{15}$ are each independently selected from the group consisting of H, $-C_{1-6}$ alkyl, - $C_{3-6}$ cycloalkyl, $-C_{3-6}$ heterocyclyl; the $-C_{1-6}$ alkyl, $-C_{3-6}$ cycloalkyl and $-C_{3-6}$ heterocyclyl are optionally substituted by $-C_{1-6}$ alkyl, $-C_{1-6}$ alkoxy, $-NR^{16}R^{17}$, $-C_{3-6}$ heterocyclyl, or heterocyclyl substituted by one or more $C_{1-6}$ alkyl; wherein, $R^{16}$ and $R^{17}$ are each independently selected from H and $-C_{1-6}$ alkyl.

In certain embodiments, $R^1$ is selected from halogen; wherein the halogen is fluorine, chlorine, bromine or iodine.
In certain embodiments, $R^1$ is fluoro or bromo.
In certain embodiments, $R^2$ is selected from the group consisting of $-C_{1-6}$ alkyl and $-C_{1-6}$ alkoxy.
In certain embodiments, $R^2$ is selected from $-C_{1-6}$ alkoxy.
In certain embodiments, $R^2$ is selected from $-CH_3$ or $-OCH_3$.
In certain embodiments, $R^4$ is selected from the group consisting of $-NR^{14}R^{15}$, aryl, heteroaryl, $-C_{3-6}$ cycloalkyl and 3-8 membered heterocyclyl; wherein the aryl, heteroaryl, $-C_{3-6}$ cycloalkyl and 3-8 membered heterocyclyl are optionally substituted by halogen, OH, $NH_2$, - $C_{1-6}$ alkyl, halogen-substituted $C_{1-6}$ alkyl, hydroxy-substituted $C_{1-6}$ alkyl, $-C_{1-6}$ alkoxy, - $NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and - $NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.
In certain embodiments, $R^4$ is a 3-8 membered heterocyclyl; the 3-8 membered heterocyclyl is optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.
In certain embodiments, $R^{14}$ and $R^{15}$ are each independently selected from the group consisting of H and $-C_{1-6}$ alkyl; wherein the $-C_{1-6}$ alkyl is optionally substituted by $-C_{1-6}$ alkoxy, $-NR^{16}R^{17}$, $-C_{3-6}$ heterocyclyl, or heterocyclyl substituted with one or more $C_{1-6}$ alkyl; wherein, $R^{16}$ and $R^{17}$ are each independently selected from H and $-C_{1-6}$ alkyl.
In certain embodiments, $R^4$ is selected from the group consisting of

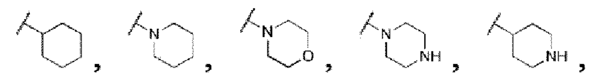
, , , , ,

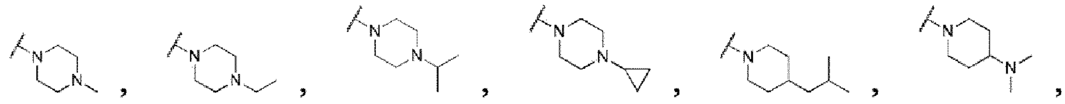
, , , , , ,

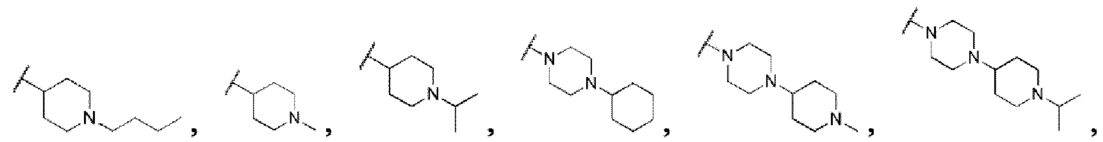
, , , , , ,

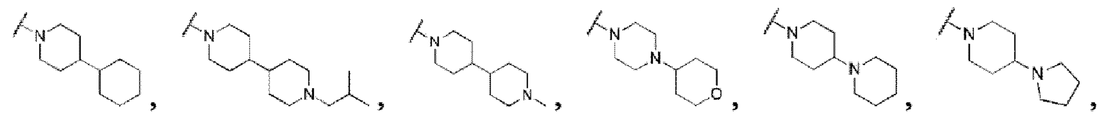
, , , , , ,

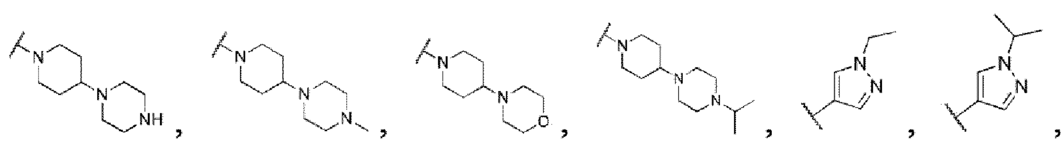
, , , , , ,

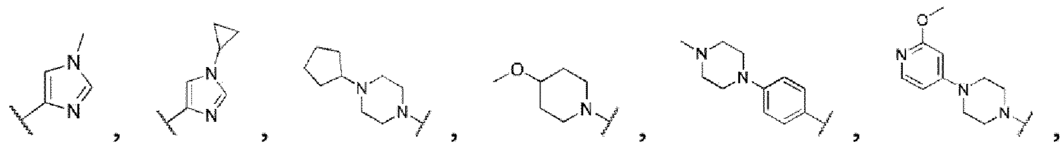
, , , , , ,

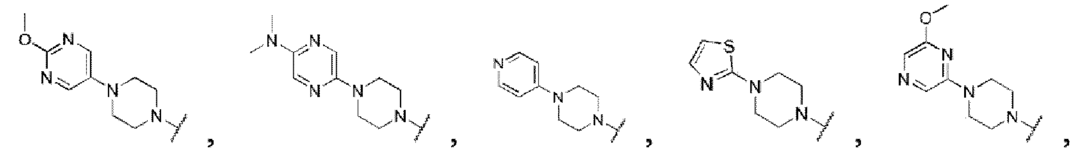
, , , , ,

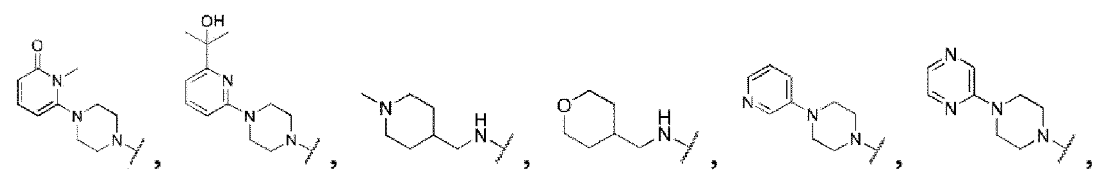
, , , , , ,

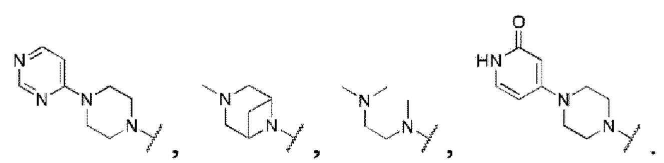
, , , .

In certain embodiments, $R^5$ is selected from the group consisting of H, halogen, CN, $NO_2$, OH, $-C_{1-6}$ alkyl, aryl, heteroaryl, $-C_{1-6}$ alkoxy; wherein the $-C_{1-6}$ alkyl, aryl, heteroaryl, and $-C_{1-6}$ alkoxy are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.

In certain embodiments, $R^5$ is selected from the group consisting of H, halogen, CN, $-C_{1-6}$ alkyl, heteroaryl,

and -$C_{1-6}$ alkoxy; wherein the -$C_{1-6}$ alkyl, heteroaryl, and -$C_{1-6}$ alkoxy are optionally substituted by halogen, OH, $NH_2$, -$C_{1-6}$ alkyl, halogen-substituted $C_{1-6}$ alkyl, hydroxy-substituted $C_{1-6}$ alkyl, -$C_{1-6}$ alkoxy, -$NR^{14}R^{15}$, -$C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and -$NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and -$NR^{14}R^{15}$.

In certain embodiments, $R^5$ is selected from the group consisting of H, CN, $CH_3$, $CH_2CH_3$, F, $OCH_3$,

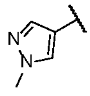 , 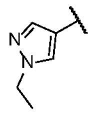 , 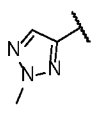 , 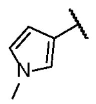 or 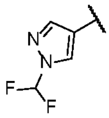 .

In certain embodiments, the compound of formula I is selected from the group consisting of :

(6-((5-bromo-2-((5-methoxy-2-methyl-4-morpholinylphenyl)amino)pyrimidin-4-yl)amino)-2-cyclopropylquinoline-5-yl)dimethylphosphine oxide;
5-((5-bromo-4-((2-cyclopropyl-5-(dimethylphosphine oxide)quinolin-6-yl)amino)pyrimidin-2-yl)amino)-4-methoxy-2-morpholinebenzonitrile;
(6-((5-bromo-2-((2-ethyl-5-methoxy-4-morpholinylphenyl)amino)pyrimidin-4-yl)amino)-2-cyclopropylquinoline-5-yl)dimethylphosphine oxide;
(6-((5-bromo-2-((2-cyclopropyl-5-methoxy-4-morpholinylphenyl)amino)pyrimidin-4-yl)amino)-2-cyclopropylquinoline-5-yl)dimethylphosphine oxide;
(6-((5-bromo-2-((2-isopropyl-5-methoxy-4-morpholinylphenyl)amino)pyrimidin-4-yl)amino)-2-cyclopropylquinoline-5-yl)dimethylphosphine oxide;
(6-((5-bromo-2-((3-methoxy-5-methyl-4-morpholinylphenyl)amino)pyrimidin-4-yl)amino)-2-cyclopropylquinoline-5-yl)dimethylphosphine oxide;
(6-((5-bromo-2-((5-methoxy-2-methyl-4-morpholinylphenyl)amino)pyrimidin-4-yl)amino)-2-(1-methyl-1H-pyrazol-4-yl)quinolin-5-yl)dimethylphosphine oxide;
(6-((5-bromo-2-((5-(1-ethyl-1H-pyrazol-4-yl)-2-methoxy-4-morpholinophenyl)amino)pyrimidine-4-yl)amino)-2-cyclopropylquinolin-5-yl)dimethylphosphine oxide;
(6-((5-bromo-2-((4-(4-(dimethylamino)piperidin-1-yl)-5-methoxy-2-methylphenyl)amino)pyrimidine-4-yl)amino)-2-cyclopropylquinolin-5-yl)dimethylphosphine oxide;
(6-((5-bromo-2-((2-methoxy-5-methyl-4-(4-methylpiperazin-1-yl)phenyl)amino)pyrimidin-4-yl)amino)-2-cyclopropylquinolin-5-yl)dimethylphosphine oxide;
(6-((5-bromo-2-((5-ethyl-2-methoxy-4-(4-methylpiperazin-1-yl)phenyl)amino)pyrimidin-4-yl)amino)-2-cyclopropylquinolin-5-yl)dimethylphosphine oxide;
5-((5-bromo-4-((2-cyclopropyl-5-(dimethylphosphine oxide)quinolin-6-yl)amino)pyrimidin-2-yl)amino)-4-methoxy-2-(4-methylpiperazin-1-yl)benzonitrile;
(6-((5-bromo-2-((2-methoxy-5-methyl-4-(4-(4-methylpiperazin-1-yl)piperidin-1-yl)phenyl)amino)pyrimidin-4-yl)amino)-2-ethynylquinolin-5-yl)dimethylphosphine oxide;
(6-((5-bromo-2-((5-ethyl-2-methoxy-4-(4-(4-methylpiperazin-1-yl)piperidin-1-yl)phenyl)amino)pyrimidin-4-yl)amino)-2-ethynylquinolin-5-yl)dimethylphosphine oxide;
(6-((5-bromo-2-((5-methoxy-2-methyl-4-(4-(4-methylpiperazin-1-yl)piperidin-1-yl)phenyl)amino)pyrimidin-4-yl)amino)-2-ethynylquinolin-5-yl)dimethylphosphine oxide;
6-((5-bromo-2-((5-ethyl-2-methoxy-4-(4-(4-methylpiperazin-1-yl)piperidin-1-yl)phenyl)amino)pyrimidin-4-yl)amino)-5-(dimethylphosphine oxide)quinoline-2-formonitrile;
(6-((5-bromo-2-((5-ethyl-2-methoxy-4-(4-(4-methylpiperazin-1-yl)piperidin-1-yl)phenyl)amino)pyrimidin-4-yl)amino)-2-(1-methyl-1H-pyrazol-4-yl)quinolin-5-yl)dimethylphosphine oxide;
(6-((5-bromo-2-((5-ethyl-2-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((5-fluoro-2-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((2,5-dimethoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
5-((5-bromo-4-((2-cyclopropyl-5-(dimethylphosphine oxide)quinolin-6-yl)amino)pyrimidin-2-yl)amino)-4-methoxy-2-(4-(4-methylpiperazin-1-yl)piperidin-1-yl)benzonitrile;
(6-((5-bromo-2-((2-methoxy-5-methyl-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((5-methoxy-2-methyl-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((2-cyclopropyl-5-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
2-((5-bromo-4-((2-cyclopropyl-5-(dimethylphosphine oxide)quinolin-6-yl)amino)pyrimidin-2-yl)amino)-4-methoxy-5-(4-(4-methylpiperazin-1-yl)piperidin-1-yl)benzonitrile;
(6-((5-bromo-2-((5-methoxy-4-(4-(4-methylpiperazin-1-yl)piperidin-1-yl)-2-(tetrahydro-2H-pyran-4-yl)phenyl)amino)pyrimidin-4-yl)amino)-2-cyclopropylquinolin-5-yl)dimethylphosphine oxide;
(6-((5-bromo-2-((5-ethyl-2-methyl-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((2-methoxy-5-methyl-4-(4-(4-methylpiperazin-1-yl)piperidin-1-yl)phenyl)amino)pyrimidin-4-yl)amino)-2-ethylquinolin-5-yl)dimethylphosphine oxide;
(6-((5-bromo-2-((3-methoxy-5-methyl-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((5-ethyl-2-methoxy-4-(1'-methyl-[4,4'-bipiperidin]-1-yl)phenyl)amino)pyrimidin-4-yl)amino)-2-cyclopropylquinolin-5-yl)dimethylphosphine oxide;
(6-((5-bromo-2-((2-ethyl-5-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((4-(4-cyclopentylpiperazin-1-yl)-2-methoxy-5-methylphenyl)amino)pyrimidin-4-yl)amino)-2-cyclopropylquinolin-5-yl)dimethylphosphine oxide;
(6-((5-bromo-2-((5-methoxy-4-(4-methoxypiperidin-1-yl)-2-methylphenyl)amino)pyrimidin-4-yl)amino)-2-cyclopropylquinolin-5-yl)dimethylphosphine oxide;
(6-((5-bromo-2-((2-methoxy-5-methyl-4-(4-(4-methylpiperazin-1-yl)piperidin-1-yl)phenyl)amino)pyrimidin-4-yl)amino)-2-isopropylquinolin-5-yl)dimethylphosphine oxide;
(6-((5-bromo-2-((5-fluoro-2-methyl-4-morpholinophenyl)amino)pyrimidin-4-yl)amino)-2-cyclopropylquinoline-5-yl)dimethylphosphine oxide;
(6-((5-bromo-2-((2,5-dimethyl-4-morpholinophenyl)amino)pyrimidin-4-yl)amino)-2-cyclopropylquinoline-5-yl)dimethylphosphine oxide;
(6-((5-bromo-2-((4-(4-cyclopentylpiperazin-1-yl)-2-methoxy-5-methylphenyl)amino)pyrimidin-4-yl)amino)-2-cyclopropylquinolin-5-yl)dimethylphosphine oxide;
(6-((5-bromo-2-((4-(4-cyclopropylpiperazin-1-yl)-2-methoxy-5-methylphenyl)amino)pyrimidin-4-yl)amino)-2-cyclopropylquinolin-5-yl)dimethylphosphine oxide;
(6-((5-bromo-2-((4-(4-cyclopentylpiperazin-1-yl)-5-methoxy-2-methylphenyl)amino)pyrimidin-4-yl)amino)-2-cyclopropylquinolin-5-yl)dimethylphosphine oxide;
(6-((5-bromo-2-((5-methoxy-2-methyl-4'-(4-methylpiperazin-1-yl)-[1,1'-biphenyl]-4-yl)amino)pyrimidin-4-yl)amino)-2-cyclopropylquinolin-5-yl)dimethylphosphine oxide;
(6-((5-bromo-2-((2-methoxy-5-methyl-4-(4-(4-methylpiperazin-1-yl)piperidin-1-yl)phenyl)amino)pyrimidin-4-yl)amino)quinolin-5-yl)dimethylphosphine oxide;
(6-((5-bromo-2-((2-methoxy-5-methyl-4-(4-(4-methylpiperazin-1-yl)piperidin-1-yl)phenyl)amino)pyrimidin-4-yl)amino)-2-propylquinolin-5-yl)dimethylphosphine oxide;
(6-((5-bromo-2-((2-methoxy-4-(4-(2-methoxypyridin-4-yl)piperazin-1-yl)-5-(1-methyl-1H-pyrazol-4-yl)phenyl)amino)pyridin-4-yl)amino)-2-cyclopropylquinolin-5-yl)dimethylphosphine oxide;
(6-((5-bromo-2-((2-methoxy-4-(4-(2-methoxypyrimidin-5-yl)piperazin-1-yl)-5-(1-methyl-1H-pyrazol-4-yl)phenyl)amino)pyrimidin-4-yl)amino)-2-cyclopropylquinolin-5-yl)dimethylphosphine oxide;
(6-((5-bromo-2-((4-(4-(5-(dimethylamino)pyrazin-2-yl)piperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide;
(6-((5-bromo-2-((2-methoxy-5-(2-methyl-2H-1,2,3-triazol-4-yl)-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((2-methoxy-5-(2-methyl-2H-1,2,3-triazol-4-yl)-4-(4-(pyridin-4-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(thiazol-2-yl)piperazin-1-yl)phenyl)amino)pyrimidin-4-yl)amino)-2-cyclopropylquinolin-5-yl)dimethylphosphine oxide;
(6-((5-bromo-2-((2-methoxy-4-(4-(6-methoxypyrazin-2-yl)piperazin-1-yl)-5-(1-methyl-1H-pyrazol-4-yl)phenyl)amino)pyrimidin-4-yl)amino)-2-cyclopropylquinolin-5-yl)dimethylphosphine oxide;
6-(4-(4-((5-bromo-4-((2-cyclopropyl-5-(dimethylphosphoryl)quinolin-6-yl)amino)pyrimidin-2-yl)amino)-5-methoxy-2-(1-methyl-1H-pyrazol-4-yl)phenyl)piperazin-1-yl)-1-methylpyridin-2(1H)-one;
(6-((5-bromo-2-((4-(4-(6-(2-hydroxypropan-2-yl)pyridin-2-yl)piperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(((1-methylpiperidine-4-yl)methyl)amino)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(((tetrahydro-2H-pyran-4-yl)methyl)amino)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-methylpiperazin-1-yl)phenyl)amino)pyrimidin-4-yl)amino)-2-cyclopropylquinolin-5-yl)dimethylphosphine oxide;
(6-((5-bromo-2-((4-(4-cyclopentylpiperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(pyridin-3-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(pyridin-4-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(pyrazin-2-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-morpholinopiperidine-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(pyrimidin-4-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(3-methyl-3,6-diazabicyclo[3.1.1]heptyl-6-yl)phenyl)amino)pyrimidin-4-yl)amino)-2-cyclopropylquinolin-5-yl)dimethylphosphine oxide;
(6-((5-bromo-2-((4-((2-(dimethylamino)ethyl)(methyl)amino)-2-methoxy-5-(1-methyl-1H-pyrazol-4-yl)phenyl)amino)pyrimidin-4-yl)amino)-2-cyclopropylquinolin-5-yl)dimethylphosphine oxide;
4-(4-(4-((5-bromo-4-((2-cyclopropyl-5-(dimethylphosphoryl)quinolin-6-yl)amino)pyrimidin-2-yl)amino)-5-methoxy-2-(1-methyl-1H-pyrazol-4-yl)phenyl)piperazin-1-yl)pyridin-2(1H)one;
(6-((5-bromo-2-((2-methoxy-5-(1-methyl-1H-pyrrol-3-yl)-4-(4-(4-methylpiperazine-1-yl)piperidin-1-yl)phenyl)amino)pyrimidin-4-yl)amino)-2-cyclopropylquinolin-5-yl)dimethylphosphine oxide;
(2-Cyclopropyl-6-((5-fluoro-2-((2-methoxy-5-(1-methyl-1H-pyrazol-4-yl)-4-(4-(4-methylpiperazin-1-yl)piperidin-1-yl)phenyl)aminopyrimidin-4-yl)amino)quinolin-5-yl)dimethylphosphine oxide;
(6-((5-bromo-2-((2-methoxy-5-(1-methyl-1H-pyrazol-4-yl)-4-(4-(1-methylpiperidine-4-yl)piperazin-1-yl)phenyl)amino)pyrimidin-4-yl)amino)-2-cyclopropylquinolin-5-yl)dimethylphosphine oxide;
(6-((5-bromo-2-((5-(1-(difluoromethyl)-1H-pyrazol-4-yl)-2-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide; and
(6-((5-bromo-2-((2-methoxy-5-(1-methyl-1H-pyrazol-4-yl)-4-(4-(4-methylpiperazine-1-yl)piperidin-1-yl)phenyl)amino)pyrimidin-4-yl)amino)-2-cyclopropylquinolin-5-yl)dimethylphosphine oxide.

The second object of the present invention is to provide a pharmaceutical composition and the pharmaceutical composition, comprising: the compound of the formula I, the formula II or the formula III, or a stereoisomer, a tautomer, a deuterated compound, a pharmaceutically acceptable salt, a prodrug, a chelate, a non-covalent complex or a solvate thereof as the active ingredient, and at least one pharmaceutically acceptable adjuvant such as a carrier or an excipient.

The third object of the present invention is to provide a method for inhibiting various forms of EGFR mutations, including one or more of L858R, Δ19del, T790M and C797S mutations. The method comprises administering any compound of formula I, or a stereoisomer, a tautomer, a deuterated compound, a pharmaceutically acceptable salt, a prodrug, a chelate, a non-covalent complex or a solvate thereof to a patient.

The fourth object of the present invention is to provide a method for treating EGFR-driven cancer. The method comprises administering a therapeutically effective amount of the compound of formula I, or a stereoisomer, a tautomer, a deuterated compound, a pharmaceutically acceptable salt, a prodrug, a chelate, a non-covalent complex or a solvate thereof to a patient in need thereof.

In certain embodiments, the EGFR-driven cancer is characterized by the presence of one or more mutations selected from the group consisting of (i) C797S, (ii) L858R and C797S, (iii) C797S and T790M, (iv) L858R, T790M, and C797S, or (v) Δ19del, T790M and C797S.

In certain embodiments, the EGFR-driven cancer is colon cancer, stomach cancer, thyroid cancer, lung cancer, leukemia, pancreatic cancer, melanoma, brain cancer, kidney cancer, prostate cancer, ovarian cancer, or breast cancer.

In certain embodiments, the lung cancer is non-small cell lung cancer carrying the $EGFR^{L858R/T790M/C797S}$ or $EGFR^{\Delta 19del/T790M/C797S}$ mutation.

The fifth object of the present invention is to provide a method for inhibiting mutant EGFR in a patient. The method comprises administering a therapeutically effective amount of the compound of formula I, or a stereoisomer, a tautomer, a deuterated compound, a pharmaceutically acceptable salt, a prodrug, a chelate, a non-covalent complex

or a solvate thereof to a patient in need thereof.

The sixth object of the present invention is to provide use of the compound of formula I of the present invention, or a stereoisomer, a tautomer, a deuterated compound, a pharmaceutically acceptable salt, a prodrug, a chelate, a non-covalent complex, or a solvate thereof, or a pharmaceutical composition thereof, in the manufacture of a medicament.

In certain embodiments, the medicament is used for treating or preventing cancer.

In certain embodiments, the cancer is colon cancer, stomach cancer, thyroid cancer, lung cancer, leukemia, pancreatic cancer, melanoma, brain cancer, kidney cancer, prostate cancer, ovarian cancer, or breast cancer.

In certain embodiments, the lung cancer is non-small cell lung cancer carrying the $EGFR^{L858R/T790M/C797S}$ or $EGFR^{\Delta 19del/T790M/C797S}$ mutation.

### Definition and Specification

The general chemical terms used in the above general formulae have their common meanings. For example, unless otherwise specified, the term "halogen" as used herein refers to fluorine, chlorine, bromine or iodine. Preferred halogen groups include F, Cl and Br.

Unless otherwise specified, alkyl as used herein includes linear or branched saturated monovalent hydrocarbyl groups. For example, alkyl includes methyl, ethyl, propyl, isopropyl, n-butyl, isobutyl, sec-butyl, tert-butyl, n-pentyl, 3-(2-methyl)butyl, 2 -pentyl, 2-methylbutyl, neopentyl, n-hexyl, 2-hexyl, 2-methylpentyl. Similarly, $C_{1-8}$ as in $C_{1-8}$ alkyl is defined as identifying the group as having a linear or branched chain of 1, 2, 3, 4, 5, 6, 7 or 8 carbon atoms.

Alkoxy is an oxo-ether formed from the aforementioned linear, branched or cyclic alkyl.

Unless otherwise indicated, the term "aryl" as used herein refers to an unsubstituted or substituted monocyclic or polycyclic ring system containing carbon ring atoms. Preferred aryl is a monocyclic or bicyclic 6-10 membered aromatic ring system. Preferred aryl comprises phenyl and naphthyl. The most preferred aryl is phenyl.

Unless otherwise specified, the term "heteroaryl" as used herein refers to an unsubstituted or substituted stable five- or six-membered monocyclic aromatic ring system, or an unsubstituted or substituted nine- or ten-membered benzo-fused heteroaromatic ring system or bicyclic heteroaromatic ring system. Preferably, a heteroaryl comprises carbon atoms and 1-4 heteroatom(s) selected from N, O or S, wherein the nitrogen or sulfur heteroatom is optionally oxidized, and the nitrogen heteroatom is optionally quaternized. Heteroaryl can be attached to any heteroatoms or carbon atoms, forming a stable structure. Examples of heteroaryl include, but are not limited to, thienyl, furyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuranyl, benzothienyl, benzisoxazolyl, benzoxazolyl, benzopyrazolyl, benzothiazolyl, benzodithiazolyl, benzotriazolyl, adeninyl, quinolinyl or isoquinolinyl.

The term "cycloalkyl" refers to a saturated cyclic alkyl chain comprising carbon atoms, such as cyclopropyl, cyclobutyl, cyclobutyl, cyclopentyl.

Unless otherwise specified, the term "heterocyclyl" as used herein refers to stable heteroatomic-containing monocyclic, bicyclic or tricyclic rings, which may be saturated or partially unsaturated, containing carbon atoms and 1-4 heteroatom(s) selected from N, O and S, and wherein the nitrogen or sulfur heteroatom is optionally oxidized, and the nitrogen heteroatom is optionally quaternized. Heterocyclyl can be attached to any heteroatoms or carbon atoms, forming a stable structure. Examples of heterocyclyl include, but are not limited to, oxiranyl, piperazinyl, morpholinyl, piperidinyl, tetrahydropyrrolyl, tetrahydrofuranyl, and the like.

The term "substituted" refers to a group in which one or more hydrogen atoms are each independently substituted by the same or different substituents. Typical substituents include, but are not limited to, halogen (F, Cl, Br or I), $C_{1-8}$ alkyl, $C_{2-12}$ cycloalkyl, $-OR^1$, $SR^1$, =O, =S, $-C(O)R^1$, $-C(S)R^1$, $=NR^1$, $-C(O)OR^1$, $-C(S)OR^1$, $-NR^1R^2$, $-C(O)NR^1R^2$, cyano, nitro, $-S(O)_2R^1$, $-OS(O_2)OR^1$, $-OS(O)_2R^1$, $-OP(O)(OR^1)(OR^2)$; wherein $R^1$ and $R^2$ are each independently selected from -H, lower alkyl, lower haloalkyl. In some embodiments, substituents are independently selected from the group consisting of -F, -Cl, -Br, -I, -OH, trifluoromethoxy, ethoxy, propoxy, isopropoxy, n-butoxy, isobutoxy, t-butoxy, $-SCH_3$, $-SC_2H_5$, formaldehyde, - $C(OCH_3)$, cyano, nitro, $CF_3$, $-OCF_3$, amino, dimethylamino, methylthio, sulfonyl and acetyl.

Examples of substituted alkyl include, but are not limited to, 2-aminoethyl, 2-hydroxyethyl, pentachloroethyl, trifluoromethyl, methoxymethyl, pentafluoroethyl, and piperazinylmethyl.

Examples of substituted alkoxy include, but are not limited to, aminomethoxy, tetrafluoromethoxy, 2-diethylaminoethoxy, 2-ethoxycarbonylethoxy, 3-hydroxypropoxy.

As used herein, the term "composition" is intended to encompass a product comprising specified ingredients in specified amounts, as well as any products that obtained, directly or indirectly, from a combination of specified ingredients in specified amounts. Accordingly, pharmaceutical compositions containing the compounds of the present invention as active ingredients and methods of preparing the compounds of the present invention are also part of the present invention. Furthermore, some of the crystalline forms of the compounds may exist as polymorphs and are therefore intended to be included in the present invention. In addition, some of the compounds may form solvates with water (i.e., hydrates) or common organic solvents, and such solvates are also included within the scope of the present invention.

The compounds of the present invention may also exist in the form of pharmaceutically acceptable salts. For using in medicine, the salts of the compounds of the present invention refer to non-toxic "pharmaceutically acceptable salts". The pharmaceutically acceptable salts include pharmaceutically acceptable acid/anionic salts or basic/cationic salts. A pharmaceutically acceptable acid/anionic salt is typically in the form in which the basic nitrogen is protonated with an inorganic or organic acid. Representative organic or inorganic acids include hydrochloric acid, hydrobromic acid, hydroiodic acid, perchloric acid, sulfuric acid, nitric acid, phosphoric acid, acetic acid, propionic acid, glycolic acid, lactic acid, succinic acid, maleic acid, fumaric acid, malic acid, tartaric acid, citric acid, benzoic acid, mandelic acid, methanesulfonic acid, isethionic acid, benzenesulfonic acid, oxalic acid, pamoic acid, 2-naphthalenesulfonic acid, p-toluenesulfonic acid, cyclohexanesulfamic acid, salicylic acid, saccharin or trifluoroacetic acid. Pharmaceutically acceptable basic/cationic salts include, but are not limited to, salts of aluminum, calcium, chloroprocaine, choline, diethanolamine, ethylenediamine, lithium, magnesium, potassium, sodium, and zinc.

Prodrugs of the compounds of the present invention are included within the scope of the present invention. In general, prodrugs refer to functional derivatives that can readily converted into the desired compounds *in vivo.* Thus, in the treating methods of the present invention, the term "administration" or "administering" shall include the treatment of various conditions described with specific disclosed compounds, or with the compounds that may not be specifically disclosed, but which are converted into the specific compounds *in vivo* after being administered to the subject. Conventional methods for the selection and preparation of suitable prodrug derivatives are described in, for example, Design of Prodrugs (Design of Prodrugs, ed. H. Bundgaard, Elsevier, 1985).

Obviously, the definition of each substituent or variable at a particular position in a molecule is independent of other positions in the molecule. It is easy to understand that those of ordinary skill in the art can select the substituents or substituted forms of the compounds of the present invention by means of the prior art and the methods described in the present invention, so as to obtain chemically stable and easy-to-synthesize compounds.

The compounds of the present invention may contain one or more asymmetric centers and may thereby give rise to diastereomers and optical isomers. The present invention includes all possible diastereomers and racemic mixtures thereof, substantially pure resolved enantiomers thereof, all possible geometric isomers and pharmaceutically acceptable salts thereof.

The above formula I does not precisely define the steric structure of the compound at a certain position. The present invention includes all stereoisomers of the compound of formula I and pharmaceutically acceptable salts thereof. Further, mixtures of stereoisomers and specific isolated stereoisomers are also included in the present invention. During synthetic procedures of preparing such compounds, or using racemization or epimerization procedures known to those of ordinary skill in the art, the resulting product may be a mixture of stereoisomers.

When a compound of formula I has a tautomer, unless otherwise stated, the present invention includes any possible tautomers and pharmaceutically acceptable salts thereof, and mixtures thereof.

When the compounds of formula I and pharmaceutically acceptable salts thereof have solvates or polymorphs, the present invention includes any possible solvates and polymorphs. The type of solvent that forms the solvate is not particularly limited, as long as the solvent is pharmacologically acceptable. For example, water, ethanol, propanol, acetone and similar solvents can be used.

The term "pharmaceutically acceptable salts" refers to salts prepared from pharmaceutically acceptable non-toxic bases or acids. When the compound provided herein is an acid, its corresponding salts can be conveniently prepared from pharmaceutically acceptable non-toxic bases, including inorganic and organic bases. Salts derived from inorganic bases include salts of aluminum, ammonium, calcium, copper (high valence and low valence), ferric, ferrous, lithium, magnesium, manganese (high valence and low valence), potassium, sodium, zinc, and the like. The salts of ammonium, calcium, magnesium, potassium and sodium are particularly preferred. Pharmaceutically acceptable nontoxic organic bases that can form salts include primary, secondary, and tertiary amines, as well as cyclic amines and substituted amines, such as natural and artificial substituted amines. Other pharmaceutically acceptable nontoxic organic bases capable of forming salts, include ion exchange resin and arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, reduced glucosamine, glucosamine, histidine, hydrabamine, isopropylamine, lysine, methylglucamine, morpholine, piperazine, piperidine, polyamine resin, procaine, purine, theobromine, triethylamine, trimethylamine, tripropylamine, tromethamine, and the like.

When the compound provided by the present invention is a base, its corresponding salts can be conveniently prepared from pharmaceutically acceptable non-toxic acids, including inorganic and organic acids. Such acids include, for example, acetic acid, benzenesulfonic acid, benzoic acid, camphorsulfonic acid, citric acid, ethanesulfonic acid, formic acid, fumaric acid, gluconic acid, glutamic acid, hydrobromic acid, hydrochloric acid, isethionic acid, lactic acid, maleic acid, malic acid, mandelic acid, methanesulfonic acid, mucic acid, nitric acid, pamoic acid, pantothenic acid, phosphoric acid, succinic acid, sulfuric acid, tartaric acid and p-toluenesulfonic acid, and the like; preferably, citric acid, hydrobromic acid, formic acid, hydrochloric acid, maleic acid, phosphoric acid, sulfuric acid and tartaric acid; more preferably, formic acid and hydrochloric acid. Since the compound of formula I will be used as a medicament, preferably

it comprises a certain purity, for example, a purity of at least 60%, more preferably at least 75%, particularly preferably at least 98% (% is weight ratio).

The pharmaceutical composition provided by the present invention includes the compound of formula I (or a pharmaceutically acceptable salt thereof) as an active component, a pharmaceutically acceptable excipient and other optional therapeutic components or adjuvants. Although the most suitable mode of administration of an active component in any given situation will depend on the particular subject being administered, the nature of the subject and the severity of the condition, the pharmaceutical compositions of the present invention include pharmaceutical compositions suitable for oral, rectal, topical and parenteral (including subcutaneous, intramuscular, intravenous) administration. The pharmaceutical composition of the present invention may conveniently be presented in any unit dosage form well known in the art and prepared by any preparation method well known in the field of medicine.

In fact, according to conventional pharmaceutical mixing techniques, the compound of formula I of the present invention, or a prodrug, a metabolite, a pharmaceutically acceptable salt thereof, as an active component, can be mixed with a pharmaceutical carrier to form a pharmaceutical composition. The said pharmaceutical carrier can be in a wide variety of forms, depending on the desired mode of administration, e.g., orally or by injection (including intravenous injection). Accordingly, the pharmaceutical compositions of the present invention may be presented in discrete units suitable for oral administration, such as capsules, cachets or tablets containing a predetermined amount of the active component. Further, the pharmaceutical composition of the present invention can be in the form of powders, granules, solutions, aqueous suspensions, non-aqueous liquids, oil-in-water emulsions, or water-in-oil emulsions. Furthermore, in addition to the common dosage forms mentioned above, the compound of formula I or a pharmaceutically acceptable salt thereof can also be administered by means of controlled release and/or delivery device. The pharmaceutical composition of the present invention can be prepared by any pharmaceutical methods. In general, such methods include the step of combining the active component with the carrier which constitutes one or more essential ingredients. In general, the pharmaceutical composition is prepared by a uniform intimate mixing of the active component with liquid carriers, or finely divided solid carriers, or a mixture of both. In addition, the product can be easily prepared into a desired appearance.

Therefore, the pharmaceutical composition of the present invention comprises a pharmaceutically acceptable carrier, and the compound of formula I or a stereoisomer, a tautomer, a polymorph, a solvate, a pharmaceutically acceptable salt thereof, or a prodrug thereof. The drug combination of the compound of formula I or a pharmaceutically acceptable salt thereof, with one or more other compounds with therapeutic activity, is also included in the pharmaceutical composition of the present invention.

The pharmaceutical carrier used in the present invention can be, for example, a solid carrier, a liquid carrier or a gaseous carrier. Solid carriers include lactose, gypsum, sucrose, talc, gelatin, agar, pectin, acacia, magnesium stearate, stearic acid. Liquid carriers include syrup, peanut oil, olive oil and water. Gas carriers, including carbon dioxide and nitrogen. In preparing a pharmaceutical oral formulation, any pharmaceutically convenient medium can be used. For example, water, ethylene glycol, oils, alcohols, flavor enhancers, preservatives, colorants, and the like can be used in oral liquid formulations such as suspensions, elixirs and solutions; and carriers such as starches, sugars, microcrystalline cellulose, diluents, granulating agents, lubricants, binders, disintegrating agents, and the like can be used for oral solid formulations such as powders, capsules and tablets. For ease of administration, tablets and capsules are preferred for oral formulations, where solid pharmaceutical carriers are employed. Optionally, tablet coatings can use standard aqueous or non-aqueous formulation techniques.

Tablets containing the compound or pharmaceutical composition of the present invention may be formed by compression or molding, optionally together with one or more adjuvant components or adjuvant drugs. Compressed tablets may be prepared by compressing, in a suitable machine, the active component in a free-flowing form such as powder or granule, mixed with a binder, a lubricant, an inert diluent, a surfactant or a dispersing agent. Molded tablets may be prepared by wetting a powdered compound or pharmaceutical composition with an inert liquid diluent and molding in a suitable machine. Preferably, each tablet contains about 0.05 mg to 5 g of active component and each cachet or capsule contains about 0.05 mg to 5 g of active component. For example, a formulation intended for oral administration to humans contains from about 0.5 mg to about 5 g of an active component, mixed with suitable and easy-to-measure adjuvant materials comprised in an amount of about 5% to 95% of the total pharmaceutical composition. A unit dosage form generally contains from about 1 mg to about 2 g of an active component, typically 25 mg, 50 mg, 100 mg, 200 mg, 300 mg, 400 mg, 500 mg, 600 mg, 800 mg or 1000 mg of an active component.

The present invention provides a pharmaceutical composition suitable for injection, including sterile aqueous solutions or dispersions. Further, the above-mentioned pharmaceutical composition can be prepared in a sterile powder form for the extemporaneous preparation of sterile injection solutions or dispersions. In any case, the final injectable form must be sterile and, for ease of injection, must be readily flowable. Furthermore, the pharmaceutical composition must be stable during manufacture and storage. Therefore, preferably, the pharmaceutical composition is preserved under conditions that are resistant to contamination by microorganisms such as bacteria and fungi. The carrier can be a solvent or dispersion medium, for example, water, ethanol, polyols (e.g., glycerol, propylene glycol, liquid polyethylene

glycol), vegetable oils, and suitable mixtures thereof.

The pharmaceutical composition provided by the present invention may be in a form suitable for topical administration, e.g., an aerosol, cream, ointment, lotion, dusting powder, or other similar dosage forms. Further, the pharmaceutical composition provided by the present invention can be in a form suitable for use in a transdermal administration device. These formulations can be prepared by conventional processing methods, using the compound of formula I of the present invention, or a pharmaceutically acceptable salt thereof. As an example, a cream or ointment with the desired consistency is prepared by adding about 5 to 10 wt. % of a hydrophilic material and water to the cream or ointment.

The pharmaceutical composition provided by the present invention can be in the form suitable for rectal administration by using a solid as a carrier. The most typical dosage form is a unit-dose suppository. Suitable adjuvants include cocoa butter and other materials commonly used in the art. Suppositories can be conveniently prepared by mixing the pharmaceutical composition with softened or melted adjuvants, following by cooling and molding.

In addition to the adjuvant components mentioned above, the above formulations may further include, where appropriate, one or more additional adjuvant components, such as diluents, buffers, flavoring agents, binders, surfactants, thickeners, lubricants and preservatives (including antioxidants), and the like. Further, other adjuvants may also include permeation enhancers that adjust the isotonic pressure of the drug and blood. The pharmaceutical composition comprising the compound of formula I, or a pharmaceutically acceptable salt thereof, can be prepared in the form of powder or concentrate.

In general, to treat the conditions or disorders indicated above, the dose level of the drug is about 0.01 mg/kg body weight to 150 mg/kg body weight per day, or 0.5 mg to 7 g per patient per day. For example, for the inflammation, cancer, psoriasis, allergies/asthma, diseases and disorders of the immune system, diseases and disorders of the central nervous system (CNS), the effective dose level of the drug is 0.01 mg/kg body weight to 50 mg/kg body weight per day, or 0.5 mg to 3.5 g per patient per day.

It will be appreciated, however, that lower or higher doses than those described above may be required. The specific dose level and treatment regimen for any particular patient will depend on a variety of factors, including the activity of the specific compound used, age, body weight, general health, sex, diet, time of administration, route of administration, excretion rate, drug combination, and the severity of the specific disease being treated.

These and other aspects will become apparent from the following description of the invention.

The following examples are provided to better illustrate the present invention. All parts and percentages are by weight and all temperatures are in degrees Celsius unless otherwise specified.

The present invention will be described in more detail by way of specific examples. The following examples are provided for illustrative purposes and are not intended to limit the invention in any manner. Those skilled in the art will readily recognize that various noncritical parameters can be varied or modified to produce substantially the same results. According to at least one assay described herein, compounds of the examples have been found to inhibit any one or more of the mutations L858R, Δ19del, T790M and C797S.

## BRIEF DESCRIPTION OF THE DRAWINGS

FIG. 1, figure showing the half effective dose $ED_{50}$ of Comparative Example 1.

## EXAMPLES

It can be understood that both the foregoing general description and the following detailed description are exemplary and explanatory only and are intended to limit any claimed subject matter. All parts and percentages are by weight and all temperatures are in degrees Celsius unless otherwise specified. The compounds described herein can be obtained from commercial sources or synthesized by conventional methods as shown below using commercially available raw materials and reagents.

The following abbreviations have been used in the examples:

AcOH: acetic acid;
DIEA: N,N-diisopropylethylamine;
DMF: N,N-dimethylformamide;
DMSO: dimethyl sulfoxide;
EA: ethyl acetate;
HEPES: 4-(2-hydroxyethyl)-1-piperazineethanesulfonic acid;
Xantphos: 4,5-bis(diphenylphosphine)-9,9-dimethylxanthene;
$Pd(OAc)_2$: Palladium acetate
n-BuOH: n-butanol;
PTSA: p-toluenesulfonic acid;

PTLC: preparative thin layer chromatography;
LCMS: liquid chromatography-mass spectrometry;
h or hrs: hour(s);
Pd/C: palladium on activated carbon;
MeOH: methanol;
NMP: N-methyl-2-pyrrolidone;
TLC: thin layer chromatography;
$Pd(dppf)Cl_2$: 1,1'-bis(diphenylphosphino)ferrocene palladium dichloride;
$Pd(PPh_3)_4$: tetrakis(triphenylphosphine)palladium;
Pd-Ruphos $G_3$: methanesulfonato(2-dicyclohexylphosphino-2',6'-diisopropoxy-1,1'-biphenyl)(2-amino-1,1 '-biphenyl)-2-yl)palladium(II);
Ruphos: 2-dicyclohexylphosphine-2',6'-diisopropoxybiphenyl;
$Cs_2CO_3$: cesium carbonate;
ACN: acetonitrile.

**Example 1 Synthesis of compound J-001**

*(6-((5-Bromo-2-((5-methoxy-2-methyl-4-morpholinylphenyl)amino)pyrimidin-4-yl)amino)-2-cyclopropylquinoline-5-yl)dimethylphosphine oxide*

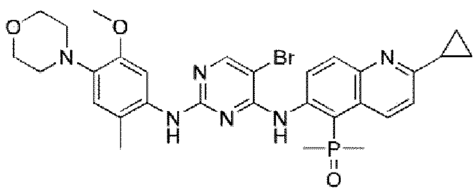

Step 1: Synthesis of Compound 1-2

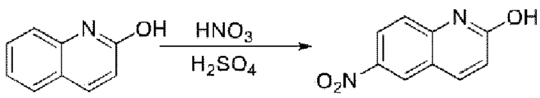


1-1 1-2

In a 100 ml single-neck flask, compound 1-1 (25 g, 172.23 mmol) was dissolved in concentrated sulfuric acid (100 mL), and concentrated nitric acid (16.28 g, 258.34 mmol) was added dropwise at 0°C. The mixture was stirred at room temperature for 2 hours. It was monitored by TLC until the raw material reacted completely. The reaction was quenched by adding the solution slowly into 2 L of ice water, and a pale yellow solid was precipitated. The reaction solution was stirred for 1 hour and filtered. The filter cake was washed with 1 L of water, collected, and dried to obtain compound 1-2 (24.5 g, 128.84 mmol, yield: 74.81%). MS: 191.04 $[M+H]^+$

Step 2: Synthesis of Compound 1-3

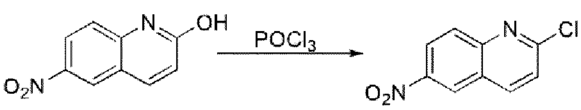


1-2 1-3

In a 500 ml single-neck flask, compound 1-2 (24.5 g, 128.84 mmol) was dissolved in phosphorus oxychloride

(200 mL), heated to 100 °C and stirred overnight. It was monitored by LCMS until the raw material reacted completely. The reaction solution was cooled to room temperature, concentrated and the residue was poured into 1 L of ice water, stirred for 0.5 hour, and filtered. The filter cake was washed with 1 L of water, collected and dried to obtain compound 1-3 (25 g, 119.85 mmol, yield: 93.02%).

Step 3: Synthesis of Compound 1-4

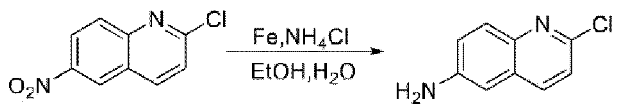


1-3 1-4

In a 500 ml single-neck flask, compound 1-3 (25 g, 119.85 mmol) was dissolved in 150 mL of ethanol. 30 mL of $H_2O$ was added, followed by adding iron powder (33.47 g, 599.23 mmol) and ammonium chloride (32.05 g, 599.23 mmol). The reaction solution was heated to 90°C, stirred for 3 hours, cooled to room temperature and filtered through diatomite. The filter cake was washed with ethanol for several times and the filtrate was collected and concentrated. The residue was purified by Flash silica gel column (A:DCM, B:MeOH; methanol 0-5%, 20 minutes) to obtain compound 1-4 (16.9 g, 94.62 mmol, yield: 78.95%). MS: 179.03 $[M+H]^+$

Step 4: Synthesis of Compound 1-5

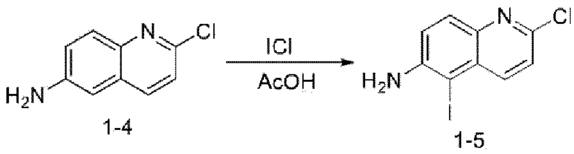


1-4 1-5

In a 250 ml single-neck flask, compound 1-4 (16.9 g, 94.62 mmol) was dissolved in glacial acetic acid (320 mL) and iodine chloride (18.43 g, 113.54 mmol) in acetic acid (80 mL) was added dropwise at room temperature. After the addition was completed, stirring was continued at room temperature for 2 hours. It was monitored by LCMS until the raw material reacted completely. 500 ml of n-hexane was added to dilute the reaction solution and a solid was precipitated, which was then filtered. The filter cake was washed with n-hexane, dried by suction, dissolved in a mixed solvent of DCM: MeOH=10:1, washed twice with saturated sodium carbonate solution, twice with saturated sodium thiosulfate solution, and once with saturated sodium chloride solution successively, dried, filtered, and concentrated. The residue was purified by Flash silica gel column (A:DCM, B:MeOH; methanol 0-5%, 20 minutes) to obtain compound 1-5 (22.23 g, 73.00 mmol, yield: 77.16%). MS: 304.93 $[M+H]^+$

Step 5: Synthesis of Compound 1-6

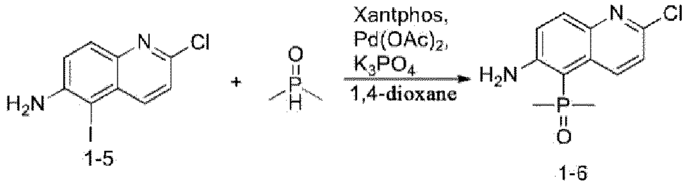


1-5 1-6

In a 250 ml single-neck flask, compound 1-5 (10.00 g, 32.84 mmol), dimethylphosphine oxide (2.69 g, 34.48 mmol), Xantphos (3.80 g, 6.57 mmol), palladium acetate (737.27 mg, 3.28 mmol) and anhydrous potassium phosphate (13.94 g, 65.68 mmol) were dissolved in 1,4-dioxane (100 mL). Nitrogen replacement was performed for three times. The mixture was heated to 100°C and stirred overnight. The temperature was lowered and the reaction solution was filtered. The filtrate was concentrated. The residue was purified by Flash silica gel column, being eluting first with

petroleum ether/ethyl acetate (ethyl acetate 0-50%, 10 minutes) and then with dichloromethane/methanol (methanol 0-6%, 20 minutes), to obtain compound 1-6 (6.18 g, 24.27 mmol, yield: 73.90%). MS: 255.04 $[M+H]^+$

Step 6: Synthesis of Compound 1-7

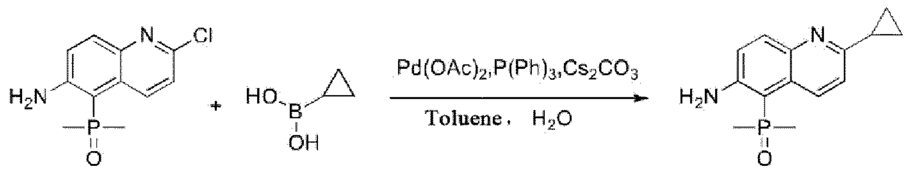


1-6 1-7

Compound 1-6 (4 g, 15.71 mmol), cyclopropylboronic acid (5.40 g, 62.83 mmol), palladium acetate (352.65 mg, 1.57 mmol), triphenylphosphine (0.82 g, 3.14 mmol) and $Cs_2CO_3$ (15.35 g, 47.12 mmol) were added to a mixed solvent of toluene (60 mL) and $H_2O$ (10 mL). The mixture was heated to 100°C under nitrogen protection, and stirred for 12 hours. It was monitored by LCMS until the reaction was completed and the reaction solution was cooled to room temperature. The reaction solution was added with 40 mL of water and the layers were separated. The organic phase was collected. The aqueous phase was extracted with ethyl acetate (3 x 30 mL). The organic phases were combined, dried with anhydrous sodium sulfate, filtered, concentrated, and purified by column chromatography (dichloromethane: methanol =15:1) to obtain a yellow solid compound 1-7 (2.2 g, 8.45 mmol, yield: 53.81%). MS: 261.11 $[M+H]^+$

Step 7: Synthesis of Compound 1-8

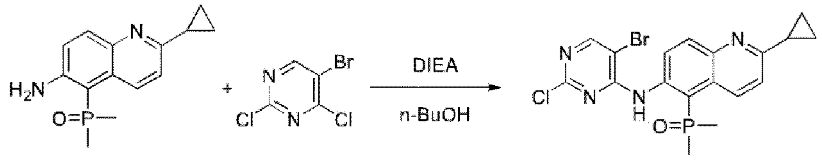


1-7 1-8

Compound 1-7 (2.2 g, 8.45 mmol), 5-bromo-2,4-dichloropyrimidine (3.85 g, 16.91 mmol), DIEA (3.28 g, 25.36 mmol, 4.42 mL) and n-BuOH (40 mL) were successively added into a reaction flask. The mixture was heated to 120°C and stirred for 10 hours. It was monitored by LCMS until the reaction was completed. The reaction solution was cooled to room temperature and filtered. The filter cake was dried to obtain a pale yellow solid compound 1-8 (2.4 g, 5.31 mmol, yield: 62.86%). MS: 451.00 $[M+H]^+$

Step 8: Synthesis of Compound 1-10

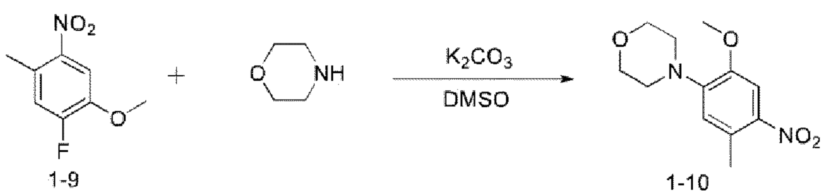


1-9 1-10

Compound 1-9 (3.00 g), morpholine (2.11 g), $K_2CO_3$ (4.48 g) and DMSO (30 mL) were successively added to a reaction flask and the temperature was raised to 90°C. The mixture stirred under heating for 12 hours. It was monitored by LCMS until the reaction was completed and then the reaction was stopped. The reaction solution was poured into 100 mL of water, and a solid was precipitated and filtered by suction. The filter cake was washed with water and dried

to obtain a yellow solid compound 1-10 (3.5 g). MS: 253.11 [M+H]+

Step 9: Synthesis of Compound 1-11

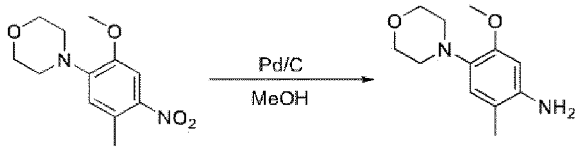


1-10 1-11

Compound 1-10 (3.50 g) and MeOH (60 mL) were added to a reaction flask. The reaction flask was then vacuumized, purged with nitrogen, added with Pd/C (1.00 g, 10%), vacuumized, and purged with $H_2$. The reaction was carried out at room temperature for 3 hours. It was monitored by LCMS until the reaction was completed and then the reaction was stopped. After filtration by suction, the filter cake was washed with methanol (20 mL). The organic phase was collected and concentrated to obtain a reddish-brown liquid compound 1-11 (3.1 g). MS: 223.14 [M+H]+

Step 10: Synthesis of Compound J-001

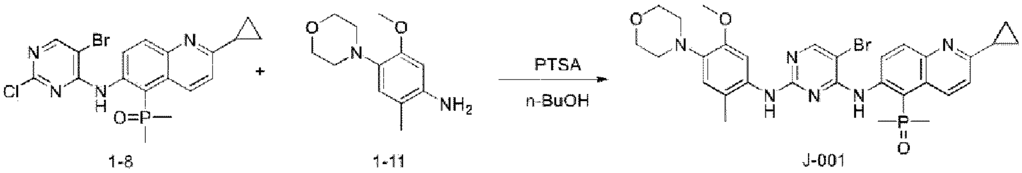


1-8 1-11 J-001

Compound 1-8 (100 mg), compound 1-11 (49.3 mg), p-toluenesulfonic acid (57.3 mg) and n-BuOH (3 mL) were successively added to a reaction flask and the mixture was stirred at 110°C overnight. It was monitored by LCMS until the reaction was completed. The reaction solution was cooled to room temperature, spin-dried, added with saturated aqueous $Na_2CO_3$ solution (10 mL), extracted with dichloromethane (3 x 10 mL), dried with anhydrous sodium sulfate, filtered, and spin-dried. The crude product was purified by PTLC (dichloromethane: methanol=10:1) to obtain compound J-001 (55 mg). MS: 637.16 [M+H]+

**Example 2 Synthesis of compound J-002**

*5-((5-Bromo-4-((2-cyclopropyl-5-(dimethylphosphine oxide)quinolin-6-yl)amino)pyrimidin-2-yl)amino)-4-methoxy-2-morpholinebenzonitrile*

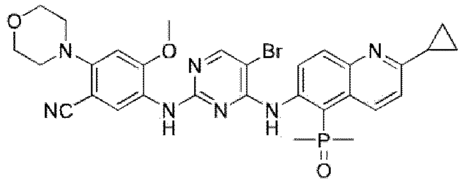

Step 1: Synthesis of Compound 2-2

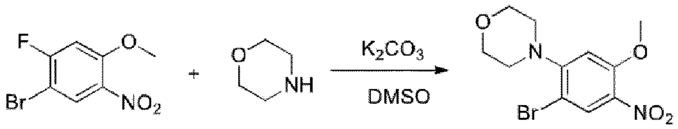


2-1 2-2

Compound 2-1 (1 g, 4.00 mmol), morpholine (696.90 mg, 8.00 mmol), $K_2CO_3$ (1.66 g, 12.00 mmol) and DMSO (10 mL) were successively added to a reaction flask and the temperature was raised to 100°C. The mixture stirred under heating for 12 hours. It was monitored by LCMS until the reaction was completed and then the reaction was stopped. The reaction solution was poured into water (20 mL) and filtered by suction. The filter cake was washed with water and dried to obtain a yellow solid compound 2-2 (1.1 g, 3.47 mmol). MS: 317.01 $[M+H]^+$

Step 2: Synthesis of Compound 2-3

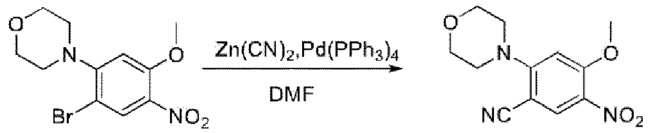


2-2 2-3

Compound 2-2 (1.1 g, 3.47 mmol), $Zn(CN)_2$ (610.94 mg, 5.20 mmol) and $Pd(PPh_3)_4$ (400.81 mg, 346.86 μmol) were dissolved in DMF (10 mL) and nitrogen replacement was performed for three times. The reaction was carried out at 120°C under microwave for 2 hours and was monitored by LCMS until the reaction was completed. The reaction solution was cooled to room temperature, added with water (20 mL), filtered, washed with a small amount of water and washed with dichloromethane (15 mL). The layers were separated. The organic phase are dried with anhydrous sodium sulfate, filtered, concentrated, and purified by column chromatography (dichloromethane: methanol = 15: 1) to obtain a white solid compound 2-3 (700 mg, 2.66 mmol, yield: 76.66%). MS: 264.00 $[M+H]^+$

Step 3: Synthesis of Compound 2-4

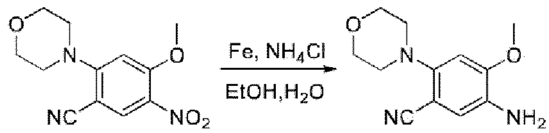


2-3 2-4

Compound 2-3 (700 mg, 2.66 mmol) was dissolved in ethanol (10 mL) and water (5 mL), followed by adding reduced iron powder (1.49 g, 26.59 mmol) and $NH_4Cl$ (1.42 g, 26.59 mmol), and the reaction was carried out at 80°C for 2 hours. It was monitored by LCMS until the reaction was completed. The reaction solution was cooled to room temperature, added with 10 mL of saturated $Na_2CO_3$aqueous solution, extracted with dichloromethane (3 × 10), dried, filtered and concentrated to obtain a pale yellow solid compound 2-4 (500 mg, 2.14 mmol, yield: 80.61%). MS: 234.12 $[M+H]^+$

Step 4: Synthesis of Compound J-002

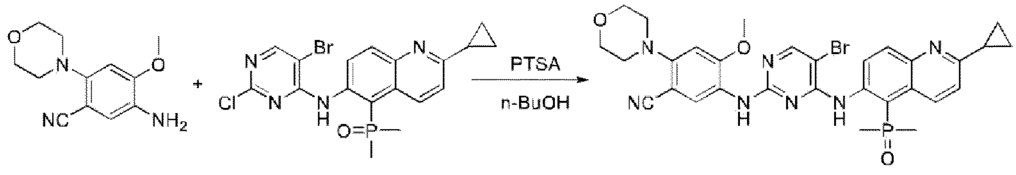


2-4 1-8 J-002

Compound 2-4 (55 mg, 235.78 μmol), compound 1-8 (117.15 mg, 259.36 μmol), p-toluenesulfonic acid (64.96 mg, 377.25 μmol) and n-BuOH (2 mL) were successively added to a reaction flask and the mixture was stirred at 110°C overnight. It was monitored by LCMS until the reaction was completed. The reaction solution was cooled to room temperature, spin-dried, added with saturated aqueous $Na_2CO_3$ solution (10 mL), extracted with dichloromethane (3 x 10 mL), dried with anhydrous sodium sulfate, filtered and spin-dried. The crude product was purified by PTLC (dichloromethane: methanol=10:1) to obtain compound J-002 (10 mg, 15.42 μmol, yield: 6.54%). MS: 648.14 $[M+H]^+$

**Example 3 Synthesis of compound J-003**

*(6-((5-Bromo-2-((2-ethyl-5-methoxy-4-morpholinylphenyl)amino)pyrimidin-4-yl)amino)-2-cyclopropylquinoline-5-yl)dimethylphosphine oxide*

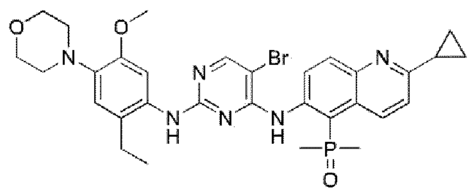

Step 1: Synthesis of Compound 3-2

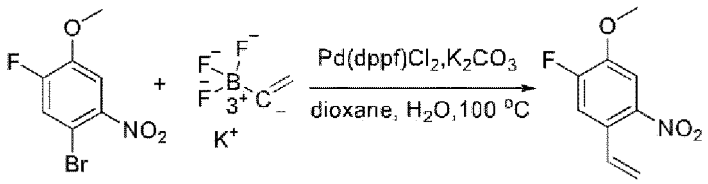


3-1 3-2

Compound 3-1 (500 mg, 2.00 mmol), potassium trifluoro(vinyl)borate (401.81 mg, 3.00 mmol), $Pd(dppf)Cl_2$ (163.31 mg, 199.98 μmol), $K_2CO_3$ (690.99 mg, 5.00 mmol), dioxane (60 mL) and $H_2O$ (15 mL) were successively added to a reaction flask and the reaction was carried out at 100°C for 6 hours. The reaction solution was cooled to room temperature, added with 25 mL water, extracted with ethyl acetate, dried with anhydrous sodium sulfate, filtered, and spin-dried. The crude product was subjected to column chromatography (dichloromethane/methanol=15:1) to obtain the yellow solid target product 3-2 (300 mg, 1.52 mmol, yield: 76.09%).

Step 3: Synthesis of Compound 3-3

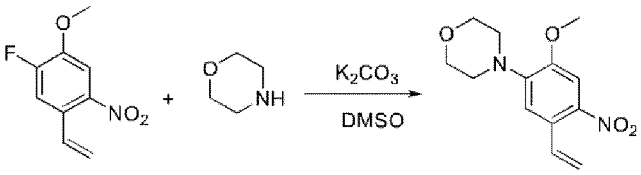


3-2 3-3

The synthesis method of compound 3-3 is the same as that of compound 1-10, except that the starting compound 1-9 is replaced by compound 3-2. MS of compound 3-3: 265.11 [M+H]$^+$

Step 3: Synthesis of Compound 3-4

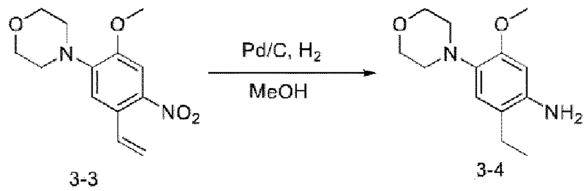


3-3 3-4

The synthesis method of compound 3-4 is the same as that of compound 1-11, except that the starting compound 1-10 is replaced by compound 3-3. MS of compound 3-4: 237.15 [M+H]$^+$

Step 4: Synthesis of Compound J-003

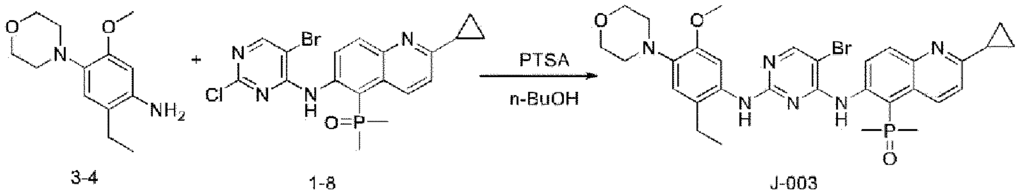


3-4 1-8 J-003

The synthesis method of compound J-003 is the same as that of compound J-001, except that the starting compound 1-11 is replaced by compound 3-4. MS of compound J-003: 651.18 [M+H]$^+$

$^1$H NMR (500 MHz, DMSO-$d_6$) δ 12.16 (s, 1H), 8.64 (s, 1H), 8.36 (s, 1H), 8.30 (d, J = 8.9 Hz, 1H), 8.15 (s, 1H), 7.53 (s, 1H), 7.41 (d, J = 8.9 Hz, 1H), 6.83 (s, 1H), 6.71 (s, 1H), 3.80-3.75 (m, 4H), 3.64 (s, 3H), 3.05-2.76 (m, 4H), 2.46-2.40 (m, 2H), 2.29-2.24 (m, 1H), 2.00 (s, 3H), 1.97 (s, 3H), 1.09 - 0.96 (m, 7H).

**Example 4 Synthesis of compound J-004**

*(6-((5-Bromo-2-((2-cyclopropyl--5-methoxy-4-morpholinylphenyl)amino)pyrimidin-4-yl)amino)-2-cyclopropylquinoline-5-yl)dimethylphosphine oxide*

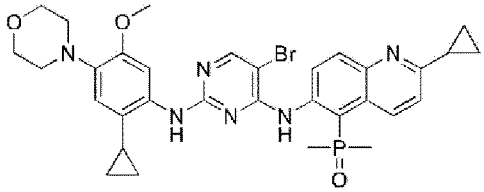

Step 1: Synthesis of Compound 4-1

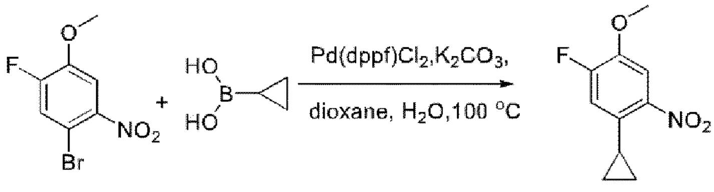


3-1

4-1

The synthesis method of compound 4-1 is the same as that of compound 3-2, except that the starting compound potassium trifluoro(vinyl)borate is replaced with cyclopropylboronic acid.

Step 2: Synthesis of Compound 4-2

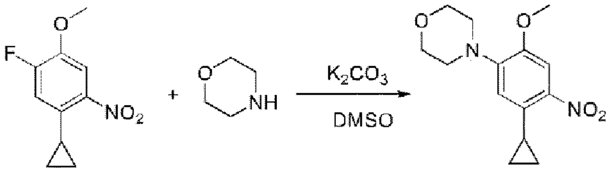


4-1

4-2

The synthesis method of compound 4-2 is the same as that of compound 1-10, except that the starting compound 1-9 is replaced by compound 4-1. MS of compound 4-2: 279.13 $[M+H]^+$

Step 3: Synthesis of Compound 4-3

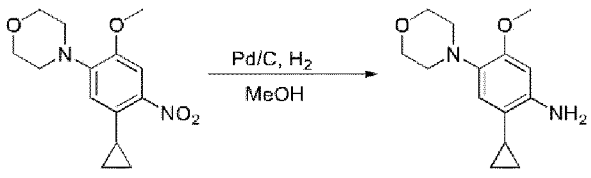


4-2

4-3

The synthesis method of compound 4-3 is the same as that of compound 1-11, except that the starting compound 1-10 is replaced by compound 4-2. MS of compound 4-3: 249.15 $[M+H]^+$

Step 4: Synthesis of Compound J-004

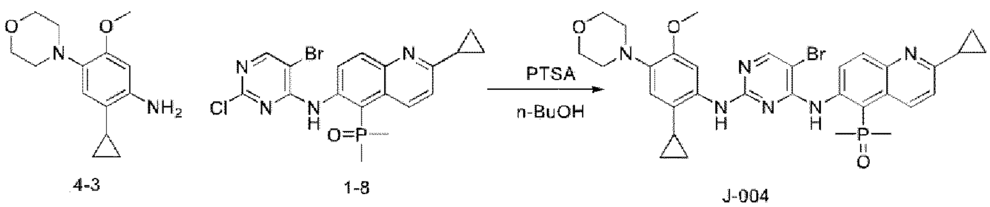


4-3

1-8

J-004

The synthesis method of compound J-004 is the same as that of compound J-001, except that the starting compound 1-11 is replaced by compound 4-3. MS of compound J-004: 663.18 $[M+H]^+$

$^1H$ NMR (500 MHz, DMSO-$d_6$) $\delta$ 12.14 (s, 1H), 8.63 (s, 1H), 8.38 (d, J = 9.2 Hz, 1H), 8.31 (d, J = 8.9 Hz, 1H), 8.18 (s, 1H), 7.54 (s, 1H), 7.41 (d, J = 9.0 Hz, 1H), 6.90 (s, 1H), 6.35 (s, 1H), 3.79-3.60 (m, 4H), 3.58 (s, 3H), 2.99-2.59 (m, 4H), 2.31 - 2.23 (m, 1H), 2.00 (s, 3H), 1.97 (s, 3H), 1.89 - 1.81 (m, 1H), 1.08 - 0.95 (m, 4H), 0.76-0.73 (m, 2H) 0.49-0.47(m, 2H).

**Example 5 Synthesis of compound J-005**

*(6-((5-Bromo-2-((2-isopropyl-5-methoxy-4-morpholinylphenyl)amino)pyrimidin-4-yl)amino)-2-cyclopropylquinoline-5-yl)dimethylphosphine oxide*

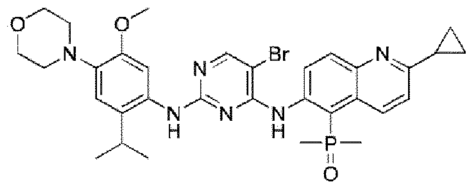

Step 1: Synthesis of Compound 5-1

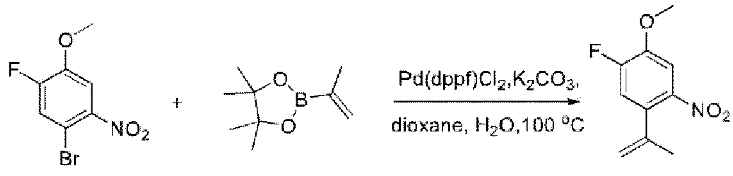


3-1

5-1

The synthesis method of compound 5-1 is the same as that of compound 3-2, except that the starting compound potassium trifluoro(vinyl)borate is replaced by 4,4,5,5-tetramethyl-2-(prop-1-en-2-yl)-1,3,2-dioxaborane.

Step 2: Synthesis of Compound 5-2

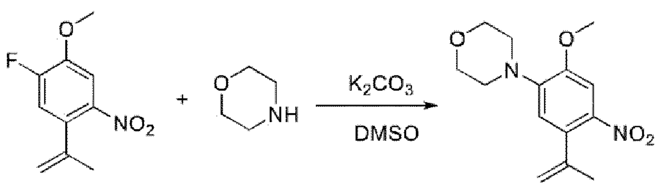


5-1

5-2

The synthesis method of compound 5-2 is the same as that of compound 1-10, except that the starting compound 1-9 is replaced by compound 5-1. MS of compound 5-2: 279.13 $[M+H]^+$

Step 3: Synthesis of Compound 5-3

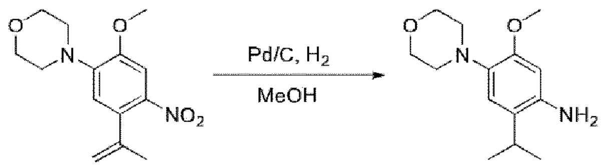


5-2 5-3

The synthesis method of compound 5-3 is the same as that of compound 1-11, except that the starting compound 1-10 is replaced by compound 5-2. MS of compound 5-3: 251.17 $[M+H]^+$

Step 4: Synthesis of Compound J-005

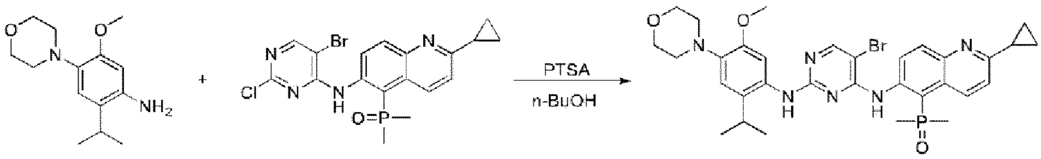


5-3 1-8 J-005

The synthesis method of compound J-005 is the same as that of compound J-001, except that the starting compound 1-11 is replaced by compound 5-3. MS of compound J-005: 665.19 $[M+H]^+$

**Example 6 Synthesis of compound J-006**

*(6-((5-Bromo-2-((3-methoxy-5-methyl-4-morpholinylphenyl)amino)pyrimidin-4-yl)amino)-2-cyclopropylquinoline-5-yl)dimethylphosphine oxide*

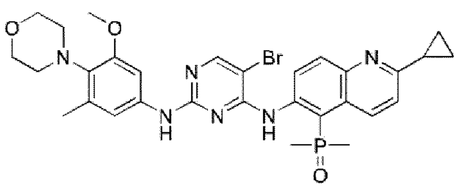

Step 1: Synthesis of Compound 6-2

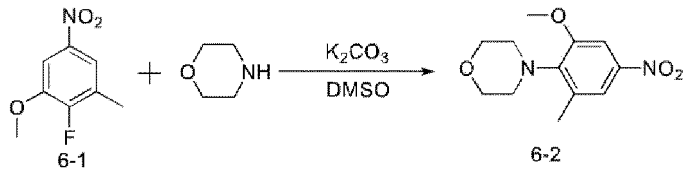


6-1 6-2

The synthesis method of compound 6-2 is the same as that of compound 1-10, except that the starting compound 1-9 is replaced by compound 6-1. MS of compound 6-2: 253.11 $[M+H]^+$

Step 2: Synthesis of Compound 6-3

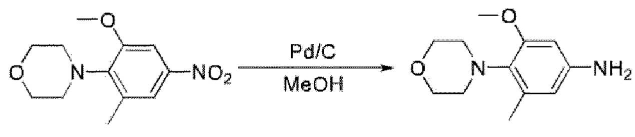


6-2 6-3

The synthesis method of compound 6-3 is the same as that of compound 1-11, except that the starting compound 1-10 is replaced by compound 6-2. MS of compound 6-3: 223.14 [M+H]+

Step 3: Synthesis of Compound J-006

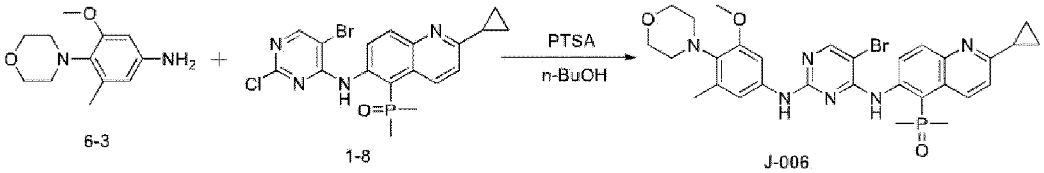


6-3 1-8 J-006

The synthesis method of compound J-006 is the same as that of compound J-001, except that the starting compound 1-11 is replaced by compound 6-3. MS of compound J-006: 637.16 [M+H]+

## Example 7 Synthesis of compound J-007

*(6-((5-Bromo-2-((5-methoxy-2-methyl-4-morpholinylphenyl)amino)pyrimidin-4-yl)amino)-2-(1-methyl-1H-pyrazol-4-yl)quinolin-5-yl)dimethylphosphine oxide*

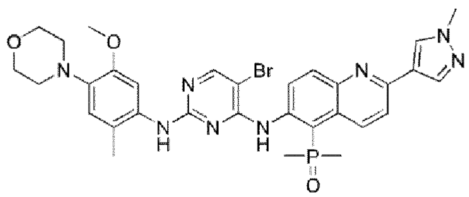

Step 1: Synthesis of Compound 7-2

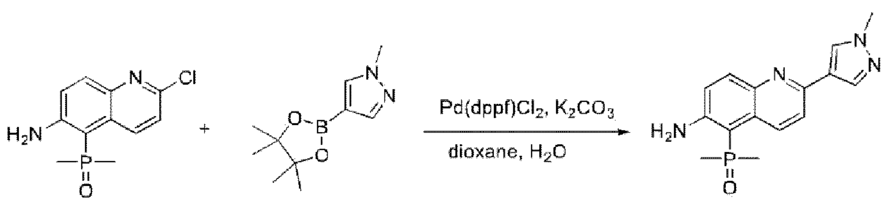


1-6 7-1 7-2

Compound 1-6 (500 mg, 1.96 mmol), compound 7-1 (490.23 mg, 2.36 mmol), Pd(dppf)$Cl_2$ (160.34 mg, 196.35 μmol), $K_2CO_3$ (678.43 mg, 4.91 mmol), dioxane (9 mL) and $H_2O$ (3 mL) were successively added to a reaction flask. Nitrogen replacement was performed for three times and the mixture was heated to 100 °C and stirred for 6 hours. It was monitored by LCMS until the reaction was completed and the reaction solution was cooled to room temperature. The reaction solution was added with 15 mL of water and the layers were separated. The organic phase was collected, and the aqueous phase was extracted with ethyl acetate (3 × 20 mL). The organic phases were combined, dried with

anhydrous sodium sulfate, filtered, spin-dried, and purified by column chromatography (dichloromethane: methanol =15:1) to obtain a yellow solid compound 7-2 (400 mg, 1.33 mmol, yield: 67.84%). MS: 301.11 $[M+H]^+$

Step 2: Synthesis of Compound 7-3

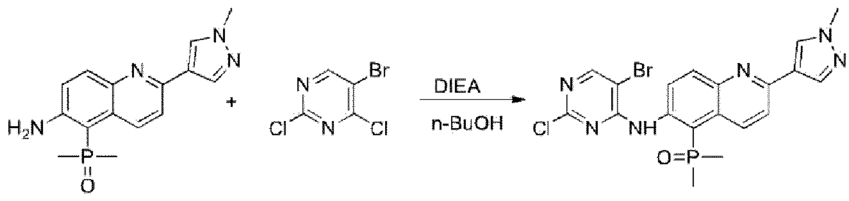


7-2 7-3

The synthesis method of compound 7-3 is the same as that of compound 1-8, except that the starting compound 1-7 is replaced by compound 7-2. MS of compound 7-3: 491.01 $[M+H]^+$

Step 3: Synthesis of Compound J-007

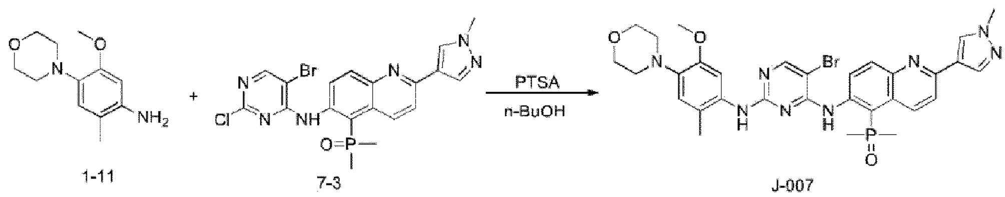


1-11 7-3 J-007

The synthesis method of compound J-007 is the same as that of compound J-001, except that the starting compound 1-8 is replaced by compound 7-3. MS of compound J-007: 677.17 $[M+H]^+$

**Example 8 Synthesis of compound J-008**

*(6-((5-Bromo-2-((5-(1-ethyl-1H-pyrazol-4-yl)-2-methoxy-4-morpholinophenyl)amino)pyrimidine-4-yl)amino)-2-cyclopropylquinolin-5-yl)dimethylphosphine oxide*

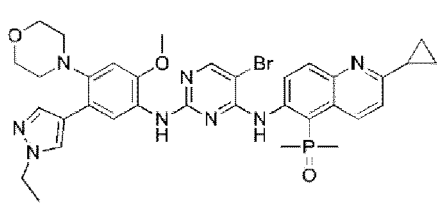

Step 1: Synthesis of Compound 8-2

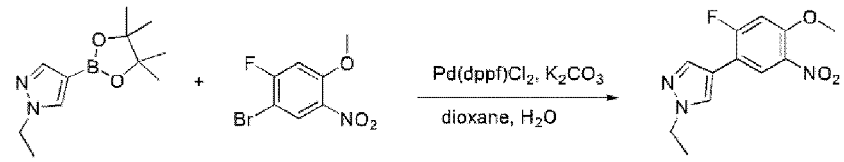


8-1 2-1 8-2

The synthesis method of compound 8-2 is the same as that of compound 7-2, except that compound 1-6 is replaced by compound 2-1, and compound 7-1 is replaced by compound 8-1. MS of compound 8-2: 266.09 $[M+H]^+$

Step 2: Synthesis of Compound 8-3

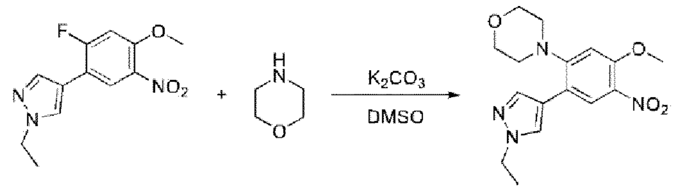


8-2 8-3

The synthesis method of compound 8-3 is the same as that of compound 1-10, except that the starting compound 1-9 is replaced by compound 8-2. MS of compound 8-3: 333.15 $[M+H]^+$

Step 3: Synthesis of Compound 8-4

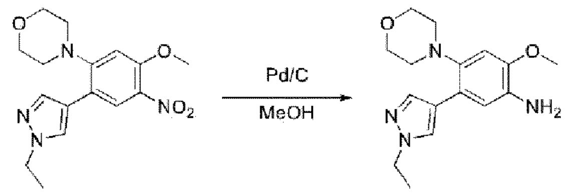


8-3 8-4

The synthesis method of compound 8-4 is the same as that of compound 1-11, except that the starting compound 1-10 is replaced by compound 8-3. MS of compound 8-4: 303.17 $[M+H]^+$

Step 4: Synthesis of Compound J-008

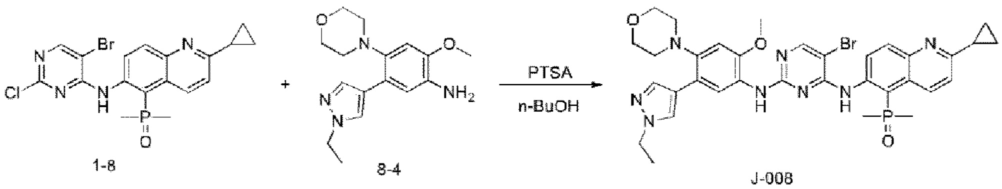


1-8 8-4 J-008

Compound 1-8 (149 mg), compound 8-4 (100 mg), p-toluenesulfonic acid (114 mg) and n-BuOH (2 mL) were successively added to a reaction flask and the temperature was raised to 110°C. The mixture was stirred under heating for 4 hours. It was monitored by LCMS until the reaction was completed and then the reaction was stopped. The reaction

solution was added with water (10 mL) and extracted with dichloromethane (3 × 10 mL). The organic phase was washed with saturated brine (3 × 10 mL), and dried with anhydrous sodium sulfate. The solvent was removed, and the residue was separated and purified by PTLC (dichloromethane: methanol = 15: 1) to obtain a brown solid compound J-008 (7.2 mg). MS: 717.20 [M+H]$^+$

**Example 9 Synthesis of compound J-009**

*(6-((5-Bromo-2-((4-(4-(dimethylamino)piperidin-1-yl)-5-methoxy-2-methylphenyl)amino)pyrimidine-4-yl)amino)-2-cyclopropylquinolin-5-yl)dimethylphosphine oxide*

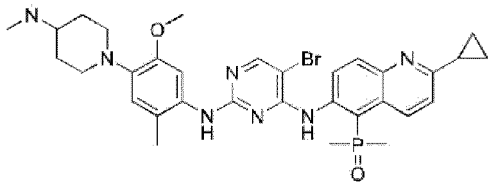

Step 1: Synthesis of Compound 9-2

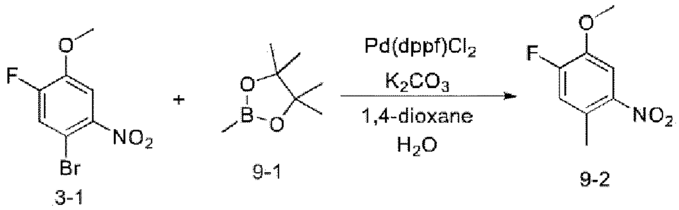


3-1 9-1 9-2

The synthesis method of compound 9-2 is the same as that of compound 3-2, except that the starting compound potassium trifluoro(vinyl)borate is replaced with compound 9-1.

Step 2: Synthesis of Compound 9-3

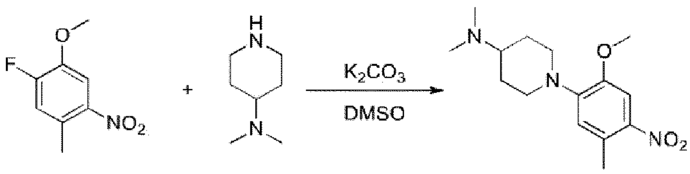


9-2 9-3

The synthesis method of compound 9-3 is the same as that of compound 1-10, except that the raw material compound 1-9 is replaced by compound 9-2, and the compound morpholine is replaced by 4-dimethylaminopiperidine. MS of compound 9-3: 294.17 [M+H]$^+$ Step 3: Synthesis of Compound 9-4

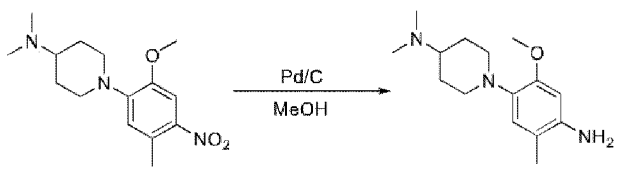


9-3 9-4

The synthesis method of compound 9-4 is the same as that of compound 1-11, except that the starting compound 1-10 is replaced by compound 9-3. MS of compound 9-4: 264.20 [M+H]+

Step 4: Synthesis of Compound J-009

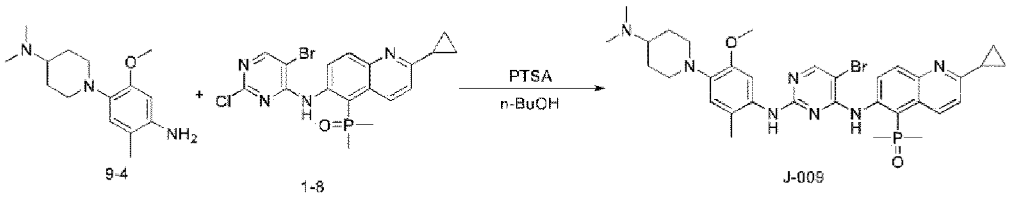


9-4 1-8 J-009

The synthesis method of compound J-009 is the same as that of compound J-001, except that the starting compound 1-11 is replaced by compound 9-4. MS of compound J-009: 678.22 [M+H]+

**Example 10 Synthesis of compound J-010**

*(6-((5-Bromo-2-((2-methoxy-5-methyl-4-(4-methylpiperazin-1-yl)phenyl)amino)pyrimidin-4-yl)amino)-2-cyclopropylquinolin-5-yl)dimethylphosphine oxide*

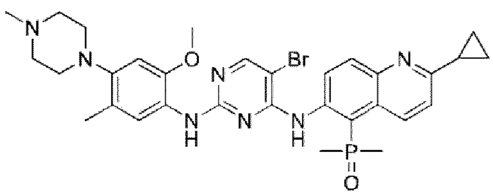

Step 1: Synthesis of Compound 10-2

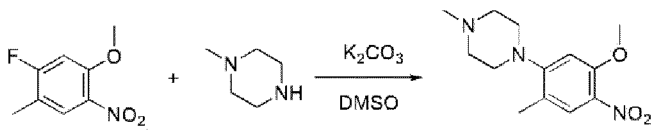


10-1 10-2

The synthesis method of compound 10-2 is the same as that of compound 1-10, except that the starting compound 1-9 is replaced by compound 10-1, and morpholine is replaced by N-methylpiperazine. MS of compound 10-2: 266.14 [M+H]+

Step 2: Synthesis of Compound 10-3

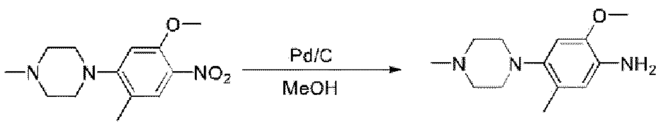


10-2 10-3

The synthesis method of compound 10-3 is the same as that of compound 1-11, except that the starting compound 1-10 is replaced by compound 10-2. MS of compound 10-3: 236.17 $[M+H]^+$

Step 3: Synthesis of Compound J-010

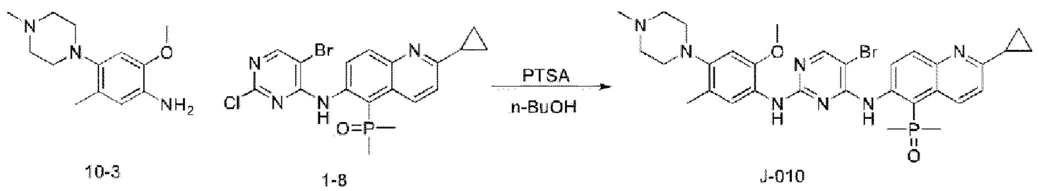


10-3 1-8 J-010

The synthesis method of compound J-010 is the same as that of compound J-001, except that the starting compound 1-11 is replaced by compound 10-3. MS of compound J-010: 650.19 $[M+H]^+$

**Example 11 Synthesis of compound J-011**

*(6-((5-Bromo-2-((5-ethyl-2-methoxy-4-(4-methylpiperazin-1-yl)phenyl)amino)pyrimidin-4-yl)amino)-2-cyclopropylquinolin-5-yl)dimethylphosphine oxide*

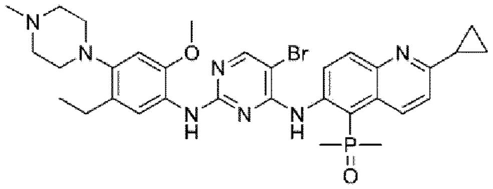

Step 1: Synthesis of Compound 11-2

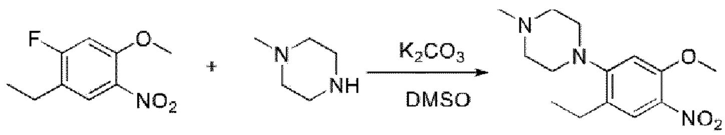


11-1 11-2

The synthesis method of compound 11-2 is the same as that of compound 10-2, except that the starting compound 10-1 is replaced by compound 11-1. MS of compound 11-2: 280.16 $[M+H]^+$

Step 2: Synthesis of Compound 11-3

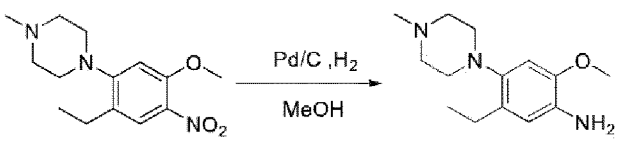


11-2 11-3

The synthesis method of compound 11-3 is the same as that of compound 1-11, except that the starting

compound 1-10 is replaced by compound 11-2. MS of compound 11-3: 250.18 $[M+H]^+$

Step 3: Synthesis of Compound J-011

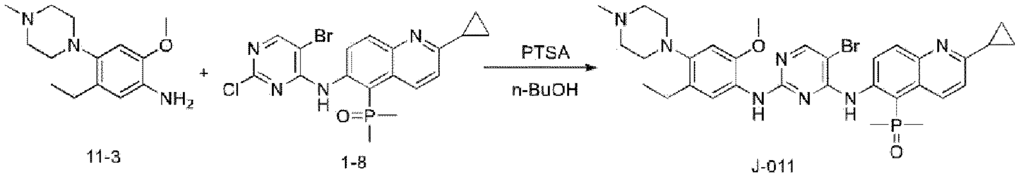


11-3 1-8 J-011

The synthesis method of compound J-011 is the same as that of compound J-001, except that the starting compound 1-11 is replaced by compound 11-3. MS of compound J-011: 664.21 $[M+H]^+$

**Example 12 Synthesis of compound J-012**

*5-((5-Bromo-4-((2-cyclopropyl-5-(dimethylphosphine oxide)quinolin-6-yl)amino)pyrimidin-2-yl)amino)-4-methoxy-2-(4-methylpiperazin-1-yl)benzonitrile*

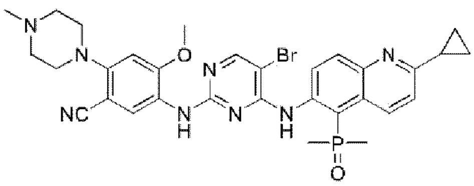

Step 1: Synthesis of Compound 12-1

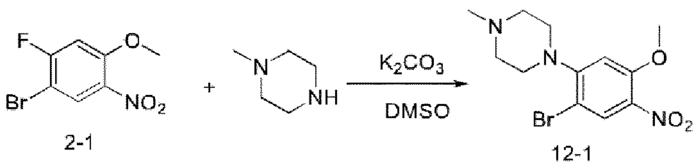


2-1 12-1

The synthesis method of compound 12-1 is the same as that of compound 2-2, except that morpholine is replaced by N-methylpiperazine. MS of compound 12-1: 330.04 $[M+H]^+$ Step 2: Synthesis of Compound 12-2

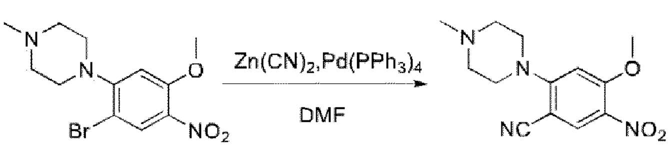


12-1 12-2

The synthesis method of compound 12-2 is the same as that of compound 2-3, except that the starting compound 2-2 is replaced by compound 12-1. MS of compound 12-2: 277.12 $[M+H]^+$

Step 3: Synthesis of Compound 12-3

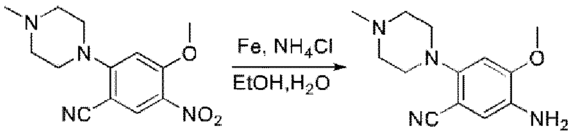


12-2 12-3

The synthesis method of compound 12-3 is the same as that of compound 2-4, except that the starting compound 2-3 is replaced by compound 12-2. MS of compound 12-3: 247.15 [M+H]+

Step 4: Synthesis of Compound J-012

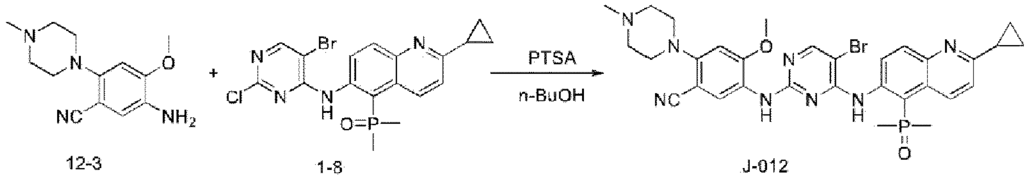


12-3 1-8 J-012

The synthesis method of compound J-012 is the same as that of compound J-001, except that the starting compound 1-11 is replaced by compound 12-3. MS of compound J-012: 661.17 [M+H]+

**Example 13 Synthesis of compound J-014**

*(6-((5-Bromo-2-((5-ethyl-2-methoxy-4-(4--yl)piperidin-1-yl)phenyl)amino)pyrimidin-4-yl)amino)-2-ethynylquinolin-5-yl)dimethylphosphine oxide*

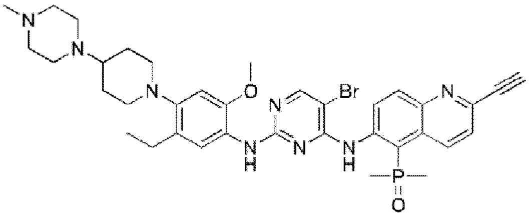

Step 1: Synthesis of Compound 13-1

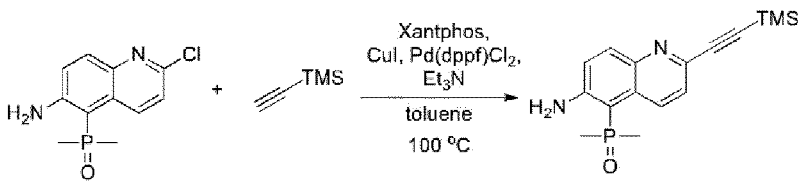


1-6 13-1

Compound 1-6 (500 mg) was dissolved in toluene (10 ml) in a reaction flask, followed by adding trimethylethynylsilicon (290 mg), Xantphos (227 mg), CuI (112 mg), Pd(dppf)$Cl_2$ (138 mg) and triethylamine (595 mg). The mixture was heated to 100° C under nitrogen protection and stirred overnight. It was monitored by LCMS until the reaction was completed. The reaction solution was cooled to room temperature and concentrated. The residue was mixed with silica gel and purified by flash silica gel column (DCM/MeOH=10/1, MeOH 0-5%, 20 minutes). The eluant was concentrated to obtain a solid compound 13-1 (390 mg, yield: 62.78%). MS: 317.12 [M+H]+

Step 2: Synthesis of Compound 13-2

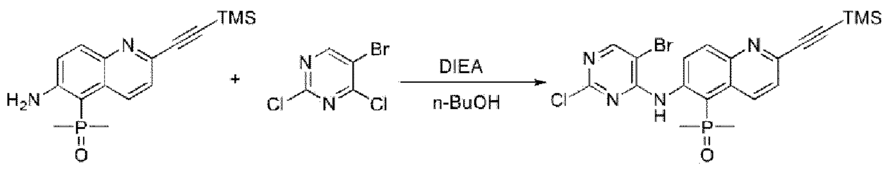


13-1 13-2

The synthesis method of compound 13-2 is the same as that of compound 1-8, except that the starting compound 1-7 is replaced by compound 13-1. MS of compound 13-2: 507.01 $[M+H]^+$

Step 3: Synthesis of Compound 13-4

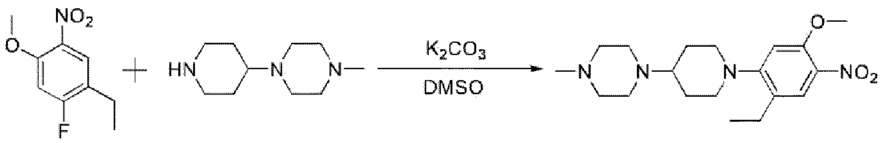


11-1 13-3 13-4

Compound 11-1 (3.00 g), compound 13-3 (4.14 g), $K_2CO_3$ (6.24 g) and DMSO (30 mL) were successively added to a reaction flask and the temperature was raised to 90°C. The mixture was stirred under heating for 12 hours. It was monitored by LCMS until the reaction was completed and then the reaction was stopped. The reaction solution was poured into water (100 mL) and filtered by suction. The filter cake was washed with water and dried to obtain a yellow solid compound 13-4 (4.20 g). MS: 363.23 $[M+H]^+$

Step 4: Synthesis of Compound 13-5

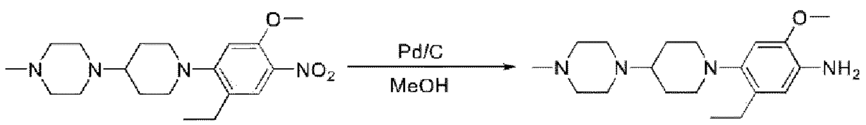


13-4 13-5

Compound 13-4 (4.20 g), Pd/C (1.00 g, 10%) and MeOH (60 mL) were successively added to a reaction flask which was then purged with $H_2$. The reaction solution was stirred at room temperature for 3 hours. It was monitored by LCMS until the reaction was completed and then the reaction was stopped. The reaction solution was filtrated by suction and washed with methanol (20 mL). The organic phase was collected and the solvent was removed to obtain a reddish-brown liquid compound 13-5 (3.5 g). MS: 333.26 $[M+H]^+$

Step 5: Synthesis of Compound 13-6

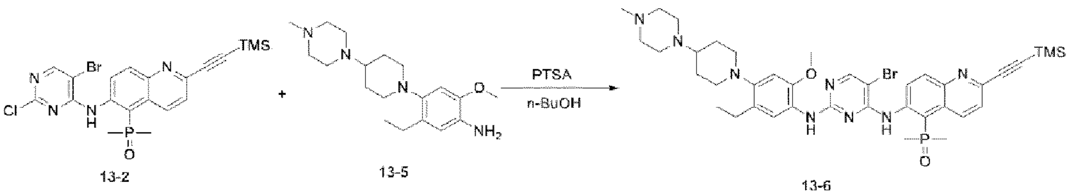


13-2 13-5 13-6

The synthesis method of compound 13-6 is the same as that of compound J-001, except that compound 1-11 is replaced by compound 13-5, and compound 1-8 is replaced by compound 13-2. MS of compound 13-6: 803.29 $[M+H]^+$

Step 6: Synthesis of Compound J-014

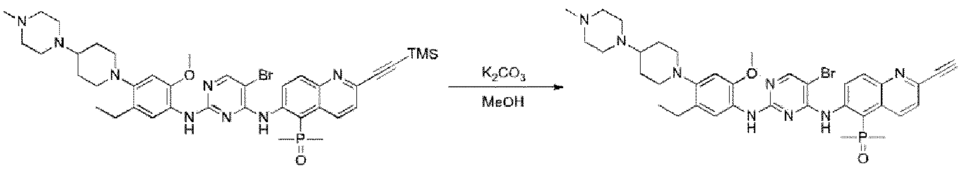


13-6 J-014

Compound 13-6 (44 mg, 54.74 μmol) was dissolved in anhydrous methanol (5 mL) in a reaction flask, followed by adding solid potassium carbonate (23 mg, 166.42 μmol). The reaction solution was stirred at room temperature for 1 hour. It was monitored by LCMS until the reaction was completed. DCM and water were added to the reaction solution and the layers were separated by stirring. The organic phase was washed once with water, dried, filtered, and concentrated. The residue was dissolved in a small amount of dichloromethane and purified by PTLC (DCM/MeOH=10/1) to obtain compound J-014 (18.3 mg, 25.01 μmol, yield: 45.69%). MS: 731.25 $[M+H]^+$

**Example 14 Synthesis of compound J-013**

*(6-((5-Bromo-2-((2-methoxy-5-methyl-4-(4-(4-methylpiperazin-1-yl)piperidin-1-yl)phenyl)amino)pyrimidin-4-yl)amino)-2-ethynylquinolin-5-yl)dimethylphosphine oxide*

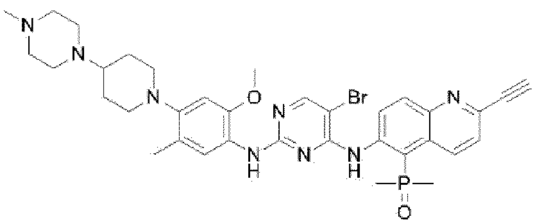

Step 1: Synthesis of Compound 14-1

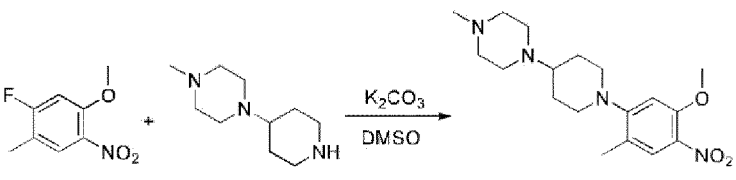


10-1 13-3 14-1

Compound 10-1 (1 g, 5.40 mmol), compound 13-3 (1.19 g, 6.48 mmol), $K_2CO_3$ (1.49 g, 10.8 mmol) and DMSO (10 mL) were successively added to the reaction flask and the temperature was raised to 90°C. The mixture was stirred under heating overnight. It was monitored by LCMS until the reaction was completed and then the reaction was stopped. The reaction solution was added with 50 mL of DCM, washed with water (100 × 2 mL), washed with 100 mL of saturated brine, dried with anhydrous magnesium sulfate, concentrated, slurried with diethyl ether, filtered by suction, and dried to obtain a yellow solid compound 14-1 (1.61 g, 4.62 mmol). MS: 349.22 $[M+H]^+$

Step 2: Synthesis of Compound 14-2

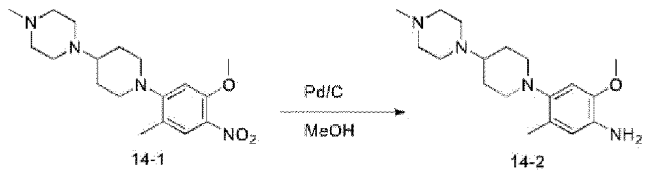


14-1 14-2

Compound 14-1 (1.61 g, 4.62 mmol), Pd/C (0.5 g, 10%) and MeOH (30 mL) were successively added to a reaction flask, which was then purged with $H_2$. The reaction solution was stirred at room temperature for 3 hours. It was monitored by LCMS until the reaction was completed and then the reaction was stopped. The reaction solution was filtrated by suction and washed with methanol (20 mL). The organic phase was collected and the solvent was removed to obtain target product compound 14-2 (1.3 g, 4.08 mmol). MS: 319.24 $[M+H]^+$

Step 3: Synthesis of Compound 14-3

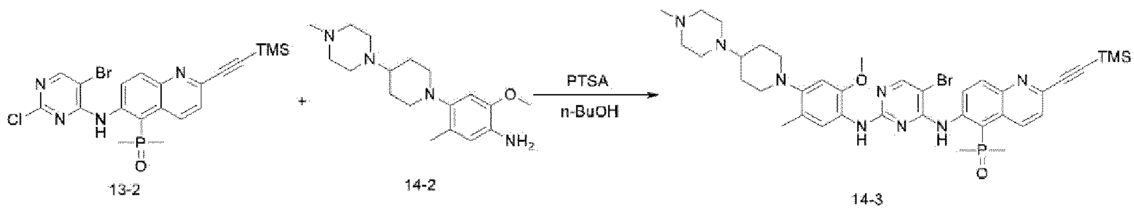


13-2 14-2 14-3

The synthesis method of compound 14-3 is the same as that of compound 13-6, except that the starting compound 13-5 is replaced by compound 14-2. MS of compound 14-3: 789.27 $[M+H]^+$

Step 4: Synthesis of Compound J-013

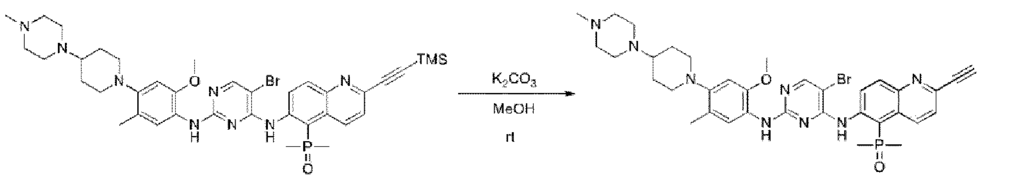


14-3 J-013

Compound 14-3 (32 mg, 40.52 μmol) was dissolved in anhydrous methanol (5 mL) in the reaction flask, followed by adding $K_2CO_3$ (17 mg, 123.01 μmol). The reaction solution was stirred at room temperature for 1 hour. It was monitored by LCMS until the reaction was completed. DCM and water were added to the reaction solution and the layers were separated by stirring. The organic phase was washed once with water, dried, filtered, and concentrated. The residue was dissolved in a small amount of dichloromethane and purified by PTLC (DCM/MeOH=10/1) to obtain compound J-013 (8.4 mg, 11.71 μmol, yield: 28.89%). MS: 717.24 $[M+H]^+$

**Example 15 Synthesis of compound J-015**

*(6-((5-Bromo-2-((5-methoxy-2-methyl-4-(4-(4-methylpiperazin-1-yl)piperidin-1-yl)phenyl)amino)pyrimidin-4-yl)amino)-2-ethynylquinolin-5-yl)dimethylphosphine oxide*

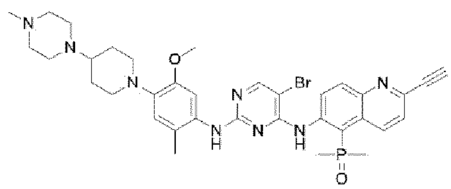

Step 1: Synthesis of Compound 15-1

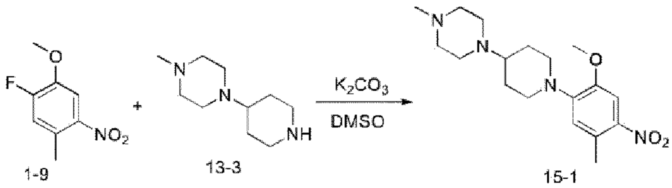


1-9 13-3 15-1

The synthesis method of compound 15-1 is the same as that of compound 1-10, except that the starting compound morpholine is replaced by compound 13-3. MS of compound 15-1: 349.22 $[M+H]^+$

Step 2: Synthesis of Compound 15-2

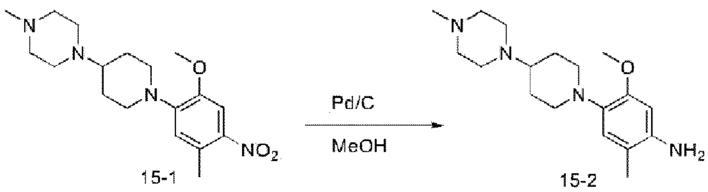


15-1 15-2

The synthesis method of compound 15-2 is the same as that of compound 1-11, except that the starting compound 1-10 is replaced by compound 15-1. MS of compound 15-2: 319.24 $[M+H]^+$

Step 3: Synthesis of Compound 15-3

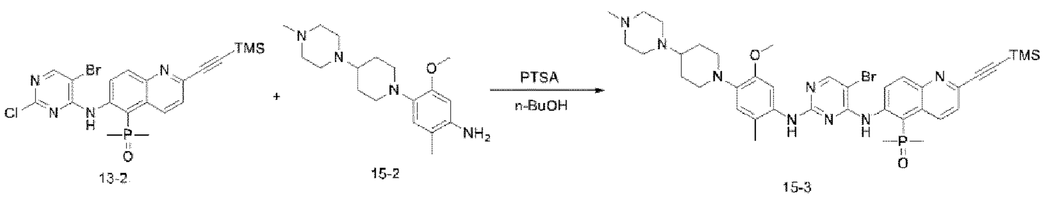


13-2 15-2 15-3

The synthesis method of compound 15-3 is the same as that of compound 13-6, except that the starting compound 13-5 is replaced by compound 15-2. MS of compound 15-3: 789.27 $[M+H]^+$

Step 4: Synthesis of Compound J-015

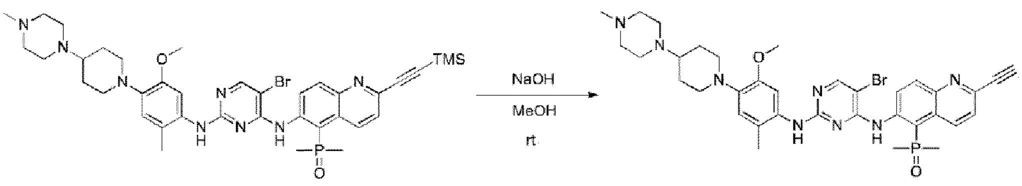


15-3 J-015

The synthesis method of compound J-015 is the same as that of compound J-014, except that the starting compound 13-6 is replaced by compound 15-3. MS of compound J-015: 717.24 $[M+H]^+$

**Example 16 Synthesis of compound J-016**

*6-((5-Bromo-2-((5-ethyl-2-methoxy-4-(4-(4-methylpiperazin-1-yl)piperidin-1-yl)phenyl)amino)pyrimidin-4-yl)amino)-5-(dimethylphosphine oxide)quinoline-2-formonitrile*

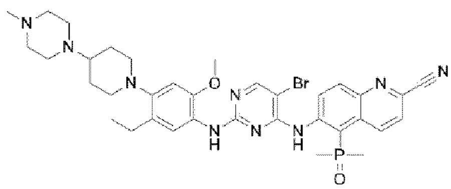

Step 1: Synthesis of Compound 16-1

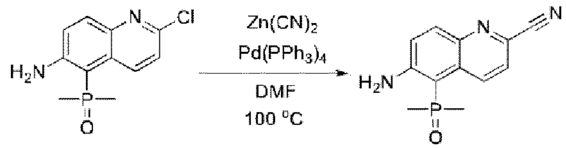


1-6 16-1

Compound 1-6 (600 mg, 2.36 mmol) was dissolved in N,N-dimethylformamide (5 mL) in a reaction flask, followed by adding zinc cyanide (552.00 mg, 4.70 mmol), $Pd(PPh_3)_4$ (272.00 mg, 235.38 μmol) successively. The mixture was heated under microwave to 100°C under nitrogen protection and stirred for 0.5 hour. It was monitored by LCMS until the reaction was completed. The reaction solution was cooled to room temperature, diluted with water, and extracted twice with DCM. The organic phases were combined, washed three times with water, then washed once with saturated aqueous sodium chloride solution, dried, and filtered. The filtrate was concentrated and mixed with silica gel and purified by Flash silica gel column (DCM/MeOH, MeOH 0-10%, 20 min). The eluant was concentrated to obtain a solid compound 16-1 (300 mg, 1.22 mmol, yield: 51.92%). MS: 246.07 $[M+H]^+$

Step 2: Synthesis of Compound 16-2

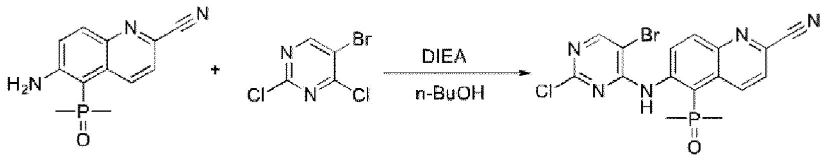


16-1

16-2

The synthesis method of compound 16-2 is the same as that of compound 1-8, except that the starting compound 1-7 is replaced by compound 16-1. MS of compound 16-2: 435.97 $[M+H]^+$

Step 3: Synthesis of Compound J-016

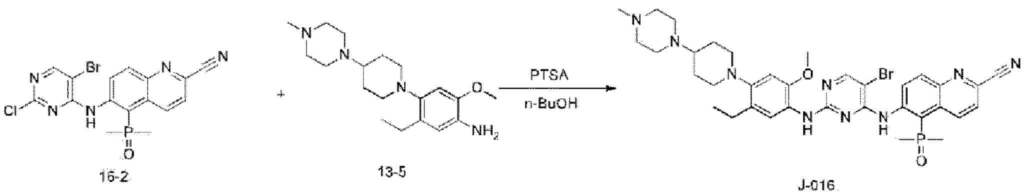


16-2

13-5

J-016

The synthesis method of compound J-016 is the same as that of compound 13-6, except that the starting compound 13-2 is replaced by compound 16-2. MS of compound J-016: 732.25 $[M+H]^+$

**Example 17 Synthesis of compound J-017**

*(6-((5-Bromo-2-((5-ethyl-2-methoxy-4-(4-(4-methylpiperazin-1-yl)piperidin-1-yl)phenyl)amino)pyrimidin-4-yl)amino)-2-(1-methyl-1H-pyrazol-4-yl)quinolin-5-yl)dimethylphosphine oxide*

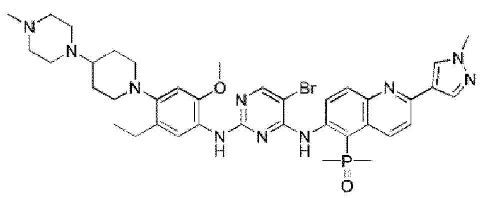

Step 1: Synthesis of Compound J-017

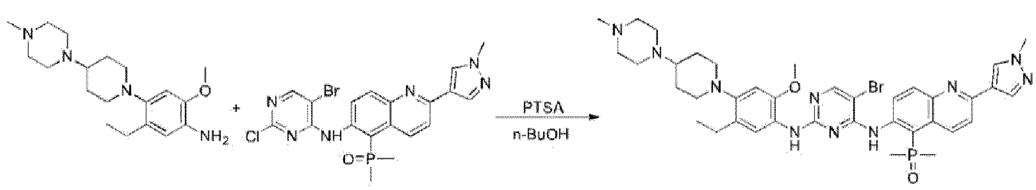


13-5

7-3

J-017

Compound 13-5 (135.24 mg, 406.75 μmol), compound 7-3 (200 mg, 406.75 μmol), p-toluenesulfonic acid (104.95 mg, 610.12 μmol) and n-BuOH (8 mL) were successively added to a reaction flask and the mixture was stirred at 120°C overnight. It was monitored by LCMS until the reaction was completed. The reaction solution was cooled to room temperature, spin-dried, added with saturated aqueous $K_2CO_3$ solution (10 mL), extracted with dichloromethane (3 × 10 mL), dried with anhydrous sodium sulfate, filtered and spin-dried. The crude product was purified by column

chromatography (dichloromethane: methanol=8:1) to obtain compound J-017 (98.6 mg, 125.17 μmol, yield: 30.77%). MS: 787.29 $[M+H]^+$

**Example 18 Synthesis of compound J-018**

*(6-((5-Bromo-2-((5-ethyl-2-methoxy-4-(4-1-yl)piperidin-1-yl)phenyl)amino)pyrimidin-4-yl)amino)-2-cyclopropylquinolin-5-yl)dimethylphosphine oxide*

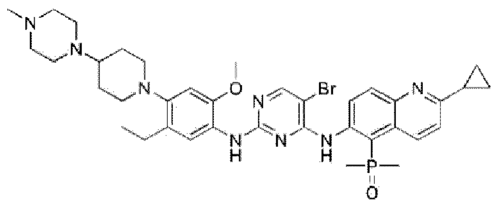

Step 1: Synthesis of Compound J-018

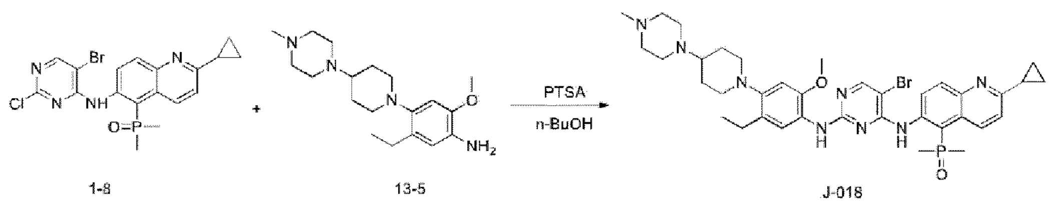


1-8 13-5 J-018

Compound 13-5 (80.0 mg, 240.61 μmol), compound 1-8 (119.55 mg, 264.68 μmol), p-toluenesulfonic acid (82.87 mg, 481.23 μmol) and n-BuOH (2 mL) were successively added to a reaction flask. The mixture was heated to 110°C and stirred overnight. It was monitored by LCMS until the reaction was completed. The reaction solution was cooled to room temperature, spin-dried, added with saturated aqueous $Na_2CO_3$ solution (10 mL), extracted with dichloromethane (3 × 10 mL), dried with anhydrous sodium sulfate, filtered and spin-dried. The crude product was purified by PTLC (dichloromethane: methanol=10:1) to obtain compound J-018 (98 mg, 131.07 μmol, yield: 54.47%). MS: 747.28 $[M+H]^+$

$^1H$ NMR (500 MHz, DMSO-$d_6$) δ 11.76 (s, 1H), 8.49 (d, J = 8.8 Hz, 1H), 8.20 (s, 2H), 8.06 (d, J = 9.7 Hz, 1H), 7.74 (d, J = 9.2 Hz, 1H), 7.44 (d, J = 8.9 Hz, 1H), 7.27 (s, 1H), 6.76 (s, 1H), 3.75 (s, 3H), 2.98-2.91 (m, 2H), 2.71-2.52 (m, 5H), 2.49-2.12 (m, 12H), 1.98 (s, 3H), 1.96 (s, 3H) 1.89-1.79 (m, 2H), 1.61-1.43 (m, 2H), 1.24-1.19 (m, 3H), 1.09-1.01 (m, 4H).

**Example 19 Synthesis of compound J-019**

*(6-((5-Bromo-2-((5-fluoro-2-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide*

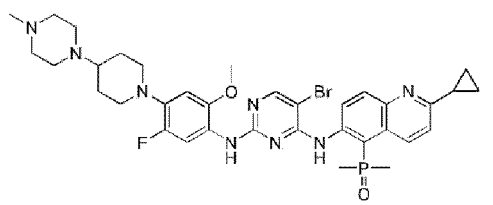

Step 1: Synthesis of Compound 19-2

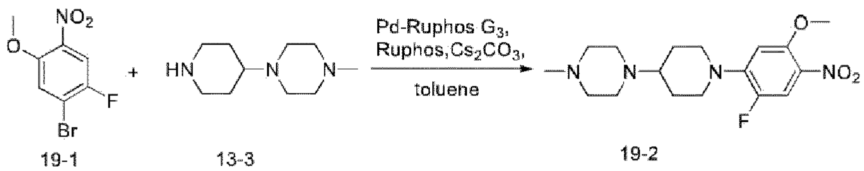


19-1 13-3 19-2

Compound 19-1 (500 mg, 2.00 mmol), compound 13-3 (439.86 mg, 2.40 mmol), Pd-Ruphos $G_3$ (186.64 mg, 400 μmol), Ruphos (167.46 mg, 200 μmol), $Cs_2CO_3$ (1.23 g, 4.00 mmol) and toluene (15 mL) were successively added to a reaction flask. The mixture was heated to 100°C and stirred for 5 hours. It was monitored by LCMS until the reaction was completed and then the reaction was stopped. The reaction solution was cooled to room temperature, filtered by suction, spin-dried, and purified with silica gel column (dichloromethane: methanol=15:1) to obtain a yellow solid compound 19-2 (4.20 g). MS: 353.19 $[M+H]^+$

Step 2: Synthesis of Compound 19-3

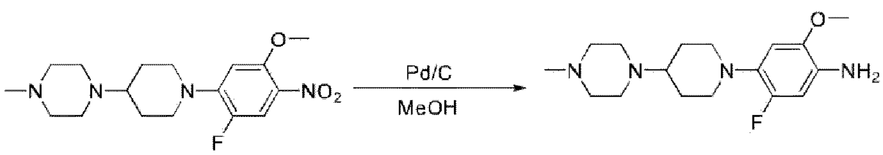


19-2 19-3

The synthesis method of compound 19-3 is the same as that of compound 1-11, except that the starting compound 1-10 is replaced by compound 19-2. MS of compound 19-3: 323.22 $[M+H]^+$

Step 3: Synthesis of Compound J-019

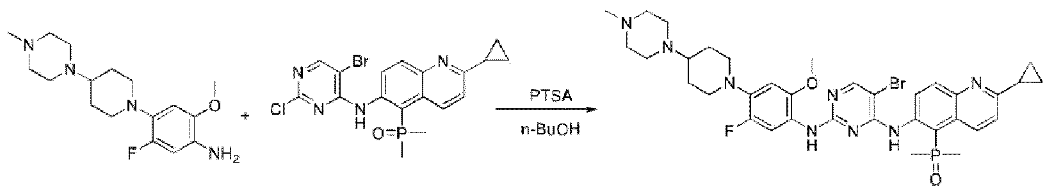


19-3 1-8 J-019

The synthesis method of compound J-019 is the same as that of compound J-001, except that the starting compound 1-11 is replaced by compound 19-3. MS of compound J-019: 737.24 $[M+H]^+$

**Example 20 Synthesis of compound J-020**

*(6-((5-Bromo-2-((2,5-dimethoxy-4-(4-(4-methylpiperazin-1-yl)piperidin-1-yl)phenyl)amino)pyrimidin-4-yl)amino)-2-cyclopropylquinolin-5-yl)dimethylphosphine oxide*

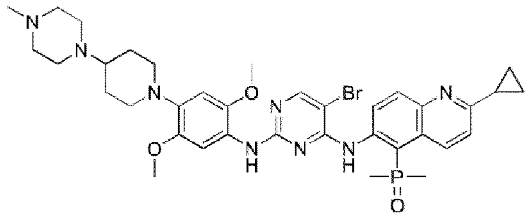

Step 1: Synthesis of Compound 20-2

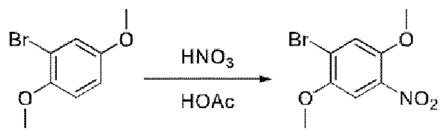


20-1 20-2

Compound 20-1 (5.0 g, 23.04 mmol) was dissolved in HOAc (15 mL), and $HNO_3$ (2 mL) was slowly added dropwise under an ice-water bath. The reaction was carried out at room temperature for 15 minutes. The reaction solution was poured into 40 mL of ice-water and a solid was precipitated and filtered. The filter cake was washed with 10 mL of water for 3 times, washed with 5 mL of diethyl ether, and dried to obtain a white solid compound 20-2 (5 g, 19.08 mmol, yield: 82.83%).

Step 2: Synthesis of Compound 20-3

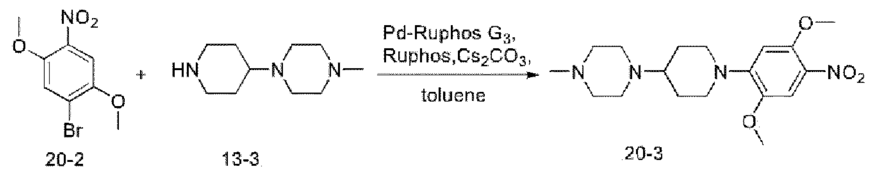


20-2 13-3 20-3

The synthesis method of compound 20-3 is the same as that of compound 19-2, except that the starting compound 19-1 is replaced by compound 20-2. MS of compound 20-3: 365.21 $[M+H]^+$

Step 3: Synthesis of Compound 20-4

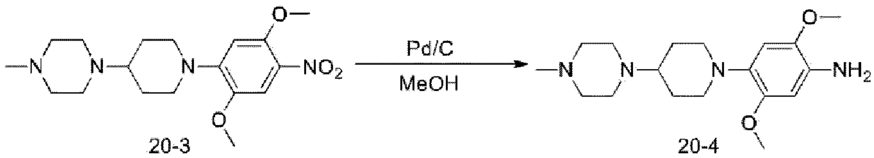


20-3 20-4

The synthesis method of compound 20-4 is the same as that of compound 1-11, except that the starting compound 1-10 is replaced by compound 20-3. MS of compound 20-4: 335.24 $[M+H]^+$

Step 4: Synthesis of Compound J-020

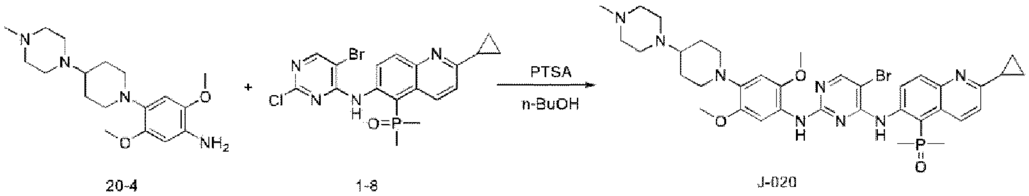


20-4 1-8 J-020

The synthesis method of compound J-020 is the same as that of compound J-001, except that the starting compound 1-11 is replaced by compound 20-4. MS of compound J-020: 749.26 $[M+H]^+$

**Example 21 Synthesis of compound J-021**

*5-((5-Bromo-4-((2-cyclopropyl-5-(dimethylphosphine oxide)quinolin-6-yl)amino)pyrimidin-2-yl)amino)-4-methoxy-2-(4-(4-methylpiperazin-1-yl)piperidin-1-yl)benzonitrile*

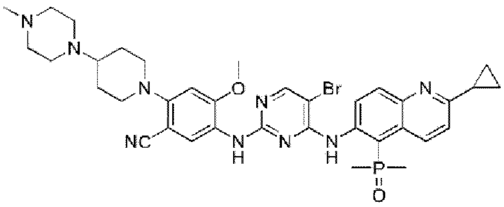

Step 1: Synthesis of Compound 21-1

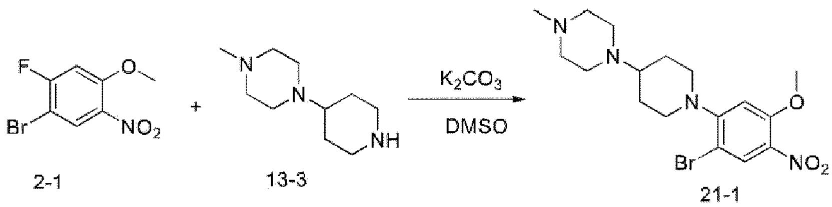


2-1 13-3 21-1

The synthesis method of compound 21-1 is the same as that of compound 2-2, except that the starting compound morpholine is replaced by compound 13-3. MS of compound 21-1: 413.11 $[M+H]^+$

Step 2: Synthesis of Compound 21-2

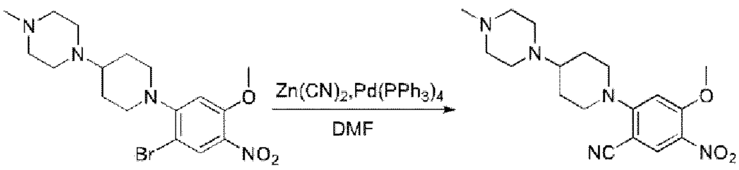


21-1 21-2

The synthesis method of compound 21-2 is the same as that of compound 2-3, except that the compound 2-2 is replaced with compound 21-1. MS of compound 21-2: 360.20 $[M+H]^+$

Step 3: Synthesis of Compound 21-3

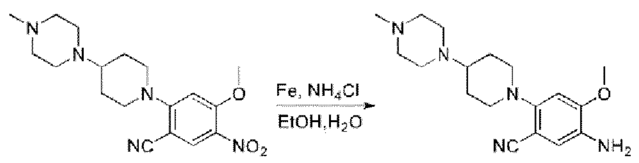


21-2 21-3

The synthesis method of compound 21-3 is the same as that of compound 2-4, except that the compound 2-3 is replaced with compound 21-2. MS of compound 21-3: 330.22 $[M+H]^+$

Step 4: Synthesis of Compound J-021

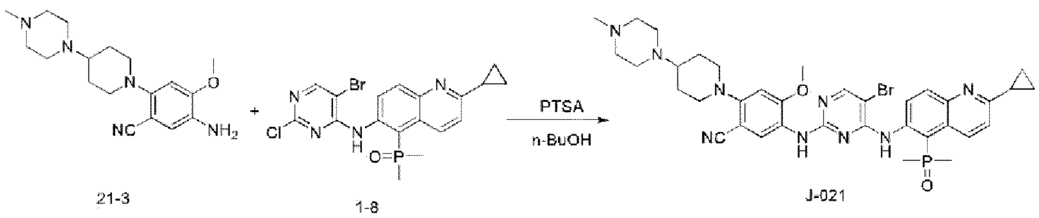


21-3 1-8 J-021

The synthesis method of compound J-021 is the same as that of compound J-001, except that the starting compound 1-11 is replaced by compound 21-3. MS of compound J-021: 744.25 $[M+H]^+$

**Example 22 Synthesis of compound J-022**

*(6-((5-Bromo-2-((2-methoxy-5-methyl-4-(4-(4-methylpiperazin-1-yl)piperidin-1-yl)phenyl)amino)pyrimidin-4-yl)amino)-2-cyclopropylquinolin-5-yl)dimethylphosphine oxide*

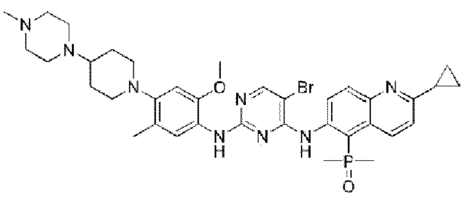

Step 1: Synthesis of Compound J-022

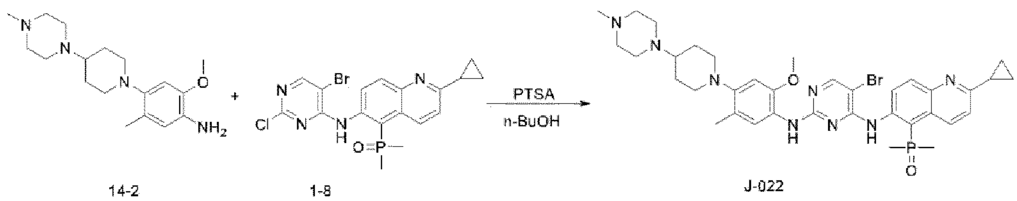


14-2 1-8 J-022

Compound 14-2 (67.05 mg, 210.54 μmol), compound 1-8 (104.61 mg, 231.59 μmol), p-toluenesulfonic acid

(65.26 mg, 378.97 μmol) and n-BuOH (2 mL) were successively added to a reaction flask. The mixture was heated to 100°C and stirred overnight. It was monitored by LCMS until the reaction was completed. The reaction solution was cooled to room temperature, spin-dried, added with saturated aqueous $Na_2CO_3$ solution (10 mL), extracted with dichloromethane (3 × 10 mL), dried with anhydrous sodium sulfate, filtered, and spin-dried. The crude product was purified by PTLC (dichloromethane: methanol=10:1) to obtain compound J-022 (24 mg, 32.71 μmol, yield: 15.54%). MS: 733.27 $[M+H]^+$

$^1$H-NMR(500 MHz, DMSO-$d_6$): δ 11.88 (s, 1H), 8.44 (d, J = 8.5 Hz, 1H), 8.27-8.26 (m, 1H), 8.19 (s, 1H), 8.03 (s, 1H), 7.75 (d, J = 9,0 Hz, 1H), 7.42 (d, J = 8.5 Hz, 1H), 7.24 (br, 1H), 6.69 (s, 1H), 3.75 (s, 3H), 3.02-3.00 (m, 2H), 2.63-2.59 (m, 2H), 2.51-2.50 (m, 4H), 2.30 - 2.26 (m, 6H), 2.14 (s, 3H), 1.98 (d, J = 13.5 Hz, 6H), 1.90 (s, 3H), 1.84-1.82 (m, 2H) 1.55-1.52 (m, 2H), 1.06-1.05 (m, 4H).

**Example 23 Synthesis of compound J-023**

*(6-((5-Bromo-2-((5-methoxy-2-methyl-4-(4-(4-methylpiperazin-1-yl)piperidin-1-yl)phenyl)amino)pyrimidin-4-yl)amino)-2-cyclopropylquinolin-5-yl)dimethylphosphine oxide*

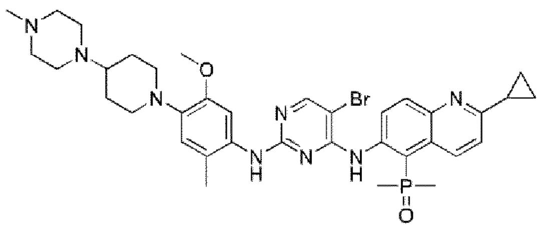

Step 1: Synthesis of Compound J-023

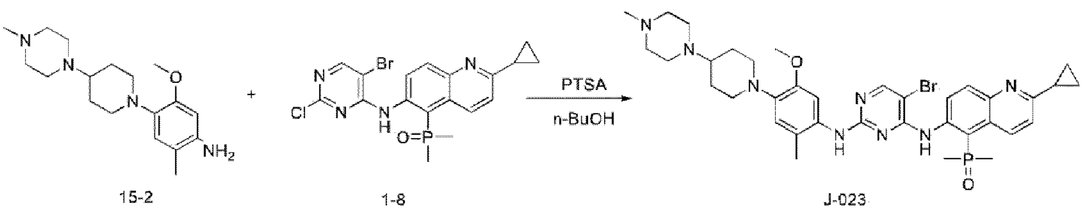


15-2 1-8 J-023

The synthesis method of compound J-023 is the same as that of compound J-001, except that the starting compound 1-11 is replaced by compound 15-2. MS of compound J-023: 733.27 $[M+H]^+$

**Example 24 Synthesis of compound J-024**

*(6-((5-Bromo-2-((2-cyclopropyl-5-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide*

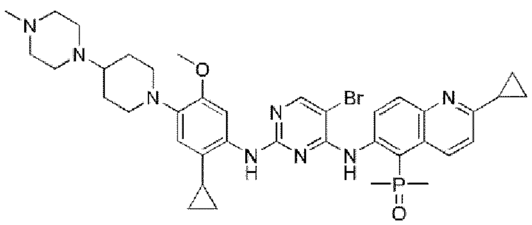

Step 1: Synthesis of Compound 24-1

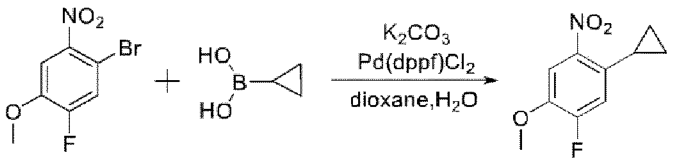


3-1 24-1

Compound 3-1 (2.00 g, 8.00 mmol), cyclopropylboronic acid (3.44 g, 39.97 mmol), Pd(dppf)$Cl_2$ (0.58 g, 0.80 mmol), $K_2CO_3$ (2.21 g, 15.99 mmol) were added to a mixed solvent of dioxane (20 mL) and $H_2O$ (4 mL). The mixture was heated to 100°C under nitrogen protection and stirred for 12 hours. It was monitored by TLC until the reaction was completed and the reaction solution was cooled to room temperature. The reaction solution was added with water (30 mL) and extracted with ethyl acetate (3 × 30 mL). The organic phase was washed with saturated brine (3 × 30 mL), dried with anhydrous sodium sulfate, and purified by column chromatography (n-hexane: ethyl acetate=15 :1). Pale yellow solid compound 24-1 (1.2 g) was obtained.

Step 2: Synthesis of Compound 24-2

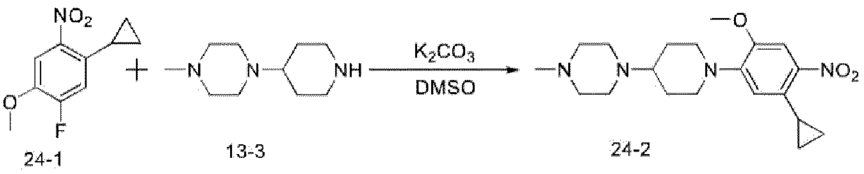


24-1 13-3 24-2

The synthesis method of compound 24-2 is the same as that of compound 13-4, except that the starting compound 11-1 is replaced by compound 24-1. MS of compound 24-2: 375.23 $[M+H]^+$

Step 3: Synthesis of Compound 24-3

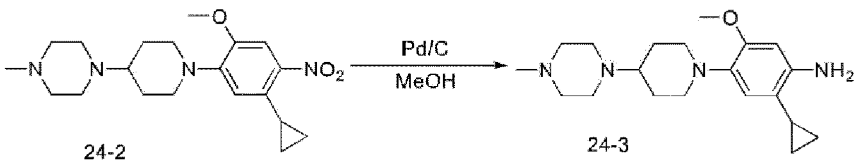


24-2 24-3

The synthesis method of compound 24-3 is the same as that of compound 1-11, except that the starting compound 1-10 is replaced by compound 24-2. MS of compound 24-3: 345.26 $[M+H]^+$

Step 4: Synthesis of Compound J-024

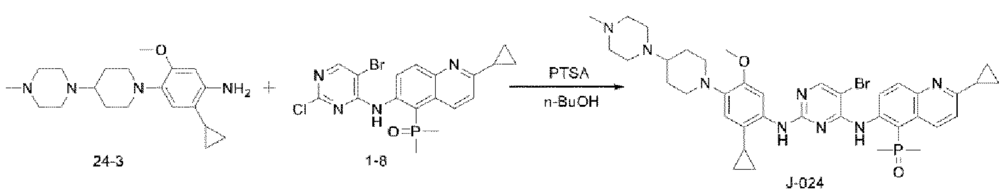


24-3 1-8 J-024

The synthesis method of compound J-024 is the same as that of compound J-001, except that the starting compound 1-11 is replaced by compound 24-3. MS of compound J-024: 759.28 [M+H]$^+$

**Example 25 Synthesis of compound J-025**

*2-((5-Bromo-4-((2-cyclopropyl-5-(dimethylphosphine oxide)quinolin-6-yl)amino)pyrimidin-2-yl)amino)-4-methoxy-5-(4-(4-methylpiperazin-1-yl)piperidin-1-yl)benzonitrile*

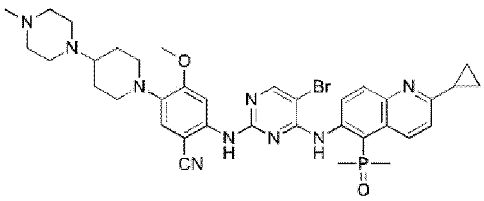

Step 1: Synthesis of Compound 25-1

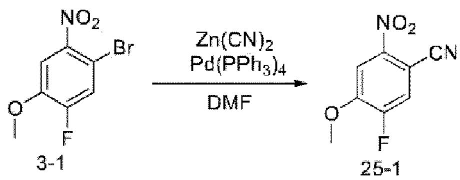


3-1 25-1

The synthesis method of compound 25-1 is the same as that of compound 2-3, except that the starting compound 2-2 is replaced by compound 3-1.

Step 2: Synthesis of Compound 25-2

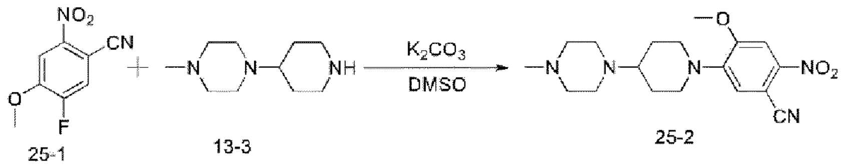


25-1 13-3 25-2

The synthesis method of compound 25-2 is the same as that of compound 13-4, except that the starting compound 11-1 is replaced by compound 25-1. MS of compound 25-2: 360.20 [M+H]$^+$

Step 3: Synthesis of Compound 25-3

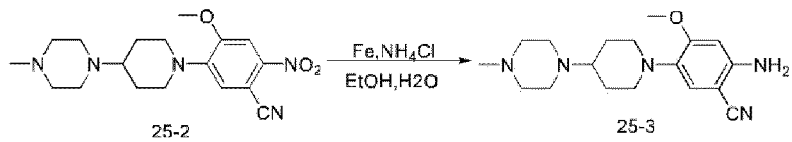


25-2 25-3

The synthesis method of compound 25-3 is the same as that of compound 2-4, except that the starting compound 2-3 is replaced by compound 25-2. MS of compound 25-3: 330.22 [M+H]$^+$

Step 4: Synthesis of Compound J-025

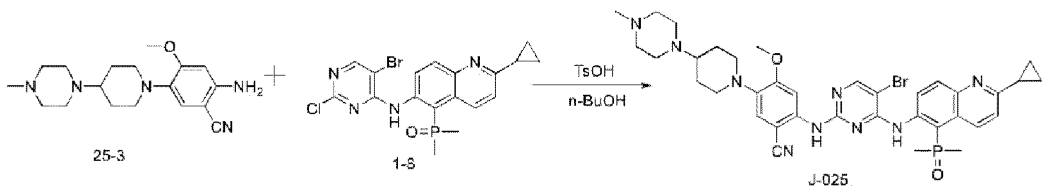


25-3 1-8 J-025

The synthesis method of compound J-025 is the same as that of compound J-001, except that the starting compound 1-11 is replaced by compound 25-3. MS of compound J-025: 744.25 $[M+H]^+$

**Example 26 Synthesis of compound J-026**

*(6-((5-Bromo-2-((5-methoxy-4-(4-(4-methylpiperazin-1-yl)piperidin-1-yl)-2-(tetrahydro-2H-pyran-4-yl)phenyl)amino)pyrimidin-4-yl)amino)-2-cyclopropylquinolin-5-yl)dimethylphosphine oxide*

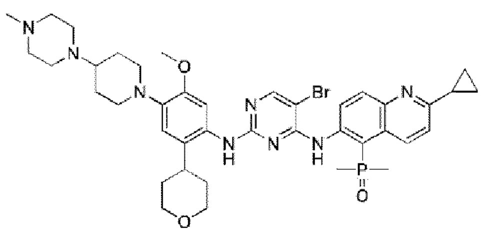

Step 1: Synthesis of Compound 26-1

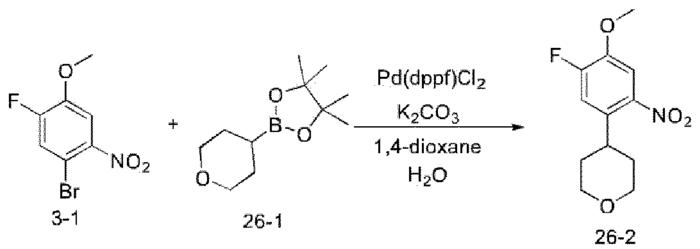


3-1 26-1 26-2

The synthesis method of compound 26-2 is the same as that of compound 24-1, except that the starting compound cyclopropylboronic acid is replaced by compound 26-1. MS of compound 26-2: 256.09 $[M+H]^+$

Step 2: Synthesis of Compound 26-3

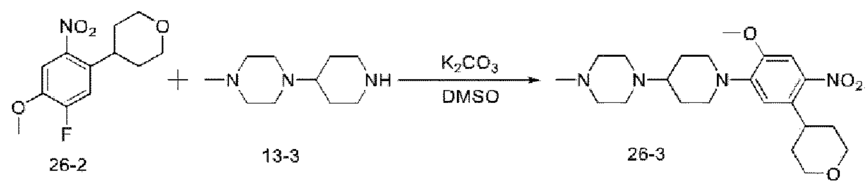


26-2 13-3 26-3

The synthesis method of compound 26-3 is the same as that of compound 13-4, except that the starting

compound 11-1 is replaced by compound 26-2. MS of compound 26-3: 419.26 $[M+H]^+$

Step 3: Synthesis of Compound 26-4

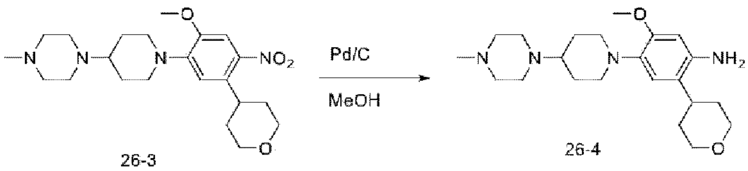


26-3 26-4

The synthesis method of compound 26-4 is the same as that of compound 1-11, except that the starting compound 1-10 is replaced by compound 26-3. MS of compound 26-4: 389.28 $[M+H]^+$

Step 4: Synthesis of Compound J-026

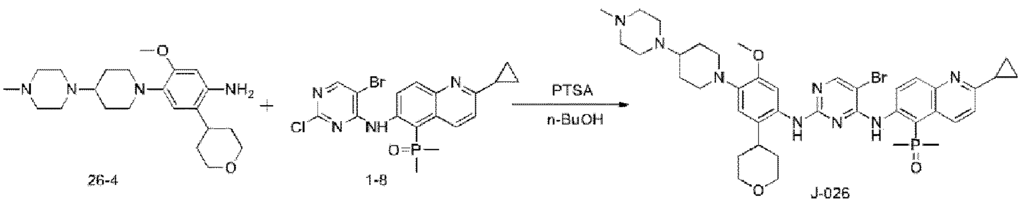


26-4 1-8 J-026

The synthesis method of compound J-026 is the same as that of compound J-001, except that the starting compound 1-11 is replaced by compound 26-4. MS of compound J-026: 803.31 $[M+H]^+$

**Example 27 Synthesis of compound J-027**

*(6-((5-Bromo-2-((5-ethyl-2-methyl-4-(4-(4-methylpiperazin-1-yl)piperidin-1-yl)phenyl)amino)pyrimidin-4-yl)amino)-2-cyclopropylquinolin-5-yl)dimethylphosphine oxide*

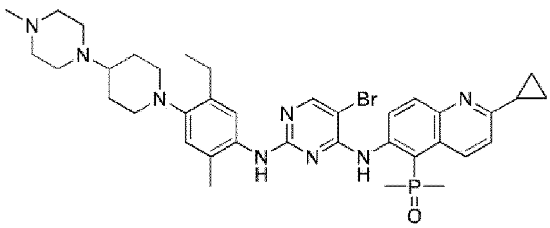

Step 1: Synthesis of Compound 27-2

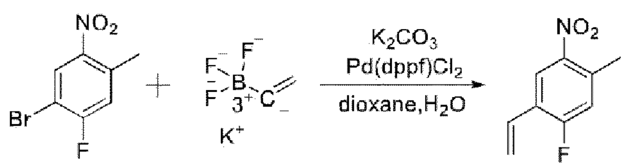


27-1 27-2

The synthesis method of compound 27-2 is the same as that of compound 3-2, except that the starting compound 3-1 is replaced by compound 27-1.

Step 2: Synthesis of Compound 27-3

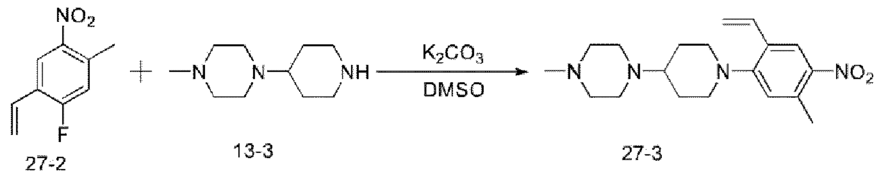


27-2 13-3 27-3

The synthesis method of compound 27-3 is the same as that of compound 13-4, except that the starting compound 11-1 is replaced by compound 27-2. MS of compound 27-3: 345.22 [M+H]$^+$

Step 3: Synthesis of Compound 27-4

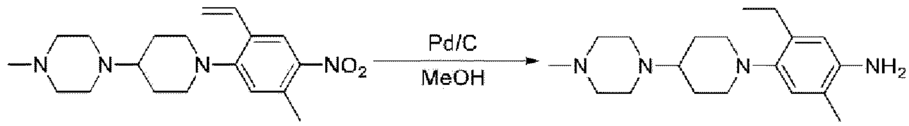


27-3 27-4

The synthesis method of compound 27-4 is the same as that of compound 1-11, except that the starting compound 1-10 is replaced by compound 27-3. MS of compound 27-3: 317.26 [M+H]$^+$

Step 4: Synthesis of Compound J-027

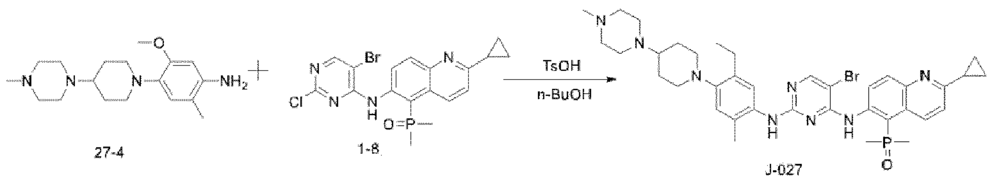


27-4 1-8 J-027

The synthesis method of compound J-027 is the same as that of compound J-001, except that the starting compound 1-11 is replaced by compound 27-4. MS of compound J-027: 731.29 [M+H]$^+$

**Example 28 Synthesis of compound J-028**

*(6-((5-Bromo-2-((2-methoxy-5-methyl-4-(4-(4-methylpiperazin-1-yl)piperidin-1-yl)phenyl)amino)pyrimidin-4-yl)amino)-2-ethylquinolin-5-yl)dimethylphosphine oxide*

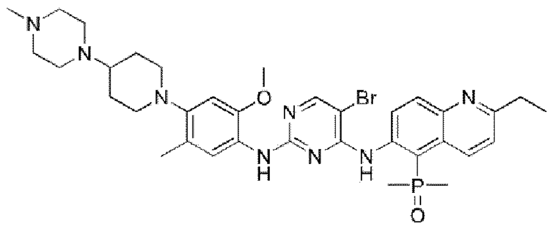

Step 1: Synthesis of Compound 28-1

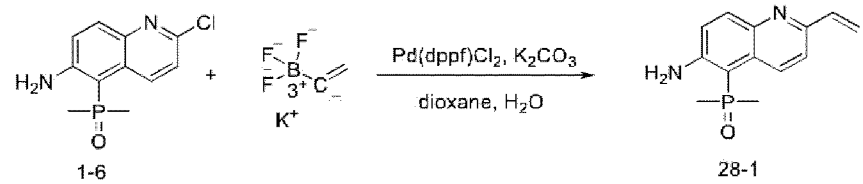


The synthesis method of compound 28-1 is the same as that of compound 7-2, except that compound 7-1 is replaced with potassium trifluoro(vinyl)borate. MS of compound 28-1: 247.09 $[M+H]^+$

Step 2: Synthesis of Compound 28-2

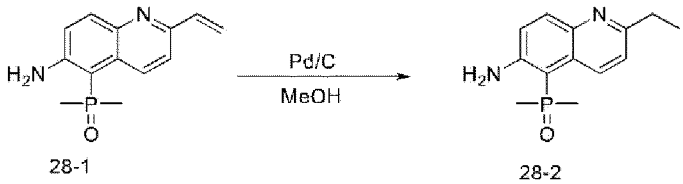


The synthesis method of compound 28-2 is the same as that of compound 1-11, except that the starting compound 1-10 is replaced by compound 28-1. MS of compound 28-2: 249.11 $[M+H]^+$

Step 3: Synthesis of Compound 28-3

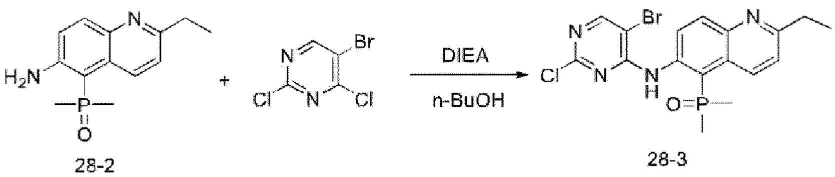


The synthesis method of compound 28-3 is the same as that of compound 1-8, except that the compound 1-7 is replaced by compound 28-2. MS of compound 28-3: 439.00 $[M+H]^+$ Step 4: Synthesis of Compound J-028

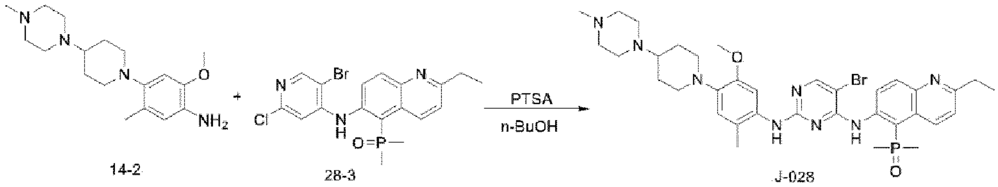

The synthesis method of compound J-028 is the same as that of compound J-022, except that the starting compound 1-8 is replaced by compound 28-3. MS of compound J-028: 721.27 [M+H]$^+$

**Example 29 Synthesis of compound J-029**

*(6-((5-Bromo-2-((3-methoxy-5-methyl-4-(4-(4-methylpiperazin-1-yl)piperidin-1-yl)phenyl)amino)pyrimidin-4-yl)amino)-2-cyclopropylquinolin-5-yl)dimethylphosphine oxide*

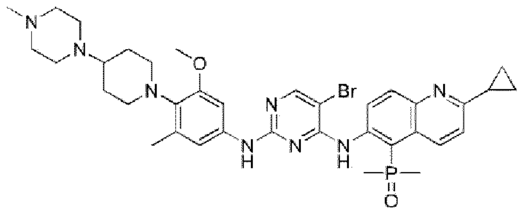

Step 1: Synthesis of Compound 29-1

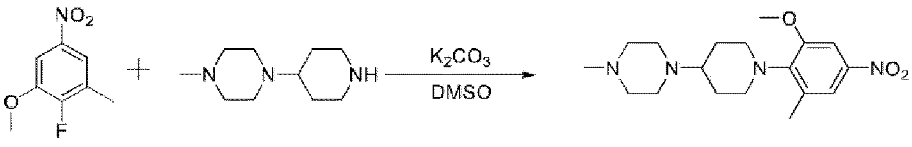


6-1 13-3 29-1

The synthesis method of compound 29-1 is the same as that of compound 6-2, except that the starting compound morpholine is replaced by compound 13-3. MS of compound 29-1: 349.22 [M+H]$^+$

Step 2: Synthesis of Compound 29-2

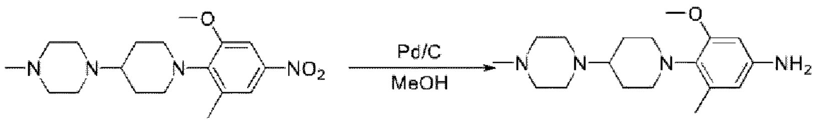


29-1 29-2

The synthesis method of compound 29-2 is the same as that of compound 1-11, except that the starting compound 1-10 is replaced by compound 29-1. MS of compound 29-2: 319.24 [M+H]$^+$

Step 3: Synthesis of Compound J-029

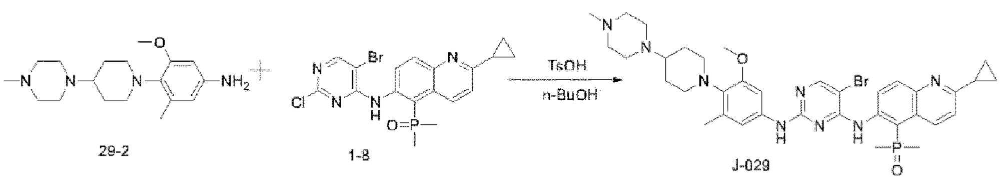


29-2 1-8 J-029

The synthesis method of compound J-029 is the same as that of compound J-001, except that the starting compound 1-11 is replaced by compound 29-2. MS of compound J-029: 733.27 [M+H]$^+$

**Example 30 Synthesis of compound J-030**

*(6-((5-Bromo-2-((5-ethyl-2-methoxy-4-(1'-methyl-[4,4'-bipiperidin]-1-yl)phenyl)amino)pyrimidin-4-yl)amino)-2-cyclopropylquinolin-5-yl)dimethylphosphine oxide*

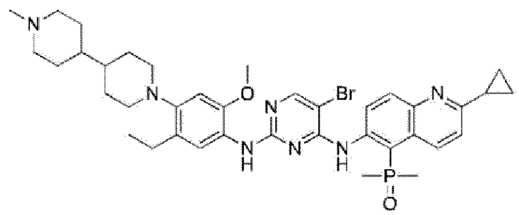

Step 1: Synthesis of Compound 30-2

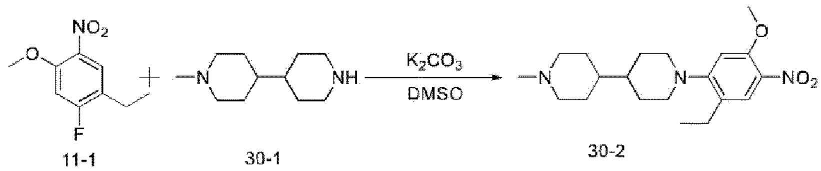


11-1 30-1 30-2

The synthesis method of compound 30-2 is the same as that of compound 13-4, except that the starting compound 13-3 is replaced by compound 30-1. MS of compound 30-2: 362.24 $[M+H]^+$

Step 2: Synthesis of Compound 30-3

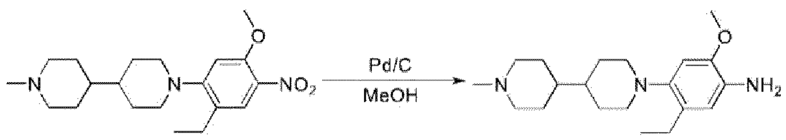


30-2 30-3

The synthesis method of compound 30-2 is the same as that of compound 1-11, except that the starting compound 1-10 is replaced by compound 30-2. MS of compound 30-3: 332.26 $[M+H]^+$

Step 3: Synthesis of Compound J-030

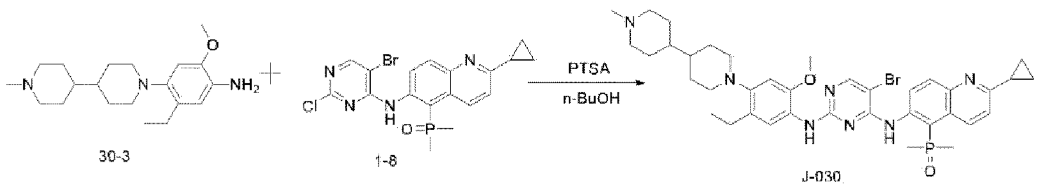


30-3 1-8 J-030

The synthesis method of compound J-030 is the same as that of compound J-001, except that the starting compound 1-11 is replaced by compound 30-3. MS of compound J-030: 746.29 $[M+H]^+$

**Example 31 Synthesis of compound J-031**

*(6-((5-Bromo-2-((2-ethyl-5-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide*

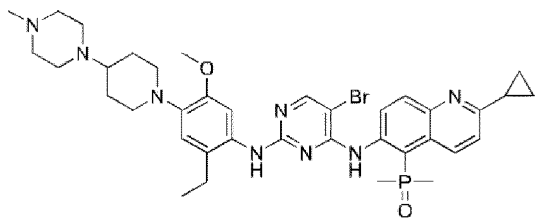

Step 1: Synthesis of Compound J-031-2

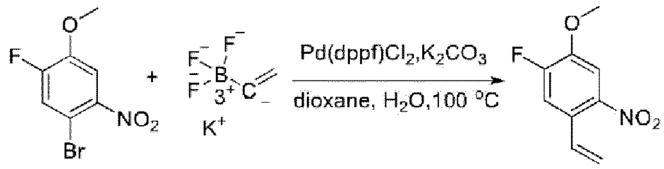


3-1 3-2

Compound 3-1 (500 mg, 2.00 mmol), potassium trifluoro(vinyl)borate (401.81 mg, 3.00 mmol), Pd(dppf)$Cl_2$ (163.31 mg, 199.98 μmol), $K_2CO_3$ (690.99 mg, 5.00 mmol), dioxane (60 mL) and $H_2O$ (15 mL) were successively added to a reaction flask. The temperature was raised to 100°C and the reaction was carried out for 6 hours. The reaction solution was cooled to room temperature, added with 25 mL of water, extracted with ethyl acetate, dried with anhydrous sodium sulfate, filtered, and spin-dried. The crude product was subjected to column chromatography (dichloromethane/methanol=15:1) to obtain the yellow solid target product 3-2 (300 mg, 1.52 mmol, yield: 76.09%).

Step 2: Synthesis of Compound J-031-3

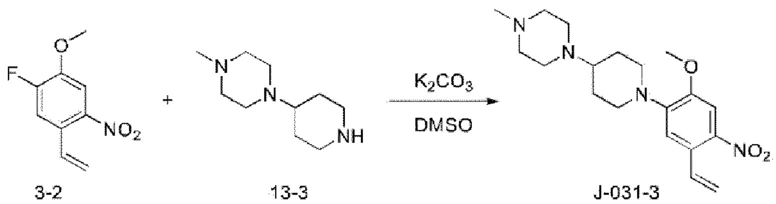


3-2 13-3 J-031-3

Compound 3-2 (0.6 g, 3.04 mmol), compound 13-3 (1.11 g, 6.08 mmol), $K_2CO_3$ (0.84 g, 6.08 mmol) and DMSO (10 mL) were successively added to a reaction flask, and the temperature was raised to 100°C. The mixture was heated and stirred 12 hours. It was monitored by LCMS until the reaction was completed and then the reaction was stopped. The reaction solution was poured into water (100 mL) and filtered by suction. The filter cake was washed with water and dried to obtain a yellow solid compound J-031-3 (0.82 g, 2.27 mmol). MS: 361.22 $[M+H]^+$

Step 3: Synthesis of Compound J-031-4

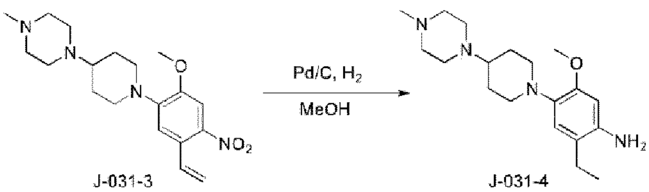


J-031-3 J-031-4

Compound J-031-3 (0.6 g) and MeOH (30 mL) were added to a reaction flask. The reaction flask was then vacuumized, purged with hydrogen, added with Pd/C (0.5 g, 10%), vacuumized, and purged with $H_2$. The reaction was carried out at room temperature for 3 hours. It was monitored by LCMS until the reaction was completed and then the reaction was stopped. After filtration by suction, the filter cake was washed with methanol (20 mL). The organic phase was collected and concentrated to obtain a reddish-brown liquid compound J-031-4 (0.45 g). MS: 333.26 $[M+H]^+$

Step 4: Synthesis of Compound J-031

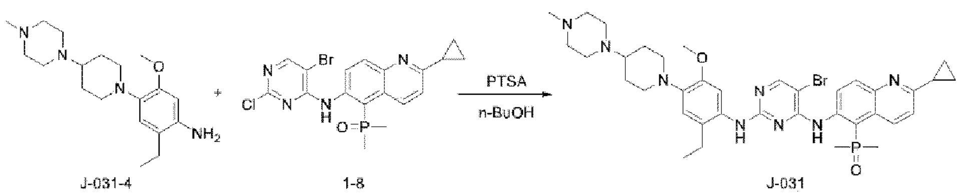


J-031-4 1-8 J-031

Compound J-031-4 (100 mg), compound 1-8 (78.2 mg), p-toluenesulfonic acid (48.6 mg) and n-BuOH (3 mL) were successively added to a reaction flask, and the mixture was stirred at 110°C overnight. It was monitored by LCMS until the reaction was completed. The reaction solution was cooled to room temperature, spin-dried, added with saturated aqueous $Na_2CO_3$ solution (10 mL), extracted with dichloromethane (3 × 10 mL), dried with anhydrous sodium sulfate, filtered, and spin-dried. The crude product was purified by PTLC (dichloromethane: methanol=10:1) to obtain compound J-031 (23 mg). MS: 747.28 $[M+H]^+$

**Example 32 Synthesis of compound J-032**

*(6-((5-Bromo-2-((4-(4-cyclopentylpiperazin-1-yl)-2-methoxy-5-methylphenyl)amino)pyrimidin-4-yl)amino)-2-cyclopropylquinolin-5-yl)dimethylphosphine oxide*

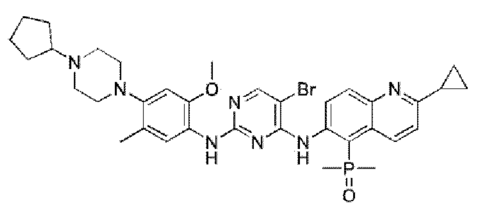

Step 1: Synthesis of Compound J-032-2

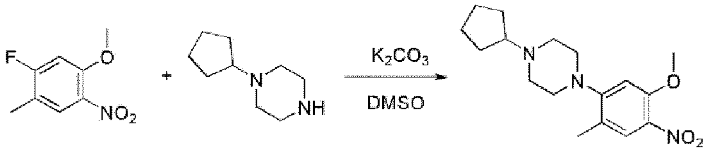


10-1 J-032-1 J-032-2

Compound 10-1 (1.0 g, 5.40 mmol), compound J-032-1 (1.66 g, 10.80 mmol), $K_2CO_3$ (1.49 g, 10.8 mmol) and DMSO (10 mL) were successively added to a reaction flask. The mixture was heated 100°C and stirred 12 hours. It was monitored by LCMS until the reaction was completed and then the reaction was stopped. The reaction solution was poured into water (20 mL) and filtered by suction. The filter cake was washed with water and dried to obtain a yellow solid compound J-032-3 (1.2 g, 3.7 mmol). MS: 320.19 $[M+H]^+$

Step 2: Synthesis of Compound J-032-3

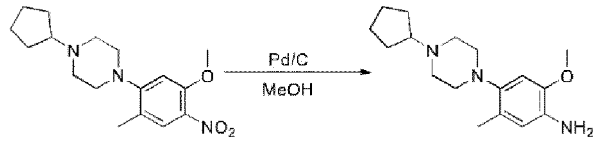


J-032-2 J-032-3

Compound J-032-2 (0.5 g) and MeOH (30 mL) were added to a reaction flask. The reaction flask was then vacuumized, purged with hydrogen, added with Pd/C (0.5 g, 10%), vacuumized, and purged with $H_2$. The reaction was carried out at room temperature for 3 hours. It was monitored by LCMS until the reaction was completed and then the reaction was stopped. After filtration by suction, the filter cake was washed with methanol (20 mL). The organic phase was collected and concentrated to obtain a reddish-brown liquid compound J-032-3 (0.3 g). MS: 290.22 $[M+H]^+$

Step 3: Synthesis of Compound J-032

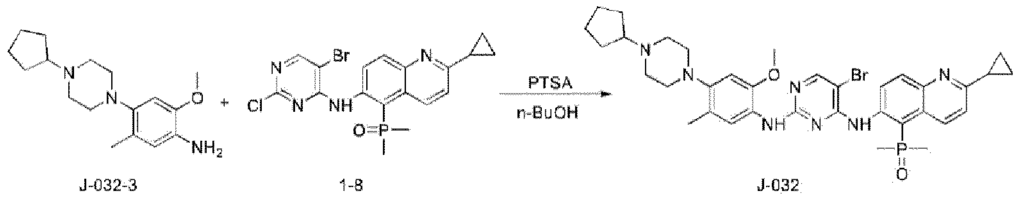


J-032-3 1-8 J-032

Compound J-032-3 (120 mg), compound 1-8 (82.6 mg), p-toluenesulfonic acid (52.3 mg) and n-BuOH (3 mL) were successively added to a reaction flask, and the mixture was stirred at 110°C overnight. It was monitored by LCMS until the reaction was completed. The reaction solution was cooled to room temperature, spin-dried, added with saturated aqueous $Na_2CO_3$ solution (10 mL), extracted with dichloromethane (3 × 10 mL), dried with anhydrous sodium sulfate, filtered, and spin-dried. The crude product was purified by PTLC (dichloromethane: methanol=10:1) to obtain compound J-032 (50 mg). MS: 704.24 $[M+H]^+$

**Example 33 Synthesis of compound J-033**

*(6-((5-Bromo-2-((5-methoxy-4-(4-methoxypiperidin-1-yl)-2-methylphenyl)amino)pyrimidin-4-yl)amino)-2-cyclopropylquinolin-5-yl)dimethylphosphine oxide*

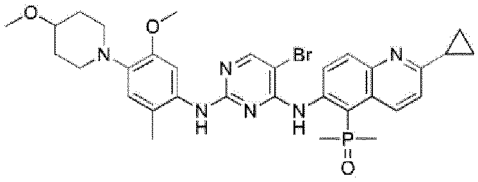

Step 1: Synthesis of Compound J-033-1

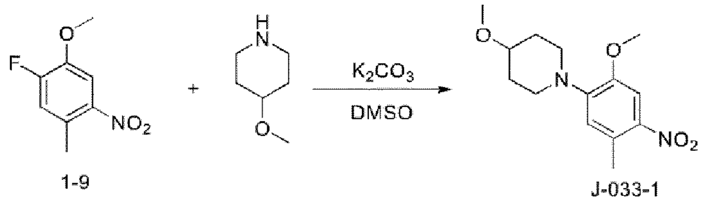


1-9 J-033-1

The synthesis method of compound J-033-1 is the same as that of compound 1-10, except that the starting compound morpholine is replaced by 4-methoxypiperidine. MS of compound J-033-1: 281.14 $[M+H]^+$

Step 2: Synthesis of Compound J-033-2

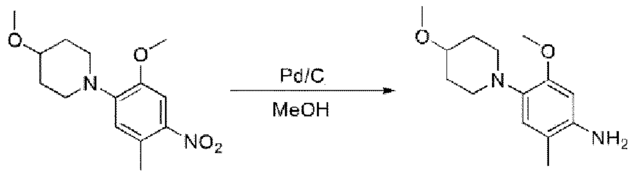


J-033-1 J-033-2

The synthesis method of compound J-033-2 is the same as that of compound 1-11, except that the starting compound 1-10 is replaced by compound J-033-1. MS of compound J-033-2: 251.17 $[M+H]^+$

Step 3: Synthesis of Compound J-033

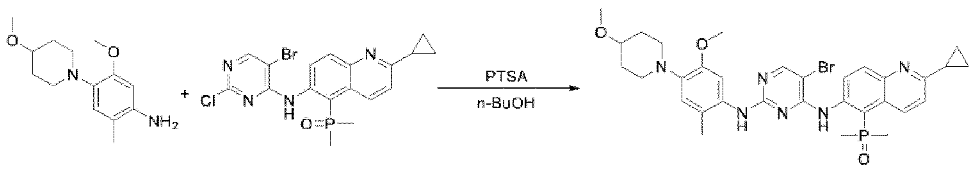


J-033-2 1-8 J-033

The synthesis method of compound J-033 is the same as that of compound J-001, except that the starting compound 1-11 is replaced by compound J-033-2. MS of compound J-033: 665.19 $[M+H]^+$

**Example 34 Synthesis of compound J-034**

*(6-((5-Bromo-2-((2-methoxy-5-methyl-4-(4-(4-methylpiperazin-1-yl)piperidin-1-yl)phenyl)amino)pyrimidin-4-yl)amino)-2-isopropylquinolin-5-yl)dimethylphosphine oxide*

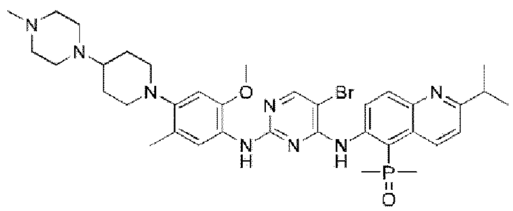

Step 1: Synthesis of Compound J-034-1

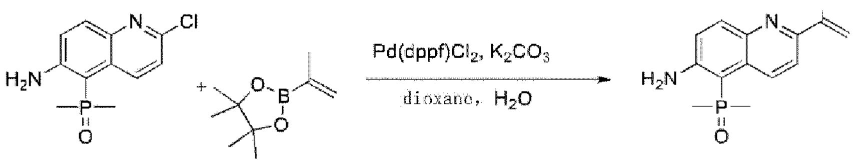


1-6 J-034-1

In a reactor with a stirrer and a nitrogen protection device, compound 1-6 (500 mg, 1.96 mmol) and 2-isopropenyl-4,4,5,5-tetramethyl-1,3,2-dioxaborane (1.32 g, 7.85 mmol) were dissolved in water (1 mL) and 1,4-dioxane (5 mL), followed by adding Pd(dppf)$Cl_2$ (160.34 mg, 196.35 μmol) and $K_2CO_3$ (678.43 mg, 4.91 mmol) at room temperature. Nitrogen replacement was performed for three times. The mixture was stirred uniformly, heated to 100°C under an oil bath, and stirred at 100°C for 1.5 hours. LCMS monitoring showed that the reaction was complete. The reaction solution was cooled to room temperature and concentrated by rotary evaporation. After concentration, organic phase was mixed with silica gel, separated and purified by column with a gradient of PE: EtOAc from 0%-100%, concentrated and dried to obtain a yellow solid compound J-034-1 (400 mg, 1.54 mmol, yield 78.27%). MS: 261.11 $[M+H]^+$

Step 2: Synthesis of Compound J-034-2

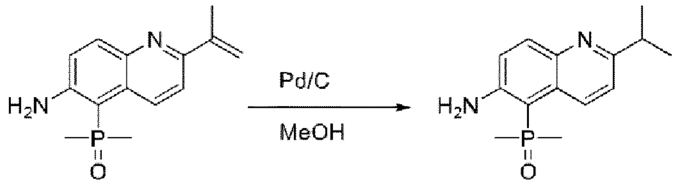


J-034-1 J-034-2

Compound J-034-1 (528.48 mg, 2.03 mmol), methanol (10 mL), Pd/C (178 mg, 10% purity) were successively added to a reaction flask. The mixture was stirred at room temperature for 12 hours under a hydrogen atmosphere. It was monitored by LCMS until the reaction was completed. The reaction solution was filtered and spin-dried to obtain a yellow solid compound J-034-2 (450 mg, 1.72 mmol, 84.50% yield), which was used directly in the next step without purification. MS: 263.12 $[M+H]^+$

Step 3: Synthesis of Compound J-034-3

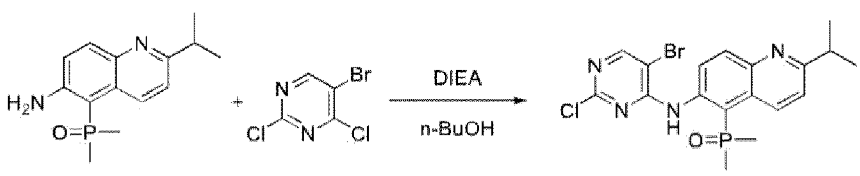


J-034-2 J-034-3

The synthesis method of compound J-034-3 is the same as that of compound 1-8, except that the starting compound 1-7 is replaced by compound J-034-2. MS of compound J-034-3: 453.02 [M+H]$^+$

Step 4: Synthesis of Compound J-034

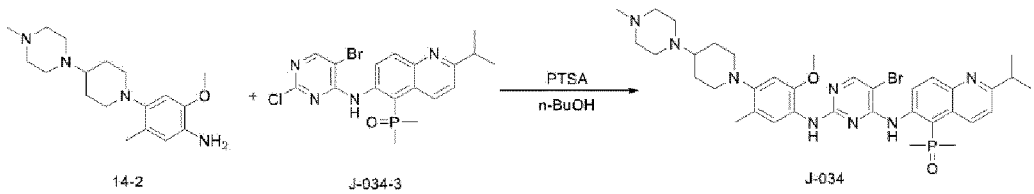


14-2 J-034-3 J-034

The synthesis method of compound J-034 is the same as that of compound J-001, except that compound 1-11 is replaced by compound 14-2, and compound 1-8 is replaced by compound J-034-3. MS of compound J-034: 735.28 [M+H]$^+$

## Example 35 Synthesis of compound J-035

*(6-((5-Bromo-2-((5-fluoro-2-methyl-4-morpholinophenyl)amino)pyrimidin-4-yl)amino)-2-cyclopropylquinoline-5-yl)dimethylphosphine oxide*

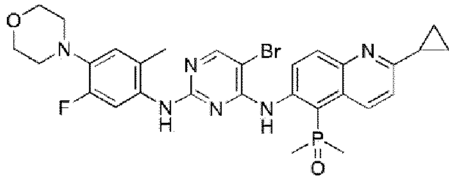

Step 1: Synthesis of Compound J-035-2

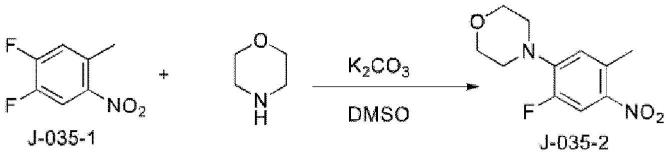


J-035-1 J-035-2

The synthesis method of compound J-035-2 is the same as that of compound 1-10, except that starting compound 1-9 is replaced by compound J-035-1. MS of compound J-035-2: 241.09 [M+H]$^+$.

Step 2: Synthesis of Compound J-035-3

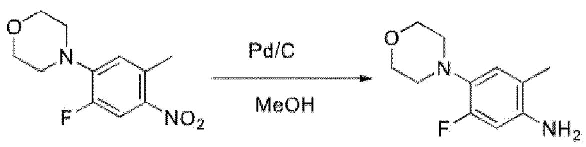


J-035-2 J-035-3

The synthesis method of compound J-035-3 is the same as that of compound 1-11, except that starting compound 1-10 is replaced by compound J-035-2. MS of compound J-035-3: 211.12 [M+H]+.

Step 3: Synthesis of Compound J-035

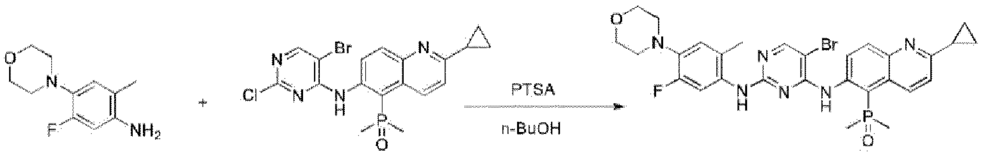


J-035-3 1-8 J-035

The synthesis method of compound J-035 is the same as that of compound J-001, except that compound 1-11 is replaced by compound J-035-3. MS of compound J-035: 625.14 [M+H]+.

**Example 36 Synthesis of compound J-036**

*(6-((5-Bromo-2-((2,5-dimethyl-4-morpholinophenyl)amino)pyrimidin-4-yl)amino)-2-cyclopropylquinoline-5-yl)dimethylphosphine oxide*

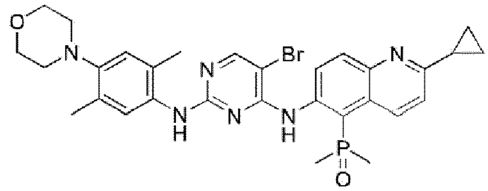

Step 1: Synthesis of Compound J-036-2

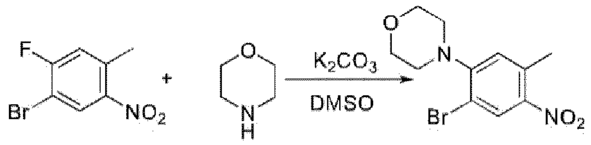


J-036-1 J-036-2

The synthesis method of compound J-036-2 is the same as that of compound 1-10, except that starting compound 1-9 is replaced by compound J-036-1. MS of compound J-036-2: 301.01 [M+H]+.

Step 2: Synthesis of Compound J-036-3

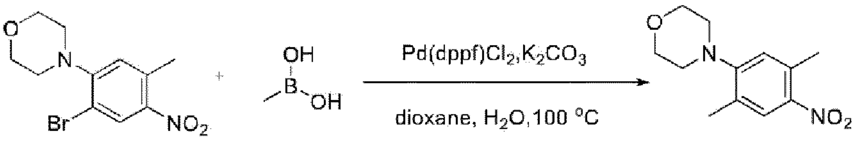


J-036-2 J-036-3

The synthesis method of compound J-036-3 is the same as that of compound 3-2, except that starting compound

3-1 is replaced by compound J-036-2, and the starting compound potassium trifluoro(vinyl)borate is replaced by methylboronic acid. MS of compound J-036-3: 237.12 [M+H]+.

Step 3: Synthesis of Compound J-036-4

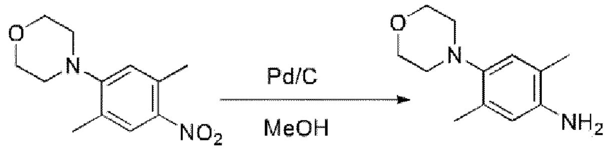


J-036-3 J-036-4

The synthesis method of compound J-036-4 is the same as that of compound 1-11, except that starting compound 1-10 is replaced by compound J-036-3. MS of compound J-036-4: 207.14 [M+H]+.

Step 4: Synthesis of Compound J-036

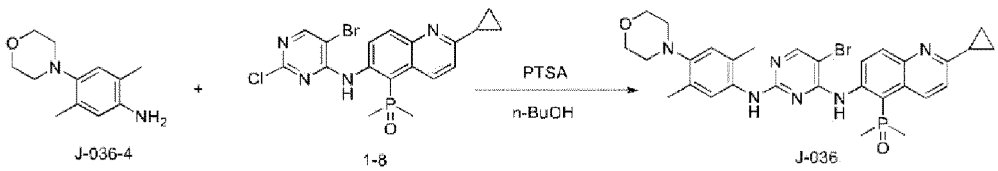


J-036-4 1-8 J-036

The synthesis method of compound J-036 is the same as that of compound J-001, except that compound 1-11 is replaced by compound J-036-4. MS of compound J-036: 621.17 [M+H]+.

**Example 37 Synthesis of compound J-037**

*(6-((5-Bromo-2-((4-(4-cyclopentylpiperazin-1-yl)-2-methoxy-5-methylphenyl)amino)pyrimidin-4-yl)amino)-2-cyclopropylquinolin-5-yl)dimethylphosphine oxide*

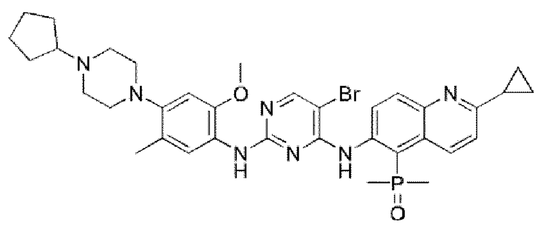

Step 1: Synthesis of Compound J-037-2

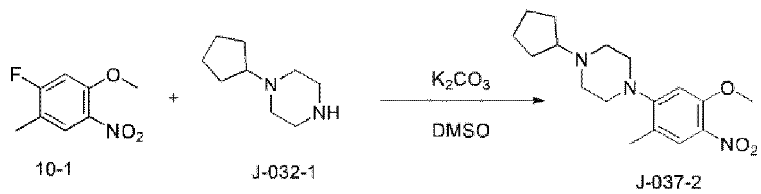


10-1 J-032-1 J-037-2

The synthesis method of compound J-037-2 is the same as that of compound 1-10, except that the starting compound 1-9 is replaced by compound 10-1, and the starting compound morpholine is replaced by compound J-032-1. MS of compound J-037-2: 320.19 $[M+H]^+$.

Step 2: Synthesis of Compound J-037-3

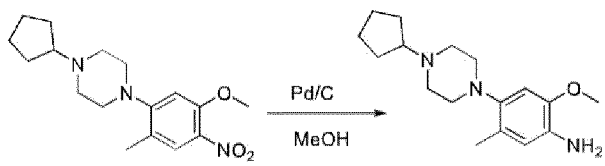


J-037-2 J-037-3

The synthesis method of compound J-037-3 is the same as that of compound 1-11, except that starting compound 1-10 is replaced by compound J-037-2. MS of compound J-037-3: 290.22 $[M+H]^+$.

Step 3: Synthesis of Compound J-037

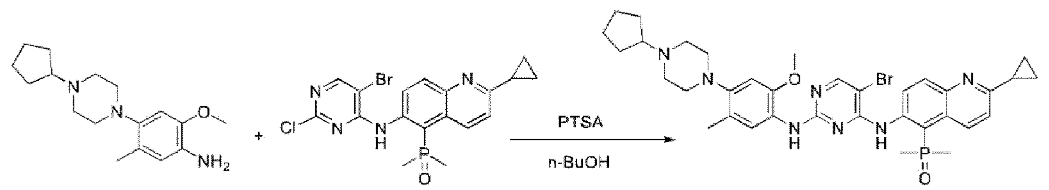


J-037-3 1-8 J-037

The synthesis method of compound J-037 is the same as that of compound J-001, except that compound 1-11 is replaced by compound J-037-3. MS of compound J-037: 704.24 $[M+H]^+$.

**Example 38 Synthesis of compound J-038**

(6-((5-Bromo-2-((4-(4-cyclopropylpiperazin-1-yl)-2-methoxy-5-methylphenyl)amino)pyrimidin-4-yl)amino)-2-cyclopropylquinolin-5-yl)dimethylphosphine oxide

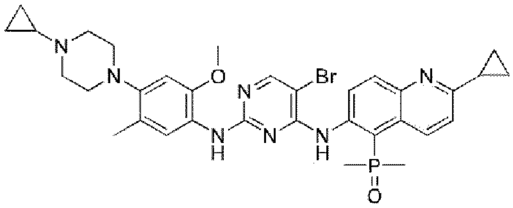

Step 1: Synthesis of Compound J-038-2

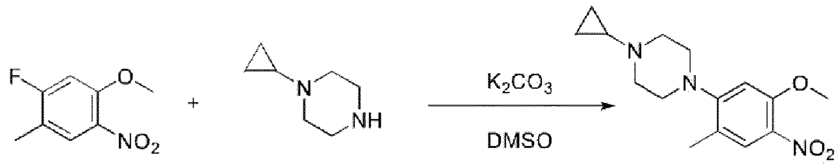


10-1 J-038-1 J-038-2

The synthesis method of compound J-038-2 is the same as that of compound 1-10, except that the starting compound 1-9 is replaced by compound 10-1, and the starting compound morpholine is replaced by compound J-038-1. MS of compound J-038-2: 292.16 $[M+H]^+$.

Step 2: Synthesis of Compound J-038-3

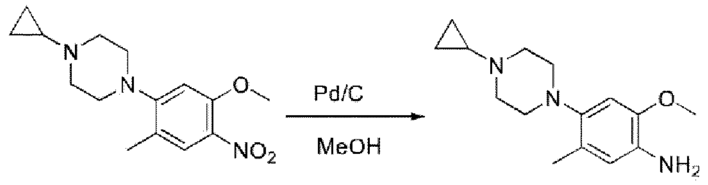


J-038-2 J-038-3

The synthesis method of compound J-038-3 is the same as that of compound 1-11, except that starting compound 1-10 is replaced by compound J-038-2. MS of compound J-038-3: 262.18 $[M+H]^+$.

Step 3: Synthesis of Compound J-038

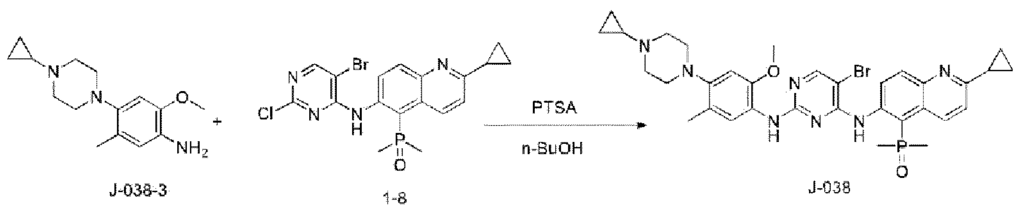


J-038-3 1-8 J-038

The synthesis method of compound J-038 is the same as that of compound J-001, except that starting compound 1-11 is replaced by compound J-038-3. MS of compound J-038: 676.21 $[M+H]^+$.

**Example 39 Synthesis of compound J-039**

*(6-((5-Bromo-2-((4-(4-cyclopentylpiperazin-1-yl)-5-methoxy-2-methylphenyl)amino)pyrimidin-4-yl)amino)-2-cyclopropylquinolin-5-yl)dimethylphosphine oxide*

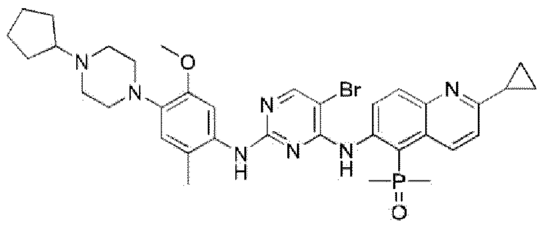

Step 1: Synthesis of Compound J-039-1

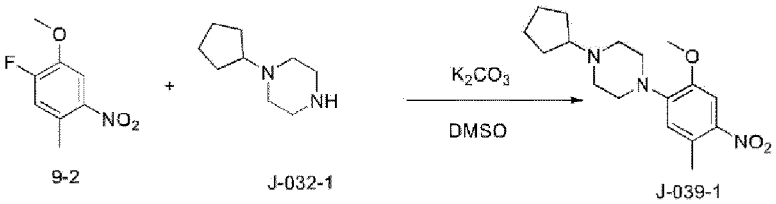


9-2 J-032-1 J-039-1

The synthesis method of compound J-039-1 is the same as that of compound 1-10, except that the starting compound 1-9 is replaced by compound 9-2, and the starting compound morpholine is replaced by compound J-032-1. MS of compound J-039-1: 320.19 $[M+H]^+$.

Step 2: Synthesis of Compound J-039-2

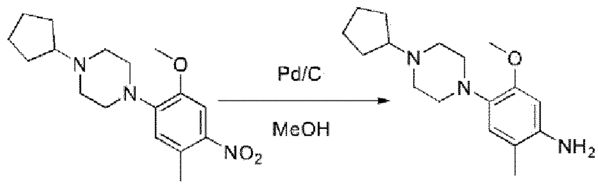


J-039-1 J-039-2

The synthesis method of compound J-039-2 is the same as that of compound 1-11, except that starting compound 1-10 is replaced by compound J-039-1. MS of compound J-039-2: 290.22 $[M+H]^+$.

Step 3: Synthesis of Compound J-039

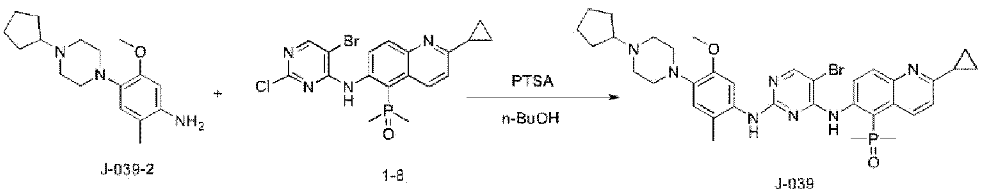


J-039-2 1-8 J-039

The synthesis method of compound J-039 is the same as that of compound J-001, except that starting compound 1-11 is replaced by compound J-039-2. MS of compound J-039: 704.24 $[M+H]^+$.

**Example 40 Synthesis of compound J-040**

*(6-((5-Bromo-2-((5-methoxy-2-methyl-4'-(4-methylpiperazin-1-yl)-[1,1'-biphenyl]-4-yl)amino)pyrimidin-4-yl)amino)-2-cyclopropylquinolin-5-yl)dimethylphosphine oxide*

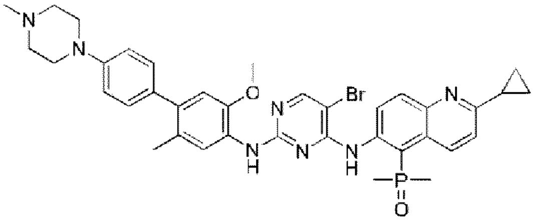

Step 1: Synthesis of Compound J-040-3

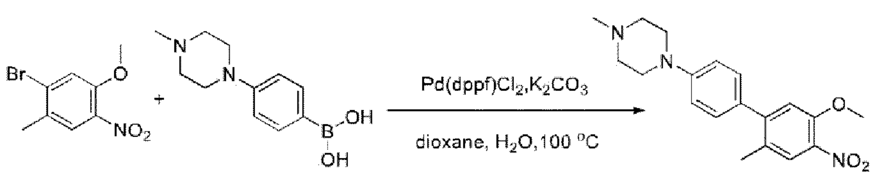


J-040-1 J-040-2 J-040-3

The synthesis method of compound J-040-3 is the same as that of compound 3-2, except that starting compound 3-1 is replaced by compound J-040-1, and the starting compound potassium trifluoro(vinyl)borate is replaced by the compound J-040-2. MS of compound J-040-3: 342.17 $[M+H]^+$.

Step 2: Synthesis of Compound J-040-4

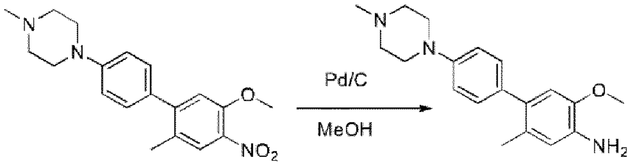


J-040-3 J-040-4

The synthesis method of compound J-040-4 is the same as that of compound 1-11, except that starting compound 1-10 is replaced by compound J-040-3. MS of compound J-040-4: 312.20 $[M+H]^+$.

Step 3: Synthesis of Compound J-040

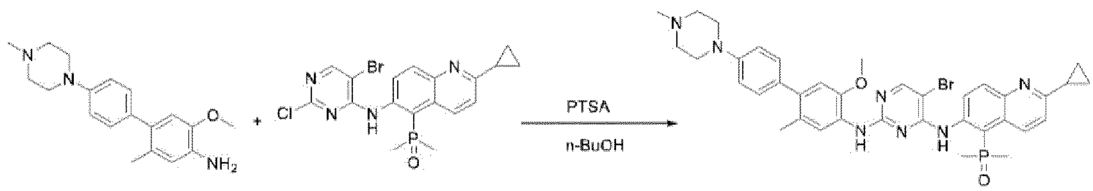


J-040-4 1-8 J-040

The synthesis method of compound J-040 is the same as that of compound J-001, except that starting compound 1-11 is replaced by compound J-040-4. MS of compound J-040: 726.22 [M+H]$^+$.

**Example 41 Synthesis of compound J-041**

*(6-((5-Bromo-2-((2-methoxy-5-methyl-4-(4-(4-methylpiperazin-1-yl)piperidin-1-yl)phenyl)amino)pyrimidin-4-yl)amino)quinolin-5-yl)dimethylphosphine oxide*

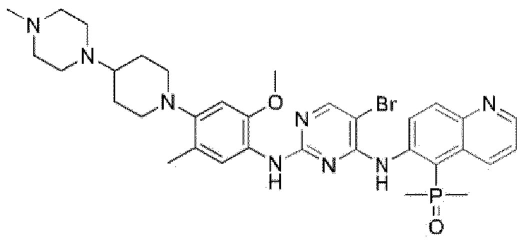

Step 1: Synthesis of Compound J-041-1

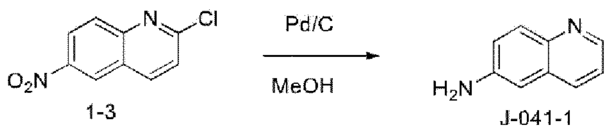


Compound 1-3 (0.5 g) was dissolved in 10 mL of methanol in a 20 ml single-neck bottle, followed by adding palladium on carbon (0.2 g, 10%). The reaction system was vacuumized and purged with hydrogen. The reaction was carried out at room temperature overnight. It was monitored by LCMS until the reaction was completed and then the reaction was stopped. After filtration by suction, the filter cake was washed with methanol (10 mL). The organic phase was collected and concentrated to obtain a reddish-grey solid compound J-041-1 (0.36 g). MS: 145.07 [M+H]$^+$

Step 2: Synthesis of Compound J-041-2

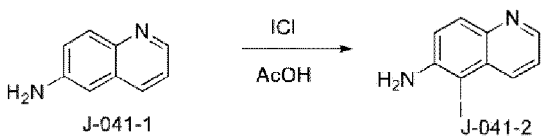


The synthesis method of compound J-041-2 is the same as that of compound 1-5, except that starting compound 1-4 is replaced by compound J-041-1. MS of compound J-041-2: 270.97 [M+H]$^+$.

Step 3: Synthesis of Compound J-041-3

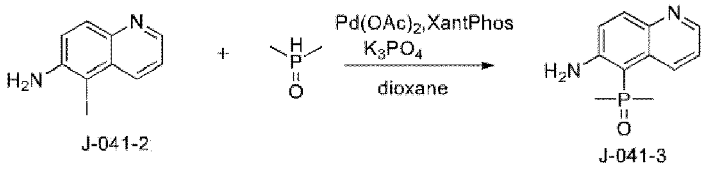

The synthesis method of compound J-041-3 is the same as that of compound 1-6, except that starting compound 1-5 is replaced by compound J-041-2. MS of compound J-041-2: 221.08 $[M+H]^+$.

Step 4: Synthesis of Compound J-041-4

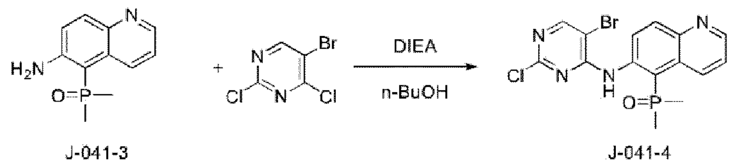


J-041-3 J-041-4

The synthesis method of compound J-041-4 is the same as that of compound 1-8, except that starting compound 1-7 is replaced by compound J-041-3. MS of compound J-041-4: 410.97 $[M+H]^+$.

Step 5: Synthesis of Compound J-041

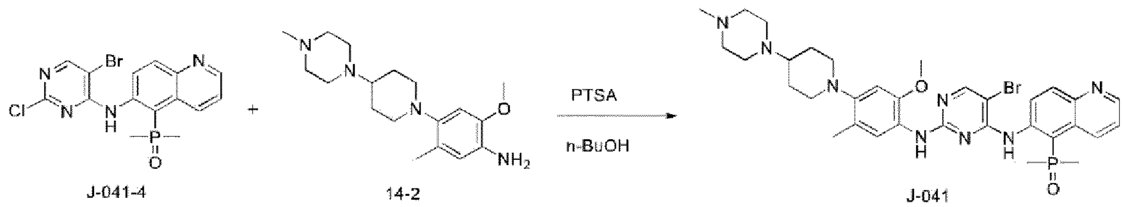


J-041-4 14-2 J-041

The synthesis method of compound J-041 is the same as that of compound J-001, except that starting compound 1-11 is replaced by compound 14-2, and compound 1-8 is replaced by compound J-041-4. MS of compound J-041: 693.24 $[M+H]^+$

**Example 42 Synthesis of compound J-042**

*(6-((5-Bromo-2-((2-methoxy-5-methyl-4-(4-(4-methylpiperazin-1-yl)piperidin-1-yl)phenyl)amino)pyrimidin-4-yl)amino)-2-propylquinolin-5-yl)dimethylphosphine oxide*

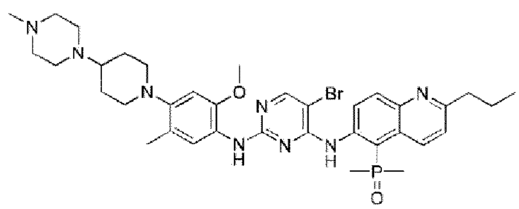

Step 1: Synthesis of Compound J-042-1

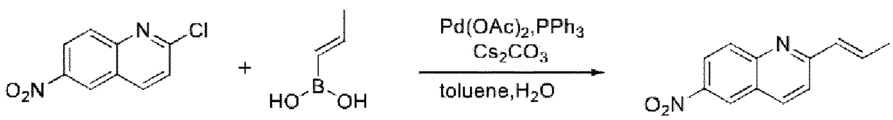


1-3 J-042-1

In a reactor with a stirrer and a nitrogen protection device, compound 1-3 (500 mg, 2.41 mmol) and trans-1-propen-1-yl-boronic acid (414 mg, 4.82 mmol) was dissolved in water (1 mL) and toluene (5 mL), followed by adding Palladium acetate (54 mg, 0.24 mmol), triphenylphosphine (126 mg, 0.48 mmol) and $Cs_2CO_3$ (1.57 g, 4.82 mmol) at room temperature. Nitrogen replacement was performed for three times. The mixture was stirred uniformly, heated to 100°C under an oil bath, and reacted overnight. It was monitored by LCMS until the reaction was completed. The reaction solution was cooled to room temperature and concentrated by rotary evaporation. After concentration, the organic phase was mixed with silica gel, separated, purified by column with a gradient of PE: EtOAc from 0% - 100%, concentrated, and dried to obtain a yellow solid compound J-042-1 (264 mg, yield 51.17%). MS: 215.07 $[M+H]^+$

Step 2: Synthesis of Compound J-042-2

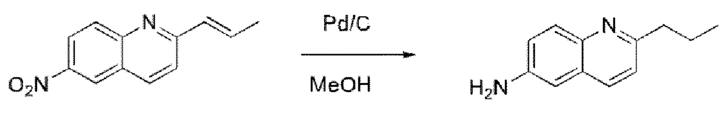


J-042-1 J-042-2

Compound J-042-1 (264 mg) was dissolved in 5 mL of methanol in a 20 ml single-neck bottle, followed by adding palladium on carbon (0.1g, 10%). The reaction system was vacuumized and purged with hydrogen. The reaction was carried out at room temperature for 4 hours. It was monitored by LCMS until the reaction was completed and then the reaction was stopped. After filtration by suction, the filter cake was washed with methanol (10 mL). The organic phase was collected and concentrated to obtain an off-white solid compound J-042-2 (220 mg). MS: 187.12 $[M+H]^+$

Step 3: Synthesis of Compound J-042-3

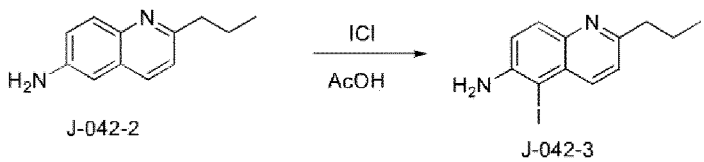


J-042-2 J-042-3

The synthesis method of compound J-042-3 is the same as that of compound 1-5, except that starting compound 1-4 is replaced by compound J-042-2. MS of compound J-042-3: 313.01 $[M+H]^+$.

Step 4: Synthesis of Compound J-042-4

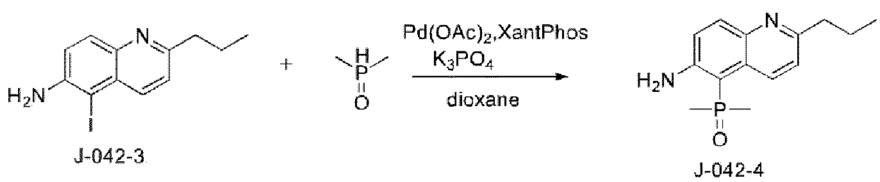


J-042-3 J-042-4

The synthesis method of compound J-042-4 is the same as that of compound 1-6, except that starting compound 1-5 is replaced by compound J-042-3. MS of compound J-042-4: 263.12 $[M+H]^+$.

Step 5: Synthesis of Compound J-041-5

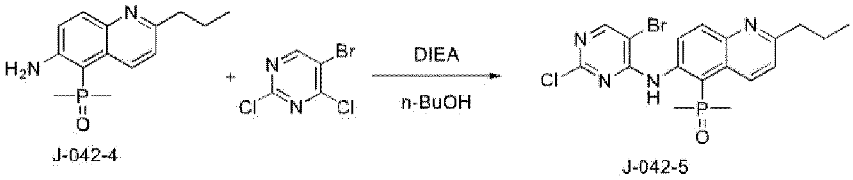


J-042-4 J-042-5

The synthesis method of compound J-042-5 is the same as that of compound 1-8, except that starting compound 1-7 is replaced by compound J-042-4. MS of compound J-042-5: 453.07 $[M+H]^+$.

Step 6: Synthesis of Compound J-042

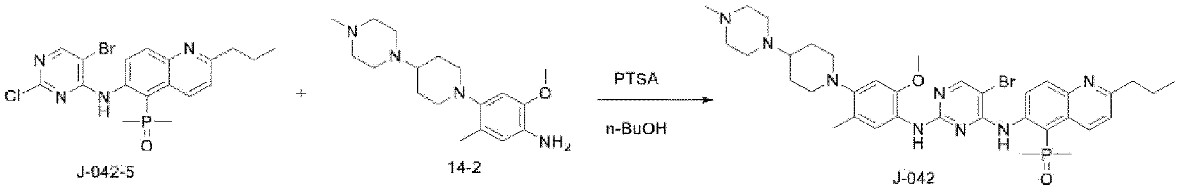


J-042-5 14-2 J-042

The synthesis method of compound J-042 is the same as that of compound J-001, except that starting compound 1-11 is replaced by compound 14-2, and compound 1-8 is replaced by compound J-042-5. MS of compound J-042: 735.28 $[M+H]^+$

**Example 43 Synthesis of compound J-043**

*(6-((5-bromo-2-((2-methoxy-4-(4-(2-methoxypyridin-4-yl)piperazin-1-yl)-5-(1-methyl-1H-pyrazol-4-yl)phenyl)amino)pyridin-4-yl)amino)-2-cyclopropylquinolin-5-yl)dimethylphosphine oxide*

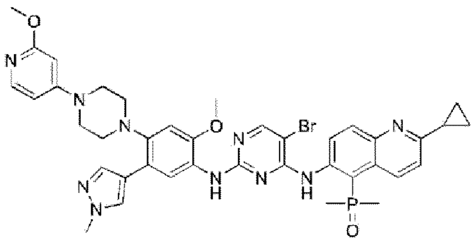

Step 1: Synthesis of Compound J-043-2

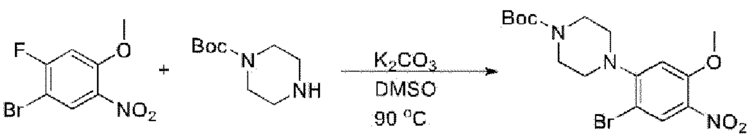


2-1 J-043-1 J-043-2

Compound 2-1 (1.00 g, 4.00 mmol, 1.0 eq), compound J-043-1 (0.82 g, 4.40 mmol, 1.1 eq), anhydrous potassium carbonate (1.106 g, 8.0 mmol, 2.0 eq) and DMSO (10 mL) were mixed uniformly in a 50 ml single-neck flask, and the reaction was carried out at 100°C for 4 hours. The reaction solution was cooled to room temperature, poured into 50 mL of water, extracted with 30 ml ethyl acetate twice. The organic phases were combined, washed three times with 50 ml water, washed once with 50 ml saturated brine, dried, filtered, and concentrated to obtain compound J-043-2 (1.634

g, 3.93 mmol, yield: 98.25%). MS: 416.07 $[M+H]^+$

Step 2: Synthesis of Compound J-043-4

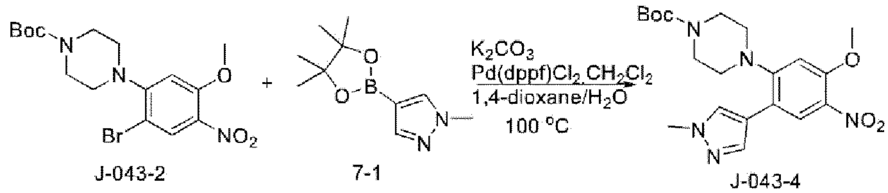


J-043-2 7-1 J-043-4

Compound J-043-2 (1.134 g, 3.93 mmol), compound 7-1 (0.980 g, 4.71 mmol), anhydrous potassium carbonate (1.086 g, 7.86 mmol) and $Pd(dppf)Cl_2.CH_2Cl_2$ (1.086 g, 7.86 mmol) was dissolved in 1,4-dioxane/water (20 mL/2 mL) in a 50 ml single-neck flask. The mixture was heated to 100°C and stirred overnight under nitrogen protection. The reaction solution was cooled to room temperature, concentrated, mixed with silica gel, and purified by flash silica gel column (DCM/MeOH; MeOH from 0-5%, 20 minutes) to obtain compound J-043-4 (1.20 g, 2.87 mmol, yield: 73.03%)). MS: 418.20 $[M+H]^+$.

Step 3: Synthesis of Compound J-043-5

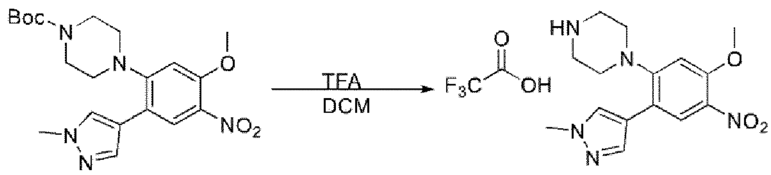


J-043-4 J-043-5

Compound J-043-4 (1.20 g, 3.93 mmol) was dissolved in dichloromethane (15 mL) in a 50 ml single-neck flask, followed by adding trifluoroformic acid (3 ml). The mixture was stirred at room temperature for 2 hours. The reaction solution was concentrated. The residue was slurried with anhydrous diethyl ether for 0.5 hour and filtered. The filter cake was washed with diethyl ether, collected, and dried to obtain compound J-043-5 (1.12 g, 2.60 mmol, yield: 90.59%). MS: 318.15 $[M+H]^+$.

Step 4: Synthesis of Compound J-043-7

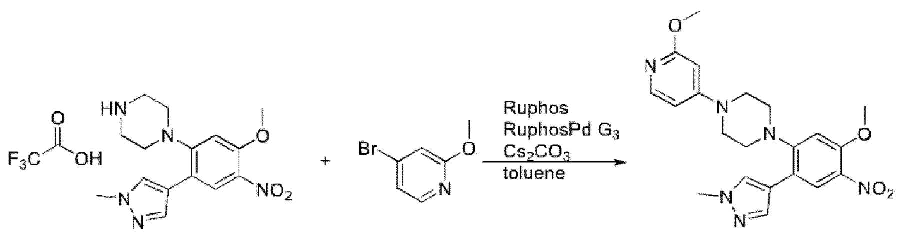


J-043-5 J-043-6 J-043-7

Compound J-043-5 (200 mg, 0.630 mmol), compound J-043-6 (178 mg, 0.945 mmol), Ruphos (59 mg, 0.126 mmol), Ruphos Pd G3 (53 mg, 0.063 mmol) and cesium carbonate (616 mg, 1.89 mmol) were dissolved in toluene (5 ml) in a 25 ml single-neck flask. The mixture was heated to 100°C and stirred overnight under nitrogen protection. The reaction solution was cooled to room temperature and concentrated. The residue was mixed with silica gel and purified by flash silica gel column (DCM/MeOH; MeOH from 0-5%, 20 minutes) to obtain compound J-043-7 (254 mg, 0.600

mmol, yield: 95.24%). MS: 425.19 [M+H]+.

Step 5: Synthesis of Compound J-043-8

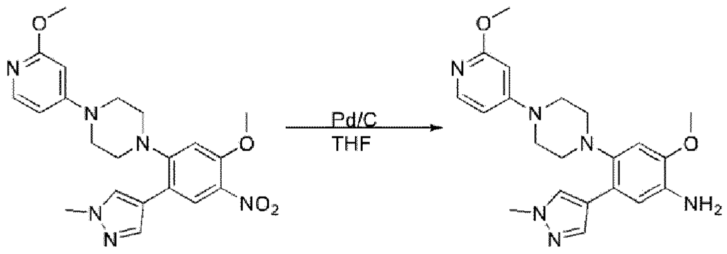


J-043-7 J-043-8

Compound J-043-7 (254 mg, 0.600 mmol) was dissolved in THF (10 mL) in a 50 ml single-neck flask, followed by adding palladium on carbon (150 mg). The reaction system was purged with hydrogen for 3 times and stirred at room temperature for 2 hours under the hydrogen atmosphere. After the reaction was completed, the reaction solution was filtered. The filter cake was washed with THF. The filtrate was collected and concentrated to obtain compound J-043-8 (200 mg, 0.507 mmol, yield: 84.5%). MS: 395.21 [M+H]+.

Step 6: Synthesis of Compound J-043

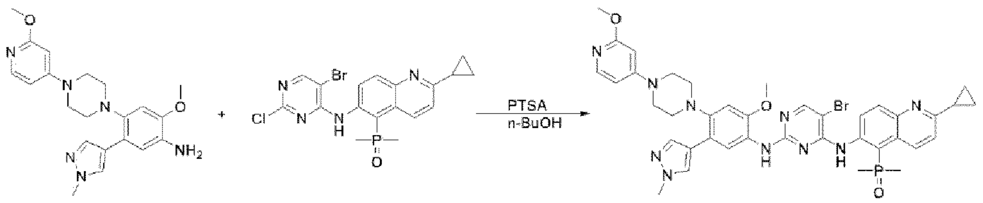


J-043-8 1-8 J-043

Compound J-043-8 (164 mg, 0.415 mmol), compound 1-8 (156 mg, 0.346 mmol) and PTSA (149 mg, 0.864 mmol) were dissolved in n-butanol (5 ml) in a 25 ml single-neck bottle. The mixture was heated to 110°C and stirred for 3 hours. After the raw materials reacted completely, the reaction solution was cooled to room temperature, concentrated, and added with 30 ml of water. The pH was adjusted to about 10 with an aqueous potassium carbonate solution and the reaction solution was extracted twice with DCM. The organic phases were combined, washed once with water, dried, and concentrated. The residue was transfered to a 1 mm thick preparative plate, with DCM/MeOH=10/1, to obtain compound J-043 (75.9 mg). MS: 809.24 [M+H]+.

**Example 44 Synthesis of compound J-044**

*(6-((5-Bromo-2-((2-methoxy-4-(4-(2-methoxypyrimidin-5-yl)piperazin-1-yl)-5-(1-methyl-1H-pyrazol-4-yl)phenyl)amino)pyrimidin-4-yl)amino)-2-cyclopropylquinolin-5-yl)dimethylphosphine oxide*

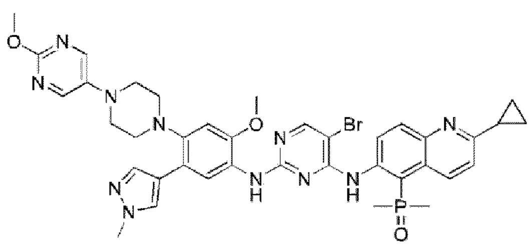

Step 1: Synthesis of Compound J-044-2

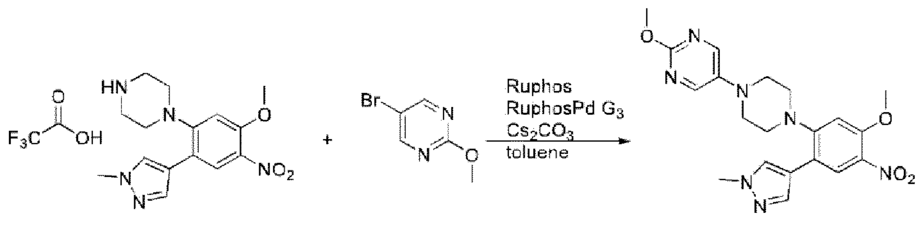


J-043-5 J-044-1 J-044-2

The synthesis method of compound J-044-2 is the same as that of compound J-043-7, except that the starting compound J-043-6 is replaced by compound J-044-1. MS of compound J-044-2: 426.18 [M+H]$^+$.

Step 2: Synthesis of Compound J-044-3

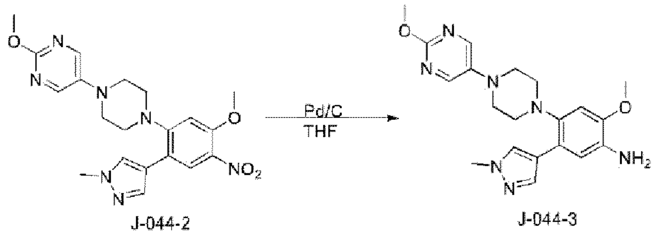


J-044-2 J-044-3

The synthesis method of compound J-044-3 is the same as that of compound J-043-8, except that the starting compound J-043-7 is replaced by compound J-044-2. MS of compound J-044-3: 396.21 [M+H]$^+$.

Step 3: Synthesis of Compound J-044

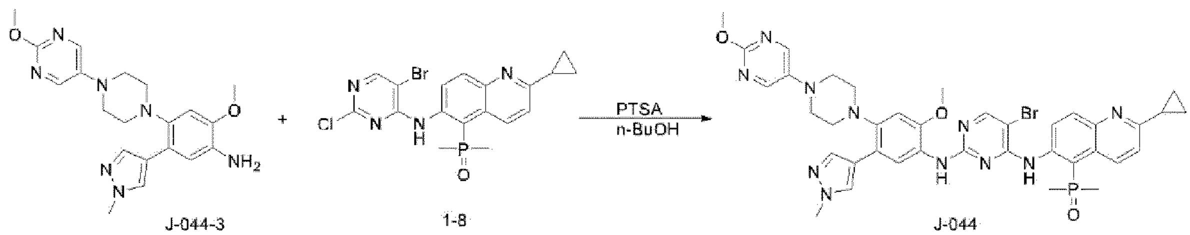


J-044-3 1-8 J-044

The synthesis method of compound J-044 is the same as that of compound J-043, except that the starting compound J-043-8 was replaced with compound J-044-3. MS of compound J-044: 810.23 [M+H]$^+$.

**Example 45 Synthesis of compound J-045**

*(6-((5-Bromo-2-((4-(4-(5-(dimethylamino)pyrazin-2-yl)piperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide*

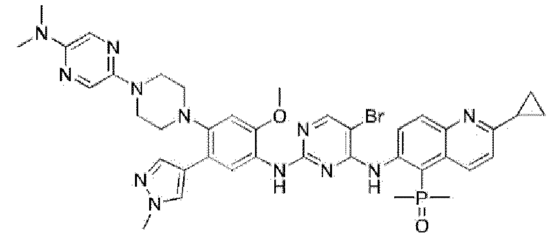

Step 1: Synthesis of Compound J-045-2

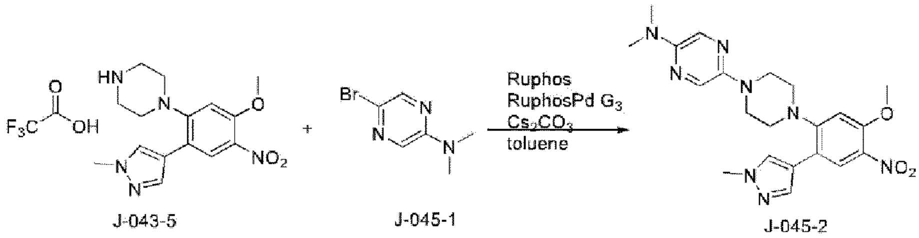


The synthesis method of compound J-045-2 is the same as that of compound J-043-7, except that the starting compound J-043-6 is replaced by compound J-045-1. MS of compound J-045-2: 439.21 $[M+H]^+$.

Step 2: Synthesis of Compound J-045-3

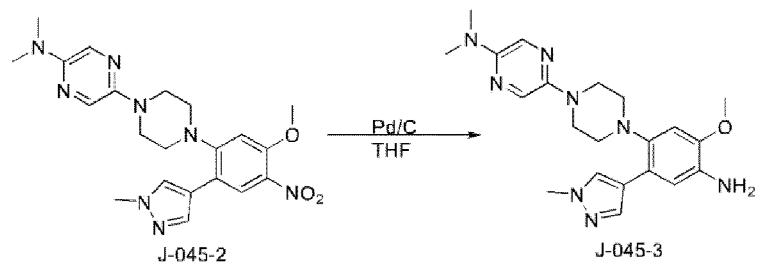


The synthesis method of compound J-045-3 is the same as that of compound J-043-8, except that the starting compound J-043-7 is replaced by compound J-045-2. MS of compound J-045-3: 409.24 $[M+H]^+$.

Step 3: Synthesis of Compound J-045

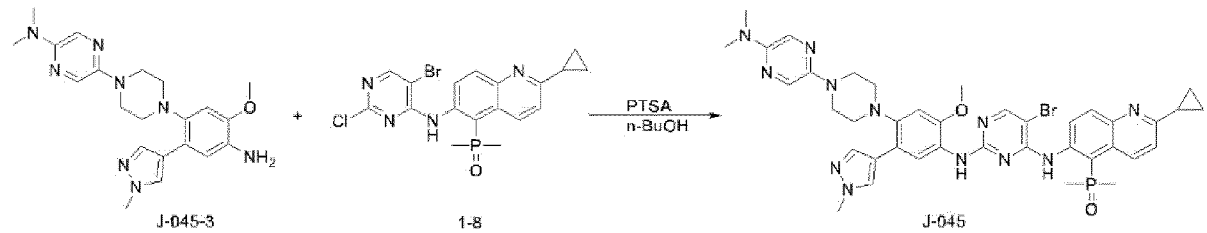


The synthesis method of compound J-045 is the same as that of compound J-043, except that the starting compound J-043-8 is replaced with compound J-045-3. MS of compound J-045: 823.26 $[M+H]^+$.

**Example 46 Synthesis of compound J-046**

*(6-((5-Bromo-2-((2-methoxy-5-(2-methyl-2H-1,2,3-triazol-4-yl)-4-(4-(4-methylpiperazin-1-yl)piperidin-1-yl)phenyl)amino)pyrimidin-4-yl)amino)-2-cyclopropylquinolin-5-yl)dimethylphosphine oxide*

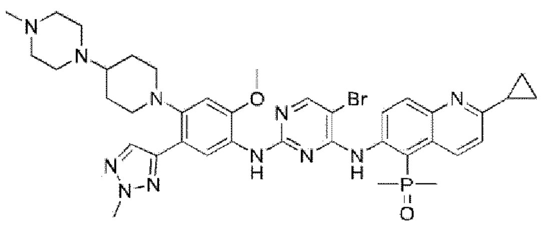

Step 1: Synthesis of Compound J-046-2

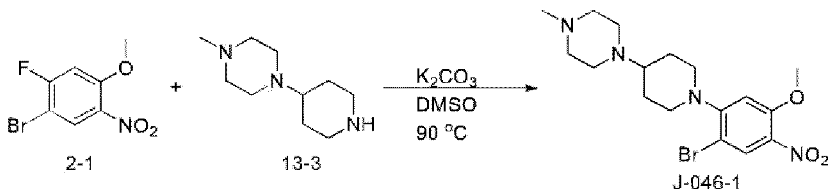


The synthesis method of compound J-046-1 is the same as that of compound J-043-2, except that the starting compound J-043-1 is replaced by compound 13-3. MS of compound J-046-1: 413.11 $[M+H]^+$.

Step 2: Synthesis of Compound J-046-3

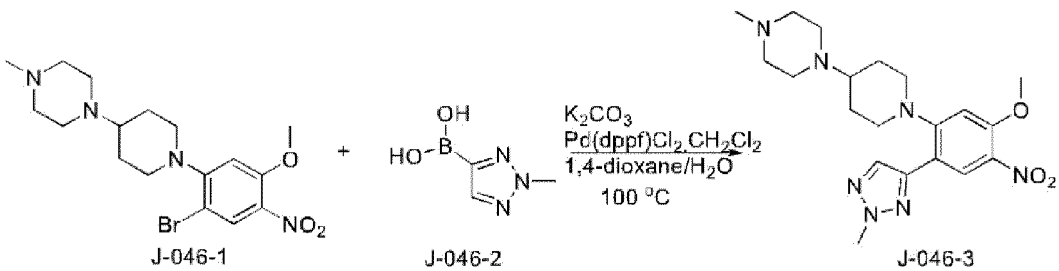


The synthesis method of compound J-046-3 is the same as that of compound J-043-4, except that the starting compound J-043-2 is replaced by compound J-046-1, and the starting compound J-043-3 is replaced by compound J-046-2. MS of compound J-046-3: 416.23 $[M+H]^+$.

Step 3: Synthesis of Compound J-046-4

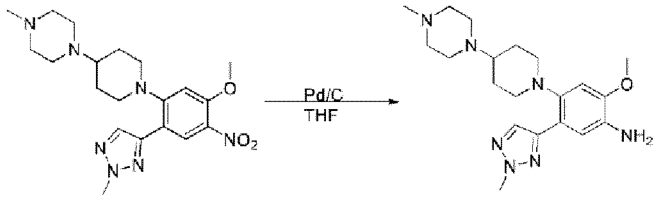


J-046-3 J-046-4

The synthesis method of compound J-046-4 is the same as that of compound J-043-8, except that the starting compound J-043-7 is replaced by compound J-046-3. MS of compound J-046-4: 386.26 $[M+H]^+$.

Step 4: Synthesis of Compound J-046

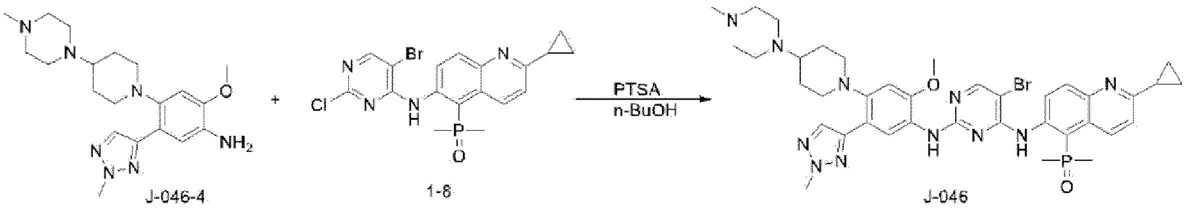


J-046-4 1-8 J-046

The synthesis method of compound J-046 is the same as that of compound J-043, except that the starting compound J-043-8 is replaced by compound J-046-4. MS of compound J-046: 800.28 $[M+H]^+$.

**Example 47 Synthesis of compound J-047**

*(6-((5-Bromo-2-((2-methoxy-5-(2-methyl-2H-1,2,3-triazol-4-yl)-4-(4-(pyridin-4-yl)piperazin-1-yl)phenyl)amino)pyrimidin-4-yl)amino)-2-cyclopropylquinolin-5-yl)dimethylphosphine oxide*

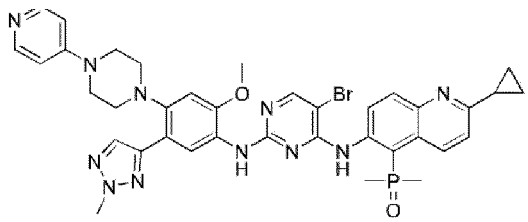

Step 1: Synthesis of Compound J-047-2

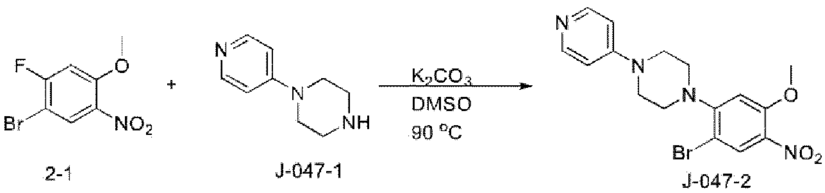


2-1 J-047-1 J-047-2

The synthesis method of compound J-047-2 is the same as that of compound J-043-2, except that the starting compound J-043-1 is replaced by compound J-047-1. MS of compound J-047-2: 393.05 $[M+H]^+$.

Step 2: Synthesis of Compound J-047-3

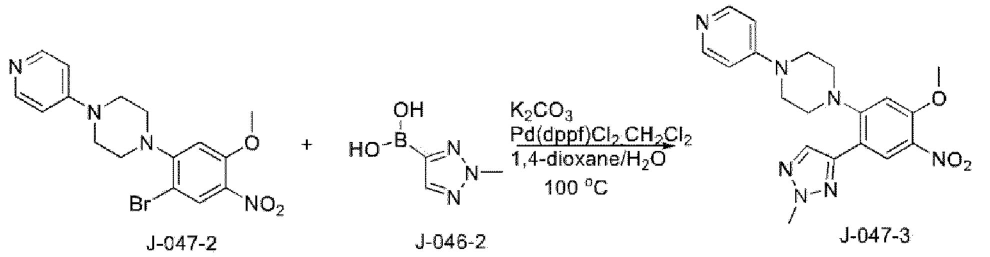


J-047-2 J-046-2 J-047-3

The synthesis method of compound J-047-3 is the same as that of compound J-043-4, except that the starting compound J-043-2 is replaced by compound J-047-2, and the starting compound J-043-3 is replaced by compound J-046-2. MS of compound J-047-3: 396.17 $[M+H]^+$

Step 3: Synthesis of Compound J-047-4

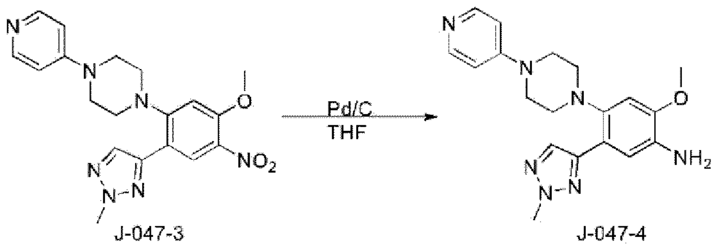


J-047-3 J-047-4

The synthesis method of compound J-047-4 is the same as that of compound J-043-8, except that the starting compound J-043-7 is replaced by compound J-047-3. MS of compound J-047-4: 366.20 $[M+H]^+$.

Step 4: Synthesis of Compound J-047

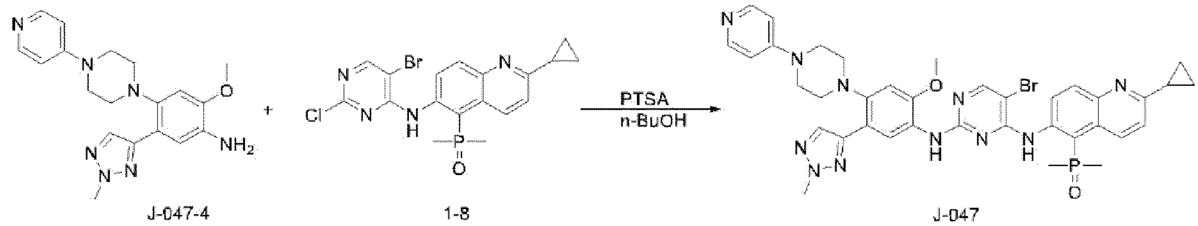


J-047-4 1-8 J-047

The synthesis method of compound J-047 is the same as that of compound J-043, except that the starting compound J-043-8 is replaced by compound J-047-4. MS of compound J-047: 780.22 $[M+H]^+$.

**Example 48 Synthesis of compound J-048**

*(6-((5-Bromo-2-((2-methoxy-5-(1-methyl-1H-pyrazol-4-yl)-4-(4-(thiazol-2-yl)piperazin-1-yl)phenyl)amino)pyrimidin-4-yl)amino)-2-cyclopropylquinolin-5-yl)dimethylphosphine oxide*

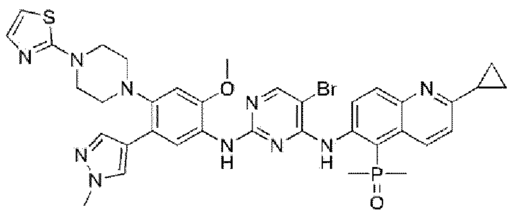

Step 1: Synthesis of Compound J-048-1

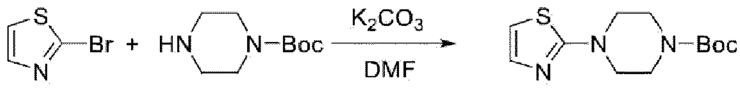


J-048-1

2-Bromothiazole (1.00 g, 6.13 mmol, 1.00 eq), tert-butyl piperazine-1-carboxylate (1.71 g, 9.20 mmol, 1.50 eq), $K_2CO_3$ (2.54 g, 18.39 mml, 3.00 eq) and DMF (20 mL) were mixed and reacted at 100°C for 72 hours. The reaction solution was cooled to room temperature, poured into 50 mL of water, filtered, washed with 15 mL of water twice, and dried to obtain the product J-048-1 (1.1 g, 4.08 mmol, yield: 66.98%). MS: 270.12 $[M+H]^+$

Step 2: Synthesis of Compound J-048-2

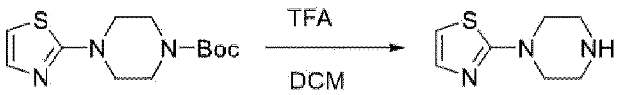


J-048-1 J-048-2

Compound J-048-1 (1.1 g, 4.08 mmol) was dissolved in DCM (10 mL), followed with adding TFA (3 mL). The mixture reacted at room temperature for 30 min. The reaction solution was spin-dried, added with 20 mL saturated aqueous sodium carbonate solution and 20 mL dichloromethane, separated, dried with anhydrous sodium sulfate, filtered, and spin-dried to obtain compound J-048-2 (570 mg, 3.37 mmol, yield: 82.47%). MS: 170.07 $[M+H]^+$

Step 3: Synthesis of Compound J-048-3

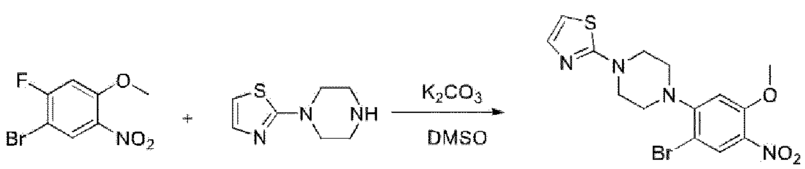


2-1 J-048-2 J-048-3

The synthesis method of compound J-048-3 is the same as that of compound 2-2, except that the starting compound morpholine is replaced by compound J-048-2. MS of compound 12-1: 399.00 $[M+H]^+$

Step 4: Synthesis of Compound J-048-4

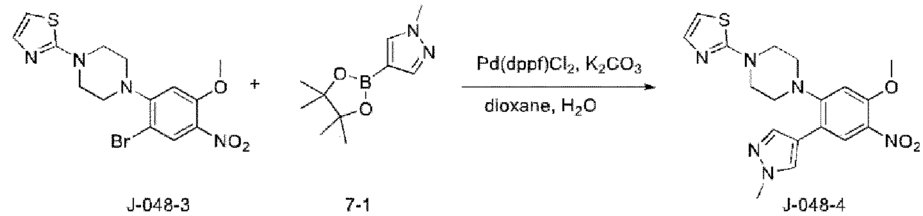


J-048-3 7-1 J-048-4

The synthesis method of compound J-048-4 is the same as that of compound 7-2, except that the starting compound 1-6 is replaced by compound J-048-3. MS of compound J-048-4: 401.13 [M+H]$^+$.

Step 5: Synthesis of Compound J-048-5

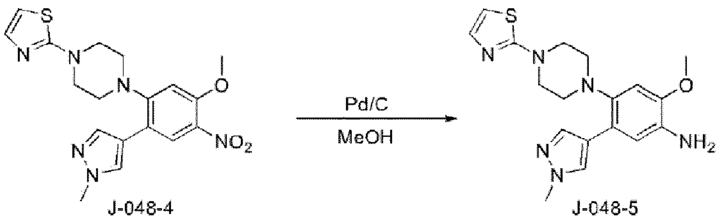


J-048-4 J-048-5

The synthesis method of compound J-048-5 is the same as that of compound 1-11, except that the starting compound 1-10 is replaced by compound J-048-4. MS of compound J-048-5: 371.16 [M+H]$^+$.

Step 6: Synthesis of Compound J-048

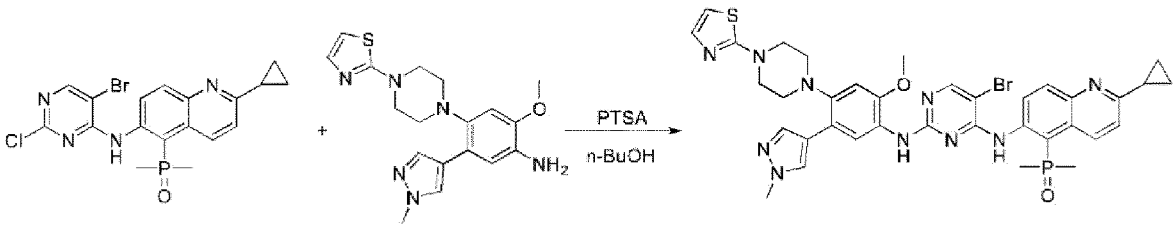


1-8 J-048-5 J-048

The synthesis method of compound J-048 is the same as that of compound J-001, except that the starting compound 1-11 is replaced by compound J-048-5. MS of compound J-048: 785.18 [M+H]$^+$.

**Example 49 Synthesis of compound J-049**

*(6-((5-Bromo-2-((2-methoxy-4-(4-(6-methoxypyrazin-2-yl)piperazin-1-yl)-5-(1-methyl-1H-pyrazol-4-yl)phenyl)amino)pyrimidin-4-yl)amino)-2-cyclopropylquinolin-5-yl)dimethylphosphine oxide*

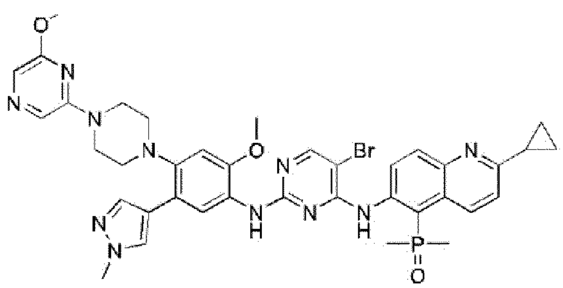

Step 1: Synthesis of Compound J-049-1

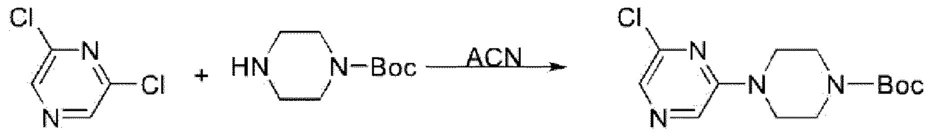


J-049-1

2,6-Dichloropyrazine (1.00 g, 6.75 mmol, 1.00 eq), tert-butyl piperazine-1-carboxylate (1.89 g, 10.13 mmol, 1.50 eq), ACN (20 mL) were mixed well and reacted at 80°C for 2 hours. It was monitored by LCMS until the reaction was completed. The reaction solution was spin-dried, and purified by column chromatography petroleum with petroleum ether : ethyl acetate (3:1) to obtain the product J-049-1 (1.17 g, 3.92 mmol, yield: 57.95%). MS: 299.12 $[M+H]^+$

Step 2: Synthesis of Compound J-049-2

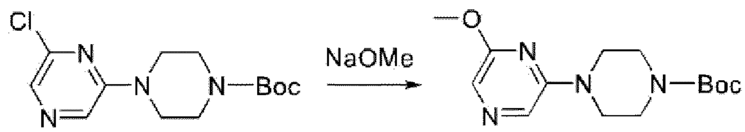


J-049-1 J-049-2

Compound J-049-1 (1.17 g, 3.92 mmol, 1.00 eq) was dissolved in MeOH (20 mL), followed by adding sodium methoxide (2.11 g, 39.16 mmol, 10.00 eq), and the reaction was refluxed for 4 days. The reaction solution was spin-dried, purified by reversed-phase chromatography with water (containing 0.05% formic acid) as mobile phase A and a mobile phase B (gradient: 0-85% B) for 25 minutes. The resultant was spin-dried to remove the solvent to obtain compound J-049-2 (780 mg, 2.65 mmol, yield: 67.67%). MS: 295.17 $[M+H]^+$

Step 3: Synthesis of Compound J-049-3

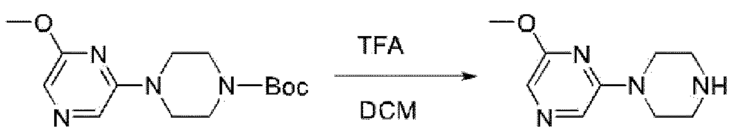


J-049-2 J-049-3

The synthesis method of compound J-049-3 is the same as that of compound J-048-2, except that the starting compound J-048-2 is replaced with compound J-049-2. MS of compound J-049-3: 195.12 $[M+H]^+$

Step 4: Synthesis of Compound J-049-4

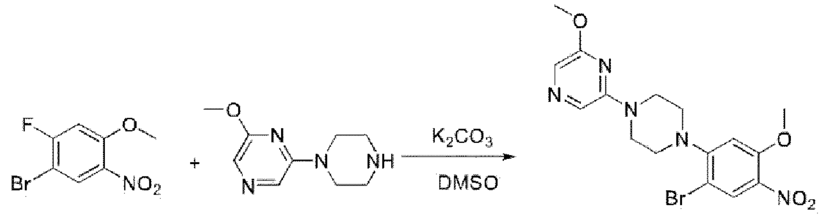


2-1 J-049-3 J-049-4

The synthesis method of compound J-048-4 is the same as that of compound 2-2, except that the starting compound morpholine is replaced by compound J-049-3. MS of compound J-049-4: 424.05 $[M+H]^+$.

Step 5: Synthesis of Compound J-049-5

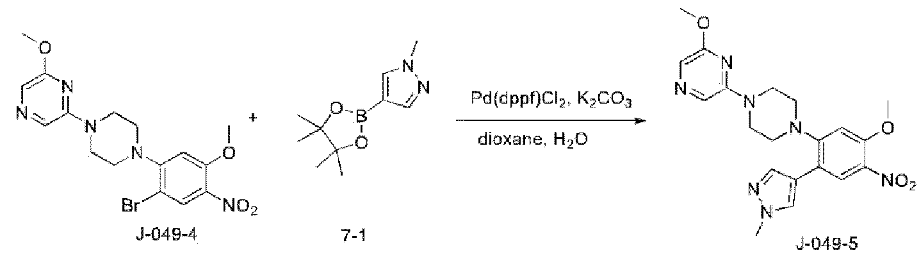


J-049-4 7-1 J-049-5

The synthesis method of compound J-049-5 is the same as that of compound 7-2, except that the starting compound 1-6 is replaced by compound J-049-4. MS of compound J-049-5: 426.18 $[M+H]^+$.

Step 6: Synthesis of Compound J-049-6

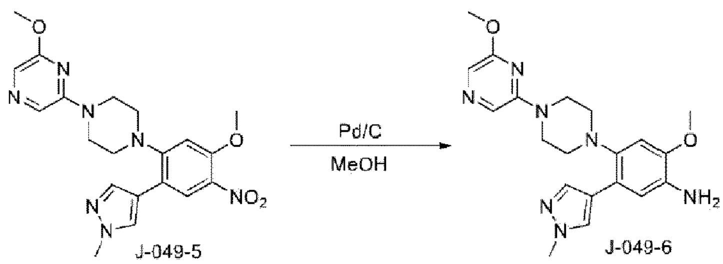


J-049-5 J-049-6

The synthesis method of compound J-049-5 is the same as that of compound 1-11, except that the starting compound 1-10 is replaced by compound J-049-4. MS of compound J-048-5: 396.21 $[M+H]^+$.

Step 6: Synthesis of Compound J-049

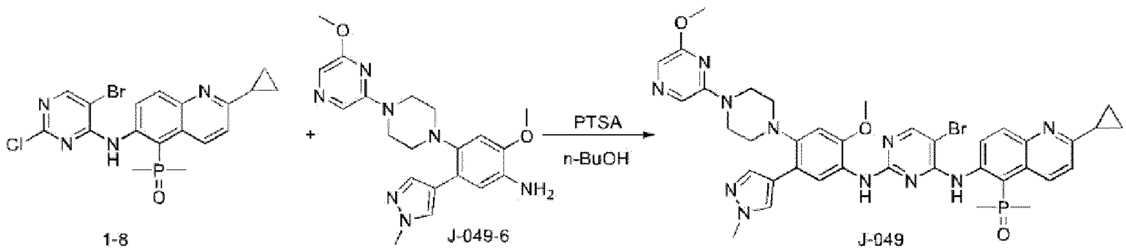


1-8 J-049-6 J-049

The synthesis method of compound J-049 is the same as that of compound J-001, except that the starting compound 1-11 is replaced by compound J-049-6. MS of compound J-049: 810.23 $[M+H]^+$.

**Example 50 Synthesis of compound J-050**

*6-(4-(4-((5-Bromo-4-((2-cyclopropyl-5-(dimethylphosphoryl)quinolin-6-yl)amino)pyrimidin-2-yl)amino)-5-methoxy-2-(1-methyl-1H-pyrazol-4-yl)phenyl)piperazin-1-yl)-1-methylpyridin-2(1H)-one*

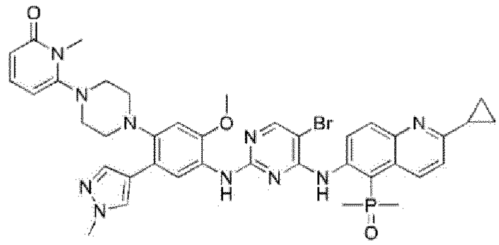

Step 1: Synthesis of Compound J-050-1

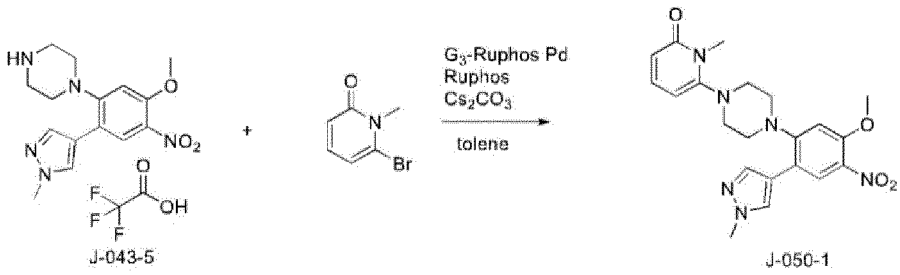


J-043-5

J-050-1

Compound J-043-5 (271 mg, 0.63 mmol, 1.00 eq), 6-bromo-1-methylpyridin-2(1H)-one (141 mg, 0.76 mmol, 1.20 eq), 2-dicyclohexylphosphine-2',6'-diisopropoxy-1,1'-biphenyl (58 mg, 0.126 mmol, 0.20 eq), RuPhos Pd G3 (52 mg, 0.06 mmol, 0.10 eq) and cesium carbonate (617 mg, 1.89 mmol, 3.00eq) were mixed well. The reaction was carried out at 100°C for 8 hours under nitrogen protection. It was monitored by LCMS until the reaction was completed. The reaction solution was spin-dried and purified by column chromatography with DCM : MeOH (15:1) to obtain the product J-050-1 (120 mg, 0.28 mmol, yield: 44%). MS: 425.19 $[M+H]^+$

Step 2: Synthesis of Compound J-050-2

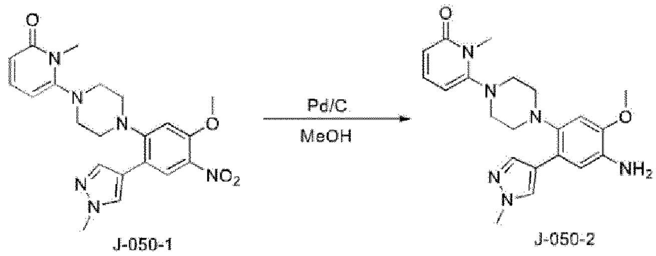


J-050-1

J-050-2

The synthesis method of compound J-050-2 is the same as that of compound 1-11, except that the starting compound 1-10 is replaced by compound J-050-1. MS of compound J-050-2: 395.21 $[M+H]^+$.

Step 3: Synthesis of Compound J-050

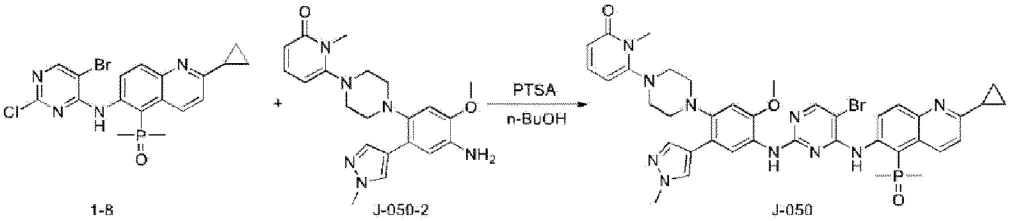


1-8 J-050-2 J-050

The synthesis method of compound J-050 is the same as that of compound J-001, except that the starting compound 1-11 is replaced by compound J-050-2. MS of compound J-049: 809.24 $[M+H]^+$.

**Example** 51 Synthesis of compound J-051

*(6-((5-Bromo-2-((4-(4-(6-(2-hydroxypropan-2-yl)pyridin-2-yl)piperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide*

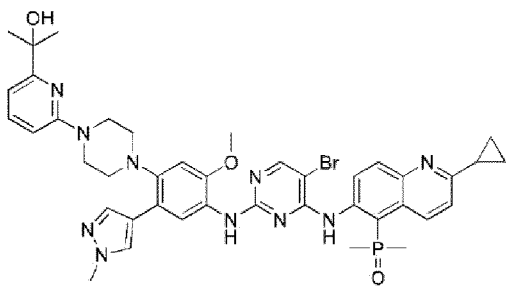

Step 1: Synthesis of Compound J-051-1

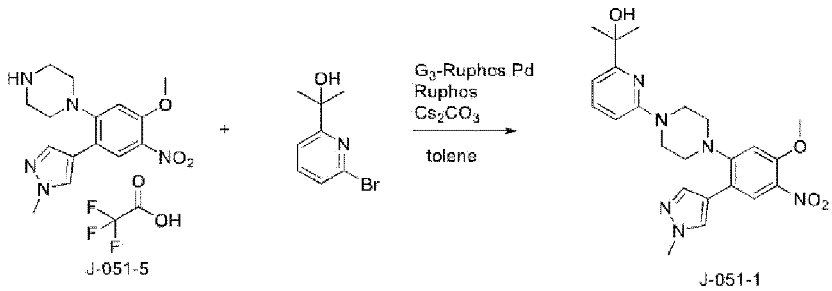


J-051-5 J-051-1

The synthesis of compound J-051-1 is the same as that of J-050-1, except that 6-bromo-1-methylpyridin-2(1H)-one is replaced by 2-(6-bromopyridin-2-yl)propan-2-ol. MS of compound J-051-1: 453.22 $[M+H]^+$

Step 2: Synthesis of Compound J-051-2

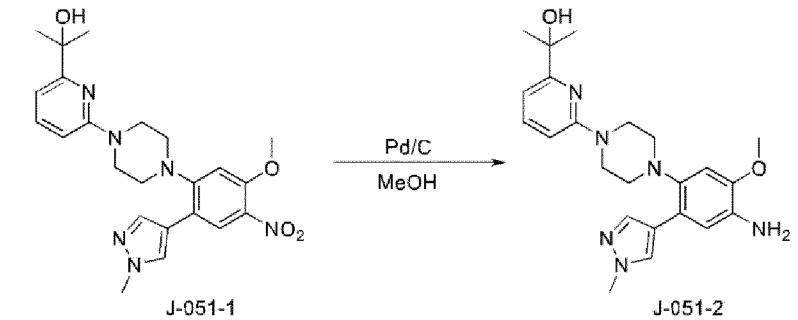


J-051-1 J-051-2

The synthesis method of compound J-051-2 is the same as that of compound 1-11, except that the starting compound 1-10 is replaced by compound J-051-1. MS of compound J-051-2: 423.24 $[M+H]^+$.

Step 3: Synthesis of Compound J-051

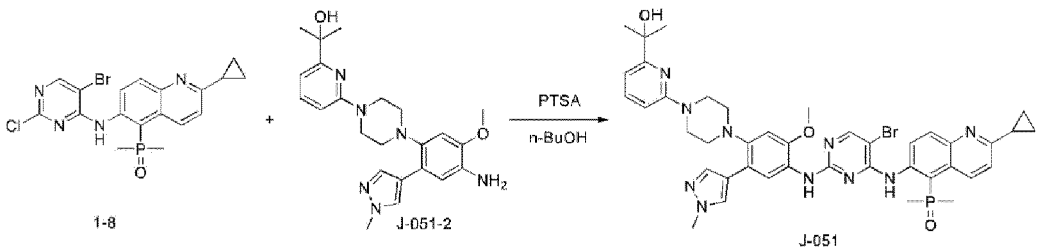


1-8 J-051-2 J-051

The synthesis method of compound J-051 is the same as that of compound J-001, except that the starting compound 1-11 is replaced by compound J-051-2. MS of compound J-051: 837.27 $[M+H]^+$.

**Example 52 Synthesis of compound J-052**

*(6-((5-Bromo-2-((2-methoxy-5-(1-methyl-1H-pyrazol-4-yl)-4-(((1-methylpiperidine-4-yl)methyl)amino)phenyl)amino)pyrimidin-4-yl)amino)-2-cyclopropylquinolin-5-yl)dimethylphosphine oxide*

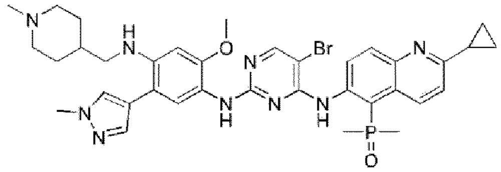

Step 1: Synthesis of Compound J-052-1

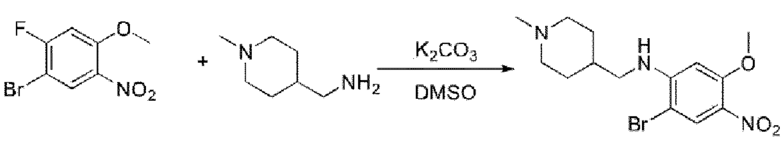


2-1 J-052-1

The synthesis method of compound J-052-1 is the same as that of compound 2-2, except that morpholine is replaced by (1-methylpiperidin-4-yl)methylamine. MS of compound J-052-1: 358.07 $[M+H]^+$.

Step 2: Synthesis of Compound J-052-2

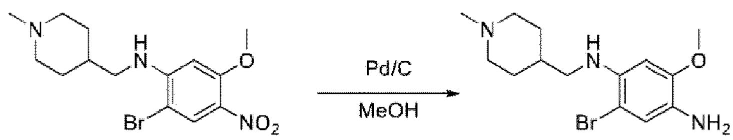


J-052-1 J-052-2

The synthesis method of compound J-052-2 is the same as that of compound 1-11, except that the starting compound 1-10 is replaced by compound J-052-2. MS of compound J-052-2: 328.09 [M+H]$^+$.

Step 3: Synthesis of Compound J-052

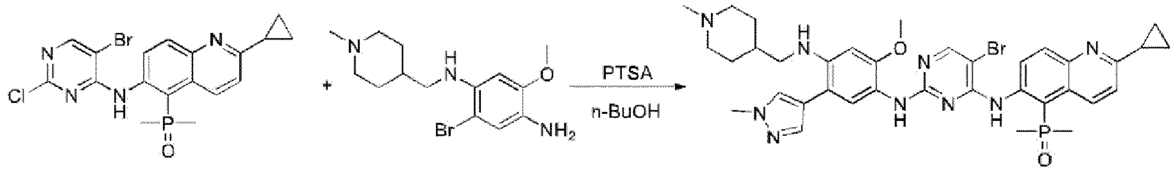


1-8 J-052-2 J-052

The synthesis method of compound J-052 is the same as that of compound J-001, except that the starting compound 1-11 is replaced by compound J-052-2. MS of compound J-052: 744.25 [M+H]$^+$.

**Example 53 Synthesis of compound J-053**

*(6-((5-Bromo-2-((2-methoxy-5-(1-methyl-1H-pyrazol-4-yl)-4-(((tetrahydro-2H-pyran-4-yl)methyl)amino)phenyl)amino)pyrimidin-4-yl)amino)-2-cyclopropylquinolin-5-yl)dimethylphosphine oxide*

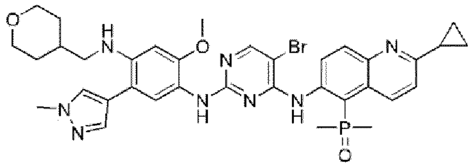

Step 1: Synthesis of Compound J-053-1

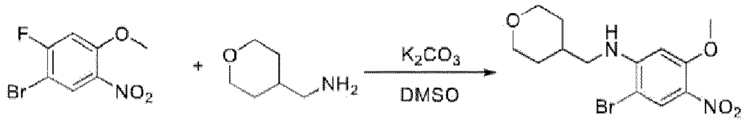


2-1 J-053-1

The synthesis method of compound J-053-1 is the same as that of compound 2-2, except that morpholine is replaced by (tetrahydro-2H-pyran-4-yl)methylamine. MS of compound J-053-1: 345.04 [M+H]$^+$.

Step 2: Synthesis of Compound J-053-2

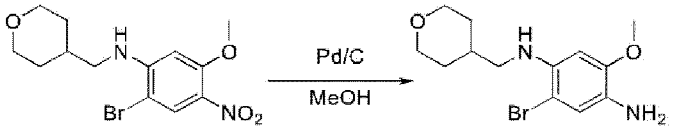


J-053-1 J-053-2

The synthesis method of compound J-053-2 is the same as that of compound 1-11, except that the starting compound 1-10 is replaced by compound J-053-2. MS of compound J-053-2: 315.06 $[M+H]^+$.

Step 3: Synthesis of Compound J-053

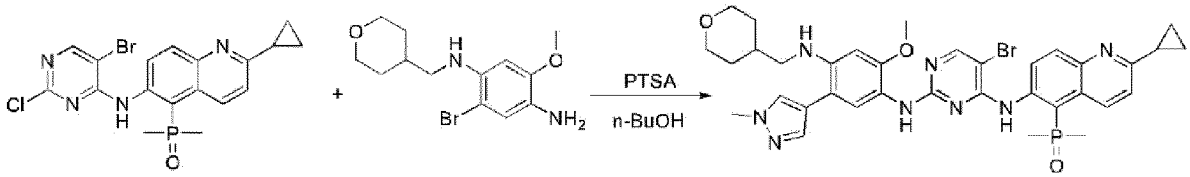


1-8 J-053-2 J-053

The synthesis method of compound J-053 is the same as that of compound J-001, except that the starting compound 1-11 is replaced by compound J-053-2. MS of compound J-053: 731.21 $[M+H]^+$.

**Example 54 Synthesis of compound J-054**

*(6-((5-Bromo-2-((2-methoxy-5-(1-methyl-1H-pyrazol-4-yl)-4-(4-methylpiperazin-1-yl)phenyl)amino)pyrimidin-4-yl)amino)-2-cyclopropylquinolin-5-yl)dimethylphosphine oxide*

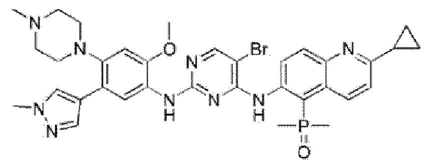

Step 1: Synthesis of Compound J-054-1

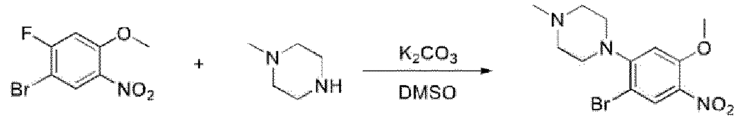


2-1 J-054-1

The synthesis method of compound J-054-1 is the same as that of compound 2-2, except that morpholine is replaced by 1-methylpiperazine. MS of compound J-054-1: 330.04 $[M+H]^+$

Step 2: Synthesis of Compound J-054-2

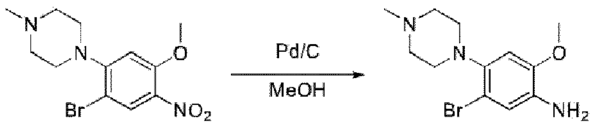


J-054-1 J-054-2

The synthesis method of compound J-054-2 is the same as that of compound 1-11, except that the starting compound 1-10 is replaced by compound J-054-1. MS of compound J-054-2: 300.06 $[M+H]^+$

Step 3: Synthesis of Compound J-054

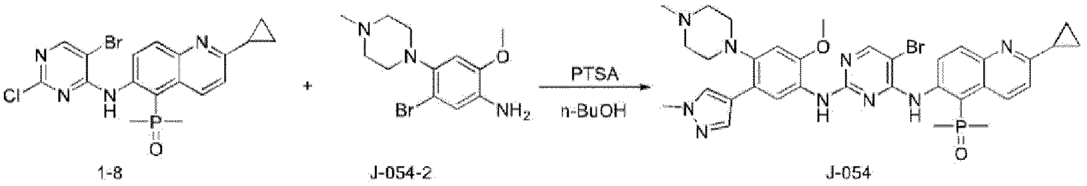


1-8 J-054-2 J-054

The synthesis method of compound J-054 is the same as that of compound J-001, except that the starting compound 1-11 is replaced by compound J-054-2. MS of compound J-054: 716.21 $[M+H]^+$

**Example 55 Synthesis of compound J-055**

*(6-((5-Bromo-2-((4-(4-cyclopentylpiperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide*

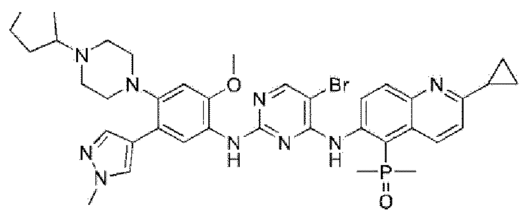

Step 1: Synthesis of Compound J-055-2

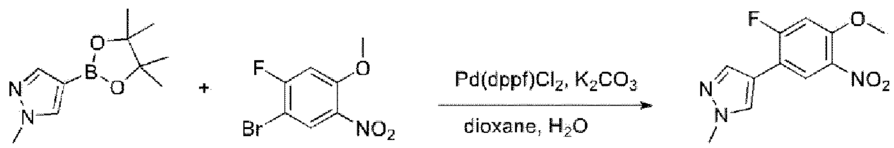


7-1 2-1 J-055-2

The synthesis method of compound J-055-2 is the same as that of compound 8-2, except that starting compound 8-1 is replaced by compound 7-1. MS of compound J-055-2: 252.07 $[M+H]^+$.

Step 2: Synthesis of Compound J-055-4

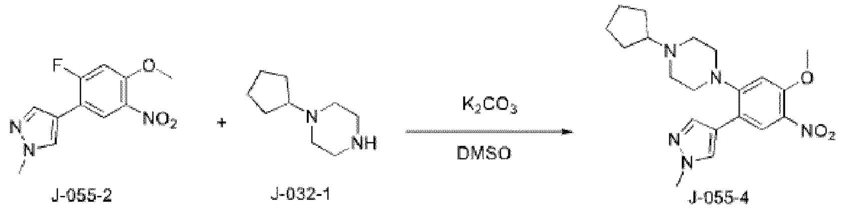


The synthesis method of compound J-055-4 is the same as that of compound 8-3, except that the starting compound 8-2 is replaced by compound J-055-2, and the starting compound morpholine is replaced by compound J-032-1. MS of compound J-055-4: 386.21 $[M+H]^+$.

Step 3: Synthesis of Compound J-055-5

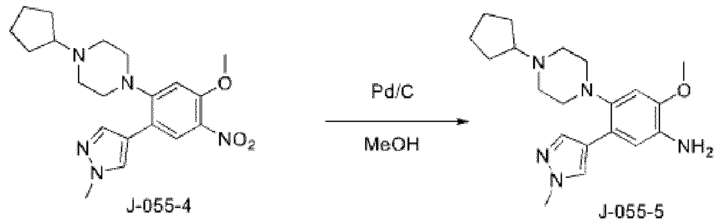


The synthesis method of compound J-055-5 is the same as that of compound 8-4, except that starting compound 8-4 is replaced by compound J-055-4. MS of compound J-055-5: 356.24 $[M+H]^+$.

Step 4: Synthesis of Compound J-055

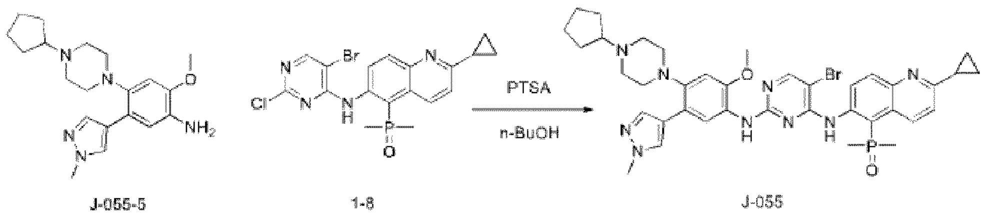


The synthesis method of compound J-055 is the same as that of compound J-008, except that starting compound 8-4 is replaced by compound J-055-5. MS of compound J-055: 770.26 $[M+H]^+$.

**Example 56 Synthesis of compound J-056**

*(6-((5-Bromo-2-((2-methoxy-5-(1-methyl-1H-pyrazol-4-yl)-4-(4-(pyridin-3-yl)piperazin-1-yl)phenyl)amino)pyrimidin-4-yl)amino)-2-cyclopropylquinolin-5-yl)dimethylphosphine oxide*

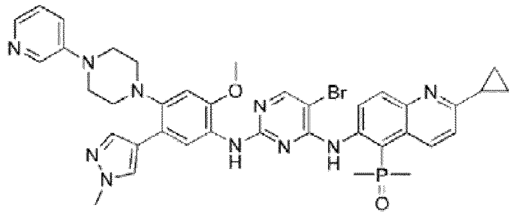

Step 1: Synthesis of Compound J-056-2

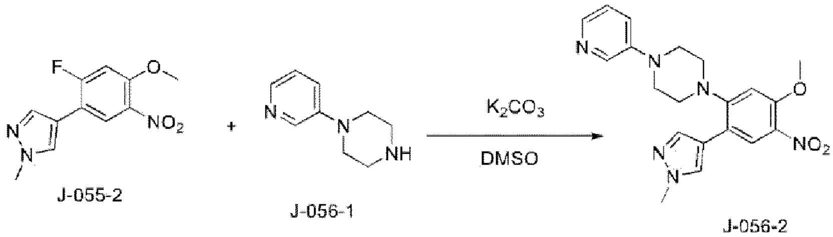


J-055-2 J-056-1 J-056-2

The synthesis method of compound J-056-2 is the same as that of compound 8-3, except that the starting compound 8-2 is replaced by compound J-055-2, and the starting compound morpholine is replaced by compound J-056-1. MS of compound J-056-2: 395.18 $[M+H]^+$.

Step 2: Synthesis of Compound J-056-3

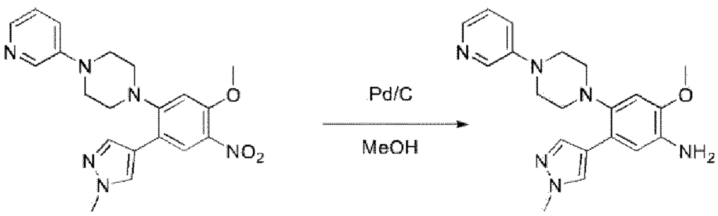


J-056-2 J-056-3

The synthesis method of compound J-056-3 is the same as that of compound 8-4, except that starting compound 8-4 is replaced by compound J-056-2. MS of compound J-056-3: 365.20 $[M+H]^+$.

Step 3: Synthesis of Compound J-056

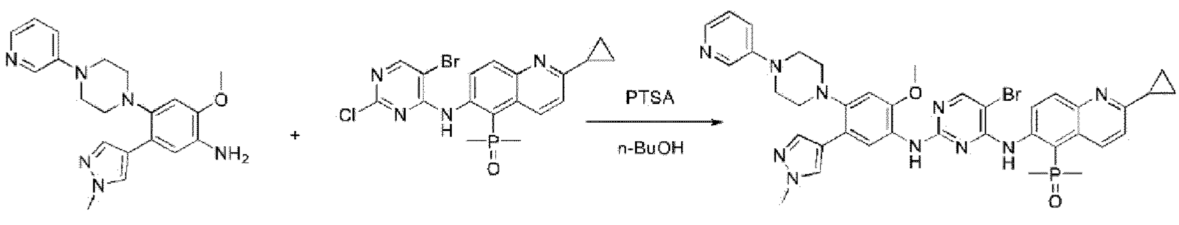


J-056-3 1-8 J-056

The synthesis method of compound J-056 is the same as that of compound J-008, except that starting compound 8-4 is replaced by compound J-056-3. MS of compound J-056: 779.23 $[M+H]^+$.

**Example 57 Synthesis of compound J-057**

*(6-((5-Bromo-2-((2-methoxy-5-(1-methyl-1H-pyrazol-4-yl)-4-(4-(pyridin-4-yl)piperazin-1-yl)phenyl)amino)pyrimidin-4-yl)amino)-2-cyclopropylquinolin-5-yl)dimethylphosphine oxide*

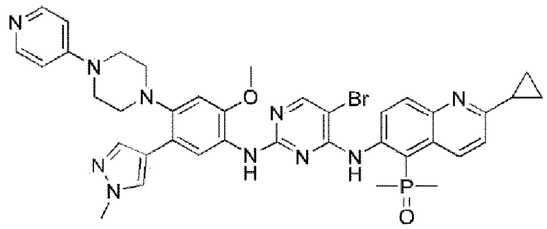

Step 1: Synthesis of Compound J-057-2

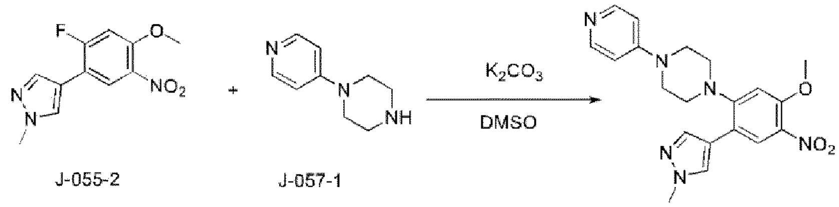


J-055-2 J-057-1 J-057-2

The synthesis method of compound J-057-2 is the same as that of compound 8-3, except that the starting compound 8-2 is replaced by compound J-055-2, and the starting compound morpholine is replaced by compound J-057-1. MS of compound J-057-2: 395.18 $[M+H]^+$.

Step 2: Synthesis of Compound J-056-3

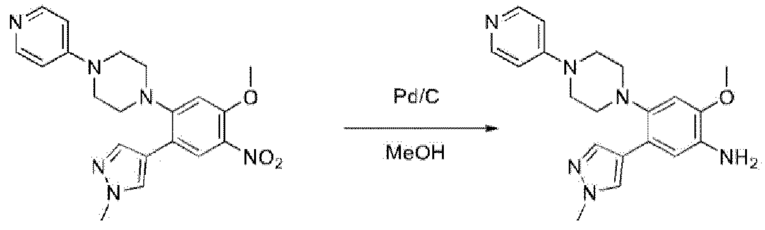


J-057-2 J-057-3

The synthesis method of compound J-057-3 is the same as that of compound 8-4, except that starting compound 8-4 is replaced by compound J-057-2. MS of compound J-057-3: 365.20 $[M+H]^+$.

Step 3: Synthesis of Compound J-057

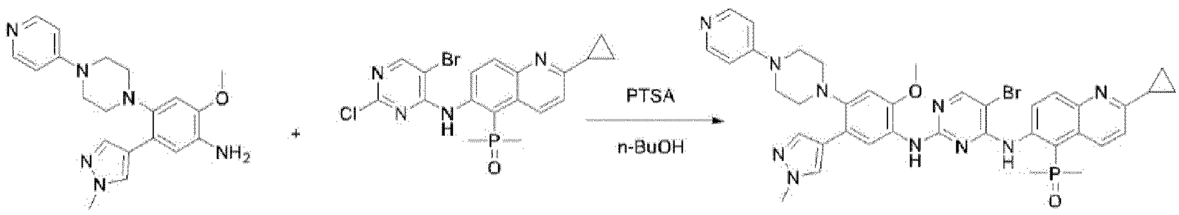


J-057-3 1-8 J-057

The synthesis method of compound J-057 is the same as that of compound J-008, except that starting compound 8-4 is replaced by compound J-057-3. MS of compound J-057: 779.23 $[M+H]^+$.

**Example 58 Synthesis of compound J-058**

*(6-((5-Bromo-2-((2-methoxy-5-(1-methyl-1H-pyrazol-4-yl)-4-(4-(pyrazin-2-yl)piperazin-1-yl)phenyl)amino)pyrimidin-4-yl)amino)-2-cyclopropylquinolin-5-yl)dimethylphosphine oxide*

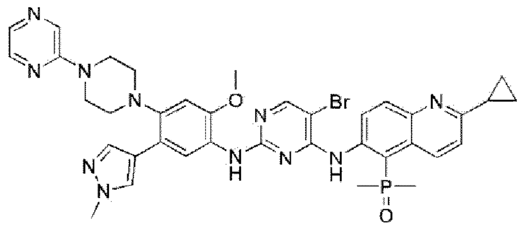

Step 1: Synthesis of Compound J-058-2

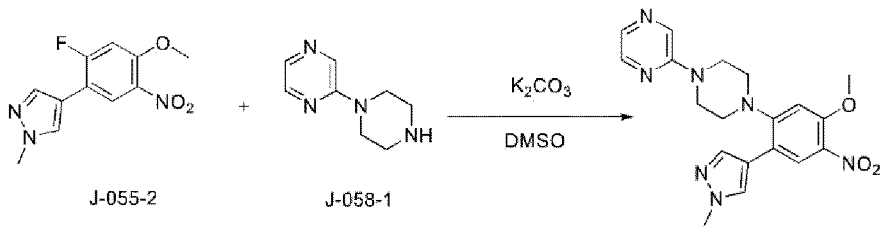


J-055-2 J-058-1 J-058-2

The synthesis method of compound J-058-2 is the same as that of compound 8-3, except that the starting compound 8-2 is replaced by compound J-055-2, and the starting compound morpholine is replaced by compound J-058-1. MS of compound J-058-2: 396.17 $[M+H]^+$.

Step 2: Synthesis of Compound J-058-3

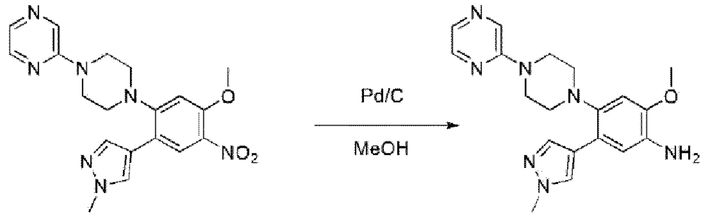


J-058-2

J-058-3

The synthesis method of compound J-058-3 is the same as that of compound 8-4, except that starting compound 8-4 is replaced by compound J-058-2. MS of compound J-058-3: 366.20 $[M+H]^+$.

Step 3: Synthesis of Compound J-058

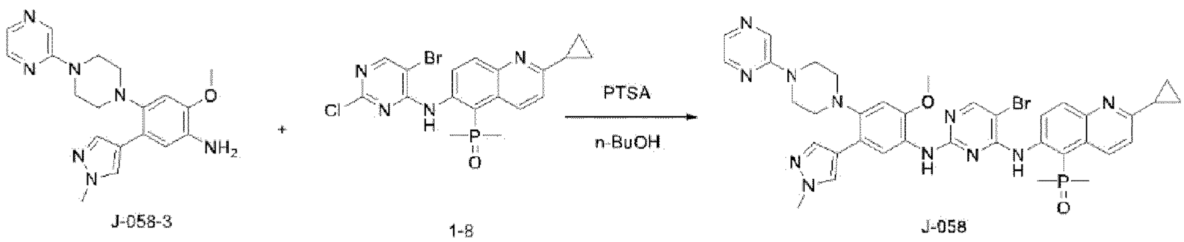


J-058-3

1-8

J-058

The synthesis method of compound J-058 is the same as that of compound J-008, except that starting compound 8-4 is replaced by compound J-058-3. MS of compound J-058: 780.22 $[M+H]^+$.

Example 59 Synthesis of compound J-059

*(6-((5-Bromo-2-((2-methoxy-5-(1-methyl-1H-pyrazol-4-yl)-4-(4-morpholinopiperidine-1-yl)phenyl)amino)pyrimidin-4-yl)amino)-2-cyclopropylquinolin-5-yl)dimethylphosphine oxide*

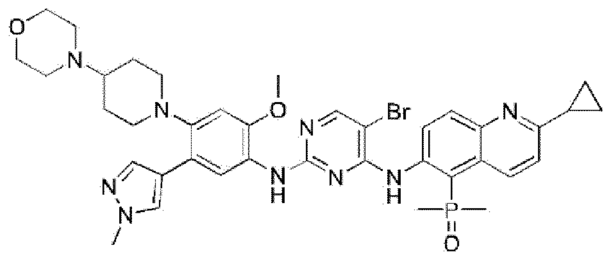

Step 1: Synthesis of Compound J-059-2

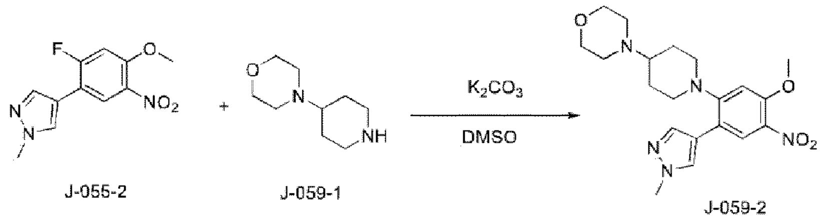


J-055-2

J-059-1

J-059-2

The synthesis method of compound J-059-2 is the same as that of compound 8-3, except that the starting compound 8-2 is replaced by compound J-055-2, and the starting compound morpholine is replaced by compound J-059-1. MS of compound J-059-2: 402.21 $[M+H]^+$.

Step 2: Synthesis of Compound J-059-3

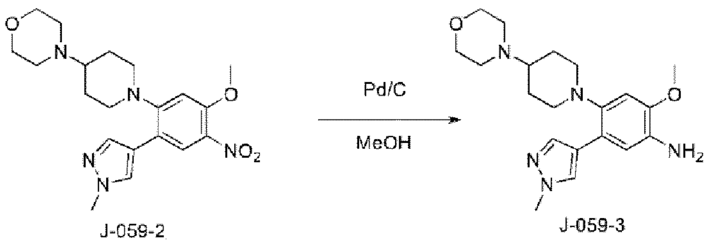


J-059-2 J-059-3

The synthesis method of compound J-059-3 is the same as that of compound 8-4, except that starting compound 8-4 is replaced by compound J-059-2. MS of compound J-059-3: 372.23 $[M+H]^+$.

Step 3: Synthesis of Compound J-059

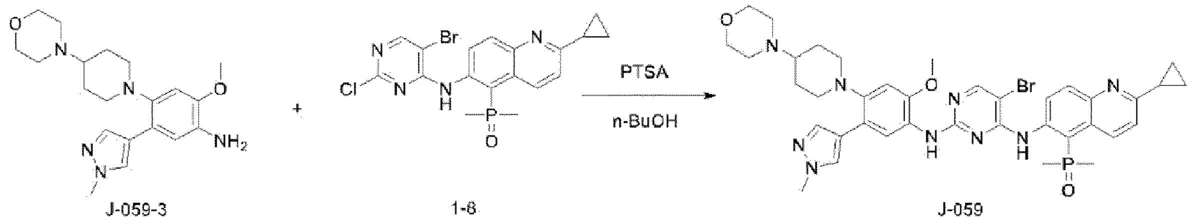


J-059-3 1-8 J-059

The synthesis method of compound J-059 is the same as that of compound J-008, except that starting compound 8-4 is replaced by compound J-059-3. MS of compound J-059: 786.26 $[M+H]^+$.

**Example 60 Synthesis of compound J-060**

*(6-((5-Bromo-2-((2-methoxy-5-(1-methyl-1H-pyrazol-4-yl)-4-(4-(pyrimidin-4-yl)piperazin-1-yl)phenyl)amino)pyrimidin-4-yl)amino)-2-cyclopropylquinolin-5-yl)dimethylphosphine oxide*

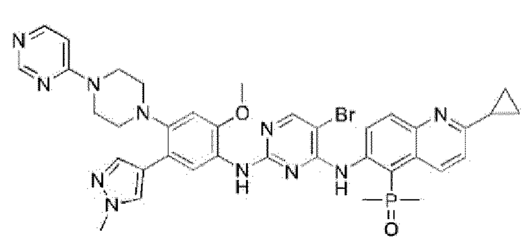

Step 1: Synthesis of Compound J-060-2

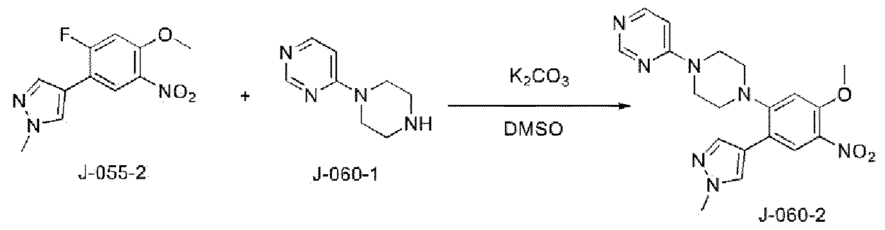


J-055-2 J-060-1 J-060-2

The synthesis method of compound J-060-2 is the same as that of compound 8-3, except that the starting compound 8-2 is replaced by compound J-055-2, and the starting compound morpholine is replaced by compound J-060-1. MS of compound J-060-2: 396.17 $[M+H]^+$.

Step 2: Synthesis of Compound J-060-3

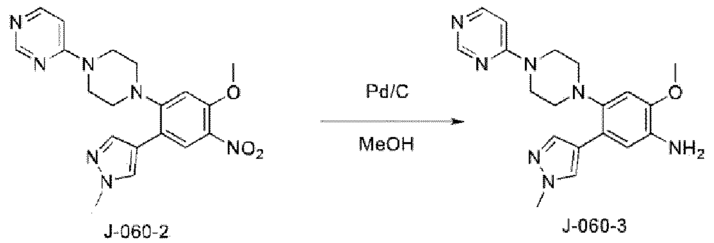


J-060-2 J-060-3

The synthesis method of compound J-060-3 is the same as that of compound 8-4, except that starting compound 8-4 is replaced by compound J-060-2. MS of compound J-060-3: 366.20 $[M+H]^+$.

Step 3: Synthesis of Compound J-060

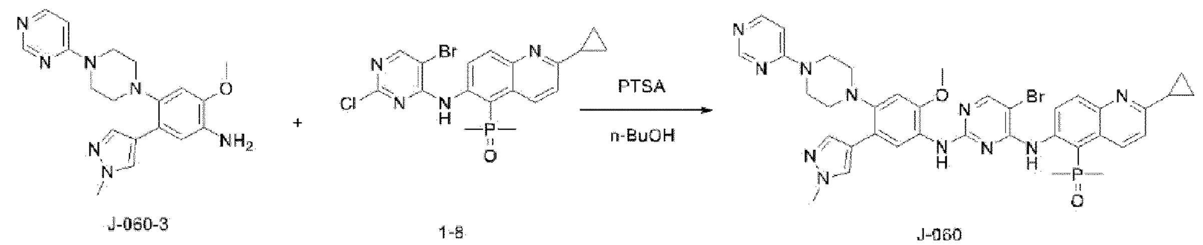


J-060-3 1-8 J-060

The synthesis method of compound J-060 is the same as that of compound J-008, except that starting compound 8-4 is replaced by compound J-060-3. MS of compound J-060: 780.22 $[M+H]^+$.

Example 61 Synthesis of compound J-061

*(6-((5-Bromo-2-((2-methoxy-5-(1-methyl-1H-pyrazol-4-yl)-4-(3-methyl-3,6-diazabicyclo[3.1.1]heptyl-6-yl)phenyl)amino)pyrimidin-4-yl)amino)-2-cyclopropylquinolin-5-yl)dimethylphosphine oxide*

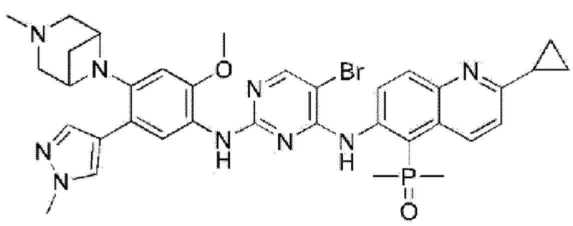

Step 1: Synthesis of Compound J-061-2

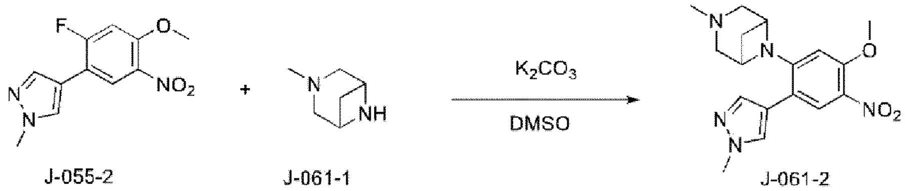


J-055-2 J-061-1 J-061-2

The synthesis method of compound J-061-2 is the same as that of compound 8-3, except that the starting compound 8-2 is replaced by compound J-055-2, and the starting compound morpholine is replaced by compound J-061-1. MS of compound J-061-2: 344.16 [M+H]$^+$.

Step 2: Synthesis of Compound J-061-3

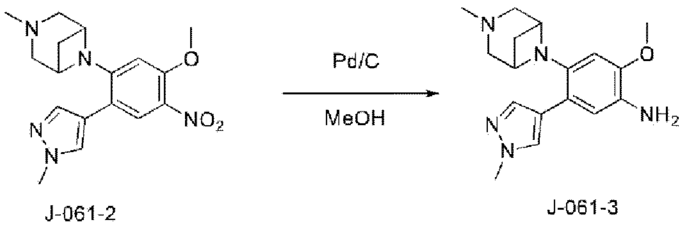


J-061-2 J-061-3

The synthesis method of compound J-061-3 is the same as that of compound 8-4, except that starting compound 8-4 is replaced by compound J-061-2. MS of compound J-061-3: 314.19 [M+H]$^+$.

Step 3: Synthesis of Compound J-061

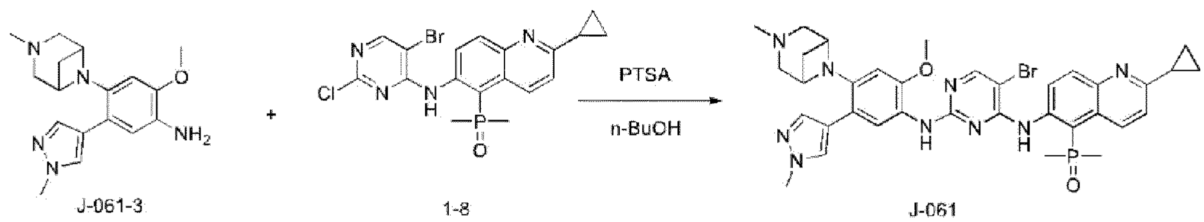


J-061-3 1-8 J-061

The synthesis method of compound J-061 is the same as that of compound J-008, except that starting compound 8-4 is replaced by compound J-061-3. MS of compound J-061: 728.21 [M+H]$^+$.

**Example 62 Synthesis of compound J-062**

*(6-((5-Bromo-2-((4-((2-(dimethylamino)ethyl)(methyl)amino)-2-methoxy-5-(1-methyl-1H-pyrazol-4-yl)phenyl)amino)pyrimidin-4-yl)amino)-2-cyclopropylquinolin-5-yl)dimethylphosphine oxide*

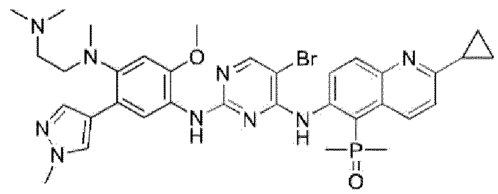

Step 1: Synthesis of Compound J-062-2

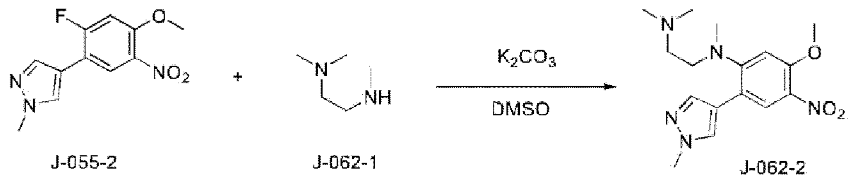


J-055-2 J-062-1 J-062-2

The synthesis method of compound J-062-2 is the same as that of compound 8-3, except that the starting compound 8-2 is replaced by compound J-055-2, and the starting compound morpholine is replaced by compound J-062-1. MS of compound J-062-2: 334.18 $[M+H]^+$.

Step 2: Synthesis of Compound J-062-3

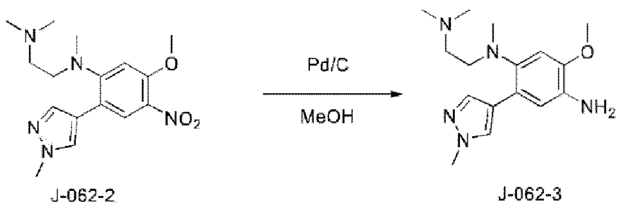


J-062-2 J-062-3

The synthesis method of compound J-062-3 is the same as that of compound 8-4, except that starting compound 8-4 is replaced by compound J-062-2. MS of compound J-062-3: 304.21 $[M+H]^+$.

Step 3: Synthesis of Compound J-062

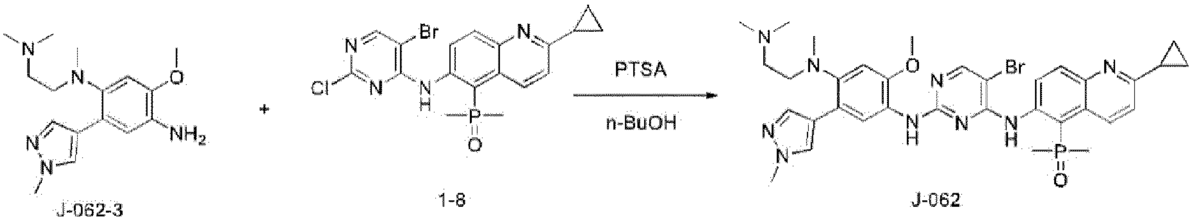


J-062-3 1-8 J-062

The synthesis method of compound J-062 is the same as that of compound J-008, except that starting compound 8-4 is replaced by compound J-062-3. MS of compound J-062: 718.23 $[M+H]^+$.

**Example 63 Synthesis of compound J-063**

*4-(4-(4-((5-bromo-4-((2-cyclopropyl-5-(dimethylphosphoryl)quinolin-6-yl)amino)pyrimidin-2-yl)amino)-5-methoxy-2-(1-methyl-1H-pyrazol-4-yl)phenyl)piperazin-1-yl)pyridin-2(1H)one*

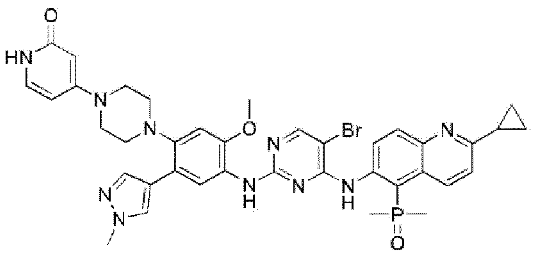

Step 1: Synthesis of Compound J-063-2

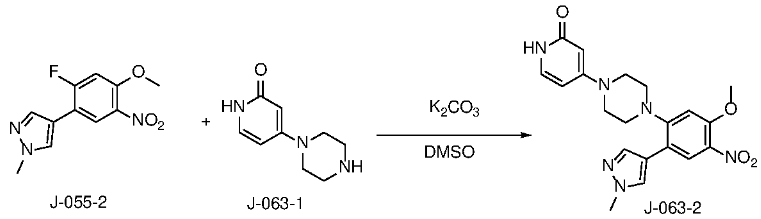


J-055-2 J-063-1 J-063-2

The synthesis method of compound J-063-2 is the same as that of compound 8-3, except that the starting compound 8-2 is replaced by compound J-055-2, and the starting compound morpholine is replaced by compound J-063-1. MS of compound J-063-2: 411.17 $[M+H]^+$.

Step 2: Synthesis of Compound J-063-3

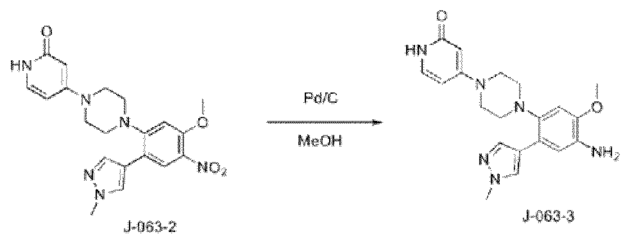


J-063-2 J-063-3

The synthesis method of compound J-063-3 is the same as that of compound 8-4, except that starting compound 8-4 is replaced by compound J-063-2. MS of compound J-063-3: 381.20 $[M+H]^+$.

Step 3: Synthesis of Compound J-063

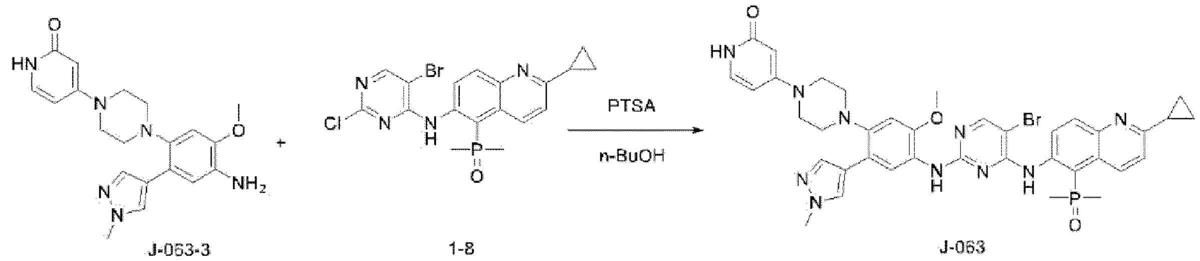


J-063-3 1-8 J-063

The synthesis method of compound J-063 is the same as that of compound J-008, except that starting compound 8-4 is replaced by compound J-063-3. MS of compound J-063: 795.22 $[M+H]^+$.

Example 64 Synthesis of compound J-064

*(6-((5-Bromo-2-((2-methoxy-5-(1-methyl-1H-pyrrol-3-yl)-4-(4-(4-methylpiperazine-1-yl)piperidin-1-yl)phenyl)amino)pyrimidin-4-yl)amino)-2-cyclopropylquinolin-5-yl)dimethylphosphine oxide*

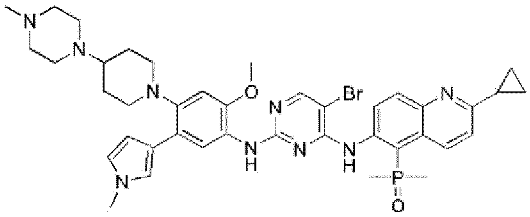

Step 1: Synthesis of Compound J-064-2

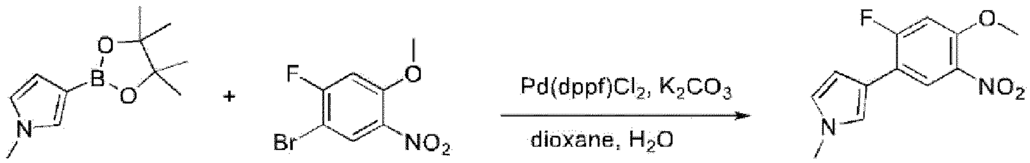


J-064-1 2-1 J-064-2

The synthesis method of compound J-064-2 is the same as that of compound 8-2, except that starting compound 8-1 is replaced by compound J-064-1. MS of compound J-064-2: 251.08 $[M+H]^+$.

Step 2: Synthesis of Compound J-064-3

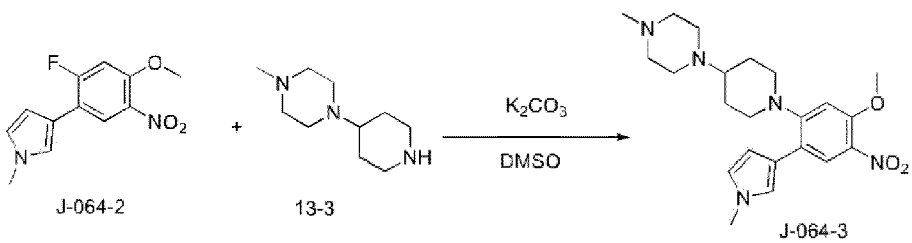


J-064-2 13-3 J-064-3

The synthesis method of compound J-064-3 is the same as that of compound 8-3, except that the starting compound 8-2 is replaced by compound J-064-2, and the starting compound morpholine is replaced by compound 13-3.

MS of compound J-064-3: 414.24 [M+H]$^+$.

Step 3: Synthesis of Compound J-064-4

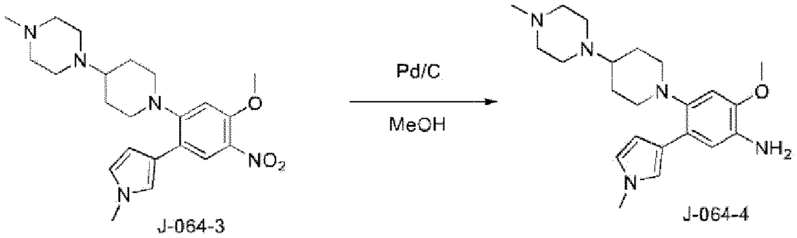


J-064-3 J-064-4

The synthesis method of compound J-064-4 is the same as that of compound 8-4, except that starting compound 8-4 is replaced by compound J-064-3. MS of compound J-064-4: 384.27 [M+H]$^+$.

Step 4: Synthesis of Compound J-064

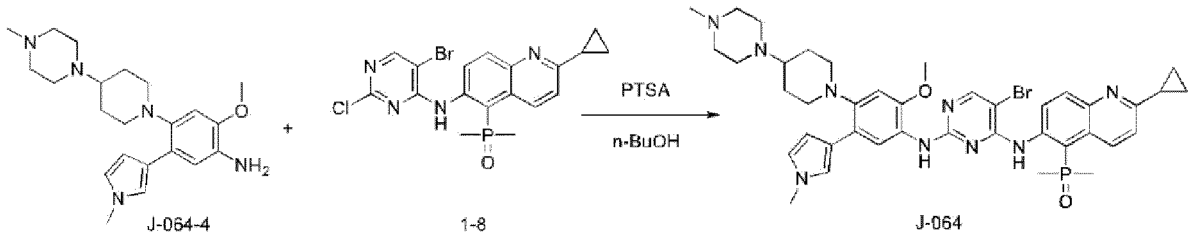


J-064-4 1-8 J-064

The synthesis method of compound J-064 is the same as that of compound J-008, except that starting compound 8-4 is replaced by compound J-064-4. MS of compound J-064: 798.29 [M+H]$^+$.

Example 65 Synthesis of compound J-065

*(2-Cyclopropyl-6-((5-fluoro-2-((2-methoxy-5-(1-methyl-1H-pyrazol-4-yl)-4-(4-(4-methylpiperazin-1-yl)piperidin-1-yl)phenyl)aminopyrimidin-4-yl)amino)quinolin-5-yl)dimethylphosphine oxide*

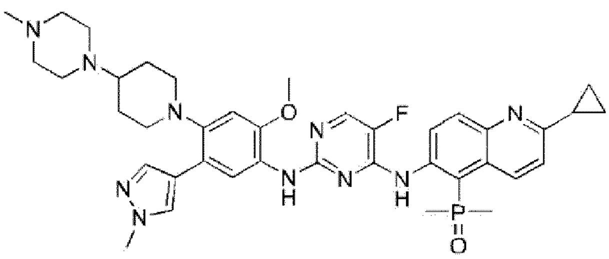

Step 1: Synthesis of Compound J-065-1

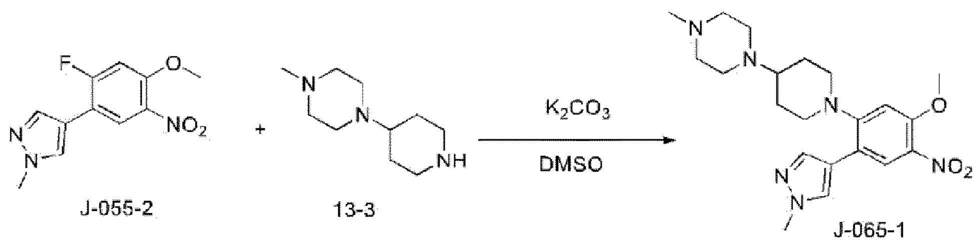


J-055-2 13-3 J-065-1

The synthesis method of compound J-065-1 is the same as that of compound 8-3, except that the starting compound 8-2 is replaced by compound J-055-2, and the starting compound morpholine is replaced by compound 13-3. MS of compound J-065-1: 415.24 $[M+H]^+$.

Step 2: Synthesis of Compound J-065-2

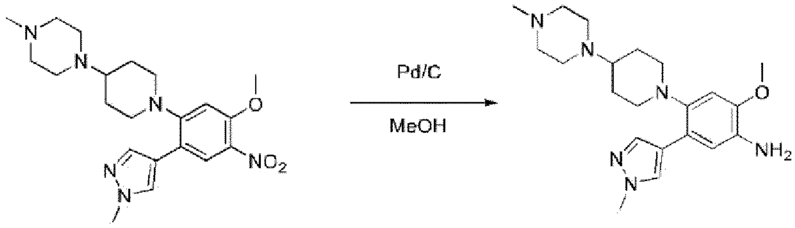


J-065-1 J-065-2

The synthesis method of compound J-065-2 is the same as that of compound 8-4, except that starting compound 8-4 is replaced by compound J-065-1. MS of compound J-065-2: 385.26 $[M+H]^+$.

Step 3: Synthesis of Compound J-065-3

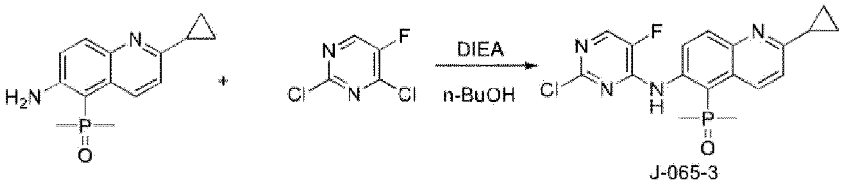


J-065-3

The synthesis method of compound J-065-3 is the same as that of compound 1-8, except that the starting compound 5-bromo-2,4-dichloropyrimidine is replaced by compound 5-fluoro-2,4-dichloropyrimidine. MS of J-065-3: 391.08 $[M+H]^+$.

Step 4: Synthesis of Compound J-065

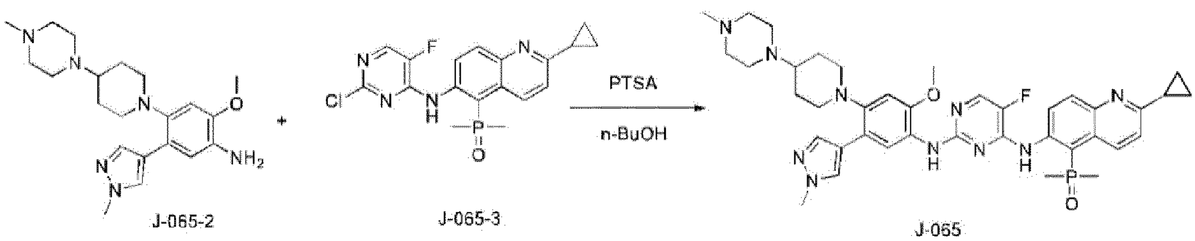


J-065-2 J-065-3 J-065

The synthesis method of compound J-065 is the same as that of compound J-008, except that the starting compound 8-4 is replaced by compound J-065-2, the starting compound 1-8 is replaced by compound J-065-3. MS of compound J-065: 739.37 [M+H]+.

**Example 66 Synthesis of compound J-066**

(6-((5-Bromo-2-((2-methoxy-5-(1-methyl-1H-pyrazol-4-yl)-4-(4-(1-methylpiperidine-4-yl)piperazin-1-yl)phenyl)amino)pyrimidin-4-yl)amino)-2-cyclopropylquinolin-5-yl)dimethylphosphine oxide

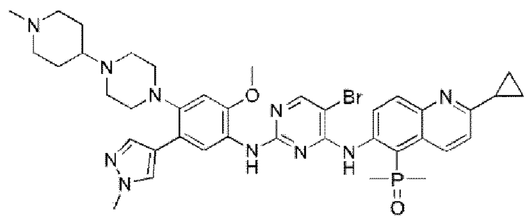

Step 1: Synthesis of Compound J-066-1

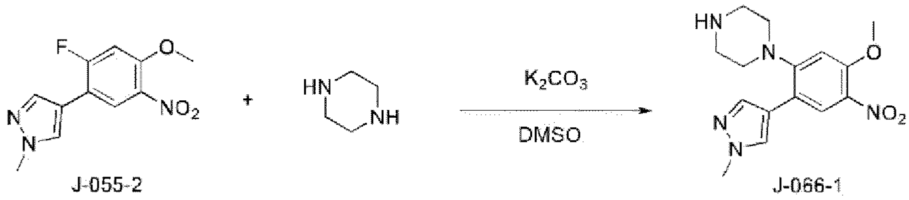


J-055-2 J-066-1

The synthesis method of compound J-066-1 is the same as that of compound 8-3, except that the raw material compound 8-2 is replaced by compound J-055-2, and the raw material compound morpholine is replaced by compound piperazine. MS of compound J-066-1: 318.15 [M+H]+.

Step 2: Synthesis of Compound J-066-2

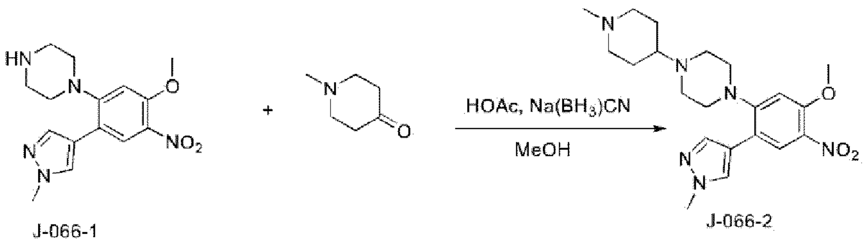


J-066-1 J-066-2

Compound J-066-1 (317 mg, 1.00 mmol), 1-methyl-4-piperidone (339 mg, 3.00 mmol) and HOAc (120 mg, 2 mmol) were added to methanol (10 mL). The mixture was heated to 65°C under nitrogen protection and stirred for 1 hour. After cooling, sodium cyanoborohydride (126 mg, 2 mmol) was added, and the mixture was stirred for 16 hours at 25°C under nitrogen protection. It was monitored by TLC until the reaction was completed. The reaction solution was spin-dried and purified by column chromatography (dichloromethane: methanol=20:1). Pale yellow solid J-066-2 (100 mg) was obtained. MS of compound J-066-2: 415.14 [M+H]+.

Step 3: Synthesis of Compound J-066-3

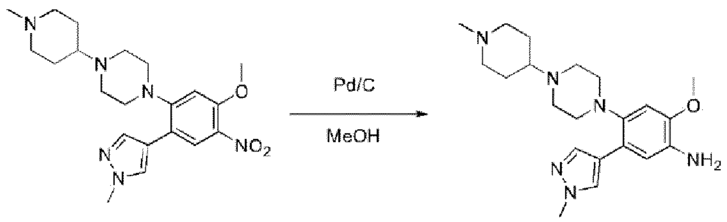


J-066-2 J-066-3

The synthesis method of compound J-066-3 is the same as that of compound 8-4, except that starting compound 8-4 is replaced by compound J-066-2. MS of compound J-066-3: 385.26 $[M+H]^+$.

Step 4: Synthesis of Compound J-066

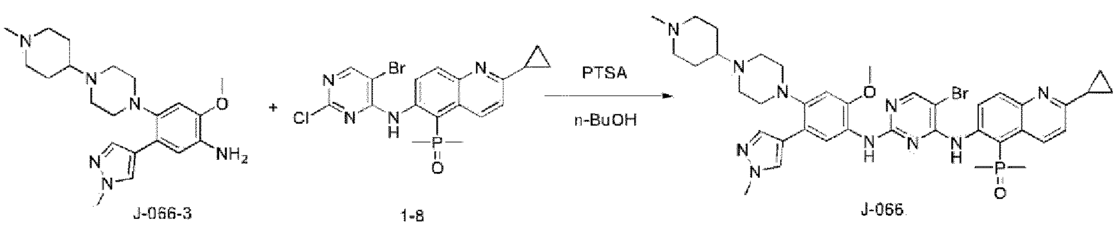


J-066-3 1-8 J-066

The synthesis method of compound J-066 is the same as that of compound J-008, except that starting compound 8-4 is replaced by compound J-066-3. MS of compound J-066: 799.29 $[M+H]^+$.

**Example 67 Synthesis of compound J-067**

*(6-((5-bromo-2-((5-(1-(difluoromethyl)-1H-pyrazol-4-yl)-2-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide*

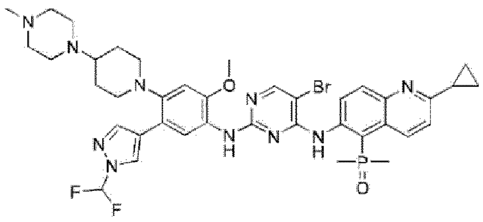

Step 1: Synthesis of Compound J-067-2

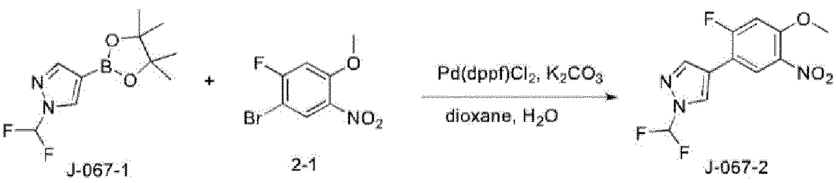


J-067-1 2-1 J-067-2

The synthesis method of compound J-067-2 is the same as that of compound 8-2, except that starting compound 8-1 is replaced by compound J-067-1. MS of compound J-067-2: 288.05 $[M+H]^+$.

Step 2: Synthesis of Compound J-067-3

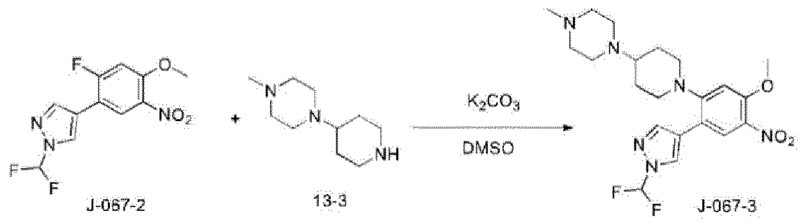


J-067-2 13-3 J-067-3

The synthesis method of compound J-067-3 is the same as that of compound 8-3, except that the starting compound 8-2 is replaced by compound J-067-2, and the starting compound morpholine is replaced by compound 13-3. MS of compound J-067-3: 451.22 [M+H]$^+$.

Step 3: Synthesis of Compound J-067-4

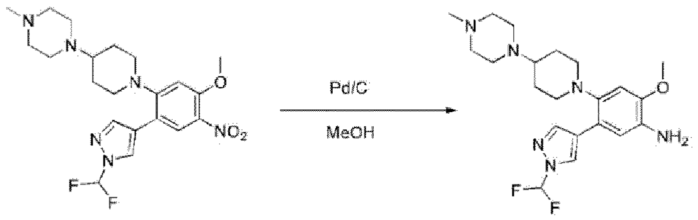


J-067-3 J-067-4

The synthesis method of compound J-067-4 is the same as that of compound 8-4, except that starting compound 8-4 is replaced by compound J-067-3. MS of compound J-067-4: 421.24 [M+H]$^+$.

Step 4: Synthesis of Compound J-067

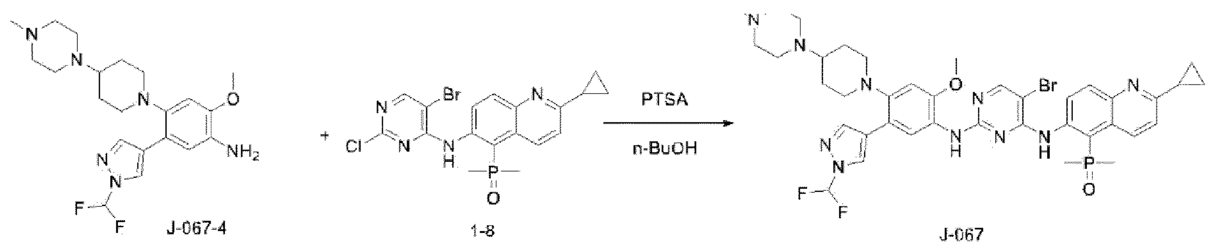


J-067-4 1-8 J-067

The synthesis method of compound J-067 is the same as that of compound J-008, except that starting compound 8-4 is replaced by compound J-067-4. MS of compound J-067: 835.27 [M+H]$^+$.

**Example 68 Synthesis of compound J-068**

(6-((5-Bromo-2-((2-methoxy-5-(1-methyl-1H-pyrazol-4-yl)-4-(4-(4-methylpiperazine-1-yl)piperidin-1-yl)phenyl)amino)pyrimidin-4-yl)amino)-2-cyclopropylquinolin-5-yl)dimethylphosphine oxide

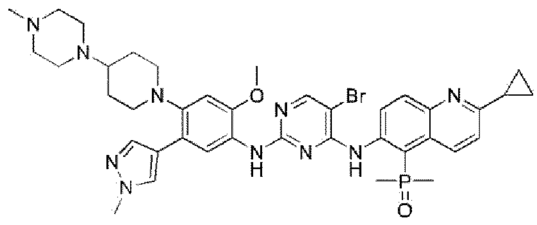

Step 1: Synthesis of Compound J-068

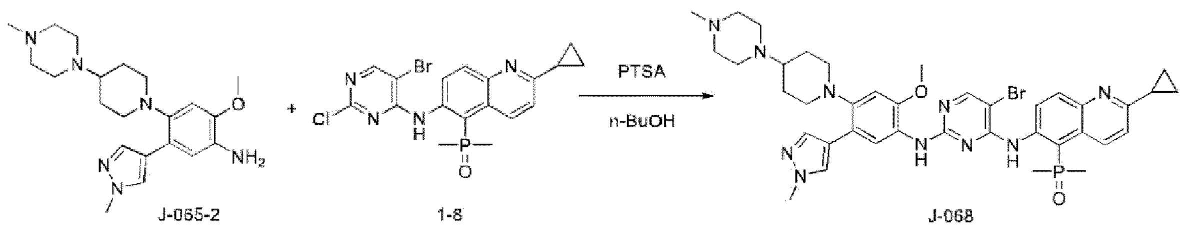


J-065-2 1-8 J-068

The synthesis method of compound J-068 is the same as that of compound J-008, except that starting compound 8-4 is replaced by compound J-065-2. MS of compound J-068: 799.29 $[M+H]^+$.

**Pharmacological experiments**

**Comparable Example 1**

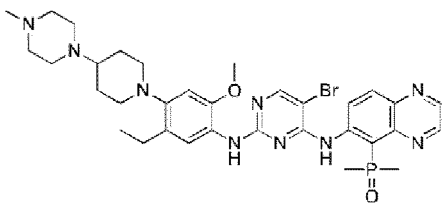

The above compound was prepared according to the synthesis process of the compound 41 in WO2019015655A1.

**Experiment 1 Kinase Inhibition Assay**

Mobility shift assays were performed to determine inhibitory activity of compounds against $EGFR^{\Delta19del/T790M/C797S}$, EGFR WT and IGF1R kinases. The enzymatic reaction scheme is as follows:

1. Preparing 1* Kinase Buffer as follows:

| 1* Kinase Buffer | Final concentration |
|---|---|
| HEPES PH7.5(mM) | 50 |
| Brij-35 | 0.0150% |
| DTT(mM) | 2 |
| $Mgcl_2$, $Mncl_2$ (mM) | 10 |

2. Preparing compound concentration gradient: the initial concentration of the compound tested in the assay was 3,000 nM or 100 nM, which is then diluted to be 100-fold final concentration in 100% DMSO in a 384-source plate. Compounds

in 10 concentrations were diluted in a 3-fold gradient with Precision. 250 nL of compound in 100-fold final concentration was transfered to OptiPlate-384F plate by using a dispenser named Echo 550.
3. Preparing kinase solution at 2.5-fold the final concentration with 1×kinase buffer.
4. Adding 10 μL of kinase solution at 2.5-fold the final concentration to compound wells and positive control wells respectively; and adding 10 μL of 1×kinase buffer to negative control wells.
5. Centrifuging the wells at 1,000 rpm for 30 seconds, mixing the reaction plate well by shaking and incubating it at room temperature for 10 minutes.
6. Preparing a mixed solution of ATP and kinase substrate at 5-fold/3-fold the final concentration with 1×Kinase buffer, respectively.
7. Initiating the reaction by adding 15 μL of the mixed solution of ATP and kinase substrate at 5-fold/3-fold the final concentration.
8. Centrifuging the 384-well plate at 1,000 rpm for 30 seconds, mixing the reaction plate well by shaking and incubating it at room temperature for a corresponding time.
9. Adding 30 μL of assay stop solution to stop the kinase reaction, and centrifuging the plate at 1,000 rpm for 30 seconds and mixing it well by shaking.
10. Reading the conversion rate with Caliper EZ Reader.
11. Calculation formula

$$\%\ \text{Inhibition} = \frac{\text{Conversion\%_max} - \text{Conversion\%_sample}}{\text{Conversion\%_max} - \text{Conversion\%_min}} \times 100,$$

wherein, "Conversion%_sample" is the conversion rate reading of the sample;
"Conversion%_min" is the mean value of the negative control wells, representing the conversion rate readings of the well without enzymatic activity; "Conversion%_max" is the mean value of the positive control wells, representing the conversion rate readings of the wells without compound inhibition.

Dose-response curve is plotted with the logarithmic concentration on the X-axis and percent inhibition on the Y-axis. The fitting of the dose-response curve is made with the module "log(inhibitor) vs. response -Variable slope" of the analysis software GraphPad Prism 5, so that the IC50 value of each compound on enzymatic activity is obtained.

The calculation formula is: Y=Bottom+(Top-Bottom)/(1+10^((LogIC50-X)*HillSlope)). The results are expressed as $IC_{50}$ values, as shown in Table 1.

Table 1

| Compound | EGFR Δ19del /T790M/C797S $IC_{50}$ (nM) | EGFR WT $IC_{50}$ (nM) | IGF1R $IC_{50}$ (nM) |
|---|---|---|---|
| **Comparable example 1** | 0.2 | 5.1 | 9.5 |
| J-001 | 0.5 | 51.7 | 63.6 |
| J-002 | 0.4 | 62.8 | 720.3 |
| J-003 | 0.5 | 172.0 | 189.4 |
| J-004 | 0.7 | 283.5 | 380.3 |
| J-005 | 0.5 | 132.2 | 74.38 |
| J-006 | 3.5 | 714 | 437 |
| J-007 | 0.2 | 30.3 | 41.0 |
| J-009 | 0.3 | 14.4 | 14.76 |
| | | | |
| J-010 | 0.2 | 79.0 | 142 |
| J-011 | 0.7 | 45.8 | 146 |
| J-012 | 0.2 | 43.9 | 279 |
| J-013 | 0.4 | 11.4 | 28.2 |
| J-014 | 0.19 | 4.7 | 58 |
| J-015 | 0.29 | 12.1 | 12.1 |

(continued)

| Compound | EGFR Δ19del /T790M/C797S $IC_{50}$ (nM) | EGFR WT $IC_{50}$ (nM) | IGF1R $IC_{50}$ (nM) |
|---|---|---|---|
| J-016 | 0.30 | 4.6 | 16.8 |
| J-017 | 0.3 | 18.4 | 246.4 |
| J-018 | 0.3 | 7.3 | 178 |
| J-019 | 0.33 | 16.0 | 222.0 |
| J-020 | 0.72 | 21 | 107 |
| J-021 | 0.3 | 19.3 | 293.4 |
| J-022 | 0.3 | 20.2 | 195 |
| J-023 | 0.28 | 17 | 27 |
| J-024 | 0.5 | 113.7 | 139.6 |
| J-025 | 43 | 2310 | >3000 |
| J-026 | 36.0 | >3000 | >3000 |
| J-027 | 0.3 | 11.1 | 61.6 |
| J-028 | 0.14 | 19 | 116 |
| J-029 | 0.22 | 41.29 | 58.05 |
| J-031 | 0.5 | 64 | 103 |
| J-032 | 1.0 | 121 | 170 |
| J-034 | 0.12 | 23 | 298 |
| J-037 | 1.0 | 138 | 210 |
| J-038 | 0.9 | 171 | 586 |
| J-039 | 0.5 | 33.5 | 21.5 |
| J-040 | 6.0 | 550 | 1162 |
| J-041 | 0.2 | 11.5 | 25 |
| J-042 | 0.3 | 21.1 | 107 |

It can be seen from Table 1 that the compounds of the present invention have a strong inhibitory effect on the $EGFR^{\Delta 19del/T790M/C797S}$ mutant kinase, but have weaker inhibitory effects on the wild-type kinase EGFR WT and IGF1R in the insulin receptor family. However, the compound of Comparative example 1 not only has a strong inhibitory effect on the $EGFR^{\Delta 19del/T790M/C797S}$ mutant kinase, but also has a strong inhibitory activity on EGFR WT and IGF1R. This shows that the compounds of the present invention have better selectivity relative to the compound of Comparative Example 1.

**Experiment 2 Cell proliferation experiment**

1. Cell Culture
Cell line:

Suspension cells: Ba/F3 cells with stable overexpression of Δ19del/T790M/C797S mutant gene, named Ba/F3-△19del/T790M/C797S; Ba/F3 cells with overexpression of EGFR WT, named Ba/F3 EGFR WT.
Adherent cells: human epidermal cancer cells A431 carrying EGFR WT.
A. Culture medium
RPMI 1640 with 10% FBS and 1% Penicillin-Streptomycin, or DMEM with 10% FBS and 1% Penicillin-Streptomycin.

B. Cell Thawing

a) Preheating a medium in a 37°C water bath.
b) Taking out the cryovial tube from the liquid nitrogen tank and placing it immediately in a 37°C water bath and making it melted completely within 1 minute.
c) Transferring the cell suspension to a 15 mL centrifuge tube containing 8 mL of medium and centrifuging it at 1,000 rpm for 5 minutes.
d) Discarding the supernatant and resuspending the cells in 1 mL medium, followed by transferring to a 75 $cm^3$ culture flask containing 15 mL medium, adding with an appropriate volume of medium, and cultivating in an incubator with 5% $CO_2$ at 37°C.

C. Cell Passaging

a) Preheating a medium in a 37°C water bath.
b) Collecting cells: for suspended cells, directly collecting the cells into a 15 mL centrifuge tube; for adherent cells, washing the cells with PBS, followed by adding an appropriate trypsin for digestion, adding medium, pipetting and transferring the cells to a 15 mL centrifuge tube. Subsequently, centrifuging the cells at 1,000 rpm for 5 minutes, discarding the supernatant, resuspending the cells, and passaging the cells according to an appropriate ratio in an incubator with 5% $CO_2$ at 37°C.

2. Compound Preparation

a) Diluting compounds being tested (20 mM stock solutions), as an initial concentration, to 10 mM with 100% DMSO, followed by diluting compounds serially in a 3-fold gradient such that each compound is diluted into 12 concentrations (Cat #P-05525, Labcyte).
b) Diluting the above compound solutions for 100-fold with culture medium to prepare 10-fold working solution.

3. Cell seeding in 96-well plate

a) Centrifuging cells in logarithmic phase at 1,000 rpm for 5 minutes, discarding the supernatant, and resuspending the cells in medium and counting.
b) Seeding cells into 96-well cell culture plate at a density of 2000 or 3000 cells/well, with 135 μL/well.

4. Compound handling

a) Adding the compound prepared in step 2 to the cell plate at 15 μL per well, resulting in a final maximum concentration of 10000 nM or 1111 nM. The compounds is diluted serially in a 3-fold gradient such that each compound is diluted into 9 concentrations, wherein the final concentration of DMSO is 0.1%. Blank control wells are added with medium (0.1% DMSO).
b) Re-incubating the cells in an incubator for 72 hours.

5. Detection

a) Adding 50 μL of CTG reagent (CellTiter Glo kit, promega, Cat#G7573) to the 96-well cell culture plate.
b) Shaking the plate for 2 minutes and reacting at room temperature for 10 minutes.
c) Reading the luminescence signal value (Lum) using PerkinElmer reader.

6. Experimental data processing
The cell survival inhibition rate of each well was calculated. The data was analyzed by GraphPad Prism 6.0 software. The dose-response curve was obtained by fitting the data with a nonlinear regression equation. The IC50 of each compound was calculated as follows:

$$\text{Cell survival inhibition rate (\%)}=(1-(\text{Lum}_{\text{compound tested}}-\text{Lum}_{\text{medium control}})/(\text{Lum}_{\text{cell control}}-\text{Lum}_{\text{medium control}}))\times 100\%$$

$$Y=Min + (Max-Min)/(1+10\text{^}((LogIC_{50}-X)*Slope));$$

X: logarithm of compound concentration. Y: cell survival inhibition rate.

The results of the cell proliferation assay were expressed as $IC_{50}$, as shown in Table 2.

Table 2

| Compound | Ba/F3/EGFR △19del /T790M/C797S $IC_{50}$ (nM) | BaF3 EGFR WT $IC_{50}$ (nM) | A431 $IC_{50}$ (nM) |
|---|---|---|---|
| **Comparable example 1** | 9 | 39.9 | 245 |
| J-001 | 26.5 | 395.6 | 1352 |
| J-003 | 25.7 | 646 | 3041 |
| J-005 | 27 | 698 | 6139 |
| J-007 | 6.0 | 146.6 | 1030 |
| J-009 | 16.3 | 168 | 383.5 |
| J-010 | 11.5 | 187.3 | 969.3 |
| J-011 | 4.7 | 190.1 | 1157 |
| J-012 | 3.4 | 129.7 | 830.2 |
| J-013 | 15.3 | 87 | 307.7 |
| J-014 | 6.0 | 108 | 514 |
| J-015 | 15.8 | 388 | 678 |
| J-016 | 5.1 | 132 | 379 |
| J-018 | 1.9 | 160 | 1501 |
| J-020 | 2.2 | 144 | 982.7 |
| J-021 | 11.7 | 238 | 1135 |
| J-022 | 6.8 | 151 | 1128 |
| J-023 | 6.4 | 117 | 877 |
| J-024 | 26.7 | 373 | 1377 |
| J-027 | 1.4 | 52 | 9010 |
| J-028 | 20.8 | 155 | 842 |
| J-029 | 12.09 | 98.52 | 203.6 |
| J-033 | 12.9 | 611 | >10000 |
| J-034 | 5.8 | 189.2 | 440.5 |
| J-039 | 12 | 256 | 1109 |
| J-041 | 28.4 | 354 | 228 |
| J-042 | 8.18 | 296 | 373 |
| J-043 | 10.9 | 67.6 | 896 |
| J-044 | 1.0 | 25.2 | 432 |
| J-045 | 13.5 | 130 | 710 |
| J-046 | 1.2 | 117 | 475 |
| J-047 | 0.5 | 21 | 176 |
| J-048 | 7.6 | 52 | 1100 |
| J-049 | 8.5 | 109 | 2295 |

(continued)

| Compound | Ba/F3/EGFR △19del /T790M/C797S $IC_{50}$ (nM) | BaF3 EGFR WT $IC_{50}$ (nM) | A431 $IC_{50}$ (nM) |
|---|---|---|---|
| J-050 | 1.8 | 42.2 | 861 |
| J-052 | 2.6 | 123 | 279 |
| J-053 | 3.9 | 54 | 753 |
| J-054 | 0.8 | 15.8 | 143 |
| J-055 | 12.4 | 79 | 1174 |
| J-056 | 0.8 | 38 | 1098 |
| J-057 | 3.0 | 54 | 328 |
| J-058 | 5.8 | 117 | 649 |
| J-059 | 0.4 | 10.6 | 264 |
| J-060 | 2.3 | 54 | 366 |
| J-062 | 1.4 | 46.2 | 157 |
| J-064 | 1.1 | 14.6 | 128 |
| J-065 | 13 | 73 | 425 |
| J-066 | 4.4 | 77.9 | 259 |
| J-067 | 2.4 | 37.3 | 632 |
| J-068 | 1.0 | 33.2 | 201 |

It can be seen from Table 2 that the compounds of the present invention have a strong inhibitory effect on the proliferation of Ba/F3-△19del/T790M /C797S cells, and have a weak or even have no obvious inhibitory effect on the wild-type EGFR cells Ba/F3 EGFR WT and A431. However, the compound of Comparative example 1 not only has a strong inhibitory effect on the proliferation of Ba/F3-△19del△790M/C797S cells, but also has a strong inhibitory effect on BaF3 EGFR WT, and also has a certain inhibitory effect on A431. This shows that the compound of the present invention has better selectivity relative to the compound of Comparative Example 1.

Experiment 3 **In** vivo experiment

**1. In** vivo efficacy experiments

In vivo efficacy experiments were performed on Ba/F3-△19del/T790M/C797S-derived xenograft (CDX) BALB/c nude mice. Female BALB/c nude mice of 6-8 weeks old with a body weight of about 18-22 g were randomly grouped, with 6 mice in each group. They were fed in an SPF environment, with each cage individually ventilated (6 mice per cage). All cages, bedding and water were disinfected prior to use. Mice were purchased from Viton Lever and were allowed to acclimate in the laboratory. Each mouse was subcutaneously inoculated with 0.1 mL cells ($5 \times 10^6$ cells, the volume ratio of matrigel to cell was 1:1) on the right upper limb. When the tumor volume was about 150 $mm^3$, the grouped mice were separately administered with compound J-022 (10 mg/kg, 100 mg/kg, 150 mg/kg) orally QD for 15 consecutive days. Tumor diameters were measured with vernier calipers twice a week. The volume of tumor is calculated as: $V=0.5a\times b^2$, wherein a and b represent the long diameter and short diameter of the tumor, respectively. The tumor growth inhibition rate TGI (%) was calculated to evaluate the tumor inhibitory effect of the compounds. TGI (%) was calculated with the following formula: TGI (%)=(1-(the average tumor volume of the compound treatment group at the end of the administration - the average tumor volume of the compound treatment group at the beginning of the administration) / (the average tumor volume of the solvent control group at the end of treatment - the average tumor volume of the solvent control group at the beginning of treatment))×100%. The experimental results are shown in Table 3.

Table 3

| **Compound tested** | **Dosage** (mg/kg/day) | **Tumor volume** ($mm^3$) | | | | | **15** day TGI% |
|---|---|---|---|---|---|---|---|
| | | **0 day** | **3 day** | **7 day** | **10day** | **15 day** | |
| **Solvent control** | N/A | 150 | 254 | 494 | 1215 | 3330 | NA |
| **Compound** J-022 | 10 | 152 | 183 | 321 | 747 | 1650 | 53% |
| | 100 | 151 | 99 | 59 | 0 | 0 | 105% |
| | 150 | 152 | 102 | 4 | 0 | 0 | 105% |

It can be seen from Table 3 that when compound J-022 was administered for 15 consecutive days with 10 mg/kg/day, the corresponding TGI was 53%. When it was administered 100 mg/kg/day or 150 mg/kg/day, tumor completely regressed.

Moreover, the data of Comparative example 1 (in the patent WO2019/015655A1) was cited in Table 4, and the TGI of each administration group was calculated. Nonlinear fitting was performed according to the administrating dosage and the tumor growth inhibition rate TGI (%) corresponding to each dosage using GraphPad Prism 8. The fitting formula is as follows and its $ED_{50}$ (half effective dose) was calculated: Y=Bottom+(Top-Bottom)/(1+10^((LogED50-X)*HillSlope)). The result is shown in Figure 1.

Table 4 TGI (%) calculated with reference to the tumor volume data at 0 day and 13 day in WO2019/015655A1

| **Compound tested** | **Dosage** (mg/kg/day) | **Tumor volume** (**$mm^3$**) | | 13 day TGI% |
|---|---|---|---|---|
| | | 0 day | 13 day | |
| **Solvent control** | N/A | 84 | 1048 | NA |
| **Control example 1** | 5 | 84 | 814 | 24% |
| | 15 | 84 | 211 | 87% |
| | 45 | 84 | 0 | 109% |

TGI was calculated according to the administration dosage and tumor growth volume of Comparative example 1. Nonlinear fitting was performed on the administration dosage and corresponding TGI (%). It was calculated that when the TGI (%) of Comparative example 1 was 50%, the corresponding dosage (the half effective dose $ED_{50}$) was 8.7 mg/kg/day. It can be seen that the half effective dose of compound J-022, which is 10 mg/kg/day, is equivalent to that of Comparative example 1.

**2. In** vivo safety experiments

The safety evaluation of the drug was carried out in BALB/c nude mice. Female BALB/c nude mice of 6-8 weeks old with a body weight of about 17-21 g were randomly grouped, with 5 mice in each group. They were fed in an SPF environment, and each cage was individually ventilated (5 mice per cage). All cages, bedding and water were disinfected prior to use. Mice were purchased from Viton Lever and were allowed to acclimate in the laboratory. Mice were administered orally QD. Comparative example 1 (75 mg/kg/day) and compound J-022 (75 mg/kg/day, 100 mg /kg/day, 150 mg/kg/day) were administered for 7 consecutive days (the first day of administration is the 0th day). The body weight was measured 3 times a week and the data is shown in Table 5. The changes in the body weight of mice were observed, so as to judge the safety and tolerance of the compounds.

Table 5

| **Compound tested** | **Dosage** (mg/kg/day) | **Body weight of mouse** (g) | | | |
|---|---|---|---|---|---|
| | | **0 days** | 2 **days** | 4 **days** | 6 days |
| **Solvent control** | N/A | 18.7 | 18.8 | 19.3 | 19.6 |
| **Control example 1** | 75 | 18.7 | 15.9 | 13.8 | **all dead** |

(continued)

| **Compound tested** | **Dosage** (mg/kg/day) | **Body weight of mouse** (g) | | | |
|---|---|---|---|---|---|
| | | **0 days** | 2 **days** | 4 **days** | 6 days |
| **Compound** J-022 | 75 | 18.7 | 18.9 | 19.2 | 19.4 |
| | 100 | 18.7 | 18.6 | 19.0 | 19.2 |
| | 150 | 18.7 | 18.3 | 18.5 | 18.4 |

It can be seen from Table 5 that, compared with the solvent control group, after oral administration of 75 mg/kg/day of Comparative example 1 to mice, the body weight of mice began to decrease on the 2nd day, decreased significantly on the 4th day, and all the 5 mice died on the 6th day. On the contrary, for the mice orally administered the compound of the present invention at 75 mg/kg/day or 100 mg/kg/day, the changes in the body weight on the 2nd, 4th, and 6th days were the same as those in the solvent control group. For mice orally administered the compound of the present invention at 150 mg/kg/day, the body weight did not decrease significantly. It shows that the compound of the present invention has higher safety and less toxicity than the compound of Comparative example 1.

In addition, when the Comparative example 1 was administered 75 mg/kg/day for 7 consecutive days, all mice died of intolerance. From Figure 1 and Table 4, it can be seen that the dose when the TGI of Comparative example 1 reaching 50% (ED50) is 8.7 mg/kg/day. Assuming that the dose corresponding to 50% TGI is the effective dose, the window from the effective dose to the intolerance dose of Comparative example 1 is less than 8.6 times. However, compound J-022 was continuously administered at 150 mg/kg/day for 7 days, and there was no significant change in the body weight of mice, indicating that it is tolerable at this dose. It can be seen from Table 3 that the TGI of compound J-022 at 10 mg/kg/day is 53%, which indicates that compound J-022 has a window greater than 15 times from the effective dose to the intolerance dose. In conclusion, the window from the effective dose to the intolerance dose of compound J-022 was greater than that of the Comparative example 1.

## Claims

1. A compound of formula I, or a stereoisomer, a tautomer, a deuterated compound, a pharmaceutically acceptable salt, a prodrug, a chelate, a non-covalent complex, or a solvate thereof:

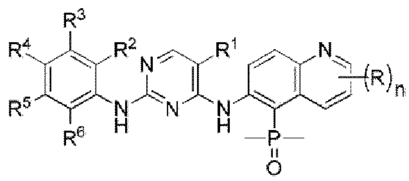

Formula I

wherein,

R is each independently selected from the group consisting of H, halogen, CN, $NO_2$, OH, -NR'R", $-C_{1-6}$ alkyl, $-C_{1-6}$ alkoxy, $-(CH_2)_p(O(CH_2)_q)_rCH_3$, $-C_{3-6}$ cycloalkyl, 3-6 membered heterocyclyl, aryl, heteroaryl, $-CR^7=CR^8$ and 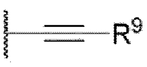 ; wherein the - $C_{1-6}$ alkyl, $-C_{1-6}$ alkoxy, 3-6 membered heterocyclyl, aryl and heteroaryl are optionally substituted by halogen, OH, $NH_2$, aryl, heteroaryl, $-C_{1-6}$ alkyl, or $-C_{3-6}$ cycloalkyl;

R', R", $R^7$, $R^8$ and $R^9$ are each independently selected from the group consisting of H, CN, $-C_{1-6}$ alkyl, $-C_{3-6}$ cycloalkyl and 3-6 membered heterocyclyl; wherein the - $C_{1-6}$ alkyl, $-C_{3-6}$ cycloalkyl and 3-6 membered heterocyclyl are optionally substituted by halogen, OH, $NH_2$, aryl, heteroaryl, $-C_{1-6}$ alkyl, $-C_{3-6}$ cycloalkyl, or 3-6 membered heterocyclyl;

$R^1$ is selected from the group consisting of H, halogen, CN, $-NR^{10}R^{11}$, OH, $-C_{1-6}$ alkyl, $-C_{1-6}$ alkoxy, $-C_{3-6}$ cycloalkyl, and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, or $-C_{1-6}$ alkyl;

$R^{10}$ and $R^{11}$ are each independently selected from H, $-C_{1-6}$ alkyl and $-C_{3-6}$ cycloalkyl;

$R^2$, $R^3$ and $R^6$ are each independently selected from the group consisting of H, halogen, CN, $-NR^{12}R^{13}$, OH, $-C_{1-6}$ alkyl, $-C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, or $-C_{1-6}$ alkyl; and $R^2$, $R^3$ and $R^6$ are not H at the same time;
$R^{12}$ and $R^{13}$ are each independently selected from the group consisting of H, $-C_{1-6}$ alkyl and $-C_{3-6}$ cycloalkyl;
$R^4$ and $R^5$ are each independently selected from the group consisting of H, halogen, CN, $NO_2$, OH, $-NR^{14}R^{15}$, $-C_{1-6}$ alkyl, aryl, heteroaryl, $-C_{1-6}$ alkoxy, $-C_{3-6}$ cycloalkyl, and 3-8 membered heterocyclyl; wherein, the $-C_{1-6}$ alkyl, aryl, heteroaryl, $-C_{1-6}$ alkoxy, $-C_{3-6}$ cycloalkyl and 3-8 membered heterocyclyl are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, substituted 3-6 membered heterocyclyl, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$; and $R^4$ and $R^5$ are not H at the same time;
$R^{14}$ and $R^{15}$ are each independently selected from the group consisting of H, $-C_{1-6}$ alkyl, $-C_{3-6}$ cycloalkyl, and $-C_{3-6}$ heterocyclyl; wherein the $-C_{1-6}$ alkyl, $-C_{3-6}$ cycloalkyl and $-C_{3-6}$ heterocyclyl are optionally substituted by $-C_{1-6}$ alkyl, $-C_{1-6}$ alkoxy, $-NR^{16}R^{17}$, $-C_{3-6}$ heterocyclyl, or heterocyclyl substituted by one or more $C_{1-6}$ alkyl; and $R^{16}$ and $R^{17}$ are each independently selected from H and $-C_{1-6}$ alkyl;
n is an integer selected from 1 to 3;
p is an integer selected from 1 to 3;
q is an integer selected from 0 to 3; and
r is an integer selected from 1 to 3.

**2.** The compound of claim 1, wherein n is 1.

**3.** The compound of claim 1, wherein R is each independently selected from the group consisting of halogen, CN, $NO_2$, OH, $-C_{1-6}$ alkyl, $-C_{3-6}$ cycloalkyl, 3-6 membered heterocyclyl, heteroaryl, and

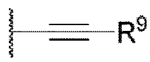

**4.** The compound of any one of claims 1-3, wherein R is each independently selected from the group consisting of halogen, CN, $NO_2$, OH, $-CF_3$, $-CH_3$, $-CH_2CH_3$, $-CH_2CH_2CH_3$, $-CH(CH_3)_2$, $-CH_2CH(CH_3)_2$,

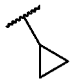, 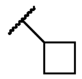, 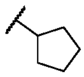, 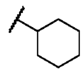, 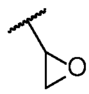,

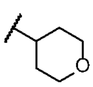, 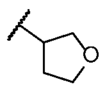, 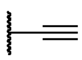, 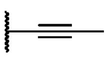, 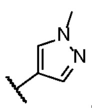, 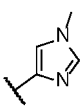, 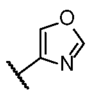 and 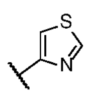.

**5.** The compound of any one of claims 1-4, wherein $R^1$ is selected from halogen, CN, $-NR^{10}R^{11}$ and OH.

**6.** The compound of claim 5, wherein $R^1$ is halogen; wherein the halogen is fluorine, chlorine, bromine or iodine.

**7.** The compound of any one of claims 1-6, wherein $R^2$ and $R^3$ are independently selected from the group consisting of H, halogen, CN, $-NR^{12}R^{13}$, OH, $-C_{1-6}$ alkyl, $-C_{1-6}$ alkoxy, $-C_{3-6}$ cycloalkyl, and 3-6 membered heterocyclyl; wherein, the $-C_{1-6}$ alkyl, $-C_{1-6}$ alkoxy, $-C_{3-6}$ cycloalkyl and 3-6 membered heterocyclyl are optionally substituted by halogen, OH, $NH_2$, or $-C_{1-6}$ alkyl; and $R^2$ and $R^3$ are not H at the same time.

**8.** The compound of claim 7, wherein $R^2$ is selected from H, $-C_{1-6}$ alkyl, and $-C_{1-6}$ alkoxy.

**9.** The compound of claim 8, wherein $R^2$ is selected from H, $-CH_3$ and $-OCH_3$.

**10.** The compound of claim 7, wherein $R^3$ is H or $-C_{1-6}$ alkoxy.

**11.** The compound of claim 10, wherein $R^3$ is H or $-OCH_3$.

**12.** The compound of any one of claims 1-11, wherein $R^4$ and $R^5$ are each independently selected from the group consisting of H, halogen, CN, $NO_2$, OH, - $NR^{14}R^{15}$, $-C_{1-6}$ alkyl, aryl, heteroaryl, $-C_{1-6}$ alkoxy, $-C_{3-6}$ cycloalkyl, and 3-6 membered heterocyclyl; wherein the $-C_{1-6}$ alkyl, aryl, heteroaryl, $-C_{1-6}$ alkoxy, $-C_{3-6}$ cycloalkyl, and 3-6 membered heterocyclyl are optionally substituted by halogen, OH, $NH_2$, $-C_{1-6}$ alkoxy, $-C_{3-6}$ cycloalkyl, substituted $C_{3-6}$ cycloalkyl, 3-6 membered heterocyclyl, substituted 3-6 membered heterocyclyl, aryl, or heteroaryl; and $R^4$ and $R^5$ are not H at the same time.

**13.** The compound of any one of claims 1-12, wherein $R^4$ is selected from the group consisting of $-NR^{14}R^{15}$, aryl, heteroaryl, $-C_{3-6}$ cycloalkyl and 3-6 membered heterocyclyl; wherein the aryl, heteroaryl, $-C_{3-6}$ cycloalkyl and 3-6 membered heterocyclyl are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.

**14.** The compound of any one of claims 1-13, wherein $R^{14}$ and $R^{15}$ are each independently selected from H, $-C_{1-6}$ alkyl and $-C_{3-6}$ cycloalkyl.

**15.** The compound of any one of claims 1-14, wherein $R^4$ is selected from the group consisting of

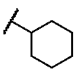, 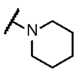, 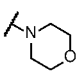, 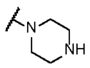, 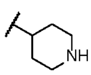, 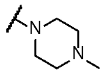, 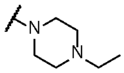,

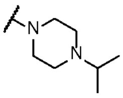, 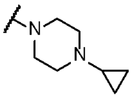, 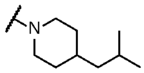, 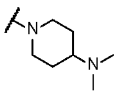, 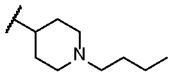, 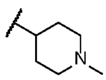, 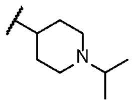,

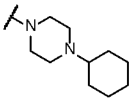, 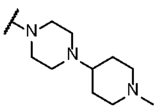, 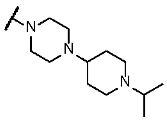, 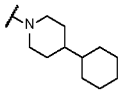, 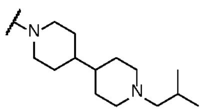, 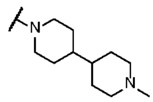,

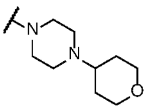, 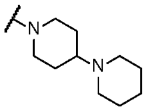, 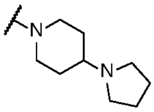, 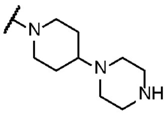, 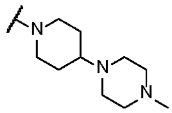, 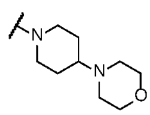,

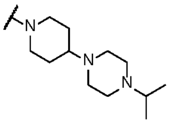, 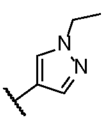, 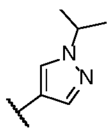, 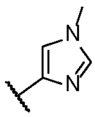, 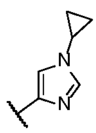, 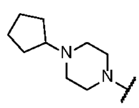, 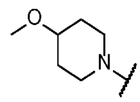,

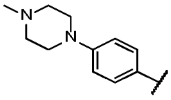, 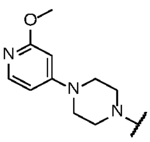, 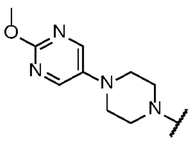, 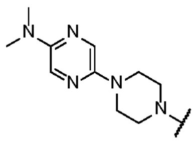, 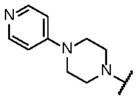, 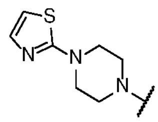,

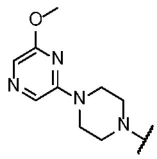, 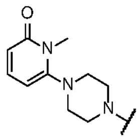, 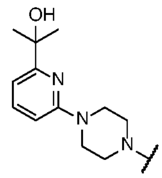, 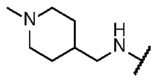, 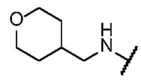, 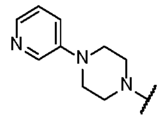,

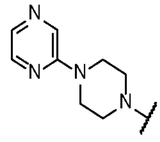, 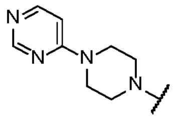, 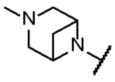, 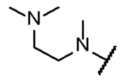 and 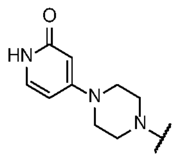.

**16.** The compound of any one of claims 1-15, wherein $R^5$ is selected from the group consisting of H, halogen, CN, $-C_{1-6}$ alkyl, heteroaryl, and $-C_{1-6}$ alkoxy; wherein the $-C_{1-6}$ alkyl, heteroaryl, and $-C_{1-6}$ alkoxy are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and - $NR^{14}R^{15}$.

**17.** The compound of any one of claims 1-16, wherein $R^5$ is selected from H, CN, $CH_3$, $CH_2CH_3$, F, $OCH_3$,

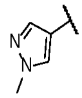, 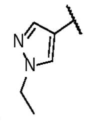, 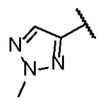, 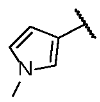 and 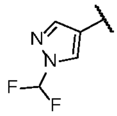.

**18.** The compound of any one of claims 1-17, wherein $R^6$ is selected from the group consisting of H, CN, $-C_{1-6}$ alkyl, $-C_{3-6}$ cycloalkyl and 3-6 membered heterocyclyl; wherein the $-C_{1-6}$ alkyl, $-C_{3-6}$ cycloalkyl and 3-6 membered heterocyclyl are optionally substituted with halogen, OH, $NH_2$, or $-C_{1-6}$ alkyl.

**19.** The compound of any one of claims 1-18, wherein $R^6$ is selected from H, CN, $CH_3$, $CH_2CH_3$, $CH(CH_3)_2$,

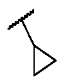, and 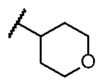.

**20.** The compound of claim 1, wherein the compound of formula I, or a stereoisomer, a tautomer, a deuterated compound, a pharmaceutically acceptable salt, a prodrug, a chelate, a non-covalent complex, or a solvate thereof is represented by formula II:

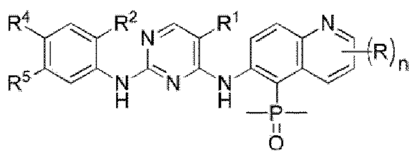

Formula II

wherein,

R is each independently selected from the group consisting of H, halogen, CN, $NO_2$, OH, -NR'R", $-C_{1-6}$ alkyl, $-C_{1-6}$ alkoxy, $-(CH_2)_p(O(CH_2)_q)_rCH_3$, $-C_{3-6}$ cycloalkyl, 3-6 membered heterocyclyl, aryl, heteroaryl, $-CR^7=CR^8$ and

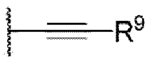;

wherein the - $C_{1-6}$ alkyl, -$C_{1-6}$ alkoxy, 3-6 membered heterocyclyl, aryl and heteroaryl are optionally substituted by halogen, OH, $NH_2$, aryl, heteroaryl, -$C_{1-6}$ alkyl, or -$C_{3-6}$ cycloalkyl;
R', R", $R^7$, $R^8$ and $R^9$ are each independently selected from the group consisting of H, CN, -$C_{1-6}$ alkyl, -$C_{3-6}$ cycloalkyl and 3-6 membered heterocyclyl; wherein the - $C_{1-6}$ alkyl, -$C_{3-6}$ cycloalkyl and 3-6 membered heterocyclyl are optionally substituted by halogen, OH, $NH_2$, aryl, heteroaryl, -$C_{1-6}$ alkyl, -$C_{3-6}$ cycloalkyl, or 3-6 membered heterocyclyl;
$R^1$ is selected from the group consisting of H, halogen, CN, -$NR^{10}R^{11}$, OH, -$C_{1-6}$ alkyl, -$C_{1-6}$ alkoxy, -$C_{3-6}$ cycloalkyl and 3-6 membered heterocyclyl; wherein the -$C_{1-6}$ alkyl, -$C_{1-6}$ alkoxy, -$C_{3-6}$ cycloalkyl and 3-6 membered heterocyclyl are optionally substituted by halogen, OH, $NH_2$, or -$C_{1-6}$ alkyl;
$R^{10}$ and $R^{11}$ are each independently selected from the group consisting of H, -$C_{1-6}$ alkyl and -$C_{3-6}$ cycloalkyl;
$R^2$ is selected from the group consisting of H, halogen, CN, -$NR^{12}R^{13}$, OH, -$C_{1-6}$ alkyl, -$C_{1-6}$ alkoxy, -$C_{3-6}$ cycloalkyl and 3-6 membered heterocyclyl; wherein the -$C_{1-6}$ alkyl, -$C_{1-6}$ alkoxy, -$C_{3-6}$ cycloalkyl and 3-6 membered heterocyclyl are optionally substituted by halogen, OH, $NH_2$, or -$C_{1-6}$ alkyl; and $R^2$, $R^3$ and $R^6$ are not H at the same time;
$R^{12}$ and $R^{13}$ are each independently selected from the group consisting of H, -$C_{1-6}$ alkyl and -$C_{3-6}$ cycloalkyl;
$R^4$ and $R^5$ are each independently selected from the group consisting of H, halogen, CN, $NO_2$, OH, -$NR^{14}R^{15}$, -$C_{1-6}$ alkyl, aryl, heteroaryl, -$C_{1-6}$ alkoxy, -$C_{3-6}$ cycloalkyl and 3-8 membered heterocyclyl; wherein the -$C_{1-6}$ alkyl, aryl, heteroaryl, - $C_{1-6}$ alkoxy, -$C_{3-6}$ cycloalkyl and 3-8 membered heterocyclyl are optionally substituted by halogen, OH, $NH_2$, -$C_{1-6}$ alkyl, halogen-substituted $C_{1-6}$ alkyl, hydroxy-substituted $C_{1-6}$ alkyl, -$C_{1-6}$ alkoxy, -$NR^{14}R^{15}$, -$C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and -$NR^{14}R^{15}$, aryl, substituted aryl, heteroaryl, or heteroaryl substituted with one or more of $C_{1-6}$ alkyl, hydroxy-substituted $C_{1-6}$ alkyl, oxo, $C_{1-6}$ alkoxy, and -$NR^{14}R^{15}$; and $R^4$ and $R^5$ are not H at the same time;
$R^{14}$ and $R^{15}$ are each independently selected from the group consisting of H, -$C_{1-6}$ alkyl, -$C_{3-6}$ cycloalkyl, and -$C_{3-6}$ heterocyclyl; wherein the -$C_{1-6}$ alkyl, -$C_{3-6}$ cycloalkyl and -$C_{3-6}$ heterocyclyl are optionally substituted by -$C_{1-6}$ alkyl, -$C_{1-6}$ alkoxy, -$NR^{16}R^{17}$, -$C_{3-6}$ heterocyclyl, or heterocyclyl substituted by one or more $C_{1-6}$ alkyl; and
$R^{16}$ and $R^{17}$ are each independently selected from H and -$C_{1-6}$ alkyl;
n is an integer selected from 1 to 3;
p is an integer selected from 1 to 3;
q is an integer selected from 0 to 3; and
r is an integer selected from 1 to 3.

**21.** The compound of claim 20, wherein n is 1.

**22.** The compound of claim 20 or 21, wherein R is each independently selected from the group consisting of halogen, CN, $NO_2$, OH, -$C_{1-6}$ alkyl, -$C_{3-6}$ cycloalkyl, 3-6 membered heterocyclyl, heteroaryl, and

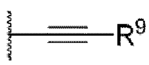.

**23.** The compound of any one of claims 20-22, wherein R is each independently selected from the group consisting of halogen, CN, $NO_2$, OH, -$CF_3$, -$CH_3$, -$CH_2CH_3$, - $CH_2CH_2CH_3$, -$CH(CH_3)_2$, -$CH_2CH(CH_3)_2$,

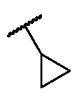, 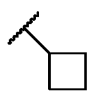, 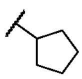, 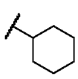, 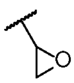,

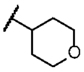, 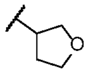, 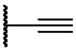, 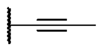, 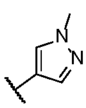, 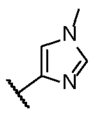, 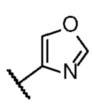 and 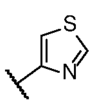.

**24.** The compound of any one of claims 20-23, wherein $R^1$ is selected from halogen, CN, $-NR^{10}R^{11}$ and OH.

**25.** The compound of claim 24, wherein $R^1$ is halogen; wherein the halogen is fluorine, chlorine, bromine or iodine.

**26.** The compound of claim 25, wherein $R^1$ is fluorine or bromine.

**27.** The compound of any one of claims 20-26, wherein $R^2$ is selected from the group consisting of H, halogen, CN, $-NR^{12}R^{13}$, OH, $-C_{1-6}$ alkyl, $-C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, or $-C_{1-6}$ alkyl; $R^{12}$ and $R^{13}$ are defined as described in any of the embodiments of the present invention.

**28.** The compound of claim 27, wherein $R^2$ is selected from H, $-C_{1-6}$ alkyl and - $C_{1-6}$ alkoxy.

**29.** The compound of claim 28, wherein $R^2$ is $-C_{1-6}$ alkoxy.

**30.** The compound of claim 28, wherein $R^2$ is H, $-CH_3$ or $-OCH_3$.

**31.** The compound of any one of claims 20-30, wherein $R^4$ is selected from the group consisting of H, halogen, CN, $NO_2$, OH, $-NR^{14}R^{15}$, aryl, heteroaryl, $-C_{3-6}$ cycloalkyl and 3-6 membered heterocyclyl; wherein the aryl, heteroaryl, $-C_{3-6}$ cycloalkyl and 3-6 membered heterocyclyl are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and - $NR^{14}R^{15}$.

**32.** The compound of claim 31, wherein $R^4$ is selected from the group consisting of $-NR^{14}$ $R^{15}$, aryl, heteroaryl, $-C_{3-6}$ cycloalkyl and 3-6 membered heterocyclyl; wherein the aryl, heteroaryl, $-C_{3-6}$ cycloalkyl and 3-6 membered heterocyclyl are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.

**33.** The compound of any one of claims 20-32, wherein $R^4$ is selected from the group consisting of

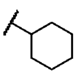, 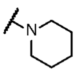, 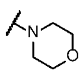, 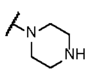, 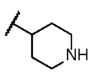, 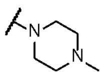, 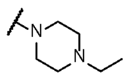,

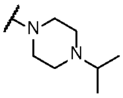, 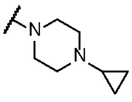, 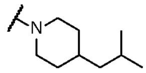, 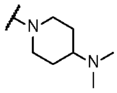, 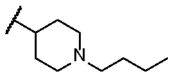, 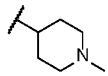, 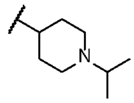,

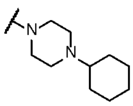, 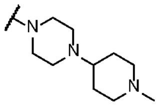, 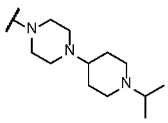, 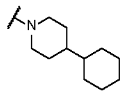, 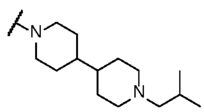, 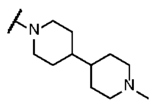,

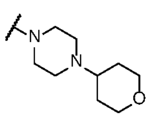, 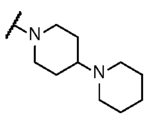, 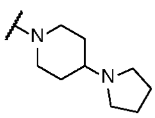, 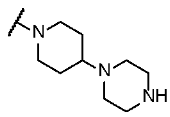, 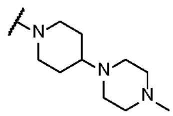, 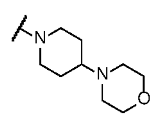,

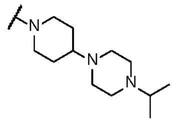, 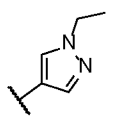, 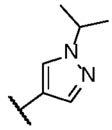, 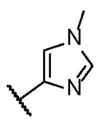, 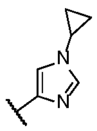, 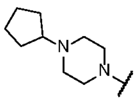, 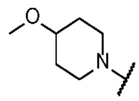,

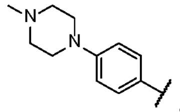, 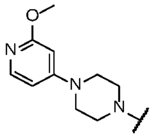, 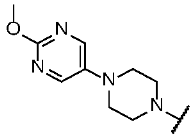, 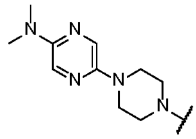, 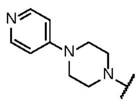, 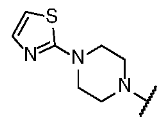,

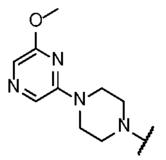, 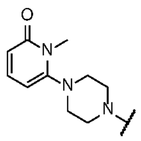, 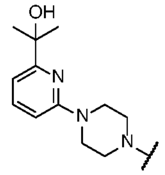, 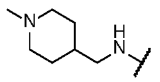, 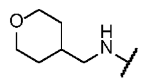, 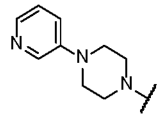,

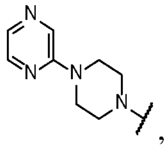, 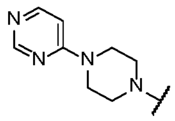, 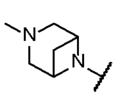, 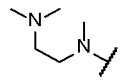, and 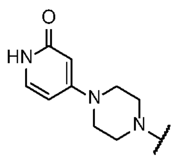.

**34.** The compound of any one of claims 20-33, wherein $R^5$ is selected from the group consisting of H, halogen, CN, $NO_2$, OH, -$C_{1-6}$ alkyl, aryl, heteroaryl, and -$C_{1-6}$ alkoxy; wherein the -$C_{1-6}$ alkyl, aryl, heteroaryl, and -$C_{1-6}$ alkoxy are optionally substituted by halogen, OH, $NH_2$, -$C_{1-6}$ alkyl, halogen-substituted $C_{1-6}$ alkyl, hydroxy-substituted $C_{1-6}$ alkyl, -$C_{1-6}$ alkoxy, -$NR^{14}R^{15}$, -$C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and -$NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and -$NR^{14}R^{15}$.

**35.** The compound of claims 34, wherein $R^5$ is selected from the group consisting of H, halogen, CN, -$C_{1-6}$ alkyl, heteroaryl, and -$C_{1-6}$ alkoxy; wherein the -$C_{1-6}$ alkyl, heteroaryl, and -$C_{1-6}$ alkoxy are optionally substituted by halogen, OH, $NH_2$, -$C_{1-6}$ alkyl, halogen-substituted $C_{1-6}$ alkyl, hydroxy-substituted $C_{1-6}$ alkyl, -$C_{1-6}$ alkoxy, - $NR^{14}R^{15}$, -$C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and -$NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and -$NR^{14}R^{15}$.

**36.** The compound of any one of claims 20-35, wherein $R^5$ is selected from H, CN, $CH_3$, $CH_2CH_3$, F, $OCH_3$,

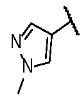, 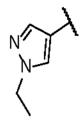, 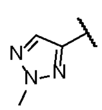, 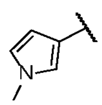, and 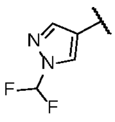.

**37.** The compound of claim 1, wherein the compound of formula I, or a stereoisomer, a tautomer, a deuterated compound, a pharmaceutically acceptable salt, a prodrug, a chelate, a non-covalent complex, or a solvate thereof is represented by formula III:

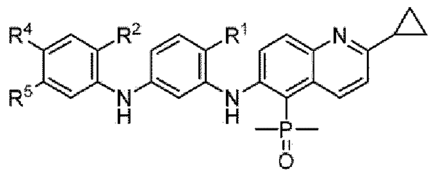

Formula III

wherein,

$R^1$ is selected from the group consisting of H, halogen, CN, $-NR^{10}R^{11}$, OH, $-C_{1-6}$ alkyl, $-C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, or $-C_{1-6}$ alkyl;
$R^{10}$ and $R^{11}$ are each independently selected from H, $-C_{1-6}$ alkyl and $-C_{3-6}$ cycloalkyl;
$R^2$ is selected from the group consisting of H, halogen, CN, $-NR^{12}R^{13}$, OH, $-C_{1-6}$ alkyl, $-C_{1-6}$ alkoxy, $-C_{3-6}$ cycloalkyl and 3-6 membered heterocyclyl; wherein the $-C_{1-6}$ alkyl, $-C_{1-6}$ alkoxy, $-C_{3-6}$ cycloalkyl and 3-6 membered heterocyclyl are optionally substituted by halogen, OH, $NH_2$, or $-C_{1-6}$ alkyl;
$R^{12}$ and $R^{13}$ are each independently selected from H, $-C_{1-6}$ alkyl and $-C_{3-6}$ cycloalkyl;
$R^4$ and $R^5$ are each independently selected from the group consisting of H, halogen, CN, $NO_2$, OH, $-NR^{14}R^{15}$, $-C_{1-6}$ alkyl, aryl, heteroaryl, $-C_{1-6}$ alkoxy, $-C_{3-6}$ cycloalkyl and 3-8 membered heterocyclyl; wherein the $-C_{1-6}$ alkyl, aryl, heteroaryl, - $C_{1-6}$ alkoxy, $-C_{3-6}$ cycloalkyl and 3-8 membered heterocyclyl are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted with one or more of $C_{1-6}$ alkyl, hydroxy-substituted $C_{1-6}$ alkyl, oxo, $C_{1-6}$ alkoxy, and $-NR^{14}R^{15}$; and $R^4$ and $R^5$ are not H at the same time; and
$R^{14}$ and $R^{15}$ are each independently selected from the group consisting of H, $-C_{1-6}$ alkyl, $-C_{3-6}$ cycloalkyl, and $-C_{3-6}$ heterocyclyl; wherein the $-C_{1-6}$ alkyl, $-C_{3-6}$ cycloalkyl and $-C_{3-6}$ heterocyclyl are optionally substituted by $-C_{1-6}$ alkyl, $-C_{1-6}$ alkoxy, $-NR^{16}R^{17}$, $-C_{3-6}$ heterocyclyl, or heterocyclyl substituted by one or more $C_{1-6}$ alkyl; wherein, $R^{16}$ and $R^{17}$ are each independently selected from H and $-C_{1-6}$ alkyl.

**38.** The compound of claim 37, wherein $R^1$ is halogen; wherein the halogen is fluorine, chlorine, bromine or iodine.

**39.** The compound of claim 38, wherein $R^1$ is fluorine or bromine.

**40.** The compound of any one of claims 37-39, wherein $R^2$ is selected from $-C_{1-6}$ alkyl and $-C_{1-6}$ alkoxy.

**41.** The compound of claim 40, wherein $R^2$ is $-C_{1-6}$ alkoxy.

**42.** The compound of claim 40, wherein $R^2$ is $-CH_3$ or $-OCH_3$.

**43.** The compound of any one of claims 37-42, wherein $R^4$ is selected from the group consisting of $-NR^{14}R^{15}$, aryl, heteroaryl, $-C_{3-6}$ cycloalkyl and 3-8 membered heterocyclyl; wherein the aryl, heteroaryl, $-C_{3-6}$ cycloalkyl and 3-8 membered heterocyclyl are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.

**44.** The compound of any one of claims 37-43, wherein $R^4$ is 3-8 membered heterocyclyl; wherein the 3-8 membered heterocyclyl is optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $-NR^{14}R^{15}$.

**45.** The compound of any one of claims 37-44, wherein $R^{14}$ and $R^{15}$ are each independently selected from H and $-C_{1-6}$

alkyl; wherein the -$C_{1-6}$ alkyl is optionally substituted by -$C_{1-6}$ alkoxy, -$NR^{16}R^{17}$, -$C_{3-6}$ heterocyclyl, or heterocyclyl substituted by one or more $C_{1-6}$ alkyl; wherein, $R^{16}$ and $R^{17}$ are each independently selected from H and -$C_{1-6}$ alkyl.

**46.** The compound of any one of claims 37-45, wherein $R^4$ is selected from the group consisting of

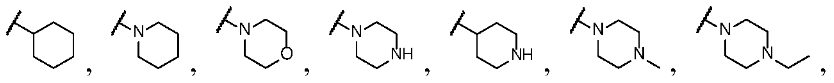

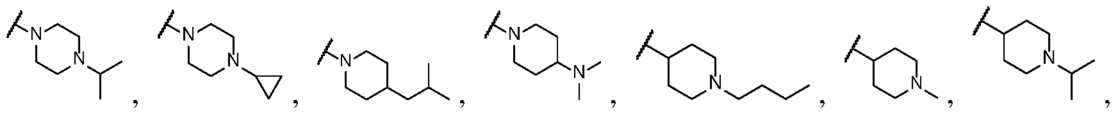

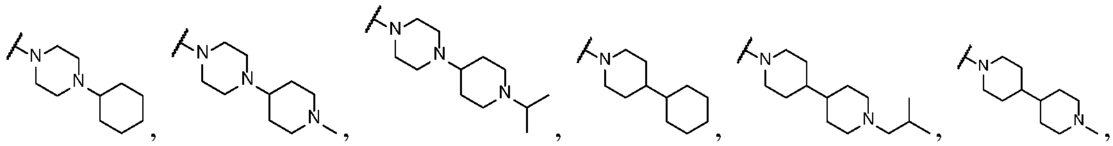

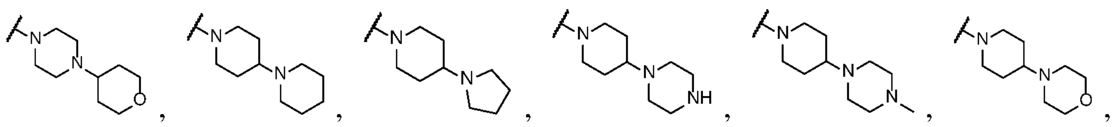

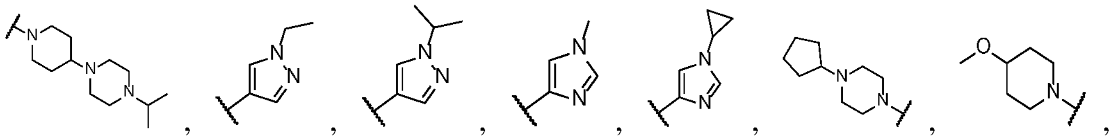

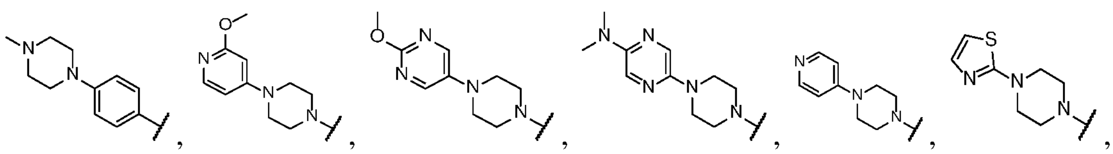

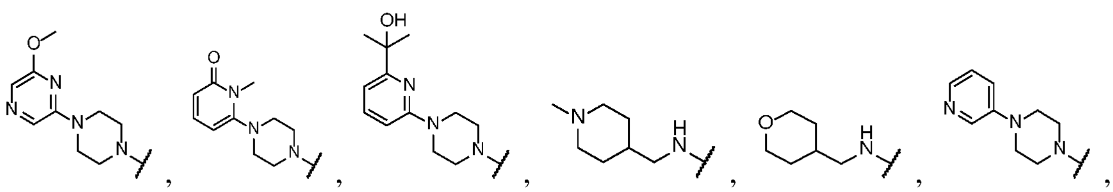

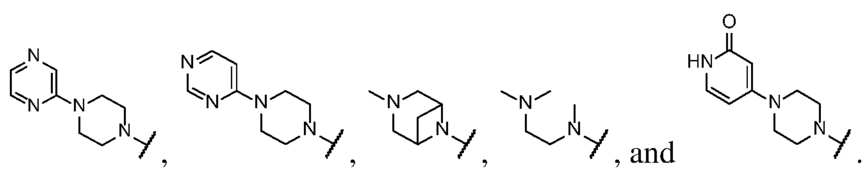

**47.** The compound of any one of claims 37-46, wherein $R^5$ is selected from the group consisting of H, halogen, CN, $NO_2$, OH, -$C_{1-6}$ alkyl, aryl, heteroaryl, and -$C_{1-6}$ alkoxy; wherein the -$C_{1-6}$ alkyl, aryl, heteroaryl, and -$C_{1-6}$ alkoxy are optionally substituted by halogen, OH, $NH_2$, -$C_{1-6}$ alkyl, halogen-substituted $C_{1-6}$ alkyl, hydroxy-substituted $C_{1-6}$ alkyl, -$C_{1-6}$ alkoxy, -$NR^{14}R^{15}$, -$C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and -$NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and -$NR^{14}R^{15}$.

**48.** The compound of any one of claims 37-47, wherein $R^5$ is selected from the group consisting of H, halogen, CN, $-C_{1-6}$ alkyl, heteroaryl, and $-C_{1-6}$ alkoxy; wherein the $-C_{1-6}$ alkyl, heteroaryl, and $-C_{1-6}$ alkoxy are optionally substituted by halogen, OH, $NH_2$, $-C_{1-6}$ alkyl, halogen-substituted $C_{1-6}$ alkyl, hydroxy-substituted $C_{1-6}$ alkyl, $-C_{1-6}$ alkoxy, $-NR^{14}R^{15}$, $-C_{3-6}$ cycloalkyl, substituted $C_{3-6}$ cycloalkyl, 3-6 membered heterocyclyl, 3-6 membered heterocyclyl substituted by one or more of $C_{1-6}$ alkyl, $C_{1-6}$ alkoxy, oxo, and $-NR^{14}R^{15}$, aryl, substituted aryl, heteroaryl, or heteroaryl substituted by one or more of $C_{1-6}$ alkyl, hydroxy-substituted $C_{1-6}$ alkyl, oxo, $C_{1-6}$ alkoxy, and $- NR^{14}R^{15}$.

**49.** The compound of any one of claims 37-48, wherein $R^5$ is selected from H, CN, $CH_3$, $CH_2CH_3$, F, $OCH_3$,

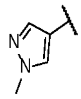 , 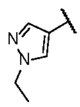 , 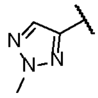 , 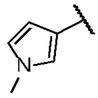 , and 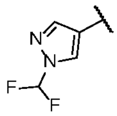 .

**50.** The compound of any one of claims 1-49, wherein the compound of formula I is selected from the group consisting of:

(6-((5-bromo-2-((5-methoxy-2-methyl-4-morpholinylphenyl)amino)pyrimidin-4-yl)amino)-2-cyclopropylquinoline-5-yl)dimethylphosphine oxide;
5-((5-bromo-4-((2-cyclopropyl-5-(dimethylphosphine oxide)quinolin-6-yl)amino)pyrimidin-2-yl)amino)-4-methoxy-2-morpholinebenzonitrile;
(6-((5-bromo-2-((2-ethyl-5-methoxy-4-morpholinylphenyl)amino)pyrimidin-4-yl)amino)-2-cyclopropylquinoline-5-yl)dimethylphosphine oxide;
(6-((5-bromo-2-((2-cyclopropyl-5-methoxy-4-morpholinylphenyl)amino)pyrimidin-4-yl)amino)-2-cyclopropylquinoline-5-yl)dimethylphosphine oxide;
(6-((5-bromo-2-((2-isopropyl-5-methoxy-4-morpholinylphenyl)amino)pyrimidin-4-yl)amino)-2-cyclopropylquinoline-5-yl)dimethylphosphine oxide;
(6-((5-bromo-2-((3-methoxy-5-methyl-4-morpholinylphenyl)amino)pyrimidin-4-yl)amino)-2-cyclopropylquinoline-5-yl)dimethylphosphine oxide;
(6-((5-bromo-2-((5-methoxy-2-methyl-4-morpholinylphenyl)amino)pyrimidin-4-yl)amino)-2-(1-methyl-1H-pyrazol-4-yl)quinolin-5-yl)dimethylphosphine oxide;
(6-((5-bromo-2-((5-(1-ethyl-1H-pyrazol-4-yl)-2-methoxy-4-morpholinophenyl)amino)pyrimidine-4-yl)amino)-2-cyclopropylquinolin-5-yl)dimethylphosphine oxide;
(6-((5-bromo-2-((4-(4-(dimethylamino)piperidin-1-yl)-5-methoxy-2-methylphenyl)amino)pyrimidine-4-yl)amino)-2-cyclopropylquinolin-5-yl)dimethylphosphine oxide;
(6-((5-bromo-2-((2-methoxy-5-methyl-4-(4-methylpiperazin-1-yl)phenyl)amino)pyrimidin-4-yl)amino)-2-cyclopropylquinolin-5-yl)dimethylphosphine oxide;
(6-((5-bromo-2-((5-ethyl-2-methoxy-4-(4-methylpiperazin-1-yl)phenyl)amino)pyrimidin-4-yl)amino)-2-cyclopropylquinolin-5-yl)dimethylphosphine oxide;
5-((5-bromo-4-((2-cyclopropyl-5-(dimethylphosphine oxide)quinolin-6-yl)amino)pyrimidin-2-yl)amino)-4-methoxy-2-(4-methylpiperazin-1-yl)benzonitrile;
(6-((5-bromo-2-((2-methoxy-5-methyl-4-(4-(4-methylpiperazin-1-yl)piperidin-1-yl)phenyl)amino)pyrimidin-4-yl)amino)-2-ethynylquinolin-5-yl)dimethylphosphine oxide;
(6-((5-bromo-2-((5-ethyl-2-methoxy-4-(4-(4-methylpiperazin-1-yl)piperidin-1-yl)phenyl)amino)pyrimidin-4-yl)amino)-2-ethynylquinolin-5-yl)dimethylphosphine oxide;
(6-((5-bromo-2-((5-methoxy-2-methyl-4-(4-(4-methylpiperazin-1-yl)piperidin-1-yl)phenyl)amino)pyrimidin-4-yl)amino)-2-ethynylquinolin-5-yl)dimethylphosphine oxide;
6-((5-bromo-2-((5-ethyl-2-methoxy-4-(4-(4-methylpiperazin-1-yl)piperidin-1-yl)phenyl)amino)pyrimidin-4-yl)amino)-5-(dimethylphosphine oxide)quinoline-2-formonitrile;
(6-((5-bromo-2-((5-ethyl-2-methoxy-4-(4-(4-methylpiperazin-1-yl)piperidin-1-yl)phenyl)amino)pyrimidin-4-yl)amino)-2-(1-methyl-1H-pyrazol-4-yl)quinolin-5-yl)dimethylphosphine oxide;
(6-((5-bromo-2-((5-ethyl-2-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((5-fluoro-2-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((2,5-dimethoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
5-((5-bromo-4-((2-cyclopropyl-5-(dimethylphosphine oxide)quinolin-6-yl)amino)pyrimidin-2-yl)amino)-4-methoxy-2-(4-(4-methylpiperazin-1-yl)piperidin-1-yl)benzonitrile;

(6-((5-bromo-2-((2-methoxy-5-methyl-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((5-methoxy-2-methyl-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((2-cyclopropyl-5-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
2-((5-bromo-4-((2-cyclopropyl-5-(dimethylphosphine oxide)quinolin-6-yl)amino)pyrimidin-2-yl)amino)-4-methoxy-5-(4-(4-methylpiperazin-1-yl)piperidin-1-yl)benzonitrile;
(6-((5-bromo-2-((5-methoxy-4-(4-(4-methylpiperazin-1-yl)piperidin-1-yl)-2-(tetrahydro-2H-pyran-4-yl)phenyl)amino)pyrimidin-4-yl)amino)-2-cyclopropylquinolin-5-yl)dimethylphosphine oxide;
(6-((5-bromo-2-((5-ethyl-2-methyl-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((2-methoxy-5-methyl-4-(4-(4-methylpiperazin-1-yl)piperidin-1-yl)phenyl)amino)pyrimidin-4-yl)amino)-2-ethylquinolin-5-yl)dimethylphosphine oxide;
(6-((5-bromo-2-((3-methoxy-5-methyl-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((5-ethyl-2-methoxy-4-(1'-methyl-[4,4'-bipiperidin]-1-yl)phenyl)amino)pyrimidin-4-yl)amino)-2-cyclopropylquinolin-5-yl)dimethylphosphine oxide;
(6-((5-bromo-2-((2-ethyl-5-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((4-(4-cyclopentylpiperazin-1-yl)-2-methoxy-5-methylphenyl)amino)pyrimidin-4-yl)amino)-2-cyclopropylquinolin-5-yl)dimethylphosphine oxide;
(6-((5-bromo-2-((5-methoxy-4-(4-methoxypiperidin-1-yl)-2-methylphenyl)amino)pyrimidin-4-yl)amino)-2-cyclopropylquinolin-5-yl)dimethylphosphine oxide;
(6-((5-bromo-2-((2-methoxy-5-methyl-4-(4-(4-methylpiperazin-1-yl)piperidin-1-yl)phenyl)amino)pyrimidin-4-yl)amino)-2-isopropylquinolin-5-yl)dimethylphosphine oxide;
(6-((5-bromo-2-((5-fluoro-2-methyl-4-morpholinophenyl)amino)pyrimidin-4-yl)amino)-2-cyclopropylquinoline-5-yl)dimethylphosphine oxide;
(6-((5-bromo-2-((2,5-dimethyl-4-morpholinophenyl)amino)pyrimidin-4-yl)amino)-2-cyclopropylquinoline-5-yl)dimethylphosphine oxide;
(6-((5-bromo-2-((4-(4-cyclopentylpiperazin-1-yl)-2-methoxy-5-methylphenyl)amino)pyrimidin-4-yl)amino)-2-cyclopropylquinolin-5-yl)dimethylphosphine oxide;
(6-((5-bromo-2-((4-(4-cyclopropylpiperazin-1-yl)-2-methoxy-5-methylphenyl)amino)pyrimidin-4-yl)amino)-2-cyclopropylquinolin-5-yl)dimethylphosphine oxide;
(6-((5-bromo-2-((4-(4-cyclopentylpiperazin-1-yl)-5-methoxy-2-methylphenyl)amino)pyrimidin-4-yl)amino)-2-cyclopropylquinolin-5-yl)dimethylphosphine oxide;
(6-((5-bromo-2-((5-methoxy-2-methyl-4'-(4-methylpiperazin-1-yl)-[1,1'-biphenyl]-4-yl)amino)pyrimidin-4-yl)amino)-2-cyclopropylquinolin-5-yl)dimethylphosphine oxide;
(6-((5-bromo-2-((2-methoxy-5-methyl-4-(4-(4-methylpiperazin-1-yl)piperidin-1-yl)phenyl)amino)pyrimidin-4-yl)amino)quinolin-5-yl)dimethylphosphine oxide;
(6-((5-bromo-2-((2-methoxy-5-methyl-4-(4-(4-methylpiperazin-1-yl)piperidin-1-yl)phenyl)amino)pyrimidin-4-yl)amino)-2-propylquinolin-5-yl)dimethylphosphine oxide;
(6-((5-bromo-2-((2-methoxy-4-(4-(2-methoxypyridin-4-yl)piperazin-1-yl)-5-(1-methyl-1H-pyrazol-4-yl)phenyl)amino)pyridin-4-yl)amino)-2-cyclopropylquinolin-5-yl)dimethylphosphine oxide;
(6-((5-bromo-2-((2-methoxy-4-(4-(2-methoxypyrimidin-5-yl)piperazin-1-yl)-5-(1-methyl-1H-pyrazol-4-yl)phenyl)amino)pyrimidin-4-yl)amino)-2-cyclopropylquinolin-5-yl)dimethylphosphine oxide;
(6-((5-bromo-2-((4-(4-(5-(dimethylamino)pyrazin-2-yl)piperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide;
(6-((5-bromo-2-((2-methoxy-5-(2-methyl-2H-1,2,3-triazol-4-yl)-4-(4-(4-methylpiperazin-1-yl)piperidin-1-yl)phenyl)amino)pyrimidin-4-yl)amino)-2-cyclopropylquinolin-5-yl)dimethylphosphine oxide;
(6-((5-bromo-2-((2-methoxy-5-(2-methyl-2H-1,2,3-triazol-4-yl)-4-(4-(pyridin-4-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(thiazol-2-yl)piperazin-1-yl)phenyl)amino)pyrimidin-4-yl)amino)-2-cyclopropylquinolin-5-yl)dimethylphosphine oxide;
(6-((5-bromo-2-((2-methoxy-4-(4-(6-methoxypyrazin-2-yl)piperazin-1-yl)-5-(1-methyl-1H-pyrazol-4-yl)phenyl)amino)pyrimidin-4-yl)amino)-2-cyclopropylquinolin-5-yl)dimethylphosphine oxide;
6-(4-(4-((5-bromo-4-(2-cyclopropyl-5-(dimethylphosphoryl)quinolin-6-yl)amino)pyrimidin-2-yl)amino)-5-methoxy-2-(1-methyl-1H-pyrazol-4-yl)phenyl)piperazin-1-yl)-1-methylpyridin-2(1H)-one;

(6-((5-bromo-2-((4-(4-(6-(2-hydroxypropan-2-yl)pyridin-2-yl)piperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(((1-methylpiperidine-4-yl)methyl)amino)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(((tetrahydro-2H-pyran-4-yl)methyl)amino)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-methylpiperazin-1-yl)phenyl)amino)pyrimidin-4-yl)amino)-2-cyclopropylquinolin-5-yl)dimethylphosphine oxide;
(6-((5-bromo-2-((4-(4-cyclopentylpiperazin-1-yl)-2-methoxy-5-(1-methyl-1H-pyrazol-4-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(pyridin-3-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(pyridin-4-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(pyrazin-2-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-morpholinopiperidine-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(4-(pyrimidin-4-yl)piperazin-1-yl)phenyl)amino)pyrimidin-4-yl)amino)-2-cyclopropylquinolin-5-yl)dimethylphosphine oxide;
(6-((5-bromo-2-((2-methoxy-5-(1-methyl-1H-pyrazol-4-yl)-4-(3-methyl-3,6-diazabicyclo[3.1.1]heptyl-6-yl)phenyl)amino)pyrimidin-4-yl)amino)-2-cyclopropylquinolin-5-yl)dimethylphosphine oxide;
(6-((5-bromo-2-((4-((2-(dimethylamino)ethyl)(methyl)amino)-2-methoxy-5-(1-methyl-1H-pyrazol-4-yl)phenyl)amino)pyrimidin-4-yl)amino)-2-cyclopropylquinolin-5-yl)dimethylphosphine oxide;
4-(4-(4-((5-bromo-4-((2-cyclopropyl-5-(dimethylphosphoryl)quinolin-6-yl)amino)pyrimidin-2-yl)amino)-5-methoxy-2-(1-methyl-1H-pyrazol-4-yl)phenyl)piperazin-1-yl)pyridin-2(1H)one;
(6-((5-bromo-2-((2-methoxy-5-(1-methyl-1H-pyrrol-3-yl)-4-(4-(4-methylpiperazine-1-yl)piperidin-1-yl)phenyl)amino)pyrimidin-4-yl)amino)-2-cyclopropylquinolin-5-yl)dimethylphosphine oxide;
(2-cyclopropyl-6-((5-fluoro-2-((2-methoxy-5-(1-methyl-1H-pyrazol-4-yl)-4-(4-(4-methylpiperazin-1-yl)piperidin-1-yl)phenyl)aminopyrimidin-4-yl)amino)quinolin-5-yl)dimethylphosphine oxide;
(6-((5-bromo-2-((2-methoxy-5-(1-methyl-1H-pyrazol-4-yl)-4-(4-(1-methylpiperidine-4-yl)piperazin-1-yl)phenyl)amino)pyrimidin-4-yl)amino)-2-cyclopropylquinolin-5-yl)dimethylphosphine oxide;
(6-((5-bromo-2-((5-(1-(difluoromethyl)-1H-pyrazol-4-yl)-2-methoxy-4-(4-(4-methylpiperazin-1-yl)piperidin-1-yl)phenyl)amino)pyrimidin-4-yl)amino)-2-cyclopropylquinolin-5-yl)dimethylphosphine oxide; and
(6-((5-bromo-2-((2-methoxy-5-(1-methyl-1H-pyrazol-4-yl)-4-(4-(4-methylpiperazine-1-yl)piperidin-1-yl)phenyl)amino)pyrimidin-4-yl)amino)-2-cyclopropylquinolin-5-yl)dimethylphosphine oxide.

**51.** A pharmaceutical composition, comprising:

the compound of formula I, or a stereoisomer, a tautomer, a deuterated compound, a pharmaceutically acceptable salt, a prodrug, a chelate, a non-covalent complex, or a solvate thereof of any one of claims 1-50, as the active ingredient; and
at least one pharmaceutically acceptable adjuvant.

**52.** A method for inhibiting various forms of EGFR mutations including one or more of L858R, Δ19del, T790M and C797S mutations, comprising administering the compound of formula I, or a stereoisomer, a tautomer, a deuterated compound, a pharmaceutically acceptable salt, a prodrug, a chelate, a non-covalent complex, or a solvate thereof of any one of claims 1-50, to a patient.

**53.** A method for treating EGFR-driven cancer, comprising administering a therapeutically effective amount of the compound of formula I, or a stereoisomer, a tautomer, a deuterated compound, a pharmaceutically acceptable salt, a prodrug, a chelate, a non-covalent complex, or a solvate thereof of any one of claims 1-50, to a patient in need thereof.

**54.** The method of claim 53, wherein the EGFR-driven cancer is **characterized by** the presence of one or more mutations selected from the group consisting of: (i) C797S, (ii) L858R and C797S, (iii) C797S and T790M, (iv) L858R, T790M, and C797S, and (v) Δ19del, T790M and C797S.

**55.** The method of claim 53 or 54, wherein the EGFR-driven cancer is colon cancer, stomach cancer, thyroid cancer,

lung cancer, leukemia, pancreatic cancer, melanoma, brain cancer, kidney cancer, prostate cancer, ovarian cancer, or breast cancer.

**56.** The method of any one of claims 53-55, wherein the lung cancer is non-small cell lung cancer carrying the EGFR$^{L858R/T790M/C797S}$ or EGFR$^{\Delta 19del/T790M/C797S}$ mutation.

**57.** A method for inhibiting mutant EGFR in a patient, comprising administering a therapeutically effective amount of the compound of formula I, or a stereoisomer, a tautomer, a deuterated compound, a pharmaceutically acceptable salt, a prodrug, a chelate, a non-covalent complex, or a solvate thereof of any one of claims 1-50, to a patient in need.

**58.** Use of the compound of formula I, or a stereoisomer, a tautomer, a deuterated compound, a pharmaceutically acceptable salt, a prodrug, a chelate, a non-covalent complex, a solvate thereof of any one of claims 1-50, or a pharmaceutical composition thereof, in the manufacture of a medicament.

**59.** The use of claim 58, wherein the medicament is used for treating or preventing cancer.

**60.** The use of claim 58 or 59, wherein the cancer is colon cancer, stomach cancer, thyroid cancer, lung cancer, leukemia, pancreatic cancer, melanoma, brain cancer, kidney cancer, prostate cancer, ovarian cancer, or breast cancer.

**61.** The use of claim 60, wherein the lung cancer is non-small cell lung cancer carrying the EGFR$^{L858R/T79OM/C797S}$ or EGFR$^{\Delta 19del/T790M/C797S}$ mutation.

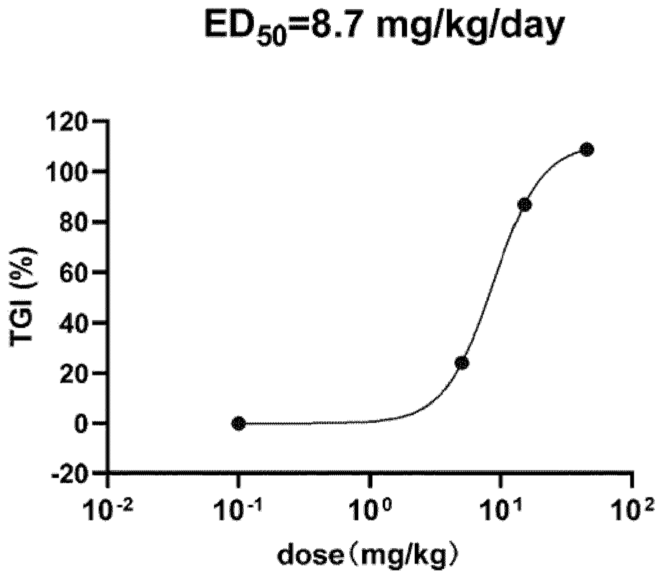
ED50=8.7 mg/kg/day
TGI (%)
120
100
80
60
40
20
0
-20
10-2
10-1
100
101
102
dose(mg/kg)

FIG. 1

**INTERNATIONAL SEARCH REPORT**

International application No.
**PCT/CN2021/075994**

**A. CLASSIFICATION OF SUBJECT MATTER**

C07D 401/12(2006.01)i； C07D 471/10(2006.01)i； C07D 487/00(2006.01)i； C07D 241/02(2006.01)i； A61K 31/66(2006.01)i； A61K 31/675(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI; SIPOABS; CNABS; CNTXT; WOTXT; USTXT; EPTXT; JPTXT; CNKI; 万方; WANFANG; STN-REG; STN-HCAPLUS; PATENTICS: 贝达药业股份有限公司, 刘湘永, 仇长勇, 刘孟强, 宋晓东, 申其超, 杜国龙, 盛海同, 丁列明, 王家炳, 喹啉, 膦氧, 嘧啶, 结构式I , EGFR, 肿瘤, 癌, +quinoline+, +phosphorus+, +oxygen+, +pyrimidine+, cancer, tumor, tumour

**C. DOCUMENTS CONSIDERED TO BE RELEVANT**

| Category* | Citation of document, with indication, where appropriate, of the relevant passages | Relevant to claim No. |
|---|---|---|
| PX | WO 2021018009 A1 (BETTA PHARMACEUTICALS CO., LTD.) 04 February 2021 (2021-02-04)<br>claims 1-25 | 1-61 |
| PX | WO 2020200191 A1 (BETTA PHARMACEUTICALS CO., LTD.) 08 October 2020 (2020-10-08)<br>claims 1-46 | 1-61 |
| X | WO 2019015655 A1 (CHIA TAI TIANQING PHARMACEUTICAL GROUP CO., LTD. et al.) 24 January 2019 (2019-01-24)<br>description, page 1, lines 5-10, and page 59, lines 5-10, and claims 1, 9 and 21 | 1-61 |
| A | WO 2013169401 A1 (ARIAD PHARMACEUTICALS, INC. et al.) 14 November 2013 (2013-11-14)<br>entire document | 1-61 |
| A | WO 2018108064 A1 (MEDSHINE DISCOVERY INC.) 21 June 2018 (2018-06-21)<br>entire document | 1-61 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 March 2021** | **27 April 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/075994**

**C. DOCUMENTS CONSIDERED TO BE RELEVANT**

| Category* | Citation of document, with indication, where appropriate, of the relevant passages | Relevant to claim No. |
|---|---|---|
| A | CN 110483485 A (INVENTISBIO (SHANGHAI) CO., LTD et al.) 22 November 2019 (2019-11-22)<br>entire document | 1-61 |
| A | WO 2017086829 A1 (R-PHARM JOINT STOCK COMPANY et al.) 26 May 2017 (2017-05-26)<br>entire document | 1-61 |
| A | WO 2017086832 A1 (R-PHARM JOINT STOCK COMPANY et al.) 26 May 2017 (2017-05-26)<br>entire document | 1-61 |

Form PCT/ISA/210 (second sheet) (January 2015)

**INTERNATIONAL SEARCH REPORT**

International application No.
**PCT/CN2021/075994**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **52-57**
because they relate to subject matter not required to be searched by this Authority, namely:

[1] Claim 52 sets forth a method for the inhibition of EGFR mutations, claims 53-56 set forth a method for the treatment of cancer, and claim 57 sets forth a method for the inhibition of EGFR in patients. The above subject matter falls within the category of a method of treatment of the human or animal body by therapy, and therefore does not warrant an international search according to the criteria set out in PCT Rule 39.1(iv). Nevertheless, a search is made on the basis of the subject matter of claim 52 which sets forth the use in the preparation of a drug for the inhibition of EGFR mutations, claims 53-56 which sets forth the use in the preparation of a drug for the treatment of EGFR-driven cancers, and claim 57 which sets forth the use in the preparation of a drug for the inhibition of mutant EGFR in patients.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/075994**

| Patent document cited in search report | Publication date (day/month/year) | Patent family member(s) | Publication date (day/month/year) |
|---|---|---|---|
| WO 2021018009 A1 | 04 February 2021 | None | |
| WO 2020200191 A1 | 08 October 2020 | None | |
| WO 2019015655 A1 | 24 January 2019 | KR 20200032146 A | 25 March 2020 |
| | | US 2020207768 A1 | 02 July 2020 |
| | | CA 3069829 A1 | 24 January 2019 |
| | | BR 112020001124 A2 | 01 September 2020 |
| | | AU 2018304757 A1 | 06 February 2020 |
| | | CN 110944989 A | 31 March 2020 |
| | | EP 3656769 A1 | 27 May 2020 |
| | | HK 40018495 A0 | 30 September 2020 |
| | | IN 202017003865 A | 02 October 2020 |
| | | JP 2020527151 W | 03 September 2020 |
| | | SG 11202000470 A1 | 27 February 2020 |
| WO 2013169401 A1 | 14 November 2013 | US 9834571 B2 | 05 December 2017 |
| | | JP 2015518490 A | 02 July 2015 |
| | | AU 2013204563 A1 | 21 November 2013 |
| | | US 2016244469 A1 | 25 August 2016 |
| | | JP 6469567 B2 | 13 February 2019 |
| | | AU 2013204563 B2 | 19 May 2016 |
| | | US 2015166591 A1 | 18 June 2015 |
| | | EP 2844642 A4 | 18 November 2015 |
| | | EP 2844642 A1 | 11 March 2015 |
| | | EP 2844642 B8 | 25 December 2019 |
| | | EP 2844642 B1 | 13 November 2019 |
| WO 2018108064 A1 | 21 June 2018 | None | |
| CN 110483485 A | 22 November 2019 | None | |
| WO 2017086829 A1 | 26 May 2017 | RU 2606951 C1 | 10 January 2017 |
| WO 2017086832 A1 | 26 May 2017 | RU 2607371 C1 | 10 January 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018108064 A
- WO 2018115218 A
- WO 2018181777 A
- WO 2019015655 A1

### Non-patent literature cited in the description

- Design of Prodrugs. Elsevier, 1985